# EUROPEAN PATENT APPLICATION

(11) **EP 1 619 184 A2**
(43) Date of publication of application: **25.01.2006**
(21) Application number: 05015480.6
(22) Date of filing: 15.08.2001
(51) Int. Cl.: C07D 213/75

(54) **Urea compounds as kinase inhibitors**

(30) Priority: 15.08.2000 US 225793 P; 14.08.2001 US 930753
(62) Divisional of application: 01964009.3
(71) Applicant: Amgen, Inc., Thousand Oaks, CA 91320-1799 (US)
(72) Inventor: Santora, Vincent, Ladera Ranch, California 92694 (US); Ghose, Arup, Thousand Oaks, California 91360 (US); Kim, Tae-Seong, Thousand Oaks, California 91360 (US); Askew, Benny, Newbury Park, California 91320 (US); Hague, Andrew, Camarillo, California 93012 (US); Laber, Ellen, Ventura, California 91362 (US); Li, Aiwen, Westlake Village, California 91362 (US); Lian, Brian, Bloomington, Indiana 47401 (US); Liu, Gang, Oak Park, California 91377 (US); Norman, Mark Henry, Thousand Oaks, California 91360 (US); Smith, Leon, Sommerset, New Jersey 08873 (US); Tasker, Andrew, Simi Valley, California 93065 (US); Tegley, Christopher, Thousand Oaks, California 91360 (US); Yang, Kevin, San Gabriel, California 91775 (US)
(74) Representative: Grünecker, Kinkeldey, Stockmair & Schwanhäusser Anwaltssozietät

(57) **Abstract**

Selected novel urea compounds are effective for prophylaxis and treatment of diseases, such as cell proliferation or apoptosis mediated diseases. The invention encompasses novel compounds, analogs, prodrugs and pharmaceutically acceptable salts thereof, pharmaceutical compositions and methods for prophylaxis and treatment of diseases and other maladies or conditions involving stroke, cancer and the like. The subject invention also relates to processes for making such compounds as well as to intermediates useful in such processes.

## Description

This application claims the benefit of U.S. Provisional Application No. 60/225,793, filed August 15, 2000, which is hereby incorporated by reference.

### FIELD OF THE INVENTION

This invention is in the field of pharmaceutical agents and specifically relates to compounds, compositions, uses and methods for treating cell proliferation-related disorders and apoptosis-related disorders.

### BACKGROUND OF THE INVENTION

Identification of therapeutic agents effective in the treatment of neoplastic diseases or for the treatment of neurological disorders is the subject of significant research efforts.

Protein kinases represent a large family of proteins which play a central role in the regulation of a wide variety of cellular processes and maintaining control over cellular function. A partial list of such kinases includes ab1, ATK, bcr-ab1, Blk, Brk, Btk, c-kit, c-met, c-src, CDK1, CDK2, CDK3, CDK4, CDK5, CDK6, CDK7, CDK8, CDK9, CDK10, cRaf1, CSF1R, CSK, EGFR, ErbB2, ErbB3, ErbB4, Erk, Fak, fes, FGFR1, FGFR2, FGFR3, FGFR4, FGFR5, Fgr, FLK-4, flt-1, Fps, Frk, Fyn, Hck, IGF-1R, INS-R, Jak, KDR, Lck, Lyn, MEK, p38, PDGFR, PIK, PKC, PYK2, ros, tie, tie2, TRK, Yes, and Zap70. As such, inhibition of kinases has become an important therapeutic target.

Cell proliferation is the rapid reproduction of cells, such as by cell division. The cell cycle, which controls cell proliferation, is itself controlled by a family of kinases called cyclin dependent kinases (CDKs). The regulation of CDK activation is complex, and requires the association of the CDK with a member of the cyclin family of regulatory subunits. A further level of regulation occurs through both activating and inactivating phosphorylations of the CDK subunit. The coordinate activation and inactivation of different cyclin/CDK complexes is necessary for normal progression through the cell cycle. Both the critical G1-S and G2-M transitions are controlled by the activation of different cyclin/CDK activities. Loss of control of CDK regulation is a frequent event in hyperproliferative diseases and cancer. (T. Noguchi et al., Am. J. Pathol., 156, 2135-47 (2000)) As such, inhibition of CDKs has become an important target in the study of chemotherapeutics (A. Senderowicz and E. Sausville, J. Nat. Canc. Instit., 92, 376-87 (2000))

Kinases have also been implicated in diseases and disorders of the central nervous system. For example, patients suffering from stroke, Alzheimer's disease or Parkinson's disease would benefit from the inhibition of kinases. Cdk5 has been shown to be involved in Alzheimer's pathology (R. Maccioni, et al., Eur. J. Biochem., 268, 1518-27 (2001)) and with neuronal development (G. Paglini and A. Caceres, Eur. J. Biochem., 268, 1528-33 (2001)).

Protein kinases also control programmed cell death, also known as apoptosis. Apoptosis is a ubiquitous physiological process used to eliminate damaged or unwanted cells in multicellular organisms. Disregulation of apoptosis is believed to be involved in the pathogenesis of many human diseases. The failure of apoptotic cell death has been implicated in various cancers, as well as autoimmune disorders. Conversely, increased apoptosis is associated with a variety of diseases involving cell loss such as neurodegenerative disorders and AIDS. As such, inhibition of apoptosis has become an important therapeutic target. Cdk5 has been shown to be involved in apoptosis pathology (A. Catania et al., Neuro-Oncology, 89-98 (April 2001)).

Substituted heterocyclic compounds are known in the pesticide art. WO00/24735, published 4 May 2000, describes 1-pyridyl-1,2,4-triazoles as pesticides. WO00/24739, published 4 May 2000, describes substituted 1,2,4-triazoles as pesticides. WO97/01552, published 16 January 1997, describes substituted 1,2,4-triazoles as antifungal agents. DE4204492 describes substituted benzamides as pesticides. WO98/57969, published 23 December 1998, describes heterocyclylpyridines as pesticides. GB2293380, published 27 March 1996, describes the use of heterocyclic compounds as pesticides. United States patent No. 5,693,667, issued Dec. 2, 1997, describes heterocyclic compounds for the treatment of take-all disease. EP468695 describes fungicide compounds. United States patent No. 5,294,596, issued March 15, 1994, describes herbicidal triazolinones. United States patent No. 5,395,818, issued March 7, 1995, describes herbicidal triazolinones.

Substituted thiazoles also are known in the pesticide art. United States patent No. 4,260,765, issued Apr. 7, 1981, describes 2-(3-pyridyl)-5-thiazolecarboxamides for the treatment of aphids. United States patent No. 5,945,380, issued Aug. 31, 1999, describes 4-(4-pyridyl)pyrazoles as insecticides. WO89/00568, published 26 January 1989, describes nicotine derivatives as fungicides.

Heterocyclic ureas are known in the pharmaceutical art. WO99/23091, published 14 May 1999, describes heterocyclic compounds as anti-inflammatories. WO99/32455, published 1 July 1999, describes heterocyclic ureas as RAF kinase inhibitors. WO99/32110 published 1 July 1999, describes heterocyclic ureas as p38 kinase inhibitors. WO99/32106, published 1 July 1999, describes heterocyclic ureas as RAF kinase inhibitors. WO99/32111, published 1 July 1999, describes heterocyclic ureas as p38 kinase inhibitors. WO99/32436 published 1 July 1999, describes urea compounds as inhibitors of RAF kinase. WO99/32463, published 1 July 1999, describes urea compounds that inhibit p38 kinase. WO98/52558, published 26 November 1998, describes urea compounds for the inhibition of p38 kinase. WO99/00357, published 7 January 1999, describes the use of urea compounds as inhibitors of p38 kinase. WO99/58502, published 18 November 1999, describes urea compounds as inhibitors of p38 kinase. US patent 5,821,245, issued Oct. 13, 1998, describes substituted naphthalene derivatives for treating cell growth. GB patent 1,437,895 describes 2-thiazolyl ureas for the treatment of ulcers. United States patent 5,364,871, issued Nov. 15, 1994 describes thiazoles as anti-ulcer compounds. WO99/21555, published 6 May 1999, describes pyridyl-substituted thiazoles as adenosine A3 receptor antagonists. WO96/23783 describes indole derivatives as 5-HT receptor antagonists. United States patent No. 5,208,248 describes indazole derivatives as 5-HT receptor antagonists. WO99/46244, published 16 September 1999 describes heterocyclic compounds as tyrosine phosphatases. GB patent 2,263,109, published 14 July 1993, describes pyridylthiazoles as PAF-receptor antagonists.

Thiazole compounds have also been described as inhibitors of CDK. WO00/26203, published 11 May 2000, describes 2-ureidothiazoles as inhibitors of cdk. WO99/65884 describes 2-aminothiazoles as inhibitors of CDK. WO99/24416 describes 2-aminothiazoles as inhibitors of CDK.

However, compounds of the current invention have not been described as inhibitors of cell proliferation or apoptosis such as for the treatment of cancer or stroke.

### DESCRIPTION OF THE INVENTION

A class of compounds useful in treating cell proliferative disorders, neurological disorders and apoptosis is defined by Formula I 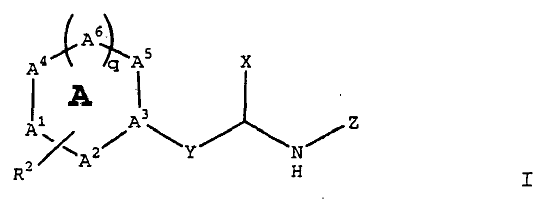 wherein each of A¹-A⁶ is selected from CH₂, CH, C, 0, S, NH and N; wherein A¹-A⁶ together form a ring A selected from
a) additionally substituted or unsubstituted 5- or 6-membered heterocyclyl,
   preferably 5- or 6-membered heteroaryl,
   more preferably 5-membered heteroaryl selected from thiazolyl, oxazolyl, imidazolyl, pyrrolyl, pyrazolyl, isoxazolyl, triazolyl and isothiazolyl, and 6-membered heteroaryl selected from pyridyl, pyrazinyl, pyrimidinyl and pyridazinyl,
   even more preferably 5-membered heteroaryl selected from thiazolyl, oxazolyl and imidazolyl, and
   6-membered heteroaryl selected from pyridyl, and pyrimidinyl,
b) additionally substituted or unsubstituted 5- or 6-membered heteroaryl fused with a phenyl group,
c) additionally substituted or unsubstituted 5- or 6-membered cycloalkenyl,
   preferably 5-membered cycloalkenyl,
   more preferably cyclopentadienyl or cyclopentenyl, and
d) additionally substituted or unsubstituted phenyl,
   wherein A is additionally substituted with one or more substituents independently selected from halo, -OR³, -SR³, -CO₂R³, -CO₂NR³R³, -COR³, -NR³R³, -SO₂NR³R³,-NR³C(O)OR³, -NR³C(O)R³, cycloalkyl, optionally substituted phenylalkylenyl, optionally substituted 5-6 membered heterocyclyl, optionally substituted heteroarylalkylenyl, optionally substituted phenyl, lower alkyl, cyano, lower hydroxyalkyl, nitro, lower alkenyl, lower alkynyl and lower haloalkyl, preferably one or more substituents independently selected from halo, -OR³, -SR³, -S(O)R³, -CO₂R³,-CO₂NR³R³, -COR³, -NR³R³, -SO₂NR³R³, -NR³C(O)OR³,-NR³C(O)R³, C₁-C₂ alkyl, cyano, C₁-C₂ hydroxyalkyl, nitro, C₂-C₃ alkenyl, C₂-C₃ alkynyl and C₁-C₂ haloalkyl,
   more preferably one or more substituents independently selected from fluoro, hydroxy, methoxy, amino and methyl;
   wherein X and Z taken together form a nitrogen containing ring selected from
   unsubstituted 5-6 membered heterocyclyl,
   unsubstituted 5-6 membered heterocyclyl fused with a phenyl group,
   5-6 membered heterocyclyl substituted with one or more substituents independently selected from R¹, and
   5-6 membered nitrogen-containing heterocyclyl, fused with a phenyl group, substituted with one or more substituents independently selected from R¹,
   preferably a ring selected from substituted or unsubstituted 5- or 6-membered nitrogen-containing heteroaryl, and substituted or unsubstituted 5- or 6-membered nitrogen-containing heteroaryl fused with a phenyl group,
   more preferably substituted or unsubstituted thiazolyl, pyridyl, pyrazinyl, pyrimidinyl, pyridazinyl, triazinyl, isoindolyl, indolyl, indazolyl, purinyl, [1,6]naphthyridinyl, 5,6,7,8-tetrahydro[1,6]naphthyridinyl, isoquinolyl and quinolyl,
   even more preferably pyridyl, pyrazinyl, pyrimidinyl, pyridazinyl, [1,6]naphthyridinyl and 5,6,7,8-tetrahydro[1,6]naphthyridinyl,
   most preferably pyridyl, pyrazinyl, pyrimidinyl and pyridazinyl,
   most preferred pyridyl;
   wherein R¹ is independently selected from H, halo, - OR³, -SR³, -CO₂R³, -CO₂NR³R³, -COR³, -CONR³R³, -NR³R³, -C(S)NR³R³, -SO₂NR³R³, -NR³C(O)OR³, -NR³C(O)R³, cycloalkyl, optionally substituted phenylalkylenyl, optionally substituted 4-10 membered heterocyclyl, optionally substituted 4-10 membered heterocyclylalkyl, optionally substituted phenyl, optionally substituted phenoxy, lower alkyl, lower cyano, lower alkenyl, lower alkynyl and lower haloalkyl,
   preferably optionally substituted pyrrolidinyl, optionally substituted piperazinyl, optionally substituted piperidinyl, morpholinyl, optionally substituted pyridyl, 1,4-dioxa-8-aza-spiro[4.5]decyl, optionally substituted phenyl, C₁-C₄ alkyl, C₁-C₂ haloalkyl, halo, C₁-C₄₋hydroxyalkyl, amino, C₁-C₄-azidoalkyl, C₁-C₄₋cyanoalkyl, C₁-C₄-aminoalkyl, hydroxy, C₁-C₄₋alkoxy-C₁-C₄-alkyl, C₁-C₄-alkylamino-C₁-C₄-alkyl, C₁-C₄-hydroxyalkylamino-C₁-C₄-alkyl, amino-C₁-C₄₋alkoxy-C₁-C₄-alkyl, C₁-C₄-alkylamino-C₁-C₄₋alkylamino-C₁-C₄-alkyl (optionally substituted pyrrolidinyl)-C₁-C₂-, (optionally substituted piperidinyl)-C₁-C₂-, (optionally substituted piperazinyl)-C₁-C₂-, 4-morpholinyl-C₁-C₂-, (optionally substituted imidazolyl)-C₁-C₂-, phthalimidylethyl, optionally substituted azepanyl-C₁-C₂-, 1,4-dioxa-8-aza-spiro[4.5]decyl-C₁-C₂-, optionally substituted pyridyloxy, optionally substituted phenoxy, tetrahydrofuryl-O-(1-aza-bicyclo[2.2.2]oct-3-yl)-oxy, optionally substituted phenoxy-C₁-C₂-, optionally substituted pyrrolidinyl-C₁-C₄-alkoxy, optionally substituted azetidinyl-C₁-C₄-alkoxy, optionally substituted piperidinyl-C₁-C₄-alkoxy, tetrahydrofuryl-C₁-C₄₋alkoxy, C₁-C₄-alkylamino-C₁-C₄-alkoxy morpholinyl-C₁-C₄-alkylenylaminocarbonyl, C₁-C₄-alkoxycarbonyl, 5-6-membered heterocyclyl-C₁-C₄₋alkylaminocarbonyl, 5-6-membered N-containing heterocyclylcarbonyl, C₁-C₄-alkylaminocarbonyl, C₁₋C₄-alkylamino-C₁-C₄-alkylaminocarbonyl, 5-6-membered N-containing heterocyclyl-C₁-C₄₋alkylamino, aminocarbonyl, C₁-C₃₋alkylaminothiocarbonyl, C₁-C₄-alkylamino and C₁-C₄₋alkylamino-C₁-C₄-alkylamino,
   more preferably 3-(N,N-dimethylamino)-1-pyrrolidinyl, 1-methyl-4-piperazinyl, 1-benzyl-4-piperazinyl, 1-(2-pyrimidinyl)-4-piperazinyl, 1-(2-pyridyl)-4-piperazinyl, 1-ethyl-4-piperazinyl, piperidinyl, morpholinyl, 4-amino-1-piperidinyl, 4-(N-hydroxyethylamino)-1-piperidinyl, 4-(N-propylamino)-1-piperidinyl, 4-(N-benzylamino)-1-piperidinyl, 4-oxo-piperidinyl, 4-(hydroxyimino)-piperidinyl, 4-morpholinyl, 1,4-dioxa-8-aza-spiro[4.5]decyl, pyridyl, phenyl, methyl, ethyl, propyl, isopropyl, butyl, sec-butyl, isobutyl, tert-butyl, amino, azidomethyl, hydroxymethyl, trifluoromethyl, difluoromethyl, pentafluoroethyl, fluoro, chloro, bromo, aminoethyl, aminomethyl, cyanomethyl, 1-pyrrolidinyl-CH₂-, 2-methoxycarbonyl-1-pyrrolidinyl-CH₂-, 2-carboxy-1-pyrrolidinyl-CH₂-, 2-hydroxymethyl-1-pyrrolidinyl-CH₂-, 1-piperidinyl-CH₂-, 4-methyl-1-piperidinyl-CH₂-, 3-methyl-1-piperidinyl-CH₂-, 2-methyl-1-piperidinyl-CH₂-, 3,5-dimethyl-1-piperidinyl-CH₂-, 4-oxo-1-piperidinyl-CH₂-, 4-hydroxy-1-piperidinyl-CH₂-, 3-hydroxy-1-piperidinyl-CH₂-, 2-ethoxycarbonyl-1-piperidinyl-CH₂-, 3-ethoxycarbonyl-1-piperidinyl-CH₂-, 3-carboxy-1-piperidinyl-CH₂-, 4-ethoxycarbonyl-1-piperidinyl-CH₂-, 4-carboxy-1-piperidinyl-CH₂-, 4-(1-pyrrolidinyl)-1-piperidinyl-CH₂-, 4-(N-hydroxyethylamino)-1-piperidinyl-CH₂-, 4-(N-propylamino)-1-piperidinyl-CH₂-, 1-methyl-4-piperazinyl-CH₂-, 4-morpholinyl-CH₂-, (2-methyl-1-imidazolyl-CH₂-, 3-(N,N-diethylamino)carbonyl-1-piperidinyl-CH₂-, phthalimidylethyleneyl, 1-azepanyl-CH₂-, 1,4-dioxa-8-aza-spiro[4.5]decyl-CH₂-, 4-(methyl)phenoxymethylenyl, 4-(N,N-dimethylaminomethylenyl)phenoxymethylenyl, methylaminothiocarbonyl, methoxymethylenyl, ethylaminothiocarbonyl, N,N-dimethylaminoethylenyl, N,N-diethylaminomethylenyl, N-methylaminoethylenyl, N-methylaminomethylenyl, N-(hydroxypropyl)aminomethylenyl, N-ethylaminomethylenyl, Boc-aminoethoxymethylenyl, aminoethoxymethylenyl, (1-aza-bicyclo[2.2.2]oct-3-yl)-oxy, 2-pyrrolidinylmethoxy, 1-methyl-2-pyrrolidinylmethoxy, azetidin-3-ylmethoxy, N-Boc-azetidin-3-ylmethoxy, N-Boc-piperidin-4-ylethoxy, 1-methyl-4-piperidinylethoxy, 4-piperidinylethoxy, 4-piperidinylmethoxy, N,N-dimethylaminoethoxy, 3-tetrahydrofuryl-O-, 3-tetrahydrofurylmethoxy, 4-tetrahydrofurylmethoxy, 4-methylphenoxy, 4-(aminoethyl)phenoxy, 4-(1-imidazolyl)phenoxy, 2,4-dimethylphenoxy, phenoxy, 4-cyanophenoxy, 4-[1,3]dioxolan-2-ylphenoxy, 4-fluorophenoxy, 3,4-difluorophenoxy, ethoxycarbonyl, morpholinylethylenylaminocarbonyl, morpholinylpropylenylaminocarbonyl, 1-piperidinylcarbonyl, methylaminocarbonyl, ethylaminocarbonyl, N,N-diethylaminocarbonyl, N-(N',N'-dimethylaminoethylenyl)aminocarbonyl, aminocarbonyl, morpholinylethylenylamino, morpholinylpropylenylamino, N,N-diethylamino, N,N-dimethylamino, N,N-diethylamino(2-propylenyl)aminomethylenyl, N,N-diethylamino(1-propylenyl)aminomethylenyl and N-(N',N'-dimethylaminoethylenyl)amino;
   wherein Y is selected from 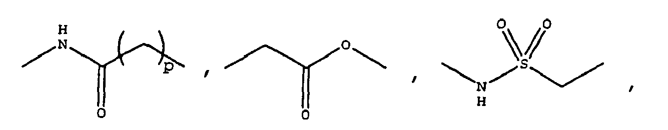 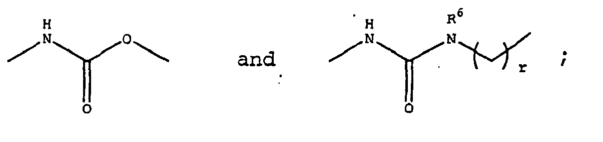
   preferably Y is selected from 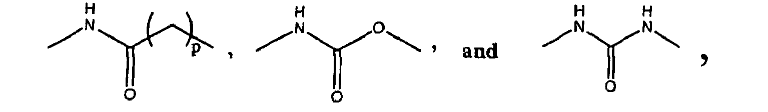
   more preferably Y is selected from 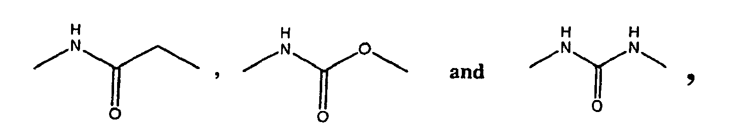
   even more preferably Y is 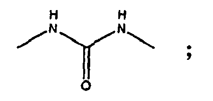
   wherein R² is selected from
   a) lower alkylaminoalkynyl,
   b) cycloalkenyl-C₂₋₃-alkynyl,
   c) cycloalkyl-C₂₋₃-alkynyl,
   d) phenyl-C₂₋₃-alkynyl,
   e) 5-6 membered heterocyclyl-C₂₋₃-alkynyl,
   f) substituted or unsubstituted cycloalkenyl,
   g) substituted or unsubstituted phenyl,
   h) substituted or unsubstituted 5-6 membered heterocyclyl, and
   i) substituted or unsubstituted 5-6 membered
      heterocyclyl bridged with a phenyl group, preferably substituted phenyl, substituted or unsubstituted 5-6 membered nitrogen-containing heteroaryl, and substituted or unsubstituted 5-6 membered nitrogen-containing heteroaryl fused with a phenyl group',
      more preferably substituted or unsubstituted substituted phenyl or a substituted or unsubstituted heterocyclyl substituent selected from thiazolyl, pyridyl, pyrazinyl, pyrimidinyl, pyridazinyl, triazinyl, isoindolyl, indolyl, indazolyl, purinyl, isoquinolyl and quinolyl,
      even more preferably phenyl, pyridyl, pyrazinyl, pyrimidinyl, pyridazinyl, purinyl, isoquinolyl and quinolyl,
      most preferably pyridyl, pyrazinyl, pyrimidinyl and pyridazinyl,
      preferred pyridyl;

   wherein substituted R² is substituted with one or more substituents independently selected from halo, -OR³, -SR³, -CO₂R³, -CO₂NR³R³, -COR³, -NR³R³, -C(O)NR³R³, - SO₂NR³R³, -NR³C(O)OR³, -NHC(O)R³, -SO₂NHC(O)R³, - C(S)NR³R³, nitro, cycloalkyl, optionally substituted phenylalkylenyl, optionally substituted 4-7 membered heterocyclyl, optionally substituted heterocyclylalkylenyl, optionally substituted phenyl, optionally substituted phenoxyalkylenyl, optionally substituted heterocyclyloxyalkyl, lower alkyl, cyano, lower hydroxyalkyl, lower alkoxyalkyl, lower azidoalkyl, lower aminoalkyl, lower (hydroxyalkyl)aminoalkyl, lower alkylaminoalkyl, lower alkylaminoalkoxy, lower aminoalkoxyalkyl, lower (alkylaminoalkyl)amino lower ((alkylamino)alkylamino)alkyl, lower alkylaminoalkylaminocarbonyl, lower cyanoalkyl, lower alkenyl, lower alkynyl and lower haloalkyl,
   preferably selected from C₁-C₄ alkyl, C₁-C₂
   haloalkyl, halo, amino, C₁-C₂-alkoxy, C₁-C₂-alkoxy-C₁-C₂-alkyl, hydroxy, C₁-C₂-alkylthio, cyano, C₁-C₂₋haloalkyloxy, aminosulfonyl, (6-membered N-containing heterocyclyl)sulfonyl, C₁-C₂₋haloalkylaminocarbonyl, nitro, C₁-C₂₋haloalkylcarbonylaminosulfonyl, C₁-C₂₋alkylaminosulfonyl, C₃-C₆-cycloalkylaminosulfonyl, phenyl-C₁-C₂-alkylaminosulfonyl, (optionally substituted phenyl)aminosulfonyl, piperidinyl, morpholinyl, C₁-C₂ alkylpiperazinyl, C₁-C₃ alkylaminothiocarbonyl, C₁-C₂-alkylamino-C₁-C₄₋alkylenyl, morpholinyl-C₁-C₄₋alkylenylaminocarbonyl, aminocarbonyl, C₁-C₂₋alkylcarbonylamino, morpholinyl-C₁-C₄₋alkylenylamino, C₁-C₂-alkylamino and C₁-C₂₋alkylamino-C₁-C₄-alkylenylamino,
   more preferably selected from nitro, methylcarbonylamino, aminosulfonyl, phenylsulfonylamino, morpholinylsulfonyl, trifluoroacetylaminosulfonyl, (4-chlorophenyl)aminosulfonyl, hydroxy, methylthio, cyano, trifluoromethoxy, bromo, chloro, fluoro, amino, methoxy, ethoxy, ethoxymethyl, trifluoromethylcarbonylamino, trifluoroethoxy, pyridyl, phenyl, methyl, ethyl, propyl, isopropyl, butyl, sec-butyl, isobutyl, tert-butyl, trifluoromethyl, difluoromethyl, pentafluoroethyl, carboxy, methylthio, piperidinyl, morpholinyl, N-methylpiperazinyl, N-ethylpiperazinyl, methylaminothiocarbonyl, N-methylamino-methylenyl, N,N-dimethylaminoethylenyl, N,N-diethylaminomethylenyl, N,N-dimethylamino, N-methylaminoethylenyl, N,N-diethylamino, morpholinylethylenylaminocarbonyl, morpholinylpropylenylaminocarbonyl, aminocarbonyl, morpholinylethylenylamino, morpholinylpropylenylamino, N,N-dimethylamino and N,N-di-methylaminoethylenylamino;
   wherein R³ is selected from H, lower alkyl, optionally substituted phenyl, optionally substituted phenylalkyl, optionally substituted heterocyclyl, optionally substituted heterocyclylalkyl, C₃-C₆ cycloalkyl, and lower haloalkyl,
   preferably H, C₁-C₃ alkyl, phenyl, 5-6 membered heteroaryl, C₅-C₆ cycloalkyl, and C₁-C₃ haloalkyl;
   more preferably H, methyl, ethyl, optionally substituted phenyl, benzyl, and trifluoromethyl;
   wherein R⁶ is selected from H, alkyl, 5-6 membered heterocyclylalkylenyl and alkylamino,
   preferably H;
   wherein p is 1-2, preferably p is 1;
   wherein q is 0 or 1; and
   wherein r is 0, 1, 2 or 3, preferably 0 or 1, more preferably 0;
   and pharmaceutically acceptable salts thereof; provided A is not thiazol-2-yl when Y is ureido;
   further provided A is not phenyl when R² is pyridyl or pyrimidyl when Y is ureido and when X and Z taken together form 1-methylindolyl; further provided A is not 1-phenylpyrazol-4-yl when Y is ureido when X and Z taken together form pyrazolyl and when R² is pyrrol-1-yl; further provided A is not thiazolyl or dihydrothiazolyl when R² is indolyl when Y is ureido and when X and Z taken together form thiazolyl or dihydrothiazolyl; provided A is not thiazolyl when R² is 3-pyridyl when Y is ureido and when X and Z taken together form 2-(3-pyridyl)thiazol-4-yl; and further provided A is not thien-3-yl when Y is ureido when X and Z taken together form thienyl and when R² is pyrrol-1-yl.

The invention also relates to compounds of Formula Ia

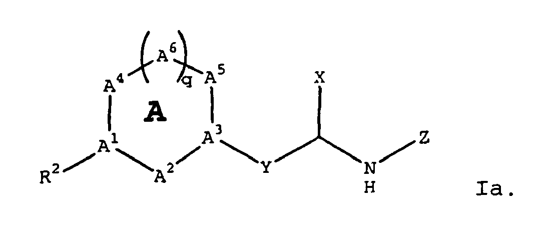

The invention also relates to compounds of Formula I wherein A is selected from 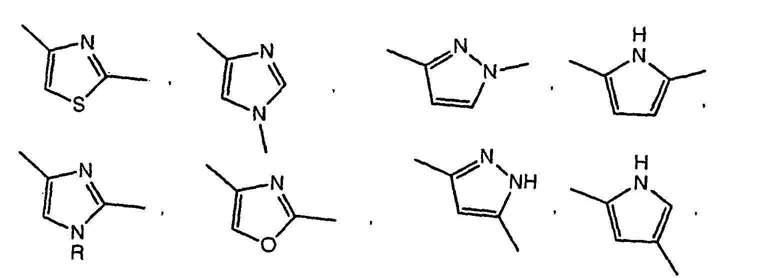 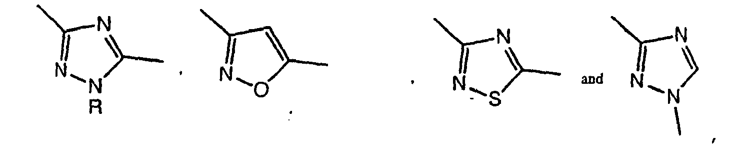 preferably A is 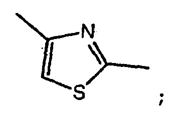 and wherein R is selected from H and C₁-C₃ alkyl; and pharmaceutically acceptable salts thereof.

The invention also relates to compounds of Formula II 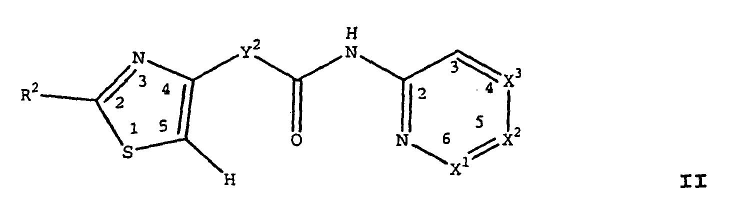
wherein X¹ is CR¹ or N; wherein X² is CR¹ or N; wherein X³ is CH or N; provided only one of X¹, X² and X³ can be N;
wherein R¹ is one or more substituents selected from H, optionally substituted pyrrolidinyl, optionally substituted piperazinyl, optionally substituted piperidinyl, morpholinyl, 1,4-dioxa-8-aza-spiro[4.5]decyl, pyridyl, phenyl, C₁-C₆-alkyl, C₁-C₂₋haloalkyl, C₁-C₄-hydroxyalkyl, amino, C₁-C₄₋azidoalkyl, C₁-C₄-cyanoalkyl, C₁-C₄-aminoalkyl, halo, hydroxy, (optionally substituted pyrrolidinyl)-C₁-C₂₋(optionally substituted piperidinyl)-C₁-C₂-, (optionally substituted piperazinyl)-C₁-C₂-, morpholinyl-C₁-C₂-, (optionally substituted imidazolyl)-C₁-C₂-, phthalimidyl-C₁-C₂-, optionally substituted azepanyl-C₁-C₂-, 1,4-dioxa-8-aza-spiro[4.5]decyl-C₁-C₂-, optionally substituted phenoxy-C₁-C₂-, C₁-C₄-alkylaminothiocarbonyl, C₁-C₄₋alkoxy-C₁-C₄-alkyl, C₁-C₄-alkylamino-C₁-C₄-alkyl, C₁₋C₄-hydroxyalkylamino-C₁-C₄-alkyl, amino-C₁-C₄-alkoxy-C₁-C₄-alkyl, (1-aza-bicyclo[2.2.2]oct-3-yl)-oxy, optionally substituted pyrrolidinyl-C₁-C₄-alkoxy, optionally substituted azetidinyl-C₁-C₄-alkoxy, optionally substituted piperidinyl-C₁-C₄-alkoxy, C₁₋C₄-alkylamino-C₁-C₄-alkoxy, tetrahydrofuryl-O-, tetrahydrofuryl-C₁-C₄-alkoxy, optionally substituted pyridyloxy, optionally substituted phenoxy, C₁-C₄₋alkoxycarbonyl, 5-6-membered heterocyclyl-C₁-C₄₋alkylaminocarbonyl, 5-6-membered N-containing heterocyclylcarbonyl, C₁-C₄-alkylaminocarbonyl, C₁₋C₄-alkylamino-C₁-C₄-alkylaminocarbonyl, aminocarbonyl, 5-6-membered N-containing heterocyclyl-C₁-C₄-alkylamino, C₁-C₄-alkylamino, C₁₋C₄-alkylamino-C₁-C₄-alkylamino-C₁-C₄-alkyl, and C₁-C₄₋alkylamino-C₁-C₄-alkylamino;
wherein R² is selected from halo, C₁-C₄-alkyl, C₁-C₄₋alkylamino-C₂-C₄-alkynyl, C₃-C₆-cycloalkyl, optionally substituted benzodioxolyl, optionally substituted indolyl, optionally substituted phenoxy, unsubstituted 5-membered oxygen or sulfur containing heteroaryl, unsubstituted 6-membered nitrogen-containing heterocyclyl, phenyl optionally substituted with one or two substituents selected
from halo, C₁-C₄-alkylamino, amino, nitro, C₁-C₄₋alkoxy, C₁-C₂-haloalkyl, hydroxy, C₁-C₄₋alkylthio, C₁-C₄-alkylcarbonylamino, (optionally substituted phenyl)sulfonylamino, cyano, C₁-C₂₋haloalkoxy, 5- or 6-membered N-containing heterocyclyl, aminosulfonyl, (6-membered N-containing heterocyclyl)sulfonyl, C₁-C₂₋haloalkylcarbonylaminosulfonyl and (optionally substituted phenyl)aminosulfonyl, and
6-membered nitrogen-containing heterocyclyl substituted with one or more substituents
independently selected from pyridyl, phenyl, C₁-C₄ alkyl, C₁-C₂ haloalkyl, C₁-C₂ alkoxy, amino, halo, piperidinyl, morpholinyl, C₁-C₂ alkylpiperazinyl, C₁-C₃ alkylaminothiocarbonyl, N,N-di-C₁-C₂-alkylamino-C₁-C₄-alkylenyl, N-C₁-C₂₋alkylamino-C₁-C₄-alkylenyl, morpholinyl-C₁-C₄₋alkylenylaminocarbonyl, aminocarbonyl, C₁-C₂₋haloalkylcarbonylamino, morpholinyl-C₁-C₄₋alkylenylamino, N,N-di-C₁-C₂-alkylamino and N,N-di-C₁-C₂-alkylamino-C₁-C₄-alkylenylamino; and
wherein Y² is selected from 0, NH and CH₂;
and pharmaceutically acceptable salts thereof.

The invention also relates to compounds of Formula III 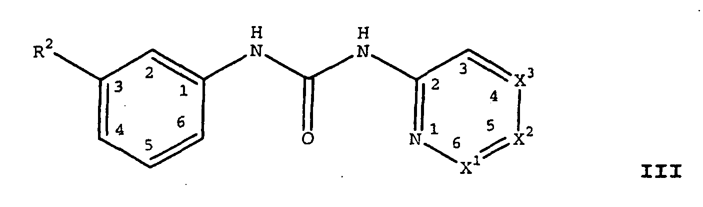
wherein X¹ is CR¹ or N; wherein X² is CR¹ or N; wherein X³ is CH or N; provided only one of X¹, X² and X³ can be N;
preferably X¹ is CR¹; X² is CR¹; X³ is CH; provided X² is CH when X¹ is not CH;
wherein R¹ is one or more substituents independently selected from H, optionally substituted pyrrolidinyl, optionally substituted piperazinyl, optionally substituted piperidinyl, morpholinyl, 1,4-dioxa-8-aza-spiro[4.5]decyl, pyridyl, phenyl, C₁-C₆-alkyl, C₁-C₂-haloalkyl, C₁-C₄-hydroxyalkyl, amino, C₁-C₄-azidoalkyl, C₁-C₄-cyanoalkyl, C₁-C₄₋aminoalkyl, halo, hydroxy, (optionally substituted pyrrolidinyl)-C₁-C₂-, (optionally substituted piperidinyl)-C₁-C₂-, (optionally substituted piperazinyl)-C₁-C₂-, morpholinyl-C₁-C₂-, (optionally substituted imidazolyl)-C₁-C₂-, phthalimidyl-C₁-C₂-, optionally substituted azepanyl-C₁-C₂-, 1,4-dioxa-8-aza-spiro[4.5]decyl-C₁-C₂-, optionally substituted phenoxy-C₁-C₂-, C₁-C₄-alkylaminothiocarbonyl, C₁-C₄₋alkoxy-C₁-C₄-alkyl, C₁-C₄-alkylamino-C₁-C₄-alkyl, C₁₋C₄-hydroxyalkylamino-C₁-C₄-alkyl, amino-C₁-C₄-alkoxy-C₁-C₄-alkyl, (1-aza-bicyclo[2.2.2] oct-3-yl)-oxy, optionally substituted pyrrolidinyl-C₁-C₄-alkoxy, optionally substituted azetidinyl-C₁-C₄-alkoxy, optionally substituted piperidinyl-C₁-C₄-alkoxy, C₁₋C₄-alkylamino-C₁-C₄-alkoxy, tetrahydrofuryl-O-, tetrahydrofuryl-C₁-C₄-alkoxy, optionally substituted pyridyloxy, optionally substituted phenoxy, C₁-C₄₋alkoxycarbonyl, 5-6-membered heterocyclyl-C₁-C₄₋alkylaminocarbonyl, 5-6-membered N-containing heterocyclylcarbonyl, C₁-C₄-alkylaminocarbonyl, C₁₋C₄-alkylamino-C₁-C₄-alkylaminocarbonyl, aminocarbonyl, 5-6-membered N-containing heterocyclyl-C₁-C₄-alkylamino, C₁-C₄-alkylamino, C₁₋C₄-alkylamino-C₁-C₄-alkylamino-C₁-C₄-alkyl, and C₁-C₄₋alkylamino-C₁-C₄-alkylamino,
preferably H, methyl, ethyl, propyl, 1-methyl-4-piperazinyl, 1-benzyl-4-piperazinyl, 1-(2-pyrimidinyl)-4-piperazinyl, 1-(2-pyridyl)-4-piperazinyl, 1-ethyl-4-piperazinyl, 1-piperidinyl-CH₂-, 4-methyl-1-piperidinyl-CH₂-, 3-methyl-1-piperidinyl-CH₂-, 2-methyl-1-piperidinyl-CH₂-, 3,5-dimethyl-1-piperidinyl-CH₂-, 4-oxo-1-piperidinyl-CH₂-, 4-hydroxy-1-piperidinyl-CH₂-, 3-hydroxy-1-piperidinyl-CH₂-, 2-ethoxycarbonyl-1-piperidinyl-CH₂-, 3-ethoxycarbonyl-1-piperidinyl-CH₂-, 3-carboxy-1-piperidinyl-CH₂-, 4-ethoxycarbonyl-1-piperidinyl-CH₂-, 4-carboxy-1-piperidinyl-CH₂-, 4-(1-pyrrolidinyl)-1-piperidinyl-CH₂-, 4-(N-hydroxyethylamino)-1-piperidinyl-CH₂-, 4-(N-propylamino)-1-piperidinyl-CH₂-, 3-(N,N-diethylamino)carbonyl-1-piperidinyl-CH₂-, 4-morpholinyl-CH₂-, N,N-dimethylaminoethylenyl, N,N-diethylaminomethylenyl, N-methylaminomethylenyl, N-ethylaminomethylenyl and N,N-diethylamino,
more preferably ethyl, propyl, 1-methyl-4-piperazinyl, 1-piperidinyl-CH₂-, 4-morpholinyl-CH₂-, N,N-diethylaminomethylenyl and N,N-diethylamino; and
wherein R² is selected from halo, C₁-C₄-alhyl, C₁-C₄₋alkylamino-C₂-C₄-alkynyl, C₃-C₆-cycloalkyl, optionally substituted benzodioxolyl, optionally substituted indolyl, optionally substituted phenoxy, unsubstituted 5-membered oxygen or sulfur containing heteroaryl, unsubstituted 5- or 6-membered nitrogen-containing heterocyclyl, phenyl optionally substituted with one or two substituents selected
from halo, C₁-C₄-alkylamino, amino, nitro, C₁-C₄₋alkoxy, C₁-C₂-haloalkyl, hydroxy, C₁-C₄₋alkylthio, C₁-C₄-alkylcarbonylamino, (optionally substituted phenyl)sulfonylamino, cyano, C₁-C₂₋haloalkoxy, 5- or 6-membered N-containing heterocyclyl, aminosulfonyl, (6-membered N-containing heterocyclyl)sulfonyl, C₁-C₂₋haloalkylcarbonylaminosulfonyl and (optionally substituted phenyl)aminosulfonyl, and
6-membered nitrogen-containing heterocyclyl substituted with one or more substituents independently selected from pyridyl, phenyl, C₁-C₄ alkyl, C₁-C₂ haloalkyl, C₁-C₂ alkoxy, amino, halo, piperidinyl, morpholinyl, C₁-C₂ alkylpiperazinyl, C₁-C₃ alkylaminothiocarbonyl, N,N-di-C₁-C₂-alkylamino-C₁-C₄-alkylenyl, N-C₁-C₂₋alkylamino-C₁-C₄-alkylenyl, morpholinyl-C₁-C₄₋alkylenylaminocarbonyl, aminocarbonyl, C₁-C₂₋haloalkylcarbonylamino, morpholinyl-C₁-C₄₋alkylenylamino, N,N-di-C₁-C₂-alkylamino and N,N-di-C₁-C₂-alkylamino-C₁-C₄-alkylenylamino,
preferably 3-(N,N-dimethylamino)-1-propynyl, 3-fluorophenyl, 4-fluorophenyl, 4-(N,N-dimethylamino)phenyl, 3-(methylcarbonylamino)phenyl, phenyl, 3-trifluoromethylphenyl, 4-trifluoromethylphenyl, 4-aminophenyl, 3-aminophenyl, 4-aminosulfonylphenyl, 4-(4-morpholinylsulfonyl)phenyl, 4-(trifluoroacetylaminosulfonyl)phenyl, 4-(trifluoromethylcarbonylaminosulfonyl)phenyl, 4-[(4-chlorophenyl)aminosulfonyl]phenyl, 3-(phenylsulfonylamino)phenyl, 2,4-difluorophenyl, 2,4-dimethoxyphenyl, 3-hydroxyphenyl, 4-hydroxyphenyl, 3-ethoxyphenyl, 3,4-dimethoxyphenyl, 4-methylthiophenyl, 4-cyanophenyl, 4-trifluoromethoxyphenyl, 4-methoxyphenyl, 3-nitrophenyl, 3-methoxyphenyl, 2-methoxyphenyl, 2-thiazolyl, 2-pyrazinyl, 5-pyrimidinyl, 4-methyl-1-piperazinyl, 4-morpholinyl, 6-methoxy-3-pyridyl, 2-methoxy-3-pyridyl, 2-ethoxy-3-pyridyl, 3,4-dichloro-4-pyridyl, 6-(trifluoromethylcarbonylamino)-3-pyridyl, 6-amino-3-pyridyl, 3,5-dichloro-4-pyridyl, 2-chloro-4-pyridyl, 3-pyridyl and 4-pyridyl,
more preferably 5-pyrimidinyl, 2-pyrazinyl, morpholinyl, 4-methylpiperazinyl, 4-fluorophenyl, 4-(N,N-dimethylamino)propynyl, 3-nitrophenyl, 3-aminophenyl, 4-aminosulfonylphenyl, 3-aminosulfonylphenyl, 3-(phenylsulfonylamino)phenyl, 3-(methylcarbonylamino)phenyl, 4-[(trifluoromethylcarbonyl)aminosulfonyl)phenyl, 4-hydroxyphenyl, 4-methoxyphenyl, 2-thiazolyl, 6-(trifluoromethylcarbonylamino)-3-pyridyl, 6-amino-3-pyridyl, 3-pyridyl and 4-pyridyl;
and pharmaceutically acceptable salts thereof.

The invention also relates to compounds of Formula IV 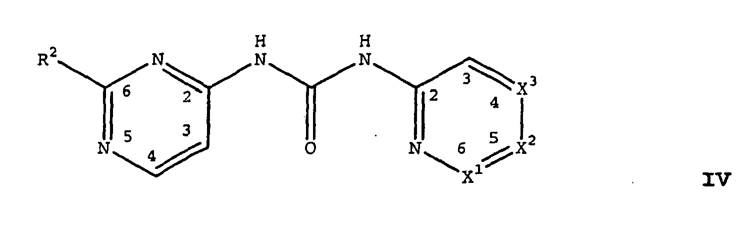
wherein X¹ is CR¹ or N; wherein X² is CR¹ or N; wherein X³ is CH or N; provided only one of X¹ X² and X³ can be N;
preferably X¹ is CR¹ ; X² is CR¹ ; X³ is CH; provided X² is CH when X¹ is not CH;
wherein R¹ is one or more substituents selected from H, optionally substituted pyrrolidinyl, optionally substituted piperazinyl, optionally substituted piperidinyl, morpholinyl, 1,4-dioxa-8-aza-spiro[4.5]decyl, pyridyl, phenyl, C₁-C₆-alkyl, C₁-C₂₋haloalkyl, C₁-C₄-hydroxyalkyl, amino, C₁-C₄₋azidoalkyl, C₁-C₄-cyanoalkyl, C₁-C₄-aminoalkyl, halo, hydroxy, (optionally substituted pyrrolidinyl)-C₁-C₂₋(optionally substituted piperidinyl)-C₁-C₂-, (optionally substituted piperazinyl)-C₁-C₂-, morpholinyl-C₁-C₂-, (optionally substituted imidazolyl)-C₁-C₂-, phthalimidyl-C₁-C₂-, optionally substituted azepanyl-C₁-C₂-, 1,4-dioxa-8-aza-spiro[4.5]decyl-C₁-C₂-, optionally substituted phenoxy-C₁-C₂-, C₁-C₄-alkylaminothiocarbonyl, C₁-C₄₋alkoxy-C₁-C₄-alkyl, C₁-C₄-alkylamino-C₁-C₄-alkyl, C₁₋C₄-hydroxyalkylamino-C₁-C₄-alkyl, amino-C₁-C₄-alkoxy-C₁-C₄-alkyl, (1-aza-bicyclo[2.2.2]oct-3-yl)-oxy, optionally substituted pyrrolidinyl-C₁-C₄-alkoxy, optionally substituted azetidinyl-C₁-C₄-alkoxy, optionally substituted piperidinyl-C₁-C₄-alkoxy, C₁₋C₄-alkylamino-C₁-C₄-alkoxy, tetrahydrofuryl-O-, tetrahydrofuryl-C₁-C₄-alkoxy, optionally substituted pyridyloxy, optionally substituted phenoxy, C₁-C₄₋alkoxycarbonyl, 5-6-membered heterocyclyl-C₁-C₄₋alkylaminocarbonyl, 5-6-membered N-containing heterocyclylcarbonyl, C₁-C₄-alkylaminocarbonyl, C₁₋C₄-alkylamino-C₁-C₄-alkylaminocarbonyl, aminocarbonyl, 5-6-membered N-containing heterocyclyl-C₁-C₄-alkylamino, C₁-C₄-alkylamino, C₁₋C₄-alkylamino-C₁-C₄-alkylamino-C₁-C₄-alkyl, and C₁-C₄₋alkylamino-C₁-C₄-alkylamino,
preferably methyl, ethyl, propyl, 1-methyl-4-piperazinyl, 1-benzyl-4-piperazinyl, 1-(2-pyrimidinyl)-4-piperazinyl, 1-(2-pyridyl)-4-piperazinyl, 1-ethyl-4-piperazinyl, 1-piperidinyl-CH₂-, 4-methyl-1-piperidinyl-CH₂ 3-methyl-1-piperidinyl-CH₂-, 2-methyl-1-piperidinyl-CH₂-, 3,5-dimethyl-1-piperidinyl-CH₂₋, 4-oxo-l-piperidinyl-CH₂-, 4-hydroxy-1-piperidinyl-CH₂-, 3-hydroxy-1-piperidinyl-CH₂-, 2-ethoxycarbonyl-1-piperidinyl-CH₂-, 3-ethoxycarbonyl-1-piperidinyl-CH₂-, 3-carboxy-1-piperidinyl-CH₂-, 4-ethoxycarbonyl-1-piperidinyl-CH₂-, 4-carboxy-1-piperidinyl-CH₂-, 4-(1-pyrrolidinyl)-1-piperidinyl-CH₂-, 4-(N-hydroxyethylamino)-1-piperidinyl-CH₂-, 4-(N-propylamino)-1-piperidinyl-CH₂-, 3-(N,N-diethylamino)carbonyl-1-piperidinyl-CH₂-, 4-morpholinyl-CH₂-, N,N-dimethylaminoethylenyl, N,N-diethylaminomethylenyl, N-methylaminomethylenyl, N-ethylaminomethylenyl and N,N-diethylamino, and
more preferably ethyl, propyl and 1-methyl-4-piperazinyl; and
wherein R² is halo, C₁-C₄-alkyl, C₁-C₄-alkylamino-C₂-C₄₋alkynyl, C₃-C₆-cycloalkyl, optionally substituted benzodioxolyl, optionally substituted indolyl, optionally substituted phenoxy, 5-membered oxygen or sulfur containing heteroaryl, 5- or 6-membered nitrogen-containing heterocyclyl, phenyl optionally substituted with one or two substituents selected from halo, C₁-C₄-alkylamino, amino, C₁-C₄-alkoxy, C₁-C₂-haloalkyl, hydroxy, C₁-C₄-alkylthio, cyano, C₁-C₂-haloalkyloxy, aminosulfonyl, (6-membered N-containing heterocyclyl)sulfonyl, C₁-C₂₋haloalkylcarbonylaminosulfonyl, and (optionally substituted phenyl)aminosulfonyl, and 6-membered nitrogen-containing heterocyclyl substituted with one or more substituents independently selected from pyridyl, phenyl, C₁₋C₄ alkyl, C₁-C₂ haloalkyl, C₁-C₂ alkoxy, halo, piperidinyl, morpholinyl, C₁-C₂ alkylpiperazinyl, C₁-C₃ alkylaminothiocarbonyl, N,N-di-C₁-C₂-alkylamino-C₁-C₄-alkylenyl, N-C₁-C₂₋alkylamino-C₁-C₄-alkylenyl, morpholinyl-C₁-C₄₋alkylenylaminocarbonyl, aminocarbonyl, morpholinyl-C₁-C₄-alkylenylamino, N,N-di-C₁-C₂₋alkylamino and N,N-di-C₁-C₂-alkylamino-C₁-C₄₋alkylenylamino,
preferably 3-fluorophenyl, 4-fluorophenyl, 4-(N,N-dimethylamino)phenyl, 3-(methylcarbonylamino)phenyl, phenyl, 3-trifluoromethylphenyl, 4-trifluoromethylphenyl, 4-aminophenyl, 3-aminophenyl, 4-aminosulfonylphenyl, 4-(4-morpholinylsulfonyl)phenyl, 4-(trifluoroacetylaminosulfonyl)phenyl, 4-(trifluoromethylcarbonylaminosulfonyl)phenyl, 4-[(4-chlorophenyl)aminosulfonyl]phenyl, 3-(phenylsulfonylamino)phenyl, 2,4-difluorophenyl, 2,4-dimethoxyphenyl, 3-hydroxyphenyl, 4-hydroxyphenyl, 3-ethoxyphenyl, 3,4-dimethoxyphenyl, 4-methylthiophenyl, 4-cyanophenyl, 4-trifluoromethoxyphenyl, 4-methoxyphenyl, 3-nitrophenyl, 3-methoxyphenyl, 2-methoxyphenyl, 2-thiazolyl, 2-pyrazinyl, 5-pyrimidinyl, 4-methyl-1-piperazinyl, 4-morpholinyl, 6-methoxy-3-pyridyl, 2-methoxy-3-pyridyl, 2-ethoxy-3-pyridyl, 3,4-dichloro-4-pyridyl, 6-(trifluoromethylcarbonylamino)-3-pyridyl, 6-amino-3-pyridyl, 3,5-dichloro-4-pyridyl, 2-chloro-4-pyridyl, 3-pyridyl and 4-pyridyl, and more preferably 4-pyridyl;
and pharmaceutically acceptable salts thereof.

The invention also relates to compounds of Formula V 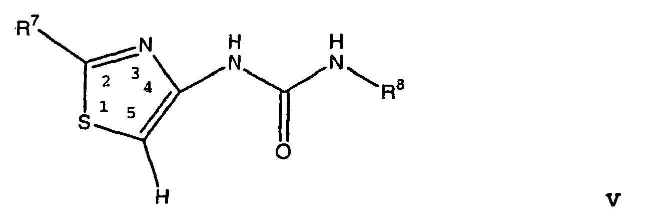
wherein R⁷ is selected from halo, C₁-C₄-alkyl, C₃-C₆₋cycloalkyl, optionally substituted benzodioxolyl, optionally substituted indolyl, optionally substituted phenoxy, 5-membered oxygen or sulfur containing heteroaryl, 6-membered nitrogen-containing heterocyclyl, phenyl optionally substituted with one or two substituents selected from halo, C₁-C₄-alkylamino, amino, C₁-C₄-alkoxy, C₁-C₂-haloalkyl, hydroxy, C₁-C₄-alkylthio, cyano, C₁-C₂-haloalkyloxy, aminosulfonyl, (6-membered N-containing heterocyclyl)sulfonyl, C₁-C₂₋haloalkylcarbonylaminosulfonyl, and (optionally substituted phenyl)aminosulfonyl, and 6-membered nitrogen-containing heterocyclyl substituted with one or more substituents independently selected from pyridyl, phenyl, C₁₋C₄ alkyl, C₁-C₂ haloalkyl, C₁-C₂ alkoxy, halo, piperidinyl, morpholinyl, C₁-C₂ alkylpiperazinyl, C₁-C₃ alkylaminothiocarbonyl, N,N-di-C₁-C₂₋alkylamino-C₁-C₄-alkylenyl, N-C₁-C₂-alkylamino-C₁₋C₄-alkylenyl, morpholinyl-C₁-C₄₋alkylenylaminocarbonyl, aminocarbonyl, morpholinyl-C₁-C₄-alkylenylamino, N,N-di-C₁-C₂₋alkylamino and N,N-di-C₁-C₂-alkylamino-C₁-C₄₋alkylenylamino,
preferably halo, C₁-C₄-alkyl, C₃-C₆-cycloalkyl, optionally substituted pyrimidinyl, morpholinyl, optionally substituted piperidinyl, optionally substituted benzodioxolyl, optionally substituted indolyl, optionally substituted phenoxy, optionally substituted thienyl, phenyl optionally substituted with one or two substituents selected from halo, C₁-C₄-alkylamino, Boc-amino, amino, C₁-C₄-alkoxy, C₁-C₂-haloalkyl, hydroxy, C₁-C₄-alkylthio, cyano, C₁-C₂₋haloalkyloxy, aminosulfonyl, (6-membered N-containing heterocyclyl)sulfonyl, C₁-C₂₋haloalkylcarbonylaminosulfonyl, and -(optionally substituted phenyl)aminosulfonyl,
and pyridyl optionally substituted with one or two substituents selected from C₁-C₃ alkyl, C₁-C₄₋alkoxy and halo,
more preferably bromo, chloro, fluoro, C₁-C₃₋alkyl, C₃-C₆-cycloalkyl, optionally substituted pyrimidinyl, morpholinyl, piperidinyl, benzodioxolyl, indolyl, phenoxy, thienyl, phenyl optionally substituted with one or two substituents selected from fluoro, N,N-dimethylamino, amino, methoxy, trifluoromethyl, Boc-amino, hydroxy, ethoxy, methylthio, cyano, trifluoromethoxy, aminosulfonyl, 4-morpholinylsulfonyl, trifluoroacetylaminosulfonyl, and (4-chlorophenyl)aminosulfonyl,
and pyridyl optionally substituted with one or two substituents selected from C₁-C₃ alkyl, methoxy, ethoxy and chloro,
even more preferably bromo, methyl, ethyl, cyclopropyl, cyclohexyl, 3-fluorophenyl, 4-fluorophenyl, 4-(N,N-dimethylamino)phenyl, phenyl, 3-trifluoromethylphenyl, 4-trifluoromethylphenyl, 4-aminophenyl, 3-aminophenyl, 4-Boc-aminophenyl, 4-aminosulfonylphenyl, 4-(4-morpholinylsulfonyl)phenyl, 4-(trifluoroacetylaminosulfonyl)phenyl, 4-[(4-chlorophenyl)aminosulfonyl]phenyl, 2,4-difluorophenyl, 5-benzodioxolyl, 2,4-dimethoxyphenyl, 3-hydroxyphenyl, 3-ethoxyphenyl, 3,4-dimethoxyphenyl, 4-methylthiophenyl, 5-indolyl, 4-cyanophenyl, 4-trifluoromethoxyphenyl, 4-methoxyphenyl, 3-methoxyphenyl, 2-methoxyphenyl, phenoxy, 2-thienyl, 4-pyrimidinyl, 2-methylthio-4-pyrimidinyl, morpholinyl, 4-piperidinyl, 6-methoxy-3-pyridyl, 2-methoxy-3-pyridyl, 2-ethoxy-3-pyridyl, 3,4-dichloro-4-pyridyl, 3,5-dichloro-4-pyridyl, 2-chloro-4-pyridyl, 3-pyridyl and 4-pyridyl; and
wherein R⁸ is selected from 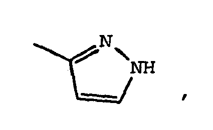 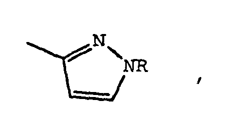 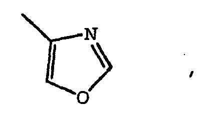 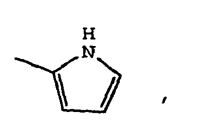 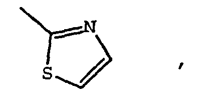 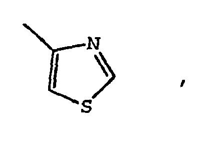 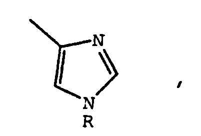 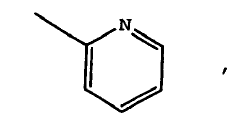 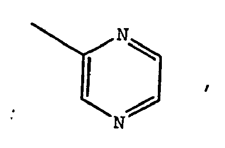 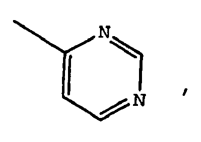 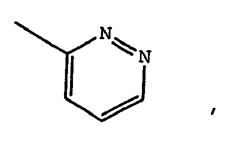 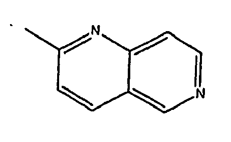 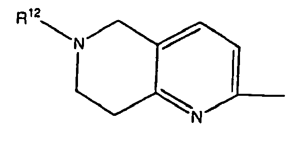 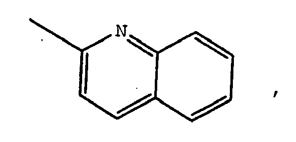 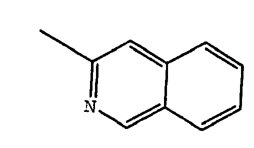 and 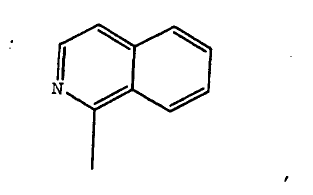 preferably 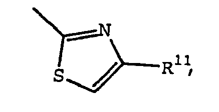 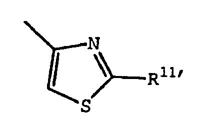 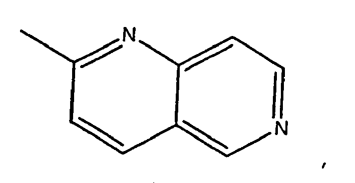 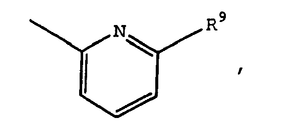 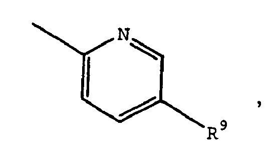 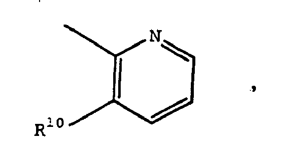 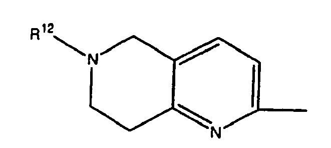 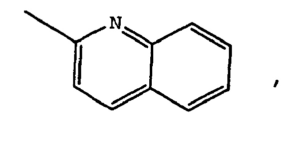 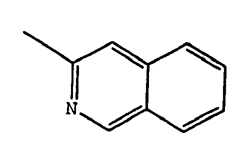 and 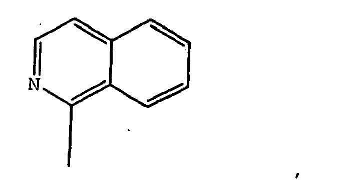 more preferably 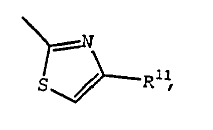 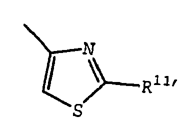 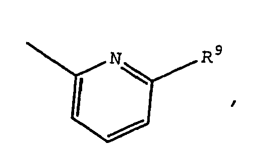 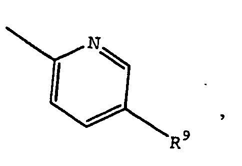 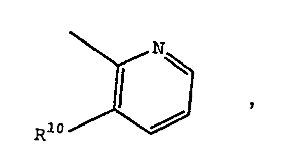 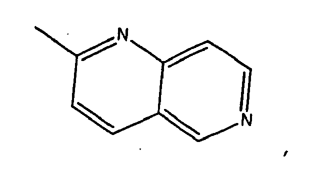 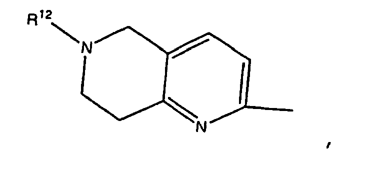 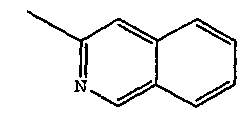 and 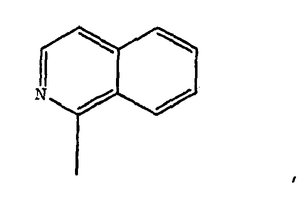 and even more preferably 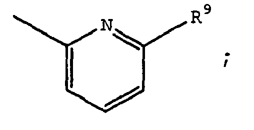
wherein R⁸ is optionally substituted with one or two substituents independently selected from H, optionally substituted pyrrolidinyl, optionally substituted piperazinyl, optionally substituted piperidinyl, morpholinyl, 1,4-dioxa-8-aza-spiro[4.5]decyl, pyridyl, phenyl, C₁-C₆-alkyl, C₁₋C₂-haloalkyl, C₁-C₄-hydroxyalkyl, amino, C₁-C₄₋azidoalkyl, C₁-C₄-cyanoalkyl, C₁-C₄-aminoalkyl, halo, hydroxy, (optionally substituted pyrrolidinyl)-C₁-C₂-, (optionally substituted piperidinyl)-C₁-C₂-, (optionally substituted piperazinyl)-C₁-C₂-, morpholinyl-C₁-C₂-, (optionally substituted imidazolyl)-C₁-C₂-, phthalimidyl-C₁-C₂-, optionally substituted azepanyl-C₁-C₂-, 1,4-dioxa-8-aza-spiro[4.5]decyl-C₁-C₂-, optionally substituted phenoxy-C₁-C₂-, C₁-C₄-alkylaminothiocarbonyl, C₁-C₄₋alkoxy-C₁-C₄-alkyl, C₁-C₄-alkylamino-C₁-C₄-alkyl, C₁₋C₄-hydroxyalkylamino-C₁-C₄-alkyl, amino-C₁-C₄-alkoxy-C₁-C₄-alkyl, (1-aza-bicyclo[2.2.2]oct-3-yl)-oxy, optionally substituted pyrrolidinyl-C₁-C₄-alkoxy, optionally substituted azetidinyl-C₁-C₄-alkoxy, optionally substituted piperidinyl-C₁-C₄-alkoxy, C₁₋C₄-alkylamino-C₁-C₄-alkoxy, tetrahydrofuryl-O-, tetrahydrofuryl-C₁-C₄-alkoxy, optionally substitute pyridyloxy, optionally substituted phenoxy, C₁-C₄₋alkoxycarbonyl, 5-6-membered heterocyclyl-C₁-C₄₋alkylaminocarbonyl, 5-6-membered N-containing heterocyclylcarbonyl, C₁-C₄-alkylaminocarbonyl, C₁₋C₄-alkylamino-C₁-C₄-alkylaminocarbonyl, aminocarbonyl, 5-6-membered N-containing heterocyclyl-C₁-C₄-alkylamino, C₁-C₄-alkylamino, C₁₋C₄-alkylamino-C₁-C₄-alkylamino-C₁-C₄-alkyl, and C₁₋C₄-alkylamino-C₁-C₄-alkylamino,
preferably unsubstituted or substituted with one or more substituents selected from pyridyl, phenyl, C₁-C₄ alkyl, C₁-C₂ haloalkyl, halo, piperidinyl, morpholinyl, methylpiperazinyl, methylaminothiocarbonyl, N,N-diethylaminomethylenyl, N-methylaminomethylenyl, morpholinylpropylenylaminocarbonyl, aminocarbonyl morpholinylpropylenylamino, N,N-diethylamino and N,N-dimethylaminoethylenylamino;
wherein R⁹ is selected from optionally substituted pyrrolidinyl, optionally substituted piperazinyl, optionally substituted piperidinyl, morpholinyl, 1,4-dioxa-8-aza-spiro[4.5]decyl, pyridyl, phenyl, C₁-C₄ alkyl, C₁-C₂ haloalkyl, C₁-C₂ hydroxyalkyl, amino, C₁-C₂ azidoalkyl, C₁-C₂ cyanoalkyl, C₁-C₂ aminoalkyl, halo, (optionally substituted pyrrolidinyl)CH₂-, (optionally substituted piperidinyl)-CH₂-, (optionally substituted piperazinyl)-CH₂-, 4-morpholinyl-CH₂-, (optionally substituted imidazolyl)-CH₂-, phthalimidylethyl, optionally substituted azepanyl-CH₂-, 1,4-dioxa-8-aza-spiro[4.5]decyl-CH₂-, optionally substituted phenoxy-CH₂-, C₁-C₄-alkylaminothiocarbonyl, C₁-C₄₋alkoxy-C₁-C₄-alkyl, C₁-C₄-alkylamino-C₁-C₄-alkyl, C₁₋C₄-hydroxyalkylamino-C₁-C₄-alkyl, Boc-aminoethoxymethylenyl, amino-C₁-C₄-alkoxy-C₁-C₄₋alkyl, (1-aza-bicyclo[2.2.2]oct-3-yl)-oxy, optionally substituted pyrrolidinyl-C₁-C₄-alkoxy, optionally substituted azetidinyl-C₁-C₄-alkoxy, optionally substituted piperidinyl-C₁-C₄-alkoxy, C₁₋C₄-alkylamino-C₁-C₄-alkoxy, tetrahydrofuryl-O-, tetrahydrofuryl-C₁-C₄-alkoxy, optionally substituted phenoxy, C₁-C₄-alkoxycarbonyl, heterocyclyl-C₁-C₄₋alkylaminocarbonyl, 1-piperidinylcarbonyl, C₁-C₄₋alkylaminocarbonyl, C₁-C₄-alkylamino-C₁-C₄₋alkylaminocarbonyl, aminocarbonyl, morpholinyl-C₁-C₄₋alkylamino, C₁-C₄-alkylamino, C₁-C₄-alkylamino-C₁-C₄₋alkylamino-C₁-C₄-alkyl, and C₁-C₄-alkylamino-C₁-C₄₋alkylamino,
preferably 3-(N,N-dimethylamino)-1-pyrrolidinyl, 1-methyl-4-piperazinyl, 1-benzyl-4-piperazinyl, 1-(2-pyrimidinyl)-4-piperazinyl, 1-(2-pyridyl)-4-piperazinyl, 1-ethyl-4-piperazinyl, 4-amino-1-piperidinyl, 4-(N-hydroxyethylamino)-1-piperidinyl, 4-(N-propylamino)-1-piperidinyl, 4-(N-benzylamino)-1-piperidinyl, 4-oxo-piperidinyl, 4-(hydroxyimino)-piperidinyl, 4-morpholinyl, 1,4-dioxa-8-aza-spiro[4.5]decyl, pyridyl, phenyl, methyl, ethyl, propyl, amino, azidomethyl, hydroxymethyl, trifluoromethyl, fluoro, chloro, bromo, aminoethyl, aminomethyl, cyanomethyl, 1-pyrrolidinyl-CH₂-, 2-methoxycarbonyl-1-pyrrolidinyl-CH₂-, 2-carboxy-1-pyrrolidinyl-CH₂-, 2-hydroxymethyl-1-pyrrolidinyl-CH₂-, 1-piperidinyl-CH₂-, 4-methyl-1-piperidinyl-CH₂-, 3-methyl-1-piperidinyl-CH₂-, 2-methyl-1-piperidinyl-CH₂-, 3,5-dimethyl-1-piperidinyl-CH₂-, 4-oxo-1-piperidinyl-CH₂-, 4-hydroxy-1-piperidinyl-CH₂-, 3-hydroxy-1-piperidinyl-CH₂-, 2-ethoxycarbonyl-1-piperidinyl-CH₂-, 3-ethoxycarbonyl-1-piperidinyl-CH₂-, 3-carboxy-1-piperidinyl-CH₂-, 4-ethoxycarbonyl-1-piperidinyl-CH₂-, 4-carboxy-1-piperidinyl-CH₂-, 4-(1-pyrrolidinyl)-1-piperidinyl-CH₂-, 4-(N-hydroxyethylamino)-1-piperidinyl-CH₂-, 4-(N-propylamino)-1-piperidinyl-CH₂-, 1-methyl-4-piperazinyl-CH₂-, 4-morpholinyl-CH₂-, (2-methyl-1-imidazolyl-CH₂-, 3-(N,N-diethylamino)carbonyl-1-piperidinyl-CH₂-, phthalimidylethyleneyl, 1-azepanyl-CH₂-, 1,4-dioxa-8-aza-spiro[4.5]decyl-CH₂-, 4-(methyl)phenoxymethylenyl, 4-(N,N-dimethylaminomethylenyl)phenoxymethylenyl, methylaminothiocarbonyl, methoxymethylenyl, ethylaminothiocarbonyl, N,N-dimethylaminoethylenyl, N,N-diethylaminomethylenyl, N-methylaminomethylenyl, N-(hydroxypropyl)aminomethylenyl, N-ethylaminomethylenyl, Boc-aminoethoxymethylenyl, aminoethoxymethylenyl, (1-aza-bicyclo[2.2.2]oct-3-yl)-oxy, 2-pyrrolidinylmethoxy, 1-methyl-2-pyrrolidinylmethoxy, azetidin-3-ylmethoxy, N-Boc-azetidin-3-ylmethoxy, N-Boc-piperidin-4-ylethoxy, 1-methyl-4-piperidinylethoxy, 4-piperidinylethoxy, 4-piperidinylmethoxy, N,N-dimethylaminoethoxy, 3-tetrahydrofuryl-O-, 3-tetrahydrofurylmethoxy, 4-tetrahydrofurylmethoxy, 4-methylphenoxy, 4-(aminoethyl)phenoxy, 4-(1-imidazolyl)phenoxy, 2,4-dimethylphenoxy, phenoxy, 4-cyanophenoxy, 4-[1,3]dioxolan-2-ylphenoxy, 4-fluorophenoxy, 3,4-difluorophenoxy, ethoxycarbonyl, morpholinylpropylenylaminocarbonyl, 1-piperidinylcarbonyl, methylaminocarbonyl, ethylaminocarbonyl, N,N-diethylaminocarbonyl, N-(N',N'-dimethylaminoethylenyl)aminocarbonyl, aminocarbonyl, morpholinylpropylenylamino, N,N-diethylamino, N,N-diethylamino(2-propylenyl)aminomethylenyl, N,N-diethylamino(1-propylenyl)aminomethylenyl and N-(N',N'-dimethylaminoethylenyl)amino;
wherein R¹⁰ is selected from H, hydroxy, and amino;
wherein R¹¹ is selected from pyridyl and pyrimidinyl, preferably pyridyl; and
wherein R¹² is selected from H, and C₁-C₄ alkyl, preferably H, methyl, ethyl and propyl;
and pharmaceutically acceptable salts thereof.

A family of specific compounds of particular interest within Formula I consists of compounds and pharmaceutically-acceptable salts thereof as follows:
N-[2-(4-Pyridinyl)-4-thiazolyl]-N'-2-[6-(1-morpholinylmethyl)pyridinyl]urea;
Ethyl 1-(6-[3-(2-(pyridin-4-yl)thiazol-4-yl)ureido]-pyridin-2-ylmethyl}-piperidine-4-carboxylate;
tert-Butyl (1-hydroxymethyl-3-methyl-butyl)-{6-[3-(2-pyridin-4-yl-thiazol-4-yl)-ureido]-pyridin-2-ylmethyl}-carbamate;
1-[6-(1,4-Dioxa-8-aza-spiro[4.5]dec-8-ylmethyl)-pyridin-2-yl]-3-(2-pyridin-4-yl-thiazol-4-yl)-urea;
1-[6-(3,5-Dimethylpiperidin-1-ylmethyl)pyridin-2-yl]-3-(2-pyridin-4-ylthiazol-4-yl)urea;
1-[6-(4-Methylpiperidin-1-ylmethyl)pyridin-2-yl]-3-(2-pyridin-4-yl-thiazol-4-yl)urea;
1-[6-(2-Methylpiperidin-1-ylmethyl)pyridin-2-yl)-3-(2-pyridin-4-yl-thiazol-4-yl)urea;
1-(2-Pyridin-4-yl-thiazol-4-yl)-3-[6-(4-pyrrolidin-1-yl-piperidin-1-ylmethyl)-pyridin-2-yl]-urea;
1-[6-(3-Hydroxy-piperidin-1-ylmethyl)-pyridin-2-yl]-3-(2-pyridin-4-yl-thiazol-4-yl)-urea;
1-[6-(2-Methyl-imidazol-1-ylmethyl)-pyridin-2-yl]-3-(2-pyridin-4-yl-thiazol-4-yl)-urea;
1-(6-Azepan-1-ylmethyl-pyridin-2-yl)-3-(2-pyridin-4-yl-thiazol-4-yl)urea;
1-[6-(4-Hydroxy-piperidin-1-ylmethyl)-pyridin-2-yl]-3-(2-pyridin-4-yl-thiazol-4-yl)-urea;
Ethyl 1-{6-[3-(2-pyridin-4-yl-thiazol-4-yl)ureido]-pyridin-2-ylmethyl}piperidine-3-carboxylate;
Ethyl 1-[6-[3- (2- (pyridin-4-yl) thiazol-4-yl) ureido] - pyridin-2-ylmethyl]piperidine-2-carboxylate;
N,N-Diethyl 1-{6-[3-(2-pyridin-4-yl-thiazol-4-yl)-ureido]pyridin-2-ylmethyl}piperidine-3-carboxamide;
1-{6-[3-(2-Pyridin-4-yl-thiazol-4-yl)-ureido]-pyridin-2-ylmethyl}-piperidine-3-carboxylic acid;
Methyl 1-{6-[3-(2-pyridin-4-yl-thiazol-4-yl)ureido]-pyridin-2-ylmethyl}-pyrrolidine-2-carboxylate;
1-[6-(3-Methyl-piperidin-1-ylmethyl)-pyridin-2-yl]-3-(2-pyridin-4-yl-thiazol-4-yl)-urea;
1-(2-Phenoxy-thiazol-4-yl)-3-(6-piperidin-1-ylmethyl-pyridin-2-yl)-urea;
tert Butyl 3-{6-[3-(2-pyridin-4-yl-thiazol-4-yl)-ureido]-pyridin-2-yloxymethyl}-azetidine-1-carboxylate;
tert Butyl 4-(2-{6-[3-(2-pyridin-4-yl-thiazol-4-yl)-ureido]pyridin-2-yloxy}ethyl)piperidine-1-carboxylate;
1-[6-(4-Dimethylaminomethyl-phenoxymethyl)-pyridin-2-yl]-3-(2-pyridin-4-yl-thiazol-4-yl)-urea;
1-(2-Pyridin-4-yl-thiazol-4-yl)-3-(6-(4-methylphenyl)oxymethylpyridin-2-yl)urea;
tert Butyl (2-{6-[3-(2-pyridin-4-yl-thiazol-4-yl)-ureido]pyridin-2-ylmethoxy}ethyl)carbamate;
tert Butyl (2-{6-[3-(2-pyridin-4-yl-thiazol-4-yl)-ureido]pyridin-3-ylmethoxy}ethyl)carbamate;
1-(5-Methoxymethyl-pyridin-2-yl)-3-(2-pyridin-4-yl.-thiazol-4-yl)-urea;
1-(5-Morpholin-4-ylmethyl-pyridin-2-yl)-3-(2-pyridin-4-yl-thiazol-4-yl)urea;
1-{6-[2-phthalimidylethyl]pyridin-2-yl}-3-(2-pyridin-4-yl-thiazol-4-yl)urea;
1-(6-Cyanomethylpyridin-2-yl)-3-(2-pyridin-4-yl-thiazol-4-yl)urea;
1-[2-(2-Chloropyridin-4-yl)thiazol-4-yl]-3-(6-morpholin-4-ylmethyl-pyridin-2-yl)urea;
1-(6-Aminopyridin-2-yl)-3-(2-pyridin-4-yl-thiazol-4-yl)urea;
1-(6-Morpholin-4-yl-pyridin-2-yl)-3-(2-pyridin-4-yl-thiazol-4-yl)urea;
1-[6-(2,4-Dimethylphenoxy)pyridin-2-yl]-3-(2-pyridin-4-yl-thiazol-4-yl)urea;
1-(6-Phenoxypyridin-2-yl)-3-(2-pyridin-4-yl-thiazol-4-yl)urea;
1-[6-(1,4-Dioxa-8-aza-spiro[4.5]dec-8-yl)-pyridin-2-yl]-3-(2-pyridin-4-yl-thiazol-4-yl)urea;
1-(2-Pyridin-4-yl-thiazol-4-yl)-3-(6-p-tolyloxy-pyridin-2-yl)-urea;
1-(4-OxO-3,4,5,6-tetrahydro-2H-[1,2']bipyridinyl-6'-yl)-3-(2-pyridin-4-yl-thiazol-4-yl)-urea;
1-(4-Benzylamino-3,4,5,6-tetrahydro-2H-[1,2']bipyridinyl-6'-yl)-3-(2-pyridin-4-yl-thiazol-4-yl)-urea;
1-(4-Propylamino-3,4,5,6-tetrahydro-2H-[1,2']bipyridinyl-6'-yl)-3-(2-pyridin-4-yl-thiazol-4-yl)-urea;
1-[4-(2-Hydroxy-ethylamino)-3,4,5,6-tetrahydro-2H-[1,2']bipyridinyl-6'-yl]-3-(2-pyridin-4-yl-thiazol-4-yl)-urea;
1-(4-Amino-3,4,5,6-tetrahydro-2H-[1,2']bipyridinyl-6'-yl)-3-(2-pyridin-4-yl-thiazol-4-yl)-urea;
1-[6-(4-Cyanophenoxy)-pyridin-2-yl]-3-(2-pyridin-4-yl-thiazol-4-yl)urea;
1-(4-Hydroxyimino-3,4,5,6-tetrahydro-2H-[1,2']bipyridinyl-6'-yl)-3-(2-pyridin-4-yl-thiazol-4-yl)-urea;
1-[6-(1-Aza-bicyclo[2.2.2]oct-3-yloxy)-pyridin-2-yl]-3-(2-pyridin-4-yl-thiazol-4-yl)-urea;
1-[6-(3-Dimethylamino-pyrrolidin-1-yl)-pyridin-2-yl]-3-(2-pyridin-4-yl-thiazol-4-yl)-urea;
1-[6-(2-Dimethylamino-ethoxy)-pyridin-2-yl]-3-(2-pyridin-4-yl-thiazol-4-yl)-urea;
1-(2-Methylthiazol-4-yl)-3-(6-phenoxy-pyridin-2-yl)urea;
1-(6-(1-Methylpyrrolidin-2-ylmethoxy)pyridin-2-yl]-3-(2-pyridin-4-yl-thiazol-4-yl)urea;
1-[6-(4-Imidazol-1-yl-phenoxy)-pyridin-2-yl]-3-(2-pyridin-4-yl-thiazol-4-yl)-urea;
1-(6-Phenoxypyridin-2-yl)-3-(2-pyridin-3-yl-thiazol-4-yl)urea;
1-[6-(4-[1,3]Dioxolan-2-yl-phenoxy)pyridin-2-yl]-3-(2-pyridin-4-yl-thiazol-4-yl)urea;
1-[6-(4-Fluorophenoxy)pyridin-2-yl]-3-(2-pyridin-4-yl-thiazol-4-yl)urea;
1-[6-(3,4-Difluorophenoxy)pyridin-2-yl]-3-(2-pyridin-4-yl-thiazol-4-yl)urea;
1-{6-[4-(2-Aminoethyl)phenoxy]pyridin-2-yl}-3-(2-pyridin-4-yl-thiazol-4-yl)urea;
1-Pyridin-3-yl-3-(2-pyridin-3-yl-thiazol-4-yl)-urea;
6-[3-(2-Pyridin-4-yl-thiazol-4-yl)-ureido]-pyridine-2-carbothioic acid methylamide;
1-(6-Diethylaminomethyl-pyridin-2-yl)-3-(2-pyridin-4-yl-thiazol-4-yl)urea;
1-(6-Methylaminomethyl-pyridin-2-yl)-3-(2-pyridin-4-yl-thiazol-4-yl)urea;
1-[6-(3-Morpholin-4-yl-propylamino)-pyridin-2-yl]-3-(2-pyridin-4-yl-thiazol-4-yl)-urea;
1-[6-(2-Dimethylamino-ethylamino)-pyridin-2-yl]-3-(2-pyridin-4-yl-thiazol-4-yl)-urea;
1-(6-Diethylamino-pyridin-2-yl)-3-(2-pyridin-4-yl-thiazol-4-yl)-urea;
6-[3-(2-Pyridin-4-yl-thiazol-4-yl)-ureido]nicotinamide;
4-{4-[3-(6-Propylpyridin-2-yl)ureido]thiazol-2-yl}-benzenesulfonamide;
tert Butyl (4-{4-[3-(6-Propylpyridin-2-yl)ureido]-thiazol-2-yl}phenyl)carbamate;
2-Dimethylaminoethyl 6-[3-(2-pyridin-4-yl-thiazol-4-yl)ureido]pyridine-2-carboxamide;
1-[6-(4-Ethylpiperazin-1-yl)-pyridin-2-yl]-3-(2-pyridin-4-yl-thiazol-4-yl)urea;
1-{2-[4-(4-Morpholinylsulfonyl)phenyl]thiazol-4-yl}-3-(6-propyl-pyridin-2-yl)urea;
1-[2-(4-Aminoghenyl)thiazol-4-yl]-3-(6-propylpyridin-2-yl)urea;
1-[6-(4-Benzylpiperazin-1-yl)pyridin-2-yl]-3-(2-pyridin-4-yl-thiazol-4-yl)urea;
1-[6-(4-Methyl-piperazin-1-ylmethyl)-pyridin-2-yl]-3-(2-pyridin-4-yl-thiazol-4-yl)-urea;
1-(6-Hydroxymethyl-pyridin-2-yl)-3-(2-pyridin-4-yl-thiazol-4-yl)-urea;
Diethyl 6-[3-(2-pyridin-4-yl-thiazol-4-yl)ureido]-pyridine-2-carboxamide;
1-[6-(4-Methylpiperazin-1-yl)pyridin-2-yl]-3-(2-pyridin-3-yl-thiazol-4-yl)urea;
1-(6-Piperidin-1-ylmethyl-pyridin-2-yl)-3-(2-pyridin-4-yl-thiazol-4-yl)-urea;
6-[3-(2-Pyridin-4-yl-thiazol-4-yl)-ureido]-pyridine-2-carboxylic acid ethyl ester;
1-[6-(Piperidine-1-carbonyl)pyridin-2-yl]-3-(2-pyridin-4-yl-thiazol-4-yl)urea;
1-[6-(4-Methylpiperazin-1-yl)pyridin-2-yl]-3-(2-pyrimidin-4-yl-thiazol-4-yl)urea;
1-(6-Diethylaminomethyl-pyridin-2-yl)-3-(2-pyrimidin-4-yl-thiazol-4-yl)urea;
1-(6-Diethylaminomethyl-pyridin-2-yl)-3-(2-pyridin-3-yl-thiazol-4-yl)urea;
Methyl 6-[3-(2-pyridin-4-yl-thiazol-4-yl)ureido]pyridine-2-carboxamide;
1-[6-(Piperidine-1-carbonyl)pyridin-2-yl]-3-(2-pyridin-3-yl-thiazol-4-yl)urea;
1-(6-Ethylaminomethylpyridin-2-yl)-3-(2-pyridin-4-yl-thiazol-4-yl)urea;
Ethyl 6-[3-(2-Pyridin-4-yl-thiazol-4-yl)ureido]pyridine-2-carboxamide;
Ethyl 6-[3-(2-pyridin-4-yl-thiazol-4-yl)ureido]-pyridine-2-thiocarboxamide;
1-(2-Pyridin-4-yl-thiazol-4-yl)-3-[6-(4-pyrimidin-2-yl-piperazin-1-yl)pyridin-2-yl]urea;
1-(6-Piperidin-1-ylmethyl-pyridin-2-yl)-3-(2-pyridin-3-yl-thiazol-4-yl)-urea;
1-(2-Pyridin-4-yl-thiazol-4-yl)-3-(6-pyrrolidin-1-ylmethyl-pyridin-2-yl)-urea;
1-[1,6]Naphthyridin-2-yl-3-(2-pyridin-4-yl-thiazol-4-yl)-urea;
1-[6-(4-Pyridin-2-yl-piperazin-1-yl)pyridin-2-yl]-3-(2-pyridin-4-yl-thiazol-4-yl)urea;
1-[6-(4-Pyridin-2-yl-piperazin-1-yl)-pyridin-2-yl]-3-(2-pyridin-4-yl-thiazol-4-yl)-urea;
1-(6-Propyl-5,6,7,8-tetrahydro-[1,6]naphthyridin-2-yl)-3-(2-pyridin-4-yl-thiazol-4-yl)-urea;
1-(6-Ethyl-5,6,7,8-tetrahydro-[1,6]naphthyridin-2-yl)-3-(2-pyridin-4-yl-thiaz ol-4-yl)-urea;
N-12-(4-Pyridinyl)-4-thiazolyl]-N'-2-[6-(1-morpholinylmethyl)pyridinyl]urea hydrochloride;
Ethyl 1-{6-[3-(2-pyridin-4-yl-thiazol-4-yl)ureido]-pyridin-2-ylmethyl}-piperidine-4-carboxylate hydrochloride;
1-[6-(3,5-Dimethylpiperidin-1-ylmethyl)pyridin-2-yl]-3-(2-pyridin-4-ylthiazol-4-yl)urea hydrochloride;
1-[6-(4-Oxo-piperidin-1-ylmethyl)pyridin-2-yl]-3-(2-pyridin-4-yl-thiazol-4-yl)urea hydrochloride ;
1-[6-(4-Methylpiperidin-1-ylmethyl)pyridin-2-yl]-3-(2-pyridin-4-yl-thiazol-4-yl)urea hydrochloride;
1-[6-(2-Methylpiperidin-1-ylmethyl)pyridin-2-yl]-3-(2-pyridin-4-yl-thiazol-4-yl)urea hydrochloride ;
Ethyl 1-{6-[3-(2-pyridin-4-yl-thiazol-4-yl)ureido]-pyridin-2-ylmethyl}piperidine-3-carboxylate hydrochloride;
1-(6-Azepan-1-ylmethyl-pyridin-2-yl)-3-(2-pyridin-4-yl-thiazol-4-yl)urea hydrochloride;
1-(6-Diethylaminomethyl-pyridin-2-yl)-3-(2-piperidin-4-yl-thiazol-4-yl)urea hydrochloride;
1-[5-Bromo-2-(pyridin-4-yl)thiazol-4-yl)-3-(6-diethylaminomethyl-pyridin-2-yl)urea hydrochloride;
Ethyl 1-{6-[3-(2-pyridin-4-yl-thiazol-4-yl)-ureido]-pyridin-2-ylmethyl}-piperidine-2-carboxylate hydrochloride;
N,N-Diethyl 1-{6-[3-(2-pyridin-4-yl-thiazol-4-yl)-ureido]pyridin-2-ylmethyl}piperidine-3-carboxamide hydrochloride;
1-[6-(Morpholin-4-ylmethyl)-pyridin-2-yl]-3-[(2-pyridin-3-yl)thiazol-4-yl]urea hydrochloride;
1-[6-(Azetidin-3-ylmethoxy)pyridin-2-yl]-3-[2-(pyridin-4-yl)thiazol-4-yl]urea;
1-[6-(2-Piperidin-4-yl-ethoxy)pyridin-2-yl]-3-[2-(pyridin-4-yl)thiazol-4-yl]urea;
N-[2-(3-Pyridinyl)-4-thiazolyl]-N'-2-[6-aminopyridin-2-yl]urea;
1-[2-(2,6-Dichloropyridin-4-yl)thiazol-4-yl]-3-[6-(piperidin-1-ylmethyl)pyridin-2-yl]urea;
1-[6-(Piperidin-1-ylmethyl)pyridin-2-yl]-3-[2-[6-(2,2,2-trifluoroethoxy)pyridin-3-yl]thiazol-4-yl]urea;
1-[6-(2-Methylimidazol-1-ylmethyl)pyridin-2-yl]-3-[2-(pyridin-3-yl)thiazol-4-yl]urea;
1-[6-(Morpholin-4-ylmethyl)-pyridin-2-yl]-3-[(2-pyridin-3-yl)thiazol-4-yl]urea;
1-{6-[3-(2-(4-Pyridinyl)-4-thiazolyl)ureido]-pyridin-2-ylmethyl}-piperidine-4-carboxylic acid;
1-{6-[(1-Hydroxymethyl-3-methylbutylamino)methyl]-pyridin-2-yl}-3-(2-pyridin-4-yl-thiazol-4-yl)urea;
1-[6-(4-Oxo-piperidin-1-ylmethyl)pyridin-2-yl]-3-(2-pyridin-4-yl-thiazol-4-yl)urea;
1-[6-[4-(Propylamino)piperidin-1-ylmethyllpyridin-2-yl]-3-(2-pyridin-4-yl-thiazol-4-yl)urea;
1-{6-[4-(2-Hydroxyethylamino)piperidin-1-ylmethyl]-pyridin-2-yl}-3- (-1-pyridin-4-yl-thiazol-4-yl) urea;
N-(6-Aminomethyl-2-pyridyl)-N'-[2-(4-pyridinyl)-4-thiazolyl]urea;
1-(6-Diethylaminomethyl-pyridin-2-yl)-3-(2-piperidin-4-yl-thiazol-4-yl)urea;
1-[5-Bromo-2-(pyridin-4-yl)thiazol-4-yl)-3-(6-diethylaminomethyl-pyridin-2-yl)urea;
1-{6-[(3-Hydroxypropylamino)methyl]-pyridin-2-yl}-3-(2-pyridin-4-yl-thiazol-4-yl)urea;
1-[6-(2-Hydroxymethylpyrrolidin-1-ylmethyl)-pyridin-2-yl]-3-(2-pyridin-4-yl-thiazol-4-yl)urea;
1-{6-[3-(2-Pyridin-4-yl-thiazol-4-yl)ureido]-pyridin-2-. ylmethyl}-pyrrolidine-2-carboxylic acid;
1-(5-Bromo-(2-pyridin-4-yl)thiazol-4-yl)-3-(6-methylpyridin-2-yl)urea;
4-{4-[3-(6-Propyl-pyridin-2-yl)-ureido]-thiazol-2-yl}-benzenesulfonamide;
1-{2-[4-(4-Morpholinylsulfonyl)phenyl]thiazol-4-yl}-3-(6-propylpyridin-2-yl)urea;
tert-Butyl (4-{4-[3-(6-propylpyridin-2-yl)ureido]-thiazol-2-yl}phenyl)carbamate;
1-[2-(4-Aminophenyl)thiazol-4-yl]-3-(6-propylpyridin-2-yl)urea;
1-{6-[2-(1-Methylpiperidin-4-yl)ethoxy]pyridin-2-yl}-3-(2-pyridin-4-yl-thiazol-4-yl)urea;
1-[6-(2-Aminoethoxymethyl)pyridin-2-yl]-3-(2-pyridin-4-yl-thiazol-4-yl)urea;
1-[5-(2-Aminoethoxymethyl)pyridin-2-yl]-3-(2-pyridin-4-yl-thiazol-4-yl)urea;
1-{6-[2-Aminoethyl]pyridin-2-yl}-3-(2-pyridin-4-yl-thiazol-4-yl)urea;
1-{6-[2-(N,N-Dimethylamino)ethyl]pyridin-2-yl}-3-(2-pyridin-4-yl-thiazol-4-yl)urea;
1-[2-(2-Ethoxypyridin-4-yl)thiazol-4-yl]-3-(6-morpholin-4-ylmethyl-pyridin-2-yl)urea;
1-[2-(2-Methoxypyridin-4-yl)thiazol-4-yl]-3-(6-morpholin-4-ylmethyl-pyridin-2-yl)urea;
1-[2-(2-Ethoxypyridin-4-yl)thiazol-4-yl]-3-(6-ethyl-pyridin-2-yl)urea;
1-[2-(6-Methoxypyridin-3-yl)thiazol-4-yl)-3-(6-piperidin-1-ylmethyl-pyridin-2-yl)urea;
1-(2-Bromothiazol-4-yl)-3-(6-piperidin-1-ylmethyl-pyridin-2-yl)urea;
1-[2-(4-Methoxyphenyl)thiazol-4-yl]-3-(6-piperidin-1-ylmethyl-pyridin-2-yl)urea;
1-(2-Benzo[1,3]dioxol-5-yl-thiazol-4-yl)-3-(6-piperidin-1-ylmethyl-pyridin-2-yl)-urea;
1-[2-(3,4-Dimethoxyphenyl)thiazol-4-yl)-3-(6-piperidin-1-ylmethyl-pyridin-2-yl)urea;
1-[2-(4-Fluorophenyl)thiazol-4-yl]-3-(6-piperidin-1-ylmethyl-pyridin-2-yl)urea;
1-[2-(3-Ethoxyphenyl)thiazol-4-yl]-3-(6-piperidin-1-ylmethyl-pyridin-2-yl)urea;
1-[2-(3-Aminophenyl)thiazol-4-yl]-3-(6-piperidin-1-ylmethyl-pyridin-2-yl)urea;
1-[2-(4-Trifluoromethylophenyl)thiazol-4-yl]-3-(6-piperidin-1-ylmethyl-pyridin-2-yl)urea;
1-[2-(3-Trifluoromethylophenyl)thiazol-4-yl]-3-(6-piperidin-1-ylmethyl-pyridin-2-yl)urea;
1-[2-(3-Fluorophenyl)thiazol-4-yl]-3-(6-piperidin-1-ylmethyl-pyridin-2-yl)urea;
1-[2-(4-Dimethylaminophenyl)thiazol-4-yl]-3-(6-piperidin-1-ylmethyl-pyridin-2-yl)urea;
1-[2-phenylthiazol-4-yl]-3-(6-piperidin-1-ylmethyl-pyridin-2-yl)urea;
1-[2-(4-Aminophenyl)thiazol-4-yl]-3-(6-piperidin-1-ylmethyl-pyridin-2-yl)urea;
1-[2-(3,5-Dichlorophenyl)thiazol-4-yl]-3-(6-piperidin-1-ylmethyl-pyridin-2-yl)urea;
1-[2-(2,4-Difluorophenyl)thiazol-4-yl]-3-(6-piperidin-1-ylmethyl-pyridin-2-yl)urea;
1-[2-(3,4-Dichlorophenyl)thiazol-4-yl]-3-(6-piperidin-1-ylmethyl-pyridin-2-yl)urea;
1-[2-(2,4-Dimethoxyphenyl)thiazol-4-yl]-3-(6-piperidin-1-ylmethyl-pyridin-2-yl)urea;
1-[2-(1H-Indol-5-yl)-thiazol-4-yl]-3-(6-piperidin-1-ylmethyl-pyridin-2-yl)-urea;
1-[2-(4-Methylthiophenyl)thiazol-4-yl]-3-(6-piperidin-1-ylmethyl-pyridin-2-yl)urea;
1-[2-(4-Cyanophenyl)thiazol-4-yl]-3-(6-piperidin-1-ylmethyl-pyridin-2-yl)urea;
1-[2-(3-Methoxyphenyl)thiazol-4-yl]-3-(6-piperidin-1-ylmethyl-pyridin-2-yl)urea;
1-[2-(2-Methoxyphenyl)thiazol-4-yl]-3-(6-piperidin-1-ylmethyl-pyridin-2-yl)urea;
1-[2-(3-Hydroxyphenyl)thiazol-4-yl]-3-(6-piperidin-1-ylmethyl-pyridin-2-yl)urea;
1-[2-(4-Methoxyphenoxymethyl)thiazol-4-yl]-3-(6-piperidin-1-ylmethyl-pyridin-2-yl)urea;
1-{6-[(2-Diethylamino-1-methylethylamino)methyl]-pyridin-2-yl}-3-(2-pyridin-4-yl-thiazol-4-yl)urea;
4-{4-[3-(6-Piperidin-1-ylmethyl-pyridin-2-yl)-ureido]-thiazol-2-yl}-benzenesulfonamide;
Ethyl 2-[3-[2-(pyridin-4-yl)-thiazol-4-yl]ureido]-thiazole-4-carboxylate;
1-(4-Cyclohexylthiazol-2-yl)-3-[2-(pyridin-4-yl)-thiazol-4-yl]urea;
1-(Pyridin-3-ylmethyl)-3-(2-pyridin-4-yl-thiazol-4-yl)urea;
1-(Pyridin-2-ylmethyl)-3-(2-pyridin-4-yl-thiazol-4-yl)urea;
1-[6-(Piperidin-1-ylmethyl)pyridin-2-yl]-3-(3-pyridin-3-yl-phenyl)urea;
1-(3-Hydroxy-pyridin-2-yl)-3-(2-pyridin-3-yl-thiazol-4-yl)-urea;
1-(3-Amino-pyridin-2-yl)-3-(2-pyridin-3-yl-thiazol-4-yl)-urea;
1-(3-Hydroxy-pyridin-2-yl)-3-(2-pyridin-4-yl-thiazol-4-yl)-urea;
1-(3-Amino-pyridin-2-yl)-3-(2-pyridin-4-yl-thiazol-4-yl)-urea;
1-(3-Piperidin-1-ylmethyl-pyridin-2-yl)-3-(2-pyridin-4-yl-thiazol-4-yl)-urea;
(1-Diethylaminomethyl-2-methyl-propyl)-{6-[3-(2-pyridin-4-yl-thiazol-4-yl)-ureido]-pyridin-2-ylmethyl}-carbamic acid tert-butyl ester;
N-[2-(4-pyridinyl)-4-thiazolyl]-N'-2-[6-(N",N"-diethylaminomethylamino)pyridinyl]urea;
N-[2-(4-pyridinyl)-4-thiazolyl]-N'-2-[6-[3-(1-morpholinyl)propyl]amino]pyridinyl]urea;
N-[2-(4-pyridinyl)-4-thiazolyl]-N'-[4-(3-pyridinyl)-2-thiazolyl] urea;
N-[3-(3-pyridinyl)phenyl]-N'-2-(6-propylpyridinyl)urea;
N-[3-(4-pyridinyl)phenyl]-N'-2-(6-propylpyridinyl)urea;
N-[2-(2-pyridinyl)-4-thiazolyl]-N'-2-(5-methylpyridinyl)urea;
N,N'-bis [2-(3-pyridinyl)-4-thiazolyl] urea;
N,N'-bis [2-(4-pyridinyl)-4-thiazolyl] urea;
N-[2-(3-pyridinyl)-4-thiazolyl]-N'-[4-(3-pyridinyl)-2-thiazolyl] urea;
N-[2-(3-pyridinyl)-4-thiazolyl]-N'-2-thiazolylurea;
N-[2-(4-pyridinyl)-4-thiazolyl]-N'-2-thiazolylurea;
N-[2-(3-pyridinyl)-4-thiazolyl]-N'-4-phenyl-2-thiazolylurea ;
N-[2-(3-pyridinyl)-4-thiazolyl]-N'-2-phenyl-4-thiazolylurea ;
N-[2-(4-pyridinyl)-4-thiazolyl]-N'-2-phenyl-4-thiazolylurea ;
N-[2-(3-pyridinyl)-4-thiazolyl]-N'-2-pyridinylurea ;
N-[2-(3-pyridinyl)-4-thiazolyl]-N'-3-pyridinylurea ;
N-[2-(4-pyridinyl)-4-thiazolyl]-N'-2-thiazolylurea ;
N-[2-(4-pyridinyl)-4-thiazolyl]-N'-2-pyridinylurea ;
N-[2-(4-pyridinyl)-4-thiazolyl]-N'-2-benzthiazolylurea;
N-[2-(4-pyridinyl)-4-thiazolyl]-N'-[2-(3-pyridinyl)-4-thiazolyl] urea ;
N-[2-(4-pyridinyl)-4-thiazolyl]-N'-[4-(3-pyridinyl)-2-thiazolyl] urea;
N-[2-(4-pyridinyl)-4-thiazolyl]-N'-2-quinolinylurea;
N-[2-(4-pyridinyl)-4-thiazolyl]-N'-3-quinolinylurea;
N-[2-(4-pyridinyl)-4-thiazolyl]-N'-4-benzimidazolylurea;
N-[2-(4-pyridinyl)-4-thiazolyl)-N'-2-(6-ethylpyridinyl)urea;
N-[2-(4-pyridinyl)-4-thiazolyl]-N'-2-(5-trifluoromethylpyridinyl)urea;
N-[2-methyl-4-thiazolyl]-N'-2-pyridinylurea
N-[2-methyl-4-thiazolyl]-N'-2-(6-ethylpyridinyl)urea;
N-[2-(3-pyridinyl)-4-thiazolyl]-N'-2-(6-ethylpyridinyl)urea;
N-[2-(4-pyridinyl)-4-thiazolyl]-N'-2-(6-propylpyridinyl)urea;
N-[2-(4-pyridinyl)-4-thiazolyl]-N'-2-(6-methylpyridinyl)urea;
N-[2-(3-pyridinyl)-4-thiazolyl]-N'-2-(6-methylpyridinyl)urea;
N-[2-(4-pyridinyl)-4-thiazolyl]-N'-2-(4-ethylpyridinyl)urea;
N-[2-(4-pyridinyl)-4-thiazolyl]-N'-2-(5-methylpyridinyl)urea;
N-[2-(4-pyridinyl)-4-thiazolyl]-N'-2-(3-methylpyridinyl)urea;
N-[2-(4-pyridinyl)-4-thiazolyl]-N'-[5-(1,1-dimethylethyl)-3-isoxazolyl]urea;
N-[2-(2-thienyl)-4-thiazolyl]-N'-2-pyridinylurea;
N-[2-(4-pyridinyl)-4-thiazolyl]-N'-2-(6-bromopyridinyl)urea;
N-[2-(4-pyridinyl)-4-thiazolyl]-N'-2-(6-chloropyridinyl)urea;
N-[2-(2-pyridinyl)-4-thiazolyl]-N'-2-(6-propylpyridinyl)urea;
N-[2-(2-pyridinyl)-4-thiazolyl)-N'-2-(6-ethylpyridinyl)urea;
N-[2-(2-pyridinyl)-4-thiazolyl]-N'-2-pyridinylurea;
N,N'-bis (2-(4-pyridinyl)-4-thiazolyl]-N'-methylurea;
N-[2-(4-pyridinyl)-4-thiazolyl]-N'-2-pyridinyl-N'-methylurea;
[4-[(1-piperidylcarbonyl)amino]-2-thiazolyl]-4-pyridine;
N-[2-(4-pyridinyl)-4-thiazolyl]-N'-2-[6-(1-piperdinyl)pyridinyl]urea;
N-[2-(2-ethyl-4-pyridinyl)-4-thiazolyl]-N'-2-(6-propylpyridinyl)urea;
[[(2-(4-pyridinyl)-4-thiazolylamino)carbonyl]amino]-6-pyridinyl-2-carboxylic acid;
N-[2-(4-pyridinyl)-4-thiazolyl]-N'-2-[6-(4-morpholinyl)pyridinyl]urea;
N-[2-(4-pyridinyl)-4-thiazolyl]-N'-2-[6-(1-methyl-4-piperazinyl)pyridinyl]urea;
N-[2-(4-pyridinyl)-4-thiazolyl]-N'-2-[6-(N".-methylaminothiocarbonyl)pyridinyl]urea;
N-[2-(4-pyridinyl)-4-thiazolyl]-N'-2-[6-(N",N"-dimethylaminomethyl)pyridinyl]urea;
N-[2-(4-pyridinyl)-4-thiazolyl]-N'-2-[6-(N"-methylaminomethyl)pyridinyl]urea;
N-[2-(4-pyridinyl)-4-thiazolyl]-N'-3-(1-bromoisoquinolinyl)urea;
N-[2-(4-pyridinyl)-4-thiazolyl]-N'-2-[[[3-(1-morpholinyl)propyl]aminocarbonyl]pyridinyl]urea;
N-[3-(2-pyridinyl)-4-thiazolyl]-N'-2-(4,6-dimethylpyridinyl)urea;
N-[2-(2-pyridinyl)-4-thiazolyl]-N'-2-(4-methylpyridinyl)urea;
N-[2-(2-pyridinyl)-4-thiazolyl]-N'-2-(5-methylpyridinyl)urea ;
N-[2-(2-pyridinyl)-4-thiazolyl]-N'-2-(4-ethylpyridinyl)urea;
N-[2-(2-pyridinyl)-4-thiazolyl]-N'-2-(3-methylpyridinyl)urea;
N-[2-(4-pyridinyl)-4-thiazolyl]-N'-3-[(1-morpholinyl)propyl]-N'-6-(2-aminopyridinyl)urea;
N-[2-(4-pyridinyl)-4-thiazolyl]-N'-2-[6-[3-(1-morpholinyl) propylamino]pyridinyl] urea;
N-[2-(2-pyridinyl)-4-thiazolyl]-N'-2-(6-methylbenzthiazolyl)urea;
N-[2-(2-thienyl)-4-thiazolyl]-N'-2-(4-ethylpyridinyl)urea;
N-[2-(2-thienyl)-4-thiazolyl]-N'-2-(3-methylpyridinyl)urea;
N-[2-(4-pyridinyl)-4-thiazolyl]-N'-2-[6-(N",N"-dimethylaminoethylamino)pyridinyl]urea;
N-[2-(4-pyridinyl)-4-thiazolyl]-N'-2-(6-(N",N"-diethylamino)pyridinyl]urea; and
[[(2-(4-pyridinyl)-4-thiazolylamino)carbonyl]amino]-6-pyridinyl-3-carboxamide.

### Indications

Compounds of the present invention would be useful for, but not limited to, the treatment of cell proliferative diseases or of apoptosis.

The compounds of the invention are endowed with kinase inhibitory activity, such as CDK/cyclin kinase inhibitory activity and KDR inhibitory activity.

The compounds of the invention are useful in therapy as antineoplasia agents.

Compounds of the invention would be useful for the treatment of neoplasia including cancer, including, but not limited to: carcinoma such as cancer of the bladder, breast, colon, kidney, liver, lung (including small cell lung cancer), esophagus, gall-bladder, ovary, pancreas, stomach, cervix, thyroid, prostate, and skin (including squamous cell carcinoma); hematopoietic tumors of lymphoid lineage (including leukemia, acute lymphocitic leukemia, acute lymphoblastic leukemia, B-cell lymphoma, T-cell-Lymphoma, Hodgkin's lymphoma, non-Hodgkin's lymphoma, hairy cell lymphoma and Burkett's lymphoma); hematopoietic tumors of myeloid lineage (including acute and chronic myelogenous leukemias, myelodysplastic syndrome and promyelocytic leukemia); tumors of mesenchymal origin (including fibrosarcoma and rhabdomyosarcoma, and other sarcomas, e.g. soft tissue and bone); tumors of the central and peripheral nervous system (including astrocytoma, neuroblastoma, glioma and schwannomas); and other tumors (including melanoma, seminoma, teratocarcinoma, osteosarcoma, xenoderoma pigmentosum, keratoctanthoma, thyroid follicular cancer and Kaposi's sarcoma).

Preferably, the compounds are useful for the treatment of neoplasia selected from lung cancer, colon cancer and breast cancer.

Due to the key role of CDKs in the regulation of cellular proliferation, these compounds are also useful in the treatment of a variety of cell proliferative disorders such as, for instance, blood vessel proliferative disorders including arthritis and restenosis; fibrotic disorders including hepatic cirrhosis and atherosclerosis; mesangial cell proliferative disorders including glomerulonephritis, diabetic nephropathy, malignant nephrosclerosis, thrombotic microangiopathy syndromes, transplant rejection and glomerulopathies; metabolic disorders including psoriasis, diabetes mellitus, chronic wound healing, inflammation, and diabetic retinopathy and other vision disorders; and others including benign prostate hyperplasia, familial adenomatosis polyposis, neuro-fibromatosis, pulmonary fibrosis, angiogenesis, metastasis, vascular smooth cell proliferation, post-surgical stenosis and hypertrophic scar formation, eczema, inflammatory bowel disease, endotoxic shock, and fungal infections.

The compounds of the invention are useful to prevent the phosphorylation of tau protein.

The compounds of the invention are useful in the treatment of neurological disorders, including neurological injuries and neurodegenerative diseases, such as, but not limited to, stroke, brain trauma, epilepsy, spinal cord injury, ischemia, multiple sclerosis, vision related disorders including but not limited to glaucoma and macular degeneration, hearing loss, AIDS-related dementia, retinitis pigmentosa, spinal muscular atrophy, cerebellar degeneration, amyotrophic lateral sclerosis, Parkinson's disease, Huntington's disease and Alzheimer's disease.

Compounds of formula I, as inhibitors of the CDKs, can modulate the level of cellular RNA and DNA synthesis. These agents would therefore be useful in the treatment of viral infections, including but not limited to HIV, human papilloma virus, herpesvirus, poxvirus, Epstein-Barr virus, Sindbis virus and adenovirus.

The compounds of this invention may also act as inhibitors of other protein kinases, e.g. KDR, IKK, JNK3, and thus be effective in the treatment of diseases associated with other protein kinases.

Besides being useful for human treatment, these compounds are also useful for veterinary treatment of companion animals, exotic animals and farm animals, including mammals, rodents, and the like. More preferred animals include horses, dogs, and cats.

Inhibitors of certain kinases may have utility in the treatment of diseases when the kinase is not misregulated, but is nonetheless essential for maintenance of the disease state. In this case, inhibition of the kinase activity would act either as a cure or palliative for these diseases. For example, many viruses, such as human papilloma virus, disrupt the cell cycle and drive cells into the S-phase of the cell cycle. Preventing cells from entering DNA synthesis after viral infection by inhibition of essential S-phase initiating activities such as CDK2, may disrupt the virus life cycle by preventing virus replication. This same principle may be used to protect normal cells of the body from toxicity of cycle-specific chemotherapeutic agents. Inhibition of CDK2 or CDK4 will prevent progression into the cycle in normal cells and limit the toxicity of cytotoxics which act in S-phase, G2 or mitosis. Furthermore, CDK2/cyclin E activity has also been shown to regulate NF-κB: Inhibition of CDK2 activity stimulates NF-κB-dependent gene expression, an event mediated through interactions with the p300 coactivator. NF-κB regulates genes involved in inflammatory responses, (such as hematopoietic growth factors chemokines and leukocyte adhesion molecules) and may be involved in the suppression of apoptotic signals within the cell.
Thus, inhibition of CDK2 may suppress apoptosis induced by cytotoxic drugs via a mechanism which involves NF-κB. Inhibition of CDK2 activity may also have utility in other cases where regulation of NF-κB plays a role in etiology of disease. A further example may be taken from fungal infections: Inhibition of the *Aspergillus* kinases Cdc2/CDC28 or Nim A may cause arrest or death in the fungi, improving the therapeutic outcome for patients with these infections.

The compounds of the invention are useful as modulators of apoptosis. As such they are useful in the prevention of AIDS development in HIV-infected individuals, autoimmune diseases (including but not limited to systemic lupus, erythematosus, autoimmune mediated glomerulonephritis, rheumatoid arthritis and autoimmune diabetes mellitus), myelodysplastic syndromes, aplastic anemia, ischemic injury associated with myocardial infarctions, stroke and reperfusion injury, vision related disorders including but not limited to glaucoma and macular degeneration, arrhythmia, atherosclerosis, toxin-induced or alcohol related liver diseases, hematological diseases (including but not limited to chronic anemia and aplastic anemia), degenerative diseases of the musculoskeletal system (including but not limited to osteoporosis) aspirin-sensitive rhinosinusitis, cystic fibrosis, kidney diseases and cancer pain.

### Definitions

The term "prevention" includes either preventing the onset of disorders altogether or delaying the onset of a preclinically evident stage of disorders in individuals. This includes prophylactic treatment of those at risk of developing a disease, such as a cancer, for example.

The phrase "therapeutically-effective" is intended to qualify the amount of each agent, which will achieve the goal of improvement in disorder severity and the frequency of incidence over treatment of each agent by itself, while avoiding adverse side effects typically associated with alternative therapies. For example, effective neuroplastic therapeutic agents prolong the survivability of the patient, inhibit the rapidly-proliferating cell growth associated with the neoplasm, or effect a regression of the neoplasm. Alternatively, effective therapeutic agents for the treatment of neurological disorders minimize the damage from injury, improve cognitive functions, and the like.

The term "H" denotes a single hydrogen atom. This radical may be attached, for example, to an oxygen atom to form a hydroxyl radical.

Where the term "alkyl" is used, either alone or within other terms such as "haloalkyl" and "alkylamino", it embraces linear or branched radicals having one to about twenty carbon atoms or, preferably, one to about twelve carbon atoms. More preferred alkyl radicals are "lower alkyl" radicals having one to about six carbon atoms. Examples of such radicals include methyl, ethyl, n-propyl, isopropyl, n-butyl, isobutyl, sec-butyl, tert-butyl, pentyl, iso-amyl, hexyl and the like. Even more preferred are lower alkyl radicals having one to four carbon atoms. The term "alkylenyl" embraces bridging divalent alkyl radicals such as methylenyl and ethyleneyl.

The term "alkenyl" embraces linear or branched radicals having at least one carbon-carbon double bond of two to about twenty carbon atoms or, preferably, two to about twelve carbon atoms. More preferred alkenyl radicals are "lower alkenyl" radicals having two to about four carbon atoms. Examples of alkenyl radicals include ethenyl, propenyl, allyl, propenyl, butenyl and 4-methylbutenyl. The terms "alkenyl" and "lower alkenyl", embrace radicals having "cis" and "trans" orientations, or alternatively, "E" and "Z" orientations.

The term "alkynyl" denotes linear or branched radicals having two to about twenty carbon atoms or, preferably, two to about twelve carbon atoms. More preferred alkynyl radicals are "lower alkynyl" radicals having two to about ten carbon atoms. Most preferred are lower alkynyl radicals having two to about four carbon atoms. Examples of such radicals include propargyl, butynyl, and the like.

The term "halo" means halogens such as fluorine, chlorine, bromine or iodine atoms.

The term "haloalkyl" embraces radicals wherein any one or more of the alkyl carbon atoms is substituted with halo as defined above. Specifically embraced are monohaloalkyl, dihaloalkyl and polyhaloalkyl radicals. A monohaloalkyl radical, for one example, may have either an iodo, bromo, chloro or fluoro atom within the radical. Dihalo and polyhaloalkyl radicals may have two or more of tie same halo atoms or a combination of different halo radicals. "Lower haloalkyl" embraces radicals having 1-6 carbon atoms. Even more preferred are lower haloalkyl radicals having one to three carbon atoms. Examples of haloalkyl radicals include fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl. "Perfluoroalkyl" means alkyl radicals having all hydrogen atoms replaced with fluoro atoms. Examples include trifluoromethyl and pentafluoroethyl.

The term "hydroxyalkyl" embraces linear or branched alkyl radicals having one to about ten carbon atoms any one of which may be substituted with one or more hydroxyl radicals. More preferred hydroxyalkyl radicals are "lower hydroxyalkyl" radicals having one to six carbon atoms and one or more hydroxyl radicals. Examples.of such radicals include hydroxymethyl, hydroxyethyl, hydroxypropyl, hydroxybutyl and hydroxyhexyl. Even more preferred are lower hydroxyalkyl radicals having one to three carbon atoms.

The term "alkoxy" embrace linear or branched oxy-containing radicals each having alkyl portions of one to about ten carbon atoms. More preferred alkoxy radicals are "lower alkoxy" radicals having one to six carbon atoms. Examples of such radicals include methoxy, ethoxy, propoxy, butoxy and tert-butoxy. Even more preferred are lower alkoxy radicals having one to three carbon atoms. The "alkoxy" radicals may be further substituted with one or more halo atoms, such as fluoro, chloro or bromo, to provide "haloalkoxy" radicals. Even more preferred are lower haloalkoxy radicals having one to three carbon atoms. Examples of such radicals include fluoromethoxy, chloromethoxy, trifluoromethoxy, trifluoroethoxy, fluoroethoxy and fluoropropoxy.

The term "aryl", alone or in combination, means a carbocyclic aromatic system containing one or two rings wherein such rings may be attached together in a pendent manner or may be fused. The term "aryl" embraces aromatic radicals such as phenyl, naphthyl, tetrahydronaphthyl, indane and biphenyl. More preferred aryl is phenyl. Said "aryl" group may have 1 to 3 substituents such as lower alkyl, hydroxyl, halo, haloalkyl, nitro, cyano, alkoxy and lower alkylamino.

The term "heterocyclyl" embraces saturated, partially saturated and unsaturated heteroatom-containing ring-shaped radicals, where the heteroatoms may be selected from nitrogen, sulfur and oxygen. It does not include rings containing -O-O-,-O-S- or -S-S-portions. Said "heterocyclyl" group may have 1 to 3 substituents such as hydroxyl, halo, haloalkyl, cyano, lower alkyl, lower aralkyl, oxo, lower alkoxy, amino and lower alkylamino.

Examples of saturated heterocyclic radicals include saturated 3 to 6-membered heteromonocyclic group containing 1 to 4 nitrogen atoms [e.g. pyrrolidinyl, imidazolidinyl, piperidino, piperazinyl]; saturated 3 to 6-membered heteromonocyclic group containing 1 to 2 oxygen atoms and 1 to 3 nitrogen atoms [e.g. morpholinyl]; saturated 3 to 6-membered heteromonocyclic group containing 1 to 2 sulfur atoms and 1 to 3 nitrogen atoms [e.g., thiazolidinyl]. Examples of partially saturated heterocyclyl radicals include dihydrothiophene, dihydropyran, dihydrofuran and dihydrothiazole.

Examples of unsaturated heterocyclic radicals, also termed "heteroaryl" radicals, include unsaturated 5 to 6 membered heteromonocyclyl group containing 1 to 4 nitrogen atoms, for example, pyrrolyl, pyrrolinyl, imidazolyl, pyrazolyl, 2-pyridyl, 3-pyridyl, 4-pyridyl, pyrimidyl, pyrazinyl, pyridazinyl, triazolyl [e.g., 4H-1,2,4-triazolyl, 1H-1,2,3-triazolyl, 2H-1,2,3-triazolyl]; unsaturated 3 to 6-membered heteromonocyclic group containing an oxygen atom, for example, pyranyl, 2-furyl, 3-furyl, etc.; unsaturated 5 to 6-membered heteromonocyclic group containing a sulfur atom, for example, 2-thienyl, 3-thienyl, etc.; unsaturated 5- to 6-membered heteromonocyclic group containing 1 to 2 oxygen atoms and 1 to 3 nitrogen atoms, for example, oxazolyl, isoxazolyl, oxadiazolyl [e.g., 1,2,4-oxadiazolyl, 1,3,4-oxadiazolyl, 1,2,5-oxadiazolyl]; unsaturated 5 to 6-membered heteromonocyclic group containing 1 to 2 sulfur atoms and 1 to 3 nitrogen atoms, for example, thiazolyl, thiadiazolyl [e.g., 1,2,4-thiadiazolyl, 1,3,4-thiadiazolyl, 1,2,5-thiadiazolyl].

The term also embraces radicals where heterocyclic radicals are fused/condensed with aryl radicals: unsaturated condensed heterocyclic group containing 1 to 5 nitrogen atoms, for example, indolyl, isoindolyl, indolizinyl, benzimidazolyl, quinolyl, isoquinolyl, indazolyl, benzotriazolyl, tetrazolopyridazinyl [e.g., tetrazolo [1,5-b]pyridazinyl]; unsaturated condensed heterocyclic group containing 1 to 2 oxygen atoms and 1 to 3 nitrogen atoms [e.g. benzoxazolyl, benzoxadiazolyl]; unsaturated condensed heterocyclic group containing 1 to 2 sulfur atoms and 1 to 3 nitrogen atoms [e.g., benzothiazolyl, benzothiadiazolyl].

The term also includes bridged, spiro and oxo-containing heterocyclic rings, such as 1,4-dioxa-8-aza-spiro[4.5]decyl, phthalimidyl, 1,4-dioxa-8-aza-spiro[4.5]decyl, and (1-aza-bicyclo[2.2.2]oct-3-yl).

Preferred heterocyclic radicals include five to ten membered fused or unfused radicals. More preferred examples of heteroaryl radicals include quinolyl, isoquinolyl, imidazolyl, pyridyl, thienyl, thiazolyl, oxazolyl, furyl, and pyrazinyl. Even more preferred heteroaryl radicals are 5- or 6-membered heteroaryl, containing one or two heteroatoms selected from sulfur nitrogen and oxygen, selected from thienyl, furanyl, pyrrolyl, thiazolyl, oxazolyl, imidazolyl, pyrazolyl, isoxazolyl, isothiazolyl, pyridyl, piperidinyl and pyrazinyl.

The term "sulfonyl", whether used alone or linked to other terms such as alkylsulfonyl, denotes respectively divalent radicals -SO₂-.

The terms "sulfamyl," "aminosulfonyl" and "sulfonamidyl," whether alone or used with terms such as "N-alkylaminosulfonyl", "N-arylaminosulfonyl", "N,N-dialkylaminosulfonyl" and "N-alkyl-N-arylaminosulfonyl", denotes a sulfonyl radical substituted with an amine radical, forming a sulfonamide (-SO₂NH₂).

The term "alkylaminosulfonyl" includes "N-alkylaminosulfonyl" and "N,N-dialkylaminosulfonyl" where sulfamyl radicals are substituted, respectively, with one alkyl radical, or two alkyl radicals. More preferred alkylaminosulfonyl radicals are "lower alkylaminosulfonyl" radicals having one to six carbon atoms. Even more preferred are lower alkylaminosulfonyl radicals having one to three carbon atoms. Examples of such lower alkylaminosulfonyl radicals include N-methylaminosulfonyl, N-ethylaminosulfonyl and N-methyl-N-ethylaminosulfonyl.

The terms "N-arylaminosulfonyl" and "N-alkyl-N-arylaminosulfonyl" denote sulfamyl radicals substituted, respectively, with one aryl radical, or one alkyl and one aryl radical. More preferred N-alkyl-N-arylaminosulfonyl radicals are "lower N-alkyl-N-arylsulfonyl" radicals having alkyl radicals of one to six carbon atoms. Even more preferred are lower N-alkyl-N-arylsulfonyl radicals having one to three carbon atoms. Examples of such lower N-alkyl-N-aryl-aminosulfonyl radicals include N-methyl-N-phenylaminosulfonyl and N-ethyl-N-phenylaminosulfonyl. Examples of such N-aryl-aminosulfonyl radicals include N-phenylaminosulfonyl.

The term "arylalkylaminosulfonyl" embraces aralkyl radicals as described above, attached to an aminosulfonyl radical. More preferred are lower arylalkylaminosulfonyl radicals having one to three carbon atoms.

The term "heterocyclylaminosulfonyl" embraces heterocyclyl radicals as described above, attached to an aminosulfonyl radical.

The terms "carboxy" or "carboxyl", whether used alone or with other terms, such as "carboxyalkyl", denotes -CO₂H.

The term "carbonyl", whether used alone or with other terms, such as "aminocarbonyl", denotes -(C=O)-.

The term "aminocarbonyl" when used by itself or with other terms such as "aminocarbonylalkyl", "N-alkylaminocarbonyl", "N-arylaminocarbonyl", "N,N-dialkylaminocarbonyl", "N-alkyl-N-arylaminocarbonyl", "N-alkyl-N-hydroxyaminocarbonyl" and "N-alkyl-N-hydroxyaminocarbonylalkyl", denotes an amide group of the formula -C (=O)NH₂.

The terms "N-alkylaminocarbonyl" and "N,N-dialkylaminocarbonyl" denote aminocarbonyl radicals which have been substituted with one alkyl radical and with two alkyl radicals, respectively. More preferred are "lower alkylaminocarbonyl" having lower alkyl radicals as described above attached to an aminocarbonyl radical.

The terms "N-arylaminocarbonyl" and "N-alkyl-N-arylaminocarbonyl" denote aminocarbonyl radicals substituted, respectively, with one aryl radical, or one alkyl and one aryl radical.

The term "aminoalkyl" embraces alkyl radicals substituted with amino radicals.

The term "alkylaminoalkyl" embraces aminoalkyl radicals having the nitrogen atom substituted with an alkyl radical. The term includes both mono- and disubstituted amines. Even more preferred are lower alkylaminoalkyl radicals having one to three carbon atoms.

The term "heterocyclylalkyl" embraces heterocyclic-substituted alkyl radicals. More preferred heterocyclylalkyl radicals are "5- or 6-membered heteroarylalkyl" radicals having alkyl portions of one to six carbon atoms and a 5- or 6-membered heteroaryl radical. Even more preferred are lower heteroarylalkyl radicals having alkyl portions of one to three carbon atoms. Examples include such radicals as pyridylmethyl and thienylmethyl.

The term "aralkyl" embraces aryl-substituted alkyl radicals. Preferable aralkyl radicals are "lower aralkyl" radicals having aryl radicals attached to alkyl radicals having one to six carbon atoms. Even more preferred are lower aralkyl radicals phenyl attached to alkyl portions having one to three carbon atoms. Examples of such radicals include benzyl, diphenylmethyl and phenylethyl. The aryl in said aralkyl may be additionally substituted with halo, alkyl, alkoxy, halkoalkyl and haloalkoxy.

The term "arylalkenyl" embraces aryl-substituted alkenyl radicals. Preferable arylalkenyl radicals are "lower arylalkenyl" radicals having aryl radicals attached to alkenyl radicals having two to six carbon atoms. Examples of such radicals include phenylethenyl. The aryl in said arylalkenyl may be additionally substituted with halo, alkyl, alkoxy, halkoalkyl and haloalkoxy.

The term "arylalkynyl" embraces aryl-substituted alkynyl radicals. Preferable arylalkynyl radicals are "lower arylalkynyl" radicals having aryl radicals attached to alkynyl radicals having two to six carbon atoms. Examples of such radicals include phenylethynyl. The aryl in said aralkyl may be additionally substituted with halo, alkyl, alkoxy, halkoalkyl and haloalkoxy. The terms benzyl and phenylmethyl are interchangeable.

The term "alkylthio" embraces radicals containing a linear or branched alkyl radical, of one to ten carbon atoms, attached to a divalent sulfur atom. Even more preferred are lower alkylthio radicals having one to three carbon atoms. An example of "alkylthio" is methylthio, (CH₃S-).

The term "haloalkylthio" embraces radicals containing a haloalkyl radical, of one to ten carbon atoms, attached to a divalent sulfur atom. Even more preferred are lower haloalkylthio radicals having one to three carbon atoms. An example of "haloalkylthio" is trifluoromethylthio.

The term "alkylsulfinyl" embraces radicals containing a linear or branched alkyl radical, of one to ten carbon atoms, attached to a divalent -S(=O)-atom. More preferred are lower alkylsulfinyl radicals having one to three carbon atoms.

The term "arylsulfinyl" embraces radicals containing an aryl radical, attached to a divalent - S(=O)- atom. Even more preferred are optionally substituted phenylsulfinyl radicals.

The term "haloalkylsulfinyl" embraces radicals containing a haloalkyl radical, of one to ten carbon atoms, attached to a divalent -S(=O)- atom. Even more preferred are lower haloalkylsulfinyl radicals having one to three carbon atoms.

The term "alkylamino" denotes amino groups which have been substituted with one alkyl radical and with two alkyl radicals, including terms "N-alkylamino" and "N,N-dialkylamino". More preferred alkylamino radicals are "lower alkylamino" radicals having one or two alkyl radicals of one to six carbon atoms, attached to a nitrogen atom. Even more preferred are lower alkylamino radicals having one to three carbon atoms. Suitable "alkylamino" may be mono or dialkylamino such as N-methylamino, N-ethylamino, N,N-dimethylamino, N,N-diethylamino or the like.

The tern "arylamino" denotes amino groups which have been substituted with one or two aryl radicals, such as N-phenylamino. The "arylamino" radicals may be further substituted on the aryl ring portion of the radical.

The term "heteroarylamino" denotes amino groups which have been substituted with one or two heteroaryl radicals, such as N-thienylamino. The "heteroarylamino" radicals may be further substituted on the heteroaryl ring portion of the radical.

The term "aralkylamino" denotes amino groups which have been substituted with one or two aralkyl radicals. More preferred are phenyl-C₁-C₃-alkylamino radicals, such as N-benzylamino. The "aralkylamino" radicals may be further substituted on the aryl ring portion of the radical.

The terms "N-alkyl-N-arylamino" and "N-aralkyl-N-alkylamino" denote amino groups which have been substituted with one aralkyl and one alkyl radical, or one aryl and one alkyl radical, respectively, to an amino group.

The term "arylthio" embraces aryl radicals of six to ten carbon atoms, attached to a divalent sulfur atom. An example of "arylthio" is phenylthio.

The term "aralkylthio" embraces aralkyl radicals as described above, attached to a divalent sulfur atom. More preferred are phenyl-C₁-C₃-alkylthio radicals. An example of "aralkylthio" is benzylthio.

The term "aryloxy" embraces optionally substituted aryl radicals, as defined above, attached to an oxygen atom. Examples of such radicals include phenoxy.

The term "aralkoxy" embraces oxy-containing aralkyl radicals attached through an oxygen atom to other radicals. More preferred aralkoxy radicals are "lower aralkoxy" radicals having optionally substituted phenyl radicals attached to lower alkoxy radical as described above.

The term "cycloalkenyl" includes carbocyclic groups have one or more carbon-carbon double bonds. "Cycloalkenyl" and "cycloalkyldienyl" compounds are included. Preferred cycloalkenyl groups include C₃-C₆ rings. More preferred compounds include, for example, cyclopentenyl, cyclopentadienyl, cyclohexenyl and cycloheptadienyl.

The term "comprising" is meant to be open ended, including the indicated component but not excluding other elements.

The present invention preferably includes compounds that selectively inhibit CDK2 and/or CDK5.

The present invention also comprises the use of a compound of the invention, or pharmaceutically acceptable salt thereof, in the manufacture of a medicament for the treatment either acutely or chronically of a cell proliferation or apoptosis mediated disease state, including those described previously. The compounds of the present invention are also useful in the manufacture of an anti-cancer medicament. The compounds of the present invention are also useful in the manufacture of a medicament to attenuate or prevent disorders through inhibition of CDKs and other kinases. The compounds of the present invention are also useful in the manufacture of a medicament to treat neurological disorders.

The present invention comprises a pharmaceutical composition comprising a therapeutically-effective amount of a compound of Formulas I-V in association with a least one pharmaceutically-acceptable carrier, adjuvant or diluent.

The present invention also comprises a method of treating cell proliferative disorders, apoptosis mediated disorders, cancer, CDK mediated disorder or neurological disorders, in a subject, the method comprising treating the subject having or susceptible to such disorder with a therapeutically-effective amount of a compound of Formula I 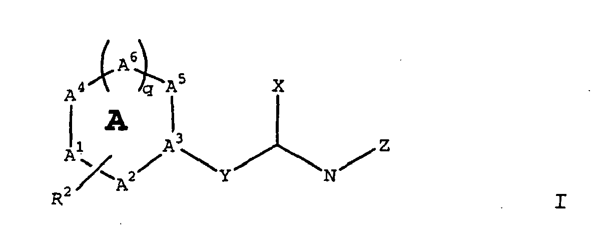
wherein each of A¹-A⁶ is selected from CH₂, CH, C, O, S, NH and N; wherein A¹-A⁶ together form a ring A selected from
additionally substituted or unsubstituted 5- or 6-membered heterocyclyl,
additionally substituted or unsubstituted 5- or 6-membered heteroaryl fused with a phenyl group,
additionally substituted or unsubstituted 5- or 6-membered cycloalkenyl, and
additionally substituted or unsubstituted phenyl, wherein the ring A is additionally substituted with one or more substituents independently selected from halo, -OR³, -SR³, -CO₂R³, -CO₂NR³R³, -COR³, -NR³R³, -SO₂NR³R³, -NR³C(O)OR³, -NR³C(O)R³, cycloalkyl, optionally substituted phenylalkylenyl, optionally substituted 5-6 membered heterocyclyl, optionally substituted heteroarylalkylenyl, optionally substituted phenyl, lower alkyl, cyano, lower hydroxyalkyl, nitro, lower alkenyl, lower alkynyl and lower haloalkyl;
wherein X and Z taken together form a nitrogen containing ring selected from
unsubstituted 5-6 membered heterocyclyl,
unsubstituted 5-6 membered heterocyclyl fused with a phenyl group,
5-6 membered heterocyclyl substituted with one or more substituents independently selected from R¹, and
5-6 membered nitrogen-containing heterocyclyl, fused with a phenyl group, substituted with one or more substituents independently selected from R¹;
wherein R¹ is independently selected from H, halo, - OR³, -SR³, -CO₂R³, -CO₂NR³R³, -COR³, -CONR³R³, -NR³R³, -C(S)NR³R³, -SO₂NR³R³, -NR³C(O)OR³, -NR³C(O)R³, cycloalkyl, optionally substituted phenylalkylenyl, optionally substituted 4-10 membered heterocyclyl, optionally substituted 4-10 membered heterocyclylalkyl, optionally substituted phenyl, optionally substituted phenoxy, lower alkyl, lower cyano, lower alkenyl, lower alkynyl and lower haloalkyl;
wherein Y is selected from, in either orientation, 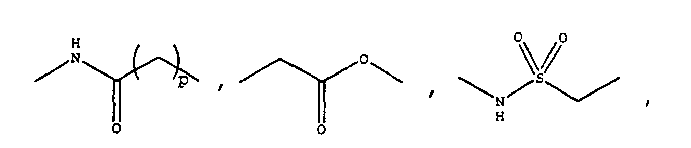 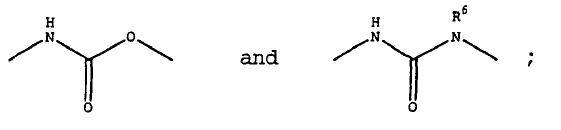
wherein R² is selected from lower alkylaminoalkynyl, substituted or unsubstituted phenyl, substituted or unsubstituted 5-6 membered heterocyclyl, and substituted or unsubstituted 5-6 membered heterocyclyl bridged with a phenyl group;
wherein substituted R² is substituted with one or more substituents independently selected from halo, -OR³, -SR³, CO₂R³, -CO₂NR³R³, -COR³, - NR³R³, -C(O)NR³R³, -SO₂NR³R³, -NR³C(O)OR³, - NHC(O)R³, -SO₂NHC(O)R³, -C(S)NR³R³, nitro, cycloalkyl, optionally substituted phenylalkylenyl, optionally substituted 4-7 membered heterocyclyl, optionally substituted heterocyclylalkylenyl, optionally substituted phenyl, optionally substituted phenoxyalkylenyl, optionally substituted heterocyclyloxyalkyl, lower alkyl, cyano, lower hydroxyalkyl, lower alkoxyalkyl, lower azidoalkyl, lower aminoalkyl, lower (hydroxyalkyl)aminoalkyl, lower alkylaminoalkyl, lower alkylaminoalkoxy, lower aminoalkoxyalkyl, lower (alkylaminoalkyl)amino lower ((alkylamino)alkylamino)alkyl, lower alkylaminoalkylaminocarbonyl, lower cyanoalkyl, lower alkenyl, lower alkynyl and lower haloalkyl;
wherein R³ is selected from H, lower alkyl, optionally substituted phenyl, optionally substituted phenylalkyl, optionally substituted heterocyclyl, optionally substituted heterocyclylalkyl, C₃-C₆ cycloalkyl, and lower haloalkyl;
wherein R⁶ is selected from H, alkyl, 5-6 membered heterocyclylalkylenyl and alkylamino;
wherein p is 1 or 2; and
wherein q is 0 or 1;
and pharmaceutically acceptable salts thereof;
provided A is not thiazol-2-yl when Y is ureido.

### COMBINATIONS

While the compounds of the invention can be administered as the sole active pharmaceutical agent, they can also be used in combination with one or more compounds of the invention or other agents. When administered as a combination, the therapeutic agents can be formulated as separate compositions that are administered at the same time or sequentially at different times, or the therapeutic agents can be given as a single composition.

The phrase "co-therapy" (or "combination-therapy"), in defining use of a compound of the present invention and another pharmaceutical agent, is intended to embrace administration of each agent in a sequential manner in a regimen that will provide beneficial effects of the drug combination, and is intended as well to embrace co-administration of these agents in a substantially simultaneous manner, such as in a single capsule having a fixed ratio of these active agents or in multiple, separate capsules for each agent.

Specifically, the administration of compounds of the present invention may be in conjunction with additional therapies known to those skilled in the art in the prevention or treatment of neoplasia, such as with radiation therapy or with cytostatic or cytotoxic agents.

If formulated as a fixed dose, such combination products employ the compounds of this invention within the accepted dosage ranges. Compounds of Formula I may also be administered sequentially with known anticancer or cytotoxic agents when a combination formulation is inappropriate. The invention is not limited in the sequence of administration; compounds of formula I may be administered either prior to or after administration of the known anticancer or cytotoxic agent.

Currently, standard treatment of primary tumors consists of surgical excision followed by either radiation or IV administered chemotherapy. The typical chemotherapy regime consists of either DNA alkylating agents, DNA intercalating agents or microtubule poisons. The chemotherapy doses used are just below the maximal tolerated dose and therefore dose limiting toxicities typically include, nausea, vomiting, diarrhea, hair loss, neutropenia etc... Experiments performed in in *vivo* animal models and in *in vitro* cell based assays have demonstrated that combining chemotherapeutic agents with cell cycle inhibitors, such as CDK inhibitors, typically results in either decreased rate of tumor growth or, in some cases, tumor regression. Combining chemotherapy with a CDK inhibitor typically results in an increased therapeutic index and lower levels of both agents are required. This ultimately results in a decrease in toxicity and an increase in efficacy.

Schwartz et al, Clin. Can. Res., 3,1467-1472 (1997) have demonstrated that combining the CDK inhibitor flavopiridol with mitomycin-C (DNA alkylating agent) resulted in an increased rate of apoptosis in gastric and breast cancer cells. Bible et al (Bible et al., Cancer Res., 57, 3375-3380 (1997) have also demonstrated therapeutic synergy exists between flavopiridol and paclitaxel, cytarabine, topotecan, doxorubicin, and etoposide (all standard chemotherapeutic agents) when tested in cell based assays using human non-small cell lung cancer cells. Preclinical models (cell culture) suggest that a cell cycle inhibitor potentiates the effect of a cytotoxic agent when administered after the chemotherapeutic agent. The chemotherapeutic agent will induce specific DNA/mitotic damage checkpoints in normal cells which in combination with a CDK inhibitor will cause a cell cycle arrest or cytostatic effect. In contrast, tumor cells will be driven into apoptosis or cell death when a chemotherapeutic agent and a CDK inhibitor are combined due to tumor cells attempting to activate defective DNA damage and cell cycle checkpoints. In addition, scheduling of a CDK inhibitor for clinical trials should include a rest period to allow the patients normal cells to recover and reduce the potential for cytotoxic side effects.

There are large numbers of antineoplastic agents available in commercial use, in clinical evaluation and in pre-clinical development, which would be selected for treatment of neoplasia by combination drug chemotherapy. Such antineoplastic agents fall into several major categories, namely, antibiotic-type agents, alkylating agents, antimetabolite agents, hormonal agents, immunological agents, interferon-type agents and a category of miscellaneous agents.

A first family of antineoplastic agents which may be used in combination with compounds of the present invention consists of antimetabolite-type/thymidilate synthase inhibitor antineoplastic agents. Suitable antimetabolite antineoplastic agents may be selected from but not limited to the group consisting of 5-FU-fibrinogen, acanthifolic acid, aminothiadiazole, brequinar sodium, carmofur, Ciba-Geigy CGP-30694, cyclopentyl cytosine, cytarabine phosphate stearate, cytarabine conjugates, Lilly DATHF, Merrel Dow DDFC, dezaguanine, dideoxycytidine, dideoxyguanosine, didox, Yoshitomi DMDC, doxifluridine, Wellcome EHNA, Merck & Co. EX-015, fazarabine, floxuridine, fludarabine phosphate, 5-fluorouracil, N-(2'-furanidyl)-5-fluorouracil, Daiichi Seiyaku FO-152, isopropyl pyrrolizine, Lilly LY-188011, Lilly LY-264618, methobenzaprim, methotrexate, Wellcome MZPES, norspermidine, NCI NSC-127716, NCI NSC-264880, NCI NSC-39661, NCI NSC-612567, Warner-Lambert PALA, pentostatin, piritrexim, plicamycin, Asahi Chemical PL-AC, Takeda TAC-788, thioguanine, tiazofurin, Erbamont TIF, trimetrexate, tyrosine kinase inhibitors, tyrosine protein kinase inhibitors, Taiho UFT and uricytin.

A second family of antineoplastic agents which may be used in combination with compounds of the present invention consists of alkylating-type antineoplastic agents. Suitable alkylating-type antineoplastic agents may be selected from but not limited to the group consisting of Shionogi 254-S, aldo-phosphamide analogues, altretamine, anaxirone, Boehringer Mannheim BBR-2207, bestrabucil, budotitane, Wakunaga CA-102, carboplatin, carmustine, Chinoin-139, Chinoin-153, chlorambucil, cisplatin, cyclophosphamide, American Cyanamid CL-286558, Sanofi CY-233, cyplatate, Degussa D-19-384, Sumimoto DACHP(Myr)2, diphenylspiromustine, diplatinum cytostatic, Erba distamycin derivatives, Chugai DWA-2114R, ITI E09, elmustine, Erbamont FCE-24517, estramustine phosphate sodium, fotemustine, Unimed G-6-M, Chinoin GYKI-17230, hepsul-fam, ifosfamide, iproplatin, lomustine, mafosfamide, mitolactol, Nippon Kayaku NK-121, NCI NSC-264395, NCI NSC-342215, oxaliplatin, Upjohn PCNU, prednimustine, Proter PTT-119, ranimustine, semustine, SmithKline SK&F-101772, Yakult Honsha SN-22, spiromus-tine, Tanabe Seiyaku TA-077, tauromustine, temozolomide, teroxirone, tetraplatin and trimelamol.

A third family of antineoplastic agents which may be used in combination with compounds of the present invention consists of antibiotic-type antineoplastic agents. Suitable antibiotic-type antineoplastic agents may be selected from but not limited to the group consisting of Taiho 4181-A, aclarubicin, actinomycin D, actinoplanone, Erbamont ADR-456, aeroplysinin derivative, Ajinomoto AN-201-II, Ajinomoto AN-3, Nippon Soda anisomycins, anthracycline, azino-mycin-A, bisucaberin, Bristol-Myers BL-6859, Bristol-Myers BMY-25067, Bristol-Myers BMY-25551, Bristol-Myers BMY-26605, Bristol-Myers BMY-27557, Bristol-Myers BMY-28438, bleomycin sulfate, bryostatin-1, Taiho C-1027, calichemycin, chromoximycin, dactinomycin, daunorubicin, Kyowa Hakko DC-102, Kyowa Hakko DC-79, Kyowa Hakko DC-88A, Kyowa Hakko DC89-A1, Kyowa Hakko DC92-B, ditrisarubicin B, Shionogi DOB-41, doxorubicin, doxorubicin-fibrinogen, elsamicin-A, epirubicin, erbstatin, esorubicin, esperamicin-A1, esperamicin-Alb; Erbamont FCE-21954, Fujisawa FK-973, fostriecin, Fujisawa FR-900482, glidobactin, gregatin-A, grincamycin, herbimycin, idarubicin, illudins, kazusamycin, kesarirhodins, Kyowa Hakko KM-5539, Kirin Brewery KRN-8602, Kyowa Hakko KT-5432, Kyowa Hakko KT-5594, Kyowa Hakko KT-6149, American Cyanamid LL-D49194, Meiji Seika ME 2303, menogaril, mitomycin, mitoxantrone, SmithKline M-TAG, neoenactin, Nippon Kayaku NK-313, Nippon Kayaku NKT-01, SRI International NSC-357704, oxalysine, oxaunomycin, peplomycin, pilatin, pirarubicin, porothramycin, pyrindanycin A, Tobishi RA-I, rapamycin, rhizoxin, rodorubicin, sibanomicin, siwenmycin, Sumitomo SM-5887, Snow Brand SN-706, Snow Brand SN-07, sorangicin-A, sparsomycin, SS Pharmaceutical SS-21020, SS Pharmaceutical SS-7313B, SS Pharmaceutical SS-9816B, steffimycin B, Taiho 4181-2, talisomycin, Takeda TAN-868A, terpentecin, thrazine, tricrozarin A, Upjohn U-73975, Kyowa Hakko UCN-10028A, Fujisawa WF-3405, Yoshitomi Y-25024 and zorubicin.

A fourth family of antineoplastic agents which may be used in combination with compounds of the present invention consists of a miscellaneous family of antineoplastic agents, including tubulin interacting agents, topoisomerase II inhibitors, topoisomerase I inhibitors and hormonal agents, selected from but not limited to the group consisting of α-carotene, α-difluoromethyl-arginine, acitretin, Biotec AD-5, Kyorin AHC-52, alstonine, amonafide, amphethinile, amsacrine, Angiostat, ankinomycin, anti-neoplaston A10, antineoplaston A2, antineoplaston A3, antineoplaston A5, antineoplaston AS2-1, Henkel APD, aphidicolin glycinate, asparaginase, Avarol, baccharin, batracylin, benfluron, benzotript, Ipsen-Beaufour BIM-23015, bisantrene, Bristol-Myers BMY-40481, Vestar boron-10, bromofosfamide, Wellcome BW-502, Wellcome BW-773, caracemide, carmethizole hydrochloride, Ajinomoto CDAF, chlorsulfaquinoxalone, Chemes CHX-2053, Chemex CHX-100, Warner-Lambert CI-921, Warner-Lambert CI-937, Warner-Lambert CI-941, Warner-Lambert CI-958, clanfenur, claviridenone, ICN compound 1259, ICN compound 4711, Contracan, Yakult Honsha CPT-11, crisnatol, curaderm, cytochalasin B. cytarabine, cytocytin, Merz D-609, DABIS maleate, dacarbazine, datelliptinium, didemnin-B, dihaematoporphyrin ether, dihydrolenperone, dinaline, distamycin, Toyo Pharmar DM-341, Toyo Pharmar DM-75, Daiichi Seiyaku DN-9693, docetaxel elliprabin, elliptinium acetate, Tsumura EPMTC, the epothilones, ergotamine, etoposide, etretinate, fenretinide, Fujisawa FR-57704, gallium nitrate, genkwadaphnin, Chugai GLA-43, Glaxo GR-63178, grifolan NMF-5N, hexadecylphosphocholine, Green Cross HO-221, homoharringtonine, hydroxyurea, BTG ICRF-187, ilmofosine, isoglutamine, isotretinoin, Otsuka JI-36, Ramot K-477, Otsuak K-76COONa, Kureha Chemical K-AM, MECT Corp KI-8110, American Cyanamid L-623, leukoregulin, lonidamine, Lundbeck LU-23-112, Lilly LY-186641, NCI (US) MAP, marycin, Merrel Dow MDL-27048, Medco MEDR-340, merbarone, merocyanlne derivatives, methylanilinoacridine, Molecular Genetics MGI-136, minactivin, mitonafide, mitoquidone mopidamol, motretinide, Zenyaku Kogyo MST-16, N-(retinoyl)amino acids, Nisshin Flour Milling N-021, N-acylated-dehydroalanines, nafazatrom, Taisho NCU-190, nocodazole derivative, Normosang, NCI NSC-145813, NCI NSC-361456, NCI NSC-604782, NCI NSC-95580, ocreotide, Ono ONO-112, oquizanocine, Akzo Org-10172, paclitaxel, pancratistatin, pazelliptine, Warner-Lambert PD-111707, Warner-Lambert PD-115934, Warner-Lambert PD-131141, Pierre Fabre PE-1001, ICRT peptide D, piroxantrone, polyhaematoporphyrin, polypreic acid, Efamol porphyrin, probimane, procarbazine, proglumide, Invitron protease nexin I, Tobishi RA-700, razoxane, Sapporo Breweries RBS, restrictin-P, retelliptine, retinoic acid, Rhone-Poulenc RP-49532, Rhone-Poulenc RP-56976, SmithKline SK&F-104864, Sumitomo SM-108, Kuraray SMANCS, SeaPharm SP-10094, spatol, spirocyclopropane derivatives, spirogermanium, Unimed, SS Pharmaceutical SS-554, strypoldinone, Stypoldione, Suntory SUN 0237, Suntory SUN 2071, superoxide dismutase, Toyama T-506, Toyama T-680, taxol, Teijin TEI-0303, teniposide, thaliblastine, Eastman Kodak TJB-29, tocotrienol, topotecan, Topostin, Teijin TT-82, Kyowa Hakko UCN-01, Kyowa Hakko UCN-1028, ukrain, Eastman Kodak USB-006, vinblastine sulfate, vincristine, vindesine, vinestramide, vinorelbine, vintriptol, vinzolidine, withanolides and Yamanouchi YM-534.

Alternatively, the present compounds may also be used in co-therapies with other anti-neoplastic agents, such as acemannan, aclarubicin, aldesleukin, alemtuzumab, alitretinoin, altretamine, amifostine, aminolevulinic acid, amrubicin, amsacrine, anagrelide, anastrozole, ANCER, ancestim, ARGLABIN, arsenic trioxide, BAM 002 (Novelos), bexarotene, bicalutamide, broxuridine, capecitabine, celecoxib, celmoleukin, cetrorelix, cladribine, clotrimazole, cytarabine ocfosfate, DA 3030 (Dong-A), daclizumab, denileukin diftitox, deslorelin, dexrazoxane, dilazep, docetaxel, docosanol, doxercalciferol, doxifluridine, doxorubicin, bromocriptine, carmustine, cytarabine, fluorouracil, HIT diclofenac, interferon alfa, daunorubicin, doxorubicin, tretinoin, edelfosine, edrecolomab, eflornithine, emitefur, epirubicin, epoetin beta, etoposide phosphate, exemestane, exisulind, fadrozole, filgrastim, finasteride, fludarabine phosphate, formestane, fotemustine, gallium nitrate, gemcitabine, gemtuzumab zogamicin, gimeracil/oteracil/tegafur combination, glycopine, goserelin, heptaplatin, human chorionic gonadotropin, human fetal alpha fetoprotein, ibandronic acid, idarubicin, (imiquimod, interferon alfa, interferon alfa, natural, interferon alfa-2, interferon alfa-2a, interferon alfa-2b, interferon alfa-N1, interferon alfa-n3, interferon alfacon-1, interferon alpha, natural, interferon beta, interferon beta-1a, interferon beta-lb, interferon gamma, natural interferon gamma-1a, interferon gamma-lb, interleukin-1 beta, iobenguane, irinotecan, irsogladine, lanreotide, LC 9018 (Yakult), leflunomide, lenograstim, lentinan sulfate, letrozole, leukocyte alpha interferon, leuprorelin, levamisole + fluorouracil, liarozole, lobaplatin, lonidamine, lovastatin, masoprocol, melarsoprol, metoclopramide, mifepristone, miltefosine, mirimostim, mismatched double stranded RNA, mitoguazone, mitolactol, mitoxantrone, molgramostim, nafarelin, naloxone + pentazocine, nartograstim, nedaplatin, nilutamide, noscapine, novel erythropoiesis stimulating protein, NSC 631570 octreotide, oprelvekin, osaterone, oxaliplatin, paclitaxel, pamidronic acid, pegaspargase, peginterferon alfa-2b, pentosan polysulfate sodium, pentostatin, picibanil, pirarubicin, rabbit antithymocyte polyclonal antibody, polyethylene glycol interferon alfa-2a, porfimer sodium, raloxifene, raltitrexed, rasburicase, rhenium Re 186 etidronate, RII retinamide, rituximab, romurtide, samarium (153 Sm) lexidronam, sargramostim, sizofiran, sobuzoxane, sonermin, strontium-89 chloride, suramin, tasonermin, tazarotene, tegafur, temoporfin, temozolomide, teniposide, tetrachlorodecaoxide, thalidomide, thymalfasin, thyrotropin alfa, topotecan, toremifene, tositumomab-iodine 131, trastuzumab, treosulfan, tretinoin, trilostane, trimetrexate, triptorelin, tumor necrosis factor alpha, natural, ubenimex, bladder cancer vaccine, Maruyama vaccine, melanoma lysate vaccine, valrubicin, verteporfin, vinorelbine, VIRULIZIN, zinostatin stimalamer, or zoledronic acid;

abarelix; AE 941 (Aeterna), ambamustine, antisense oligonucleotide, bcl-2 (Genta), APC 8015 (Dendreon), cetuximab, decitabine, dexaminoglutethimide, diaziquone, EL 532 (Elan), EM 800 (Endorecherche), eniluracil, etanidazole, fenretinide, filgrastim SD01 (Amgen), fulvestrant, galocitabine, gastrin 17 immunogen, HLA-B7 gene therapy (Vical), granulocyte macrophage colony stimulating factor, histamine dihydrochloride, ibritumomab tiuxetan, ilomastat, IM 862 (Cytran), interleukin-2, iproxifene, LDI 200 (Milkhaus), leridistim, lintuzumab, CA 125 MAb (Biomira), cancer MAb (Japan Pharmaceutical Development), HER-2 and Fc MAb (Medarex), idiotypic 105AD7 MAb (CRC Technology), idiotypic CEA MAb (Trilex), LYM-1-iodine 131 MAb (Techniclone), polymorphic epithelial mucin-yttrium 90 MAb (Antisoma), marimastat, menogaril, mitumomab, motexafin gadolinium, MX 6 (Galderma), nelarabine, nolatrexed, P 30 protein, pegvisomant, pemetrexed, porfiromycin, prinomastat, RL 0903 (Shire), rubitecan, satraplatin, sodium phenylacetate, sparfosic acid, SRL 172 (SR Pharma), SU 5416 (SUGEN), TA 077 (Tanabe), tetrathiomolybdate, thaliblastine, thrombopoietin, tin ethyl etiopurpurin, tirapazamine, cancer vaccine (Biomira), melanoma vaccine (New York University), melanoma vaccine (Sloan Kettering Institute), melanoma oncolysate vaccine (New York Medical College), viral melanoma cell lysates vaccine (Royal Newcastle Hospital), or valspodar.

Alternatively, the present compounds may also be used in co-therapies with other anti-neoplastic agents, such as other kinase inhibitors including KDR inhibitors, p38 inhibitors, TNF inhibitors, metallomatrix proteases inhibitors (MMP), COX-2 inhibitors, NSAID's, SOD mimics or αᵥβ₃ inhibitors.

Alternatively, the present compounds may also be used in co-therapies with other treatments for neurological treatments such as thrombolytic and anticoagulant agents including tPA, urokinase and inhibitors of platelet aggregation, p38 inhibitors, IL1ra, NMDA inhibitors, antiparkinsonian agents including carbidopa and levodopa, and inhibitors of lipid peroxidation, for example.

The present invention comprises a process for the preparation of a compound of Formula I-V.

Compounds of the present invention can possess, in general, one or more asymmetric carbon atoms and are thus capable of existing in the form of optical isomers as well as in the form of racemic or non-racemic mixtures thereof. The optical isomers can be obtained by resolution of the racemic mixtures according to conventional processes, e.g., by formation of diastereoisomeric salts, by treatment with an optically active acid or base. Examples of appropriate acids are tartaric, diacetyltartaric, dibenzoyltartaric, ditoluoyltartaric, and camphorsulfonic acid and then separation of the mixture of diastereoisomers by crystallization followed by liberation of the optically active bases from these salts. A different process for separation of optical isomers involves the use of a chiral chromatography column optimally chosen to maximize the separation of the enantiomers. Still another available method involves synthesis of covalent diastereoisomeric molecules by reacting compounds of the invention with an optically pure acid in an activated form or ah optically pure isocyanate. The synthesized diastereoisomers can be separated by conventional means such as chromatography, distillation, crystallization or sublimation, and then hydrolyzed to deliver the enantiomerically pure compound. The optically active compounds of the invention can likewise be obtained by using active starting materials. These isomers may be in the form of a free acid, a free base, an ester or a salt.

Compounds of the present invention can possess, in general, tautomeric forms, which are included in the family of compounds in Formula I.

Also included in the family of compounds of Formula I-V are the pharmaceutically-acceptable salts thereof. The term "pharmaceutically-acceptable salts" embraces salts commonly used to form alkali metal salts and to form addition salts of free acids or free bases. The nature of the salt is not critical, provided that it is pharmaceutically-acceptable. Suitable pharmaceutically-acceptable acid addition salts of compounds of Formula I-V may be prepared from an inorganic acid or from an organic acid. Examples of such inorganic acids are hydrochloric, hydrobromic, hydroiodic, nitric, carbonic, sulfuric and phosphoric acid. Appropriate organic acids may be selected from aliphatic, cycloaliphatic, aromatic, arylaliphatic, heterocyclic, carboxylic and sulfonic classes of organic acids, example of which are formic, acetic, adipic, butyric, propionic, succinic, glycolic, gluconic, lactic, malic, tartaric, citric, ascorbic, glucuronic, maleic, fumaric, pyruvic, aspartic, glutamic, benzoic, anthranilic, -mesylic, 4-hydroxybenzoic, phenylacetic, mandelic, embonic (pamoic), methanesulfonic, ethanesulfonic, benzenesulfonic, pantothenic, 2-hydroxyethanesulfonic, toluenesulfonic, sulfanilic, cyclohexylaminosulfonic, camphoric, camphorsulfonic, digluconic, cyclopentanepropionic, dodecylsulfonic, glucoheptanoic, glycerophosphonic, heptanoic, hexanoic, 2-hydroxy-ethanesulfonic, nicotinic, 2-naphthalenesulfonic, oxalic, palmoic, pectinic, persulfuric, 2-phenylpropionic, picric, pivalic propionic, succinic, tartaric, thiocyanic, mesylic, undecanoic, stearic, algenic, β-hydroxybutyric, salicylic, galactaric and galacturonic acid. Suitable pharmaceutically-acceptable base addition salts of compounds of Formula I-V include metallic salts, such as salts made from aluminum, calcium, lithium, magnesium, potassium, sodium and zinc, or salts made from organic bases including primary, secondary and tertiary amines, substituted amines including cyclic amines, such as caffeine, arginine, diethylamine, N-ethyl piperidine, aistidine, glucamine, isopropylamine, lysine, morpholine, N-ethyl morpholine, piperazine, piperidine, triethylamine, trimethylamine. All of these salts may be prepared by conventional means from the corresponding compound of the invention by reacting, for example, the appropriate acid or base with the compound of Formula I-V.

Also, the basic nitrogen-containing groups can be quaternised with such agents as lower alkyl halides, such as methyl, ethyl, propyl, and butyl chloride, bromides and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids that may be employed to from pharmaceutically acceptable acid addition salts include such inorganic acids as hydrochloric acid, sulphuric acid and phosphoric acid and such organic acids as oxalic acid, maleic acid, succinic acid and citric acid. Other examples include salts with alkali metals or alkaline earth metals, such as sodium, potassium, calcium or magnesium or with organic bases.

Additional examples of such salts can be found in Berge et al., J. Pharm. Sci./, 66, 1 (1977).

### GENERAL SYNTHETIC PROCEDURES

The compounds of the invention can be synthesized according to the following procedures of Schemes 1-17, wherein the substituents are as defined for Formulas I-V, above, except where further noted.

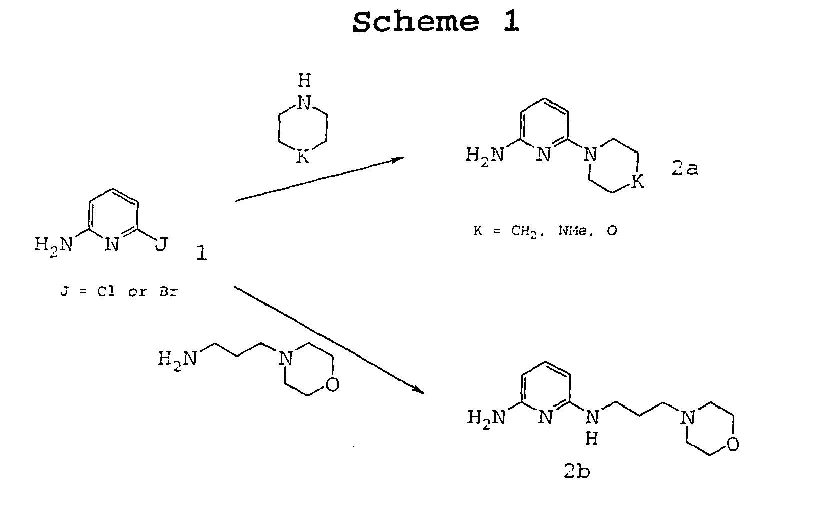

Substituted pyridines can be prepared according to the method set out in Scheme 1. A mixture of halo-aniline 1, substituted amine and phenol was reacted, preferably at a temperature above RT and more preferably at temperature of about 150°C, to yield the heterocyclyl derivative 2a or substituted amine derivative 2b.

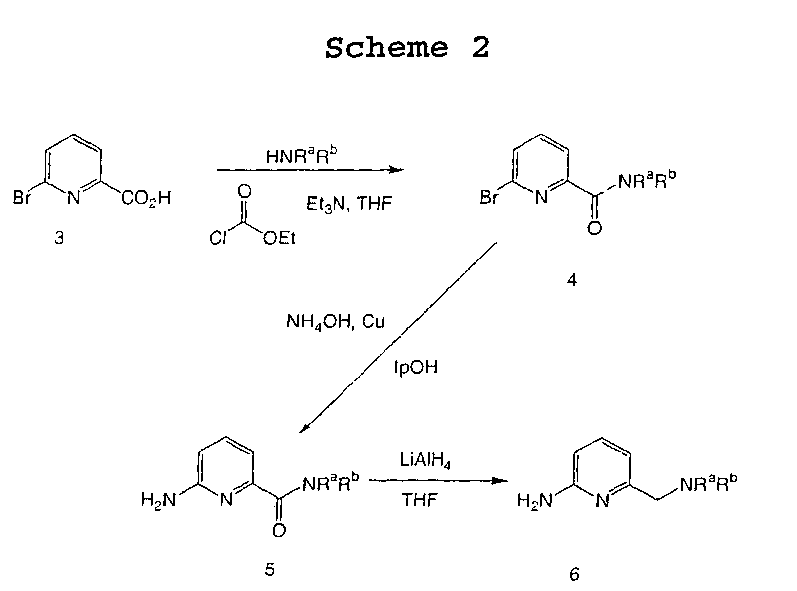

Substituted pyridines can be prepared according to the method set out in Scheme 2. A halopicolinic acid **3** is reacted with substituted amines (where Rₐ and R_{b} are H, alkyl, substituted alkyl, etc.) in the presence of chloroformate esters and base in a suitable solvent to form the halopyridyl amide derivatives **4**. Preferably the reaction is at a temperature below RT, more preferably the reaction occurs at a temperature of about 0°C. The halopyridyl amide **4** is dehalogenated, such as with NH₄OH and Cu powder in an appropriate solvent, such as IpOH to form the aniline derivative **5.** Preferably the reaction occurs at a temperature above RT, more preferably the reaction occurs at about 100°C. The aniline derivative **5** is reduced, such as with LiAlH₄ in Et₂O to form the aminoalkyl derivative **6**.

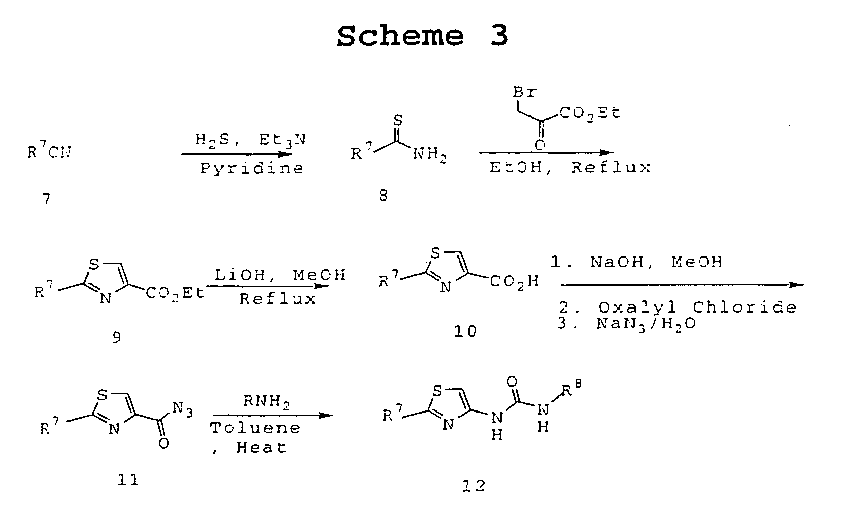

Substituted 4-thiazolylurea compounds **12** are prepared from the corresponding nitriles **7** according to the method set out in Scheme 3. Substituted nitriles **7** are added to base at about RT and H₂S is bubbled through the solution, to yield the thione **8**^{.} The thione 8 is combined with ethyl bromopyruvate and heated to form the thiazolyl carboxylate ester **9**. Aqueous LiOH is heated with the ester **9** at a temperature above RT and preferably at reflux to give the thiazole carboxylic acid **10.** Treatment of the substituted thiazolyl carboxylic acid **10** with base in a suitable solvent at about RT yields a salt. At about 0°C, oxalyl chloride is added to a suspension of the salt in solvent followed by a catalytic amount of DMF. Afterwards, aqueous NaN₃ is added to yield the thiazolyl carbonyl azide **11**. The carbonyl azide **11** is added to substituted amines to form the thiazolyl urea compound **12**.

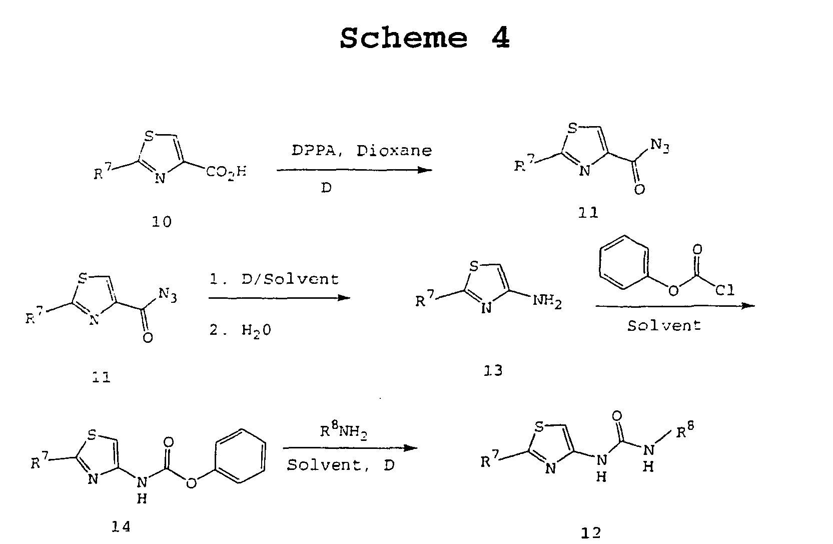

Substituted 4-thiazolylurea compounds **12** are alternatively prepared from the corresponding thiazole acids **10** according to the method set out in Scheme 4. Substituted acids **10** are reacted with diphenylphosphoryl azide in solvent to yield the azido compound **11**. The azido compound is hydrolyzed to yield the aniline **13**. Acylating agents, such as acid chlorides or anhydrides, are added to the aniline to form the carbamate **14**. The carbamates **14** are reacted with substituted amines to form the thiazolyl urea compounds **12**.

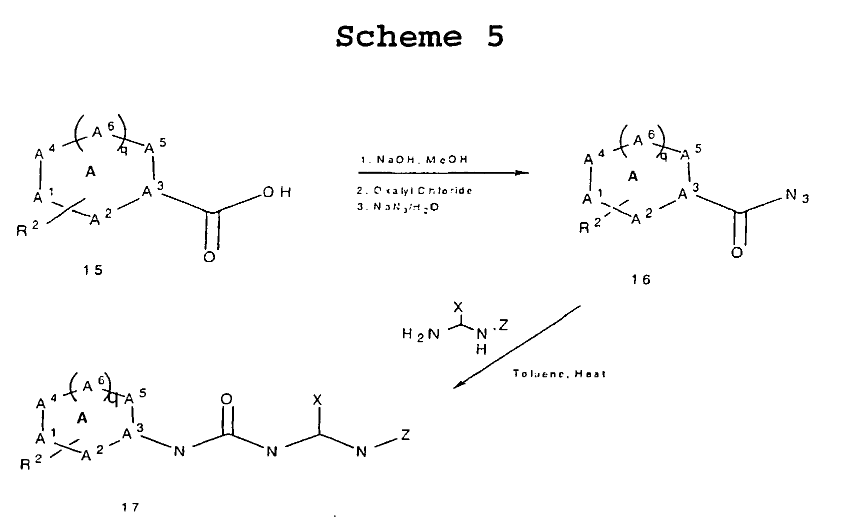

Substituted urea compounds **17** are prepared from the corresponding acids **15** according to the method set out in Scheme 5. Treatment of substituted carboxylic acids **15** with base in a suitable solvent at RT yields the sodium salt. A suspension of the salt in solvent is cooled in an ice bath and oxalyl chloride is added followed by a catalytic amount of DMF. Afterwards, aqueous sodium azide is added yielding the carbonyl azides **16**. The carbonyl azides **16** are added to substituted amines to form the ureas **17**.

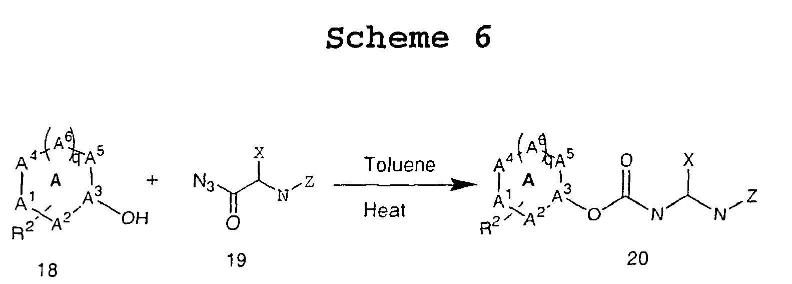

Substituted carbamates **20** are prepared from the corresponding alcohols **18** and azides **19** according to the method set out in Scheme 6. Treatment of substituted alcohols **18** with azides **19** in a suitable solvent yields the carbamates **20**.

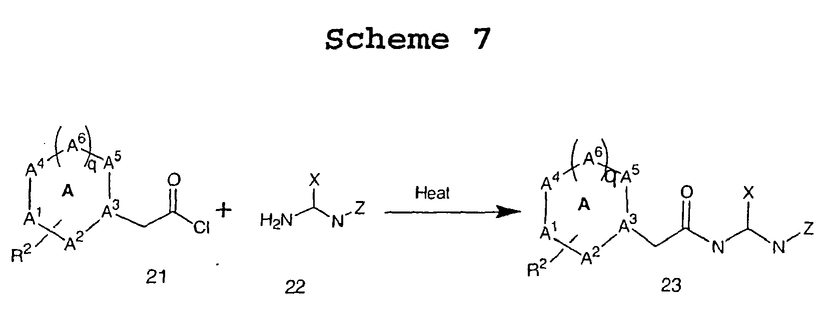

Substituted amides **23** are prepared from the corresponding acylating agents **21** (such as acid chlorides or anhydrides) and amines **22** according to the method set out in Scheme 7. Treatment of substituted amines **22** with acylating agents **21** in a suitable solvent yields the amides **23**.

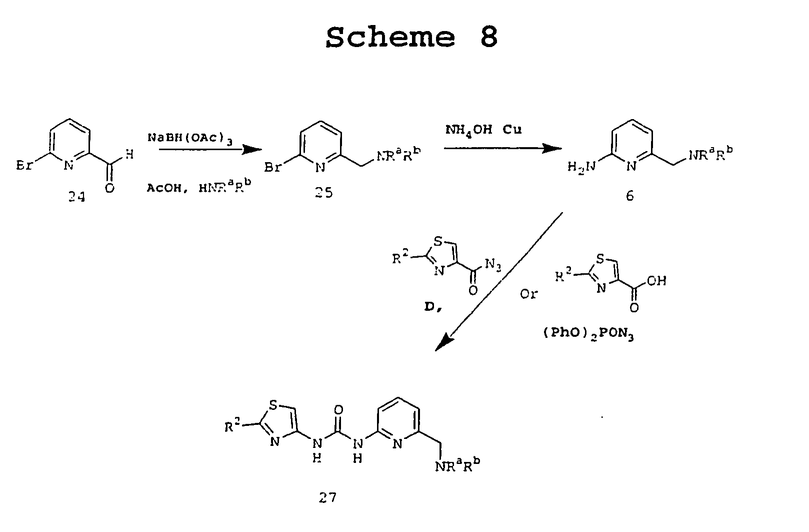

Substituted 4-thiazolylurea compounds **27** are prepared from the corresponding pyridines **24** according to the method set out in Scheme 8. Reductive amination with an amine (including nitrogen-containing heterocycles) and 6-bromo-2-pyridinecarboxaldehyde **24**, is achieved such as in a halocarbon solvent such as dichloromethane, in the presence of NaBH(OAc)₃ and acid, such as AcOH, to give 2-aminomethyl-6-bromopyridine **25**. The 2-aminomethyl-6-bromo-pyridine 25 is aminated, such as with NH₄OH in the presence of Cu powder, such as in the presence of an alcohol solvent, at a temperature above about 50°C and preferably at about 100°C, such as in a sealed tube to give the corresponding aniline **6**. A substituted thiazolylcarbonylazide, such as in dry hydrocarbon solvent such as toluene was heated at a temperature above about 50°C and preferably above about 85°C and reacted with the aniline **6** to give the 4-thiazolylurea compounds **27**.

Alternatively, the aniline **6** can be coupled with thiazolyl carboxylic acid, such as with (PhO)₂PON₃ in the presence of base, such as TEA, and molecular sieves in a solvent like THF. The reaction can be heated at a temperature above about 50°C and preferably at about reflux yielding the 4-thiazolylurea compounds **27**.

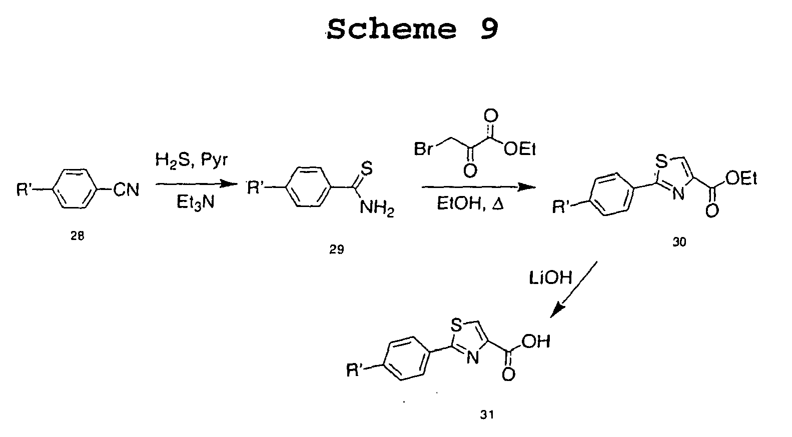

Thiazolyl carboxylic acid **31** (especially appropriate where R' is a sulfonamide or amine) are prepared from the corresponding benzonitriles **28** as described in Scheme 9. H₂S was added to the substituted 4-cyanobenzene **28** in the presence of base, such as Et₃N to afford the thiobenzamide **29**. The thiobenzamide **29** was reacted with ethyl bromopyruvate, such as in an alcohol solvent like EtOH, at a temperature greater than about 50°C, and preferably at about 75°C to give the thiazolyl ester **30**. The thiazolyl ester **30** is hydrolyzed, such as with LiOH monohydrate in an alcohol like aqueous MeOH, at a temperature greater than about 50°C, and preferably at about 75°C, to provide the acid **31**. The acid can be used similar to that described in Scheme 8.

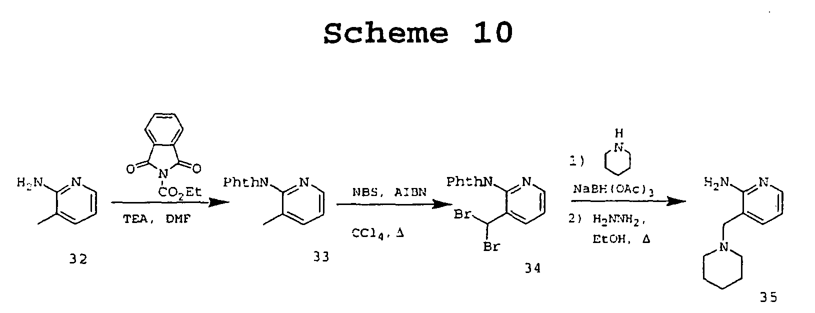

Substituted anilines **35** are prepared from the corresponding methyl compounds **32** as described in Scheme 10. 2-Amino-3-picoline was protected such as with solid carboethoxyphthalimide and base like TEA to provide the phthalimide (Phth) protected aniline **32**. The protected 3-methylaniline is brominated, such as with NBS and 2,2'-asobisisobutyrlnitrile (AIBN) at a temperature above 50ºC and preferably at about reflux. Additional AIBN and NBS may be needed to push the reaction to completeness. The dibromomethyl aniline **34** is reacted with an amine, preferably a secondary amine such as substituted or unsubstituted nitrogen containing heterocyclics like piperidines and piperazines, in the presence of acid like glacial AcOH and halocarbon solvent such as CH₂Cl₂. Treatment with NaBH(OAc)₃ provided the protected substituted methyl compound which was deported, such as by treatment with hydrazine monohydrate at a temperature greater than about 50°C, and preferably at reflux to provide the substituted aniline **35**.

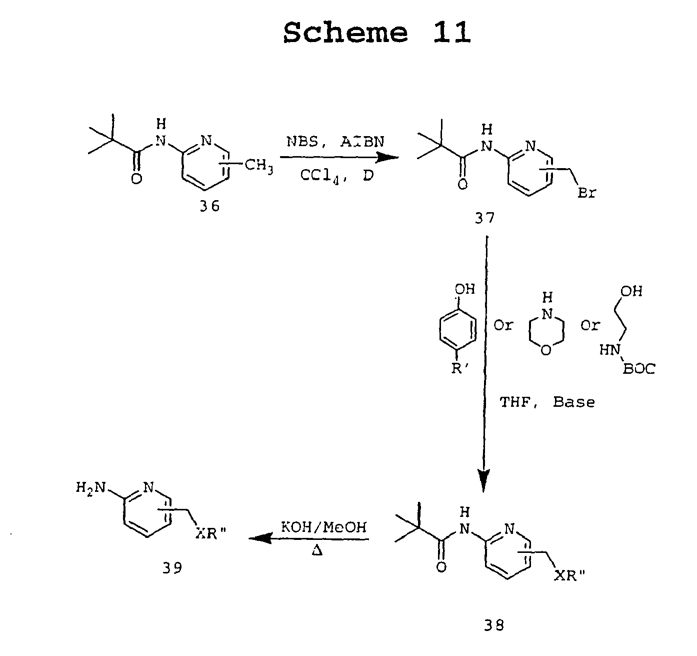

Substituted anilines **39** are prepared from the corresponding methyl compounds **36** as described in Scheme 11. *N*-Pivaloyl-2-amino-6-bromomethylpyridine **37** was prepared by the method of M.V. Papadopoulou, et al. (*J. Heterocyclic Chem.,* 1995, *32*, 675-681). The protected bromomethyl compound was treated with an alcohol or amine in the presence of base, such as NaH to yield the corresponding ether or amino alkyl compounds **38** (where X is 0 or N). The protected ether or amino alkyl compounds **38** was treated with base, such as in methanolic KOH and warmed to a temperature greater than about RT, and preferably at about 55°C, to provide the substituted anilines **39**. 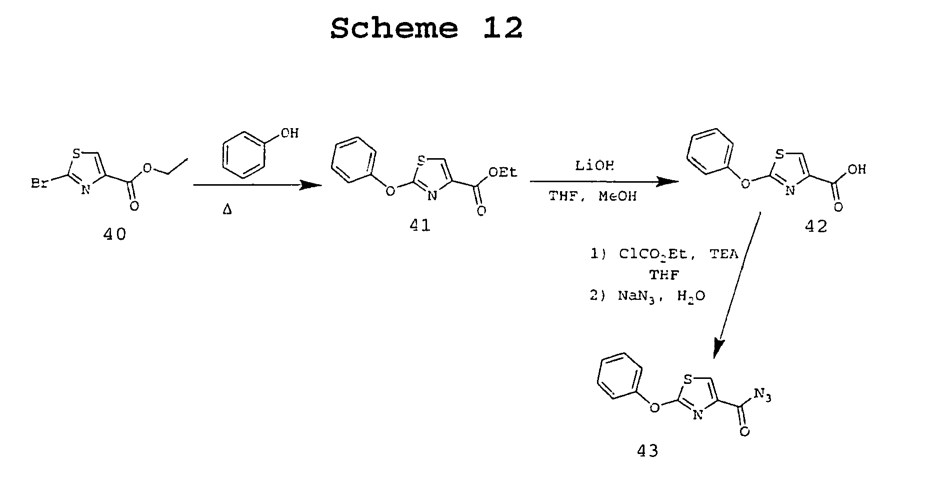 Thiazolylcarbonylazides **43** are prepared as described in Scheme 12. Bromothiazole was coupled with an aryl alcohol, such as phenol, at a temperature greater than about 100°C, and preferably at about 180°C, to provide the phenoxy compound **41**. The thiazolyl ester **41** was hydrolyzed, such as with LiOH monohydrate in an alcohol like aqueous MeOH, at a temperature greater than about 50°C, and preferably at about 75°C, to provide the acid **42**. Acid **42** is added to ethyl chloroformate and NaN₃ , in the presence of base such as TEA, to provide the azide **43**, which can be used as described in Scheme 8.

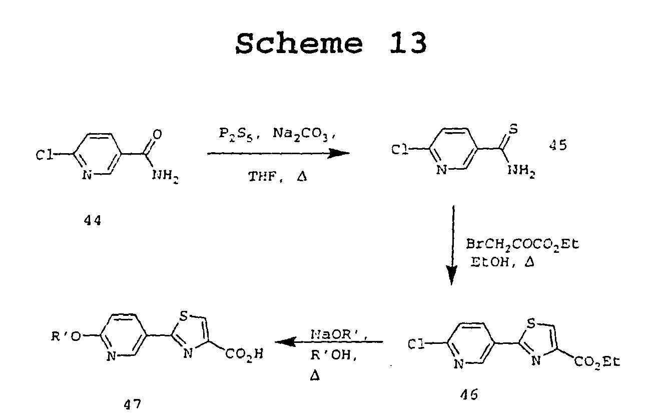

Pyridyl-2-thiazoles **47** are prepared as described in Scheme 13. 4-Chloronicotinamide **44** was converted to the thioamide **45** such as be treatment with P₂S₅, in the presence of base, such as Na₂CO₃, at a temperature greater than about 50°C, and preferably at about reflux. The thioamide **45** is converted to the thiazole ester 46 by treatment with bromoethylpyruvate and heating at a temperature greater than about 50°C, and preferably at about reflux. The ethyl ester is transesterified to the methyl ester with treatment with base, such as NaOMe. Further addition of base and heating at a temperature greater than about 50°C, and preferably at about reflux, hydrolyzed the ester to the acid. Additional NaOMe, in the presence of MeOH, and heating at a temperature greater than about 50°C, and preferably at about reflux, provided the methoxy substituted pyridine compound **47**. Use of other bases and alcohols provide alternative alkoxy substituted compounds.

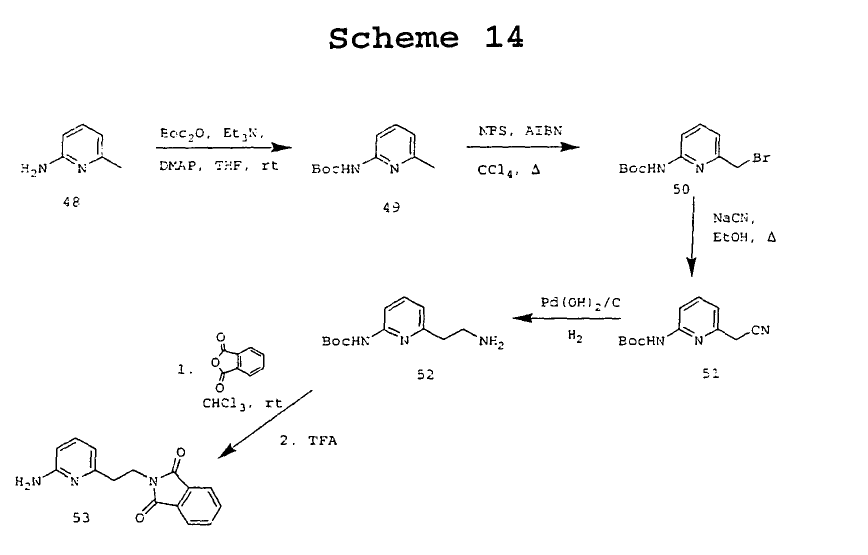

Protected aminoalkyl pyridines **53** are prepared from the 2-amino-6-methylpyridine **48** as described in Scheme 14. The amino group cf 2-amino-6-methylpyridine **48** is protected, such as with BOC and normal coupling chemistry, such as with di-tert-butyl dicarbonate and base, like TEA, and DMAP. The protected compound **49** is brominated such as with NBS and AIBN and heating at a temperature greater than about 50°C, and preferably at reflux to provide the bromomethyl derivative **50**. The bromomethyl derivative **50** is converted to the cyanomethyl compound **51** such as with treatment with NaCN in the presence of alcohol solvent such as EtOH, and heating at a temperature greater than about 50°C, and preferably at reflux. The cyanomethyl compound 51 is hydrogenated to the aminoethyl derivative **52** such as with hydrogen in the presence of Pd(OH)₂/C at a temperature about RT. The aminoethyl derivative **52** is converted to the di-protected compound such as with phthalic anhydride and heating at a temperature between RT and about 70°C. Upon treatment with strong acid, such as TFA, provides the 2-aminopyridyl compound **53**.

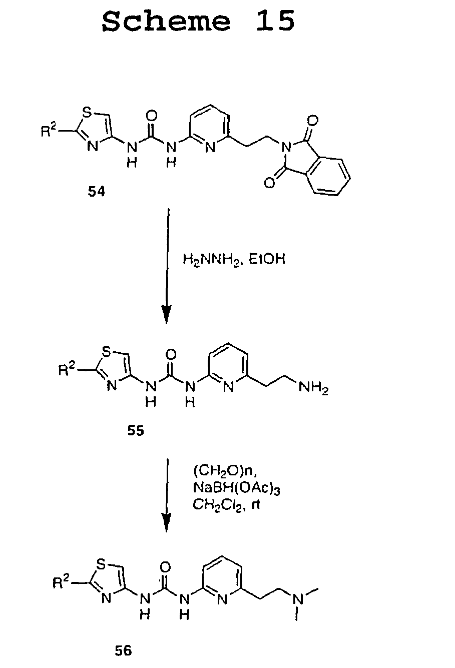

Compounds of Formula I are prepared as described in Scheme 15. Phthalimidylethyl compounds **54** are prepared from the coupling of compounds prepared similar to those described in Scheme 14 and thiazolyl acylazides as described in Scheme 8. Treatment of **54** with hydrazine hydrate and heating at a temperature greater than about 50°C, and preferably at reflux, provides the aminoethyl derivatives **55**. Alkylation of the amine **55**, such as with paraformaldehyde and NaBH(OAc)₃ in a haloalkyl solvent, such as CH₂Cl₂ provides the dimethylamine **56**.

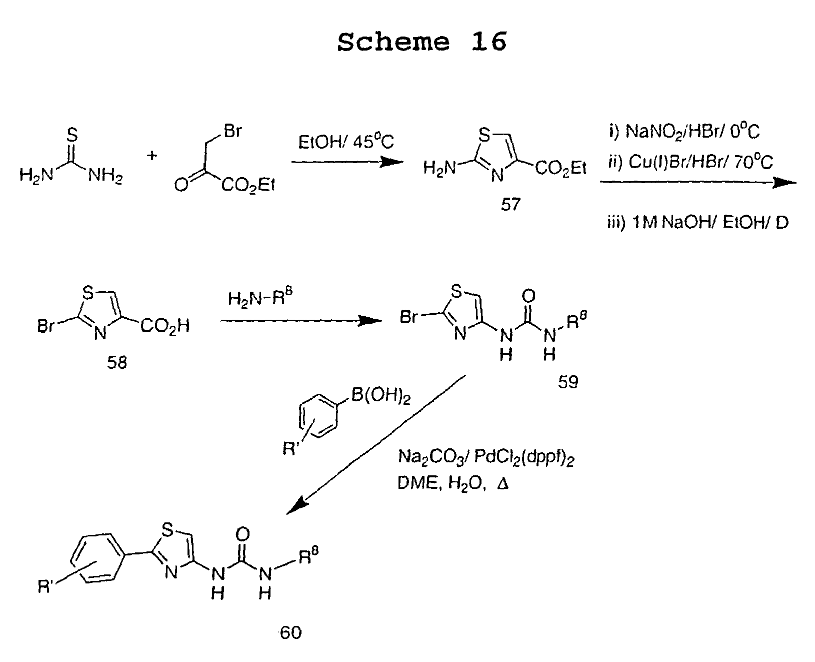

Compounds of Formula I (where R⁷ is optionally substituted phenyl) are prepared as described in Scheme 16. The 2-aminothiazole **57** was prepared from thiourea and ethyl bromopyruvate, in an alcoholic solvent like ethanol, at a temperature greater than about RT, and preferably at about 45°C. Treatment of the ethyl 2-aminothiazole-4-carboxylate with HBr, NaNO₂, CuBr and heating at a temperature greater than about 50°C, and preferably at about 70°C, provides the bromo thiazole ester. Hydrolysis of the ester, such as with aqueous NaOH and alcohol, such as EtOH and heating at a temperature greater than about 50°C, and preferably at reflux provides the bromothiazole acid **58**. Coupling with substituted amines, similar to that described in Scheme 8, provides the 2-bromothiazolyl urea **59**. Suzuki coupling of 2-bromothiazolyl urea **59** with phenyl boronic acids provides the compounds where R⁷ is optionally substituted phenyl **60**.

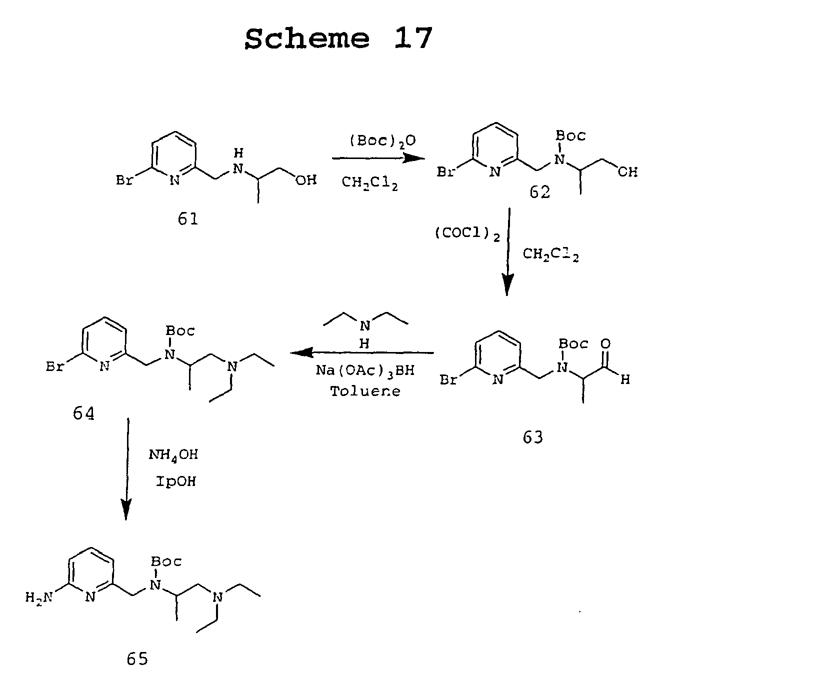

Substituted aminopyridines **65** are prepared by the method described in Scheme 17. 2-[(6-Bromo-2-pyridyl)methyl)aminopropan-1-ol **61** was protected such as with Boc with di-tert-butyldicarbonate in dry CH₂Cl₂. Conversion to the aldehyde **63** was accomplished by treatment with oxalyl chloride (in CH₂Cl₂), and DMSO at a temperature below about -23°C and preferably at about -63°C, until all the starting material was consumed. Addition of base such as DEA to the aldehyde 63, and heating to reflux in a Dean-Stark trap, followed by the addition of a solution of NaBH(OAc)₃ in acid such as AcOH at RT provided the aminoalkylaminoalkyl derivative **64**. The aminopyridine 65 is prepared as described above.

The following examples contain detailed descriptions of the methods of preparation of compounds of Formulas I-V. These detailed descriptions fall within the scope, and serve to exemplify, the above described General Synthetic Procedures which form part of the invention. These detailed descriptions are presented for illustrative purposes only and are not intended as a restriction on the scope of the invention. All parts are by weight and temperatures are in Degrees centigrade unless otherwise indicated. All compounds showed NMR spectra consistent with their assigned structures.
The following abbreviations are used:
- RT -: RT
- H₂O -: water
- Na₂SO₄: sodium sulfate
- Na₂CO₃: sodium carbonate
- Et₂O -: diethyl ether
- DMSO -: dimethylsulfoxide
- NaOMe: sodium methoxide
- NaCl -: sodium chloride
- MgCl₂ -: magnesium chloride
- EDTA -: ethylenediaminetetraacetic acid
- ESA -: bovine serum albumin
- ATP -: adenosine triphosphate
- NaN₃ -: sodium azide
- Tris-HC1: -Tris(hydroxymethyl)aminomethane hydrochloride salt
- EGTA -: ethylene glycol-bis (β-aminoethyl ether)-N,N,N', N'-tetraacetic acid
- DTT -: dithiothreitol
- NaOH -: sodium hydroxide
- mg -: milligram
- g -: gram
- ml -: milliliter
- EtOAc -: ethyl acetate
- h -: hour
- min -: minutes
- Et₃N, TEA -: triethylamine
- DEA -: diethylamine
- KOH -: potassium hydroxide
- THF -: tetrahydrofuran
- LiOH -: lithium hydroxide
- Et₂NH -: diethylamine
- IpOH -: isopropanol
- MeOH -: methanol
- EtOH -: ethanol
- CH₃CN -: acetonitrile
- DMF -: dimethylformamide
- MgSO₄ -: magnesium sulfate
- NH₄OH -: ammonium hydroxide
- LiAlH₄ -: lithium aluminum hydride
- NH₃ -: ammonia
- CH₂Cl₂ -: dichloromethane
- F₂S₅ -: phosphorous pentasulfide
- HCl -: hydrochloric acid
- HBr -: hydrobromic acid
- SOV -: sodium orthovanadate
- MnCl₂ -: manganese chloride
- Cu -: copper
- CuBr-: copper (I) bromide
- H₂S -: hydrogen sulfide
- AcOH -: acetic acid
- NaBH(OAc)₃ -: sodium trisacetoxy borohydride
- NaH -: sodium hydride
- TEA -: triethylamine
- BOC -: tert-butyloxycarbonyl
- DMAP -: 4-(dimethylamino)pyridine
- Na₂HCO₃ -: sodium bicarbonate
- DIEA -: diisopropylethylamine
- EDC -: 1-(3-dimethylaminopropyl)-3-ethylcarbodiimide hydrochloride
- AcCN -: acetonitrile
- PtO₂ -: platinum oxide
- TFA -: trifluoroacetic acid
- NaCNBH₃ -: sodium cyanoborohydride
- NaBH₄ -: sodium borohydride
- HOEt -: hydroxybenzotriazole
- BOP-Cl -: bis(2-oxo-3-oxazolidinyl)phosphinic chloride
- (PhO)₂PON₃ -: diphenylphosphoryl azide
- NBS -: N-bromosuccinimide
- Pd(OH)₂/C -: palladium hydroxide on carbon

### Procedure A: 2-Amino-6-morpholinopyridine:

A mixture of 2-chloro-6-aminopyridine (200 mg, 1.49 mmol), morpholine (326 mg, 3.75 mmol) and phenol (2 g) was heated at 150°C for 20 h. After cooling to RT, 3N NaOH (10ml) was added and the mixture was extracted with EtOAc (3×50m1). The combined organic layers were washed with brine, dried over Na₂SO₄, filtered and concentrated *in vacuo.* The crude was purified by chromatography on silica gel (1:10 MeOH/CH₂Cl₂) to afford the morpholino derivative as an amber oil. MS m/z: 180 (M+1).

### Procedure B: 2-Bromo-6-N,N-diethylamidopyridine:

Ethyl chloroformate (1.76 g, 16.3 mmol) was added dropwise to a mixture of 6-bromopicolinic acid (3 g, 14.8 mmol) and Et₃N (1.8 g, 17.8 mmol) in THF (150 ml) at 0°C. After the mixture was stirred for 1 h, DEA (1.3 g, 17.8 mmol) was added slowly to the mixture at 0°C. The resulting mixture was stirred at RT for 5 h. H₂O (200 ml) was added and the mixture was extracted with EtOAc (3x120 ml). The combined organic layers were washed with 1N NaOH and brine, dried over Na₂SO₄, and filtered. The filtrate was concentrated *in vacuo* to afford 2-bromo-6-N,N-diethylamidopyridine as an amber oil. MS m/z: 259 (M+1).

### Procedure C: 2-Amino-6-N,N-diethylamidopyridine:

A mixture of 2-bromo-6-N,N-diethylamidopyridine (3.5 g), 50 ml of 37% NH₄OH and 0.8 g of Cu powder in 40 ml of IpOH was heated at 100°C in sealed tube for 20 h. After cooling to RT, brine was added and the mixture was extracted with EtOAc (3X120 ml). The combined organic layers were washed with brine, dried over Na₂SO₄, filtered and concentrated *in vacuo* to afford the amino derivative as a light amber solid. MS m/z: 194 (M+1).

### Procedure D: 2-Amino-6-N,N-diethylaminomethylpyridine:

To a solution of 2-amino-6-N,N-diethylamidopyridine (2.2 g, 11.4 mmol) in 200 ml of THF was added slowly 34.2 ml of LiAlH₄ (1.3 g, 34.2 mmol) solution in Et₂O at 0°C. The resulting mixture was heated at reflux for 6 h. After cooling to 0°C, 2 ml of H₂O, 1.3 ml of 15% NaOH and 7.5 ml of H₂O was added to the mixture sequentially. After stirring for 2 h at RT, the mixture was filtered through Celite®. The filtrate was concentrated and purified by chromatography on silica gel (1:10 MeOH(NH₃)/CH₂Cl₂) to afford the aminomethyl compound as an amber oil. MS m/z: 180 (M+1).

### Procedure E: 2-Amino-6-(N-methylpiperazinyl)pyridine:

A mixture of 2-bromo-6-aminopyridine (3 g, 17.34 mmol), 1-methylpiperizine (2.3 g, 22.54 mmol) and Cu powder (0.5 g, 7.87 mmol) in 5 ml of 2,4-diethylphenol was heated at 150°C for 20 h. After cooling to RT, 3N HCl (30 ml) was added and the mixture was extracted with Et₂O (2x100 ml). The aqueous layer was basified with concentrated NH₄OH to pH>10 and then extracted with EtOAc (3x100 ml). The combined organic layers were washed with brine, dried over Na₂SO₄, filtered and concentrated *in vacuo.* The crude was purified by chromatography on silica gel (1:10 MeOH(NH₃)/CH₂Cl₂) to afford the piperazinyl compound as a light amber solid. MS m/z: 193 (M+1).

### Procedure F: 2-Amino-6-(4-morpholino)propylamino-pyridine:

A mixture of 2-bromo-6-aminopyridine (0.5 g, 2.92 mmol), 4-(3-aminopropyl)morpholine (1.5 g 10.42 mmol) and Cu powder (0.6 g, 9.52 mmol) in 15 ml of IpOH and 5 ml of H₂O was heated at 100°C in a sealed tube for 24 h. After cooling to RT, water was added and the mixture was extracted with EtOAc (3X50 ml). The combined organic layers were washed with brine, dried over Na₂SO₄, filtered and concentrated *in vacuo.* The crude was purified by chromatography on silica gel (1:10 MeOH(NH₃)/CH₂Cl₂) to afford the morpholino compound as an amber oil. MS m/z: 237 (M+1).

### Procedure G: 2-Amino-6-(2-N,N-dimethylamino) ethylaminopyridine:

A mixture of 2-bromo-6-aminopyridine (0.3 g, 1.17 mmol), N,N-dimethylethylenediamine (1 g, 11.36 mmol) and Cu powder (0.74 g, 11.7 mmol) in 30 ml of IpOH was heated at 100°C in seal tube for 20 h. After cooling to RT, water was added and the mixture was extracted with EtOAc (3x50 ml). The combined organic layers were washed with brine, dried over Na₂SO₄, filtered and concentrated *in vacuo.* The crude was purified by chromatography on silica gel (1:10 MeOH(NH₃)/CH₂Cl₂) to afford the aminoethyl compound as an oil . MS m/z: 181 (M+1).

### Procedure H: Amino-2-pyridylmethane-1-thione:

2-Cyanopyridine (2.6 g, 0.025 mol) was added to a solution of TEA (5.5 ml) and dry pyridine (50 ml) at RT. H₂S was bubbled through the solution for 1 h. Afterwards, H₂O (150 ml) was added and the mixture was extracted with EtOAc (3×50ml). The EtOAc extracts were dried over Na₂SO₄, filtered, and the solvent was removed under vacuum. The resulting residue was purified by column chromatography eluting with hexanes:EtOAc (4:1) to give amino-2-pyridylmethane-1-thione as a light yellow solid. GC/MS m/z: 139 (M+H); GC Retention time: 7.93 minutes.

### Procedure I: 2-(2-Pyridinyl)thiazole-4-carboxylic acid:

Amino-2-pyridylmethane-1-thione (1.88 g, 0.0136 mol), ethyl bromopyruvate (1.80 ml, 0.0143 mol) and EtOH (30 ml) were combined and heated to reflux. GC/MS of reaction mixture after 3 h showed total consumption of the starting materials. After cooling to RT, the solvent was removed under vacuum resulting in a dark brown oil (GC/MS m/z: 235 (M+H); GC Retention time: 10.69 minutes). The material was taken up in MeOH (20 ml), 1.0M LiOH-H₂O (20 ml) was added and the mixture was heated to 100°C for 14 h. After cooling to RT, the excess MeOH was evaporated and the resulting brown solid filtered. The material was washed with a minimum of H₂O and dried *in vacuo* to give the thiazole as a brown solid.

### Procedure J: 2-(4-Pyridinyl)-4-thiazolylcarbonylazide:

To a suspension of 2-(4-pyridinyl)-4-thiazolylcarboxylic acid (Maybridge Chem., 6.0g, 29.1 mmol) in 150 ml MeOH at RT was added NaGH (1.28g, 32.0 mmol) and the mixture was stirred at RT for 45 min. The reaction mixture was concentrated in vacuo then dried under high vacuum for 60 h (overnight drying is a minimum). The crude salt was suspended in 150 ml of CH₂Cl₂ and cooled in an ice bath. Oxalyl chloride (2.8 ml) was added slowly to the suspension followed by a catalytic amount of DMF (0.2 ml). The mixture was stirred for 2 h and warmed to RT. The reaction was cooled in an ice bath and a solution of NaN₃ (2.27g) in water (90 mL) was added and stirring was continued for 3 h. The reaction mixture was diluted with water (90 ml) and extracted with CH₂Cl₂ (3×75ml) . The combined organic layers were filtered through Celite® (-12 g) washed with 90 mL brine, dried with MgSO4 and concentrated *in vacuo.* Drying the crude compound on the vacuum line afforded the azido derivative as a light brown solid. MS *m*/*z:* 204.5 (M-N₂+H).

### Procedure K: 2-(3-Pyridinyl)-4-thiazolylcarbonylazide:

In a manner similar to that described for the preparation of 2-(4-pyridinyl)-4-thiazolylcarbonylazide, 6.0 g of 2-(3-pyridinyl)-4-thiazolylcarboxylic acid was treated successively with NaOH, oxalyl chloride and a solution of NaN₃ in water to give the 3-pyridinylazide as a pale brown solid. MS *m*/*z:* 204.5 (M-N₂+H).

### Procedure L: 2-(2-Pyridinyl)-4-thiazolylcarbonylazide:

In a manner similar to that described for the preparation of 2-(4-pyridinyl)-4-thiazolylcarbonylazide, 2-(2-pyridinyl)-4-thiazolylcarboxylic acid (1.0g)was treated successively with NaOH, oxalyl chloride and a solution of NaN₃ in water to give the 2-pyridinyl azide as a pale brown solid: m.p. 112-114°C. MS m/z: 232 (M+H).

### Procedure M: 2-Phenyl-4-thiazolylcarbonylazide:

In a manner similar to that described for the preparation of 2-(4-pyridinyl)-4-thiazolylcarbonylazide, 1.0g of 2-phenyl-4-thiazolylcarboxylic acid was treated successively with NaOH, oxalyl chloride and a solution of NaN₃ in water to give the phenylazide as an off white solid. MS m/z: 203.5 (M-N₂+H) .

### Procedure N: 4-(6-Bromo-pyridin-2-ylmethyl)-morpholine

To a stirred solution of 6-bromo-2-pyridine-carboxaldehyde (200 mg, 1.08 mmol) in dichloroethane (10 mL) was added morpholine (0.14 mL, 1.62 mmol) followed by NaBH(OAc)₃ (458 mg, 2.16 mmol) and AcOH (0.25 mL, 4.32 mmol). The resulting mixture was stirred at RT for 12 h. The reaction was quenched with 2M Na₂CO₃ solution and stirred 1 h. The mixture was poured into Et₂O and washed with 2 M Na₂CO₃ solution. The organic layer was collected, dried over Na₂SO₄ and concentrated in vacuo to give 2-bromo-6-morpholinylmethylpyridine as a white solid. MS m/z: 256.9 (M+H).

The following compounds were prepared in a manner similar to that described above:
1] 1-(6-Bromopyridin-2-ylmethyl)-piperidine-4-carboxylic acid ethyl ester, as a pale yellow solid, was prepared in a manner similar to that described in General Procedure N [6-bromo-2-pyridinecarboxaldehyde (400 mg, 2.16 mmol) was added to ethyl isonipecotate (0.5 mL, 3.24 mmol) in dry CH₂Cl₂ (10 mL)] . MS m/z: 327.0 (M+H). Calc' d for C₁₄H₁₉BrN₂O₂: 326.90.
2] To 6-bromo-2-pyridinecarboxaldehyde (400 mg, 2.16 mmol) was added L-leucinol (0.42 mL, 3.24 mmol) in dry CH₂Cl₂ (10 mL) to give 2-[(6-bromo-pyridin-2-ylmethyl)-amino]-4-methyl-pentan-1-ol as brown solid. MS m/z: 287.6 (M+H). Calc'd for C₁₂H₁₉BrN₂O : 287.2.
3] To 6-bromo-2-pyridinecarboxaldehyde (500 mg, 2.69 mmol) was added 1,4-dioxa-8-azaspiro-[4,5]-decane (0.5 mL, 4.03 mmol) in dry CH₂Cl₂ (10 mL) to give 2-bromo-6-(4-ethoxyacetal)-piperidinylmethylpyridine as white solid. MS m/z: 313 (M+H) . Calc'd for C₁₃H₁₇BrN₂O2 : 313.2.
4] To 6-bromo-2-pyridinecarboxaldehyde (400 mg, 2.15 mmol) was added 3,5-dimethylpiperidine (0.4 mL, 3.22 mmol) in dry CH₂Cl₂ (10 mL) to give 2-bromo-6-(3,5-dimethyl)piperidinylmethyl pyridine as white solid. MS m/z: 283.2 (M+H). Calc'd for C₁₃H₁₉BrN₂: 283.2
5] To 6-bromo-2-pyridinecarboxaldehyde (400 mg, 2.15 mmol) was added 4-methylpiperidine (0.4 mL, 3.22 mmol) in dry CH₂Cl₂ (10 mL) to give 2-bromo-6-[(4-methyl)piperidinylmethyl]pyridine as a white solid. MS m/z: 269.4 (M+H). Calc'd for C₁₂H₁₇BrN₂: 269.18 .
6] To 6-bromo-2-pyridinecarboxaldehyde (400 mg, 2.15 mmol) was added 2-methylpiperidine (0.4 mL, 3.22 mmol) in dry CH₂Cl₂ (10 mL) to give 2-bromo-6-[(2-methylpiperidinyl)methyl]pyridine as a pale yellow solid. MS m/z: 269.1(M+H). Calc'd for C₁₂H₁₇BrN₂: 269.18.
7] To 6-bromo-2-pyridinecarboxaldehyde (400 mg, 2.15 mmol) was added 4-(1-pyrrolidinyl)-piperidine (500 mg, 3.22 mmol) in dry CH₂Cl₂ (15 mL) to give 2-bromo-6-[4-(1-pyrrolidinyl)-piperidinylmethyl]pyridine as a pale yellow solid. MS m/z: 326.1(M+2H). Calc'd for C₁₅H₂₂BrN₃: 324.26.
8] To 6-bromo-2-pyridinecarboxaldehyde (400 mg, 2.15 mmol) was added 3-hydroxypiperidine (326 mg, 3.22 mmol) in dry CH₂Cl₂ (15mL) to give 2-bromo-6-(3-hydroxypiperidinyl)methyl pyridine as pale yellow solid. MS m/z: 271.2 (M+H). Calc'd for C₁₁H₁₅BrN₂O: 271.15.
9] To 6-bromo-2-pyridinecarboxaldehyde (300 mg, 1.62 mmol) was added hexamethyleneimine (0.27 mL, 2.43 mmol) in dry CH₂Cl₂ (10 mL) to give 2-bromo-6-(azaperhydroepinylmethyl)pyridine as a white solid. MS m/z: 270.3(M+H). Calc'd for C₁₂H₁₇BrN₂: 269.18.
10] To 4-hydroxypiperidine (143 mg, 1.41 mmol) was added a solution of 6-bromo-2-pyridine-carboxaldehyde (200 mg, 1.08 mmol) to give 2-bromo-6-[(4-hydroxypiperidyl)methyl]-pyridine as a white solid. MS m/z: 271.0 (M+H). Calc'd for C₁₁H₁₅BrN₂O - 271.15.
11] 3-Hydroxypropylamine (0.15 mL, 2.02 mmol) was added to a solution of 6-bromo-2-pyridine-carboxaldehyde (250 mg, 1.35 mmol) to give 2-bromo-6-[(3-hydroxypropyl)amino]-methylpyridine as a white solid. MS *m*/*z*: 245.1 (M+H). Calc'd for C₁₁H₁₃BrN₂O - 245.19.
12] Ethyl (piperidyl-3-carboxylate (0.92 mL, 5.92 mmol) was added to a solution of 6-bromo-2-pyridinecarboxaldehyde (1.0 g, 5.38 mmol) to give ethyl 1-[(6-bromopyridin-2-yl)methyl]-piperidine-3-carboxylate as a colorless oil. MS *m*/*z*: 327.1 (M+H). Calc'd for C₁₄H₁₉BrN₂O₂ - 327.22.
13] Ethyl (2-piperidyl)carboxylate (0.92 mL, 5.92 mmol) was added to a solution of 6-bromo-2-pyridinecarboxaldehyde (1.0 g, 5.38 mmol) to give ethyl 1-[(6-bromopyridin-2-yl)methyl]-piperidine-2-carboxylate as a colorless oil). MS *mlz:* 327.1 (M+H). Calc'd for C₁₄H₁₉BrN₂O₂ - 327.22.
14] N,N-Diethylcarbamoyl-piperidine-3-carboxamide (0.92 mL, 5.92 mmol) was added to a solution of 6-bromo-2-pyridinecarboxaldehyde (1.0 g, 5.38 mmol) to give N,N-diethyl 1-(6-bromopyridin-2-ylmethyl)piperidine-3-carboxamide as a colorless oil. MS m/z: 354.1 (M+H). Calc'd for C₁₆H₂₄BrN₃O - 354.29.
15] 2-Pyrrolidine carboxylic acid (0.68 g, 5.92 mmol) was added to a solution of 6-bromo-2-pyridine-carboxaldehyde (1.0 g, 5.38 mmol) to give 1-(6-bromopyridin-2-ylmethyl)-pyrrolidine-2-carboxylic acid as a white solid. MS *m*/*z:* 285.1 (M+H). Calc'd for C₁₁H₁₃BrN₂O₂ - 285.14.
16] 3-Methylpiperidine (0.33 mL, 2.8 mmol) was added to a solution of 6-bromo-2-pyridine-carboxaldehyde (350 mg, 1.88 mmol) to give 2-bromo-6-[(3-methylpiperidyl)methyl]pyridine as a white solid. MS m/z: 269.1 (M+H). Calc'd for C₁₂H₁₇BrN₂ - 269.18.

### Procedure O: 6-Morpholin-4-ylmethyl-pyridin-2-ylamine

NH₄OH (2 mL) and Cu powder (10 mg, 0.15 mmol) were added to a solution of 2-bromo-6-morpholinylpyridine (231 mg, 0.90 mmol) in IpOH (5 mL) and the resulting mixture was heated at 100°C for 36 h in a sealed tube. After cooling to RT, the mixture was partitioned between H₂O and EtOAc. The organic layer was collected, washed with brine, and dried over Na₂SO₄. Concentration in vacuo gave the tilted compound as a pale yellow solid. MS *m*/*z:* 194.1 (M+H).

The following amines were prepared from the corresponding bromo compounds (prepared by Procedure N) in a manner similar to that described in General Procedure O:
1]1-(6-amino-pyridin-2-ylmethyl)-piperidine-4-carboxylic acid ethyl ester as brown liquid. MS m/z: 264.2 (M+H). Calc' d for C₁₄H₂₁N₃O₂: 263.34.
2]2-amino-6-[N'-*tert*-butoxycarbonyl-N'-2-(1-hydroxy-4-methyl)pentylamino]methylpyridine as a brown liquid. MS m/z: 324.3 (M+H). Calc'd for C₁₇H₂₉N₃O₃: 323.2.
3] 2-amino-6-(4-ethoxyacetalpiperidinyl)-methylpyridine as a white solid. MS m/z: 250 (M+2H). Calc'd for C₁₃H₁₉N₃O₂: 249.1.
4]2-Amino-6-(3,5-dimethylpiperidinyl)methylpyridine as a yellow solid. MS m/z: 220.3 (M+H). Calc' d for C₁₃H₂₁N₃: 219.
5] 2-Amino-6-(4-methylpiperidinyl)methylpyridine as a yellow solid. MS m/z: 206.3 (M+H). Calc'd for C₁₂H₁₉N₃: 205.28.
6]2-Amino-6-(2-methylpiperidinyl)methylpyridine as a yellow liquid. MS m/z: 206.3 (M+H). Calc' d for C₁₂H₁₉N₃: 205.28.
7] 2-Amino-6-[[4-(1-pyrrolidinyl)piperidinyl]methyl]-pyridine as a brown liquid (335 mg, 93%) . MS m/z: 261.1 (M+2H). Calc'd for C₁₂H₁₉N₃: 260.
8] 2-Amino-6-(3-hydroxypiperidinyl)methylpyridine as a yellow liquid. MS m/z: 410.9 (M+H). Calc' d for C₁₁H₁₇N₃O: 410.5.
9] 2-Amino-6-(azaperhydroepinylmethyl)pyridine as a white solid. MS m/z: 206.1 (M+H). Calc'd for C₁₂H₁₉N₃ : 205.32.
10] 2-Amino-6-[(4-hydroxypiperidyl)methyl]pyridine as a pale yellow oil. MS *m*/*z:* 208.1 (M+H). Calc'd for C₁₁H₁₇N₃O - 207.27.
11] 2-Amino-6-[(N-*tert*-butoxycarbonyl-N-(3-hydroxypropyl)amino]methylpyridine as a pale yellow oil. MS *m*/*z*: 282.3 (M+H). Calc'd for C₁₄H₂₃N₃O₃ - 231.35.
12] Ethyl 1-[(6-aminopyridin-2-yl)methyl]-piperidine-3-carboxylate as a pale yellow oil. MS *m*/*z*: 264.1 (M+H). Calc'd for C₁₄H₂₁N₃O₂ - 263.34.
13] Ethyl 1-[(6-aminopyridin-2-yl)methyl]-piperidine-2-carboxylate as a pale yellow oil. MS *m*/*z*: 264.1 (M+H). Calc'd for C₁₄H₂₁N₃O₂ - 263.34.
14] N,N-Diethyl 1-(6-aminopyridin-2-ylmethyl)-piperidine-3-carboxamide as a pale yellow oil. MS *m*/*z:* 291.5 (M+H). Calc'd for C₁₆H₂₆N₄O - 290.40.
15] 1-(6-Aminopyridin-2-ylmethyl)-pyrrolidine-2-carboxylic acid as a white solid. MS m/z: 220.3 (M-H). Calc'd for C₁₁H₁₅N₃O₂ -221.26.
16] 2-Amino-6-[(3-methylpiperidyl)methyl]pyridine as a pale yellow solid (250 mg, 68%). MS m/z: 206.5 (M+H). Calc'd for C₁₂H₁₉N₃ - 205.30.
17] 1-(6-Aminopyridin-2-ylmethyl)-piperidine-3-carboxylic acid as a pale yellow oil. MS *mlz:* 235.0 (M+H). Calc'd for C₁₂H₁₇N₃O₂ -235.28.

### Procedure P: 4-(6-Aminopyridin-2-yloxy)-benzonitrile

To a stirred solution of 4-cyanophenol (1.7 g, 14.3 mmol) in 45 mL dry DMF was added NaH (0.71 g, 17.7 mmol). After stirring at RT for 15 min, 2,6-dibromopyridine (3.2 g, 13.4 mmol) was added and the mixture was heated at 95°C for 24 h. After cooling to RT, 100 mL of H₂O was added and the mixture was extracted with EtOAc (2x100 mL). The combined organic layers were washed with 40 mL brine, dried over MgSO₄ and concentrated in vacuo. The crude intermediate was dissolved in 20mL IpOH, transferred to a Teflon lined pressure vessel and 20 mL of conc. NH₄OH was added. Powdered Cu (1 g) was added and the vessel was sealed and heated at 140°C for 24 h. After cooling to RT, the Cu was removed by filtration and the filtrate was diluted with 75 mL of H₂O and extracted with EtOAc (2x75 mL). The organic layers were washed with brine, dried over MgSO₄ and concentrated in vacuo. The compound was purified by chromatography on silica gel using 10:1 CHCl₃/ (~2M NH₃/MeOH) as eluent to afford the title compound (1.5 g, 55 %) as a dark oil. MS m/z: 212.2 (M+H).

The following compounds were prepared from 2,6-dibromopyridine in a manner similar to that described in General Procedure P:
1] 6-Phenoxy-pyridin-2-ylamine: MS m/z: 187.2 (M+H). Calc'd for C₁₁H₁₀N₂O: 186.08.
3] 6-(4-Methylphenyloxy)pyridin-2-ylamine: MS m/z: 201.3 (M+H). Calc'd for C₁₂H₁₂N₂O: 200.09.
3] 6-(2,4-Dimethylphenyloxy)pyridin-2-ylamine: MS m/z: 215.3 (M+H). Calc'd for C₁₃H₁₄N₂O: 214.11.
4] 6-[4-(1-Imidazolyl)phenyloxy]pyridin-2-ylamine: MS m/z: 253.3 (M+H). Calc' d for C₁₄H₁₂N₄O: 252.10.
5] 6-[4-[1,3]Dioxolan-2-yl-phenoxy)pyridin-2-ylamine: MS m/z: 259.3 (M+H). Calc'd for C₁₄H₁₄N₂O₃: 258.10.
6] 6-(4-Fluorophenyloxy)pyridin-2-ylamine: MS m/z: 205.2 (M+H). Calc'd for C₁₁H₉FN₂O: 204.07.
7] 6-(4-Difluorophenyloxy)pyridin-2-ylamine: MS m/z: 223.2 (M+H). Calc'd for C₁₁H₈F₂N₂O: 222.06.
8] tert-Butyl {2-[4-(6-aminopyridin-2-yloxy)phenyl]ethyl}carbamate: MS m/z: 330.4 (M+H). Calc'd for C₁₉H₂₃N₃O₃ : 329.17.
9] 6-(2-Dimethylaminoethoxy)pyridin-2-ylamine: MS m/z: 182.2 (M+H). Calc'd for C₉H₁₅N₃O: 181.12.
10] 6-[(1-Methylpyrrolidin-2-yl)methoxy]pyridin-2-ylamine: MS m/z. 208.3 (M+H). Calc'd for C₁₁H₁₇N₃O: 207.14.
11] 6-(1-Aza-bicyclo[2.2.2]oct-3-yloxy)pyridin-2-ylamine: MS m/z: 220.3 (M+H). Calc'd for C₁₂H₁₇N₃O: 219.14.
12] tert-Butyl 3-[(6-aminopyridin-2-yl)oxymethyl]-azetidine-1-carboxylate: MS m/z: 280 (M+H). Calc'd for C₁₄H₂₁N₃O₃: 279.16.
13] tert-Butyl 4-[2-(6-Aminopyridin-2-yloxy)ethyl]-piperidine-1-carboxylate: MS m/z: 322 (M+H). Calc'd for C₁₇H₂₇N₃O₃: 321.21.

### Preparation Q: 2-Bromo-6-[N'-tert-butoxycarbonyl-N'-2-(1-hydroxy-4-methyl)pentylamino]methylpyridine

To 2-bromo-6-[2-N-(1-hydroxy-4-methyl)-pentylamino]methylpyridine (550 mg, 1.91 mmol) in dry CH₂Cl₂ (10 mL) was added (Boc)₂O (460 mg, 2.106 mmol). The resulting mixture was stirred under N₂ at RT for 15 h. The solvent was removed and the residue was extracted with CHCl₃. The organic layer was wash with H₂O, brine, and dried over MgSO₄ and removed to give a yellow liquid. MS m/z:387.6(M+H). Calc'd for C₁₇H₂₇BrN₂O₃: 387.32.

The following BOC protected compounds were prepared from the corresponding amines (prepared by Procedure N) in a manner similar to that described in General Procedure Q:
1] 2-Bromo-6-[(N-*tert*-butoxycarbonyl-N-(3-hydroxypropyl)amino]methylpyridine was prepared from 2-bromo-6-[(3-hydroxypropyl)-amino]-methylpyridine (300 mg, 1.22 mmol) [purified by chromatography on silica gel (hexane/acetone, 80/20]) as a colorless oil. MS *m*/*z:* 345.1 (M+H). Calc'd for C₁₄H₂₁BrN₂O₃ - 345.23.

### Preparation R: 2,2-Dimethyl-N-[6-(2-methylimidazol-1-ylmethyl)pyridin-2-yl]propionamide

A solution of 2-methylimidazole (68 mg, 0.83 mmol) in dry THF (8 mL) was treated under N₂ with NaH (33 mg, 0.83 mmol, 60% in mineral oil) at 0°C. After the addition, the mixture was warmed to RT and stirred for 0.5 h. It was then treated dropwise with a solution of N-pivaloyl-2-amino-6-bromomethylpyridine (150 mg, 0.55 mmol; M.V. Papadopoulou, et al., J. *Heterocyclic Chem.,* 1995, 32, 675-681) in dry THF (10 mL) over period of 15 min. After the addition, it was stirred for 1 h. The resulting mixture was quenched with saturated NH₄Cl (3 mL). Solvent was removed and the residue was extracted with CHCl₃. The organic layer was washed with H₂O, brine, dried over MgSO₄, and concentrated in vacuo to yield the title compound as light brownish solid (145 mg, 96%). MS *m*/*z:* 272.2 (M+H) . Calc'd. for C₁₆H₂₁N₃O - 271.37.

The following amines were prepared from the corresponding bromomethylpyridine in a manner similar to that described in Preparation R:
1] 2,2-Dimethyl-N-[6-(4-(N,N-dimethylaminomethyl)phenyloxymethyl)pyridin-2-yl]propionamide. MS *m*/*z*: 342 (M+H).

### Preparation S: N-(6-Azidomethylpyridin-2-yl)-2,2-dimethylpropionamide

*N*-Pivaloyl-2-amino-6-bromomethylpyridine (1.1 g, 4.05 mmol; M.V. Papadopoulou, et al., J. *Heterocyclic* Chem., **1995,** *32,* 675-681) was dissolved in dry THF (15 mL). NaN₃ (530 mg, 8.1 mmol) and dry DMF (5 mL) was added and the resulting mixture was heated to reflux under N₂ for 2 h. After cooling to RT, solvent was removed and the residue was partitioned between H₂O and CHCl₃. The organic layer was washed with H₂O, brine, dried over MgSO₄, and concentrated in vacuo to give the title compound as a pale yellow solid. MS m/z: 234.1 (M+H). Calc'd. for C₁₁H₁₅N₅O - 233.28.

### Preparation T: 6-Azidomethyl-pyridin-2-ylamine

2-(N'-Pivaloyl)amino-6-azidomethylpyridine (680 mg, 2.91 mmol) was dissolved in MeOH (20 mL) and KOH was added (3.4 g, 60.6 mmol). The resulting mixture was heated to reflux under N₂ for 2 h. After cooling to RT, pH was adjusted to 7 followed by removing the solvent. The residue was partitioned between H2O and CHCl₃ and the aqueous layer was extracted more with CHCl₃. The combined organic layers was washed with H₂O, brine, dried over MgSO₄, and concentrated *in vacuo* to yield the title compound as brown solid. MS m/z: 150.3 (M+H). Calc'd. for C₆H₇N₅: 149.15.

The following amines were prepared from the corresponding bromo compounds (prepared by Preparations R-S, and AA) in a manner similar to that described in Preparation T:
1] 3-(2-Methylimidazol-1-ylmethyl)phenylamine. MS *m*/*z* : 189.3 (M+H). Calc'd. for C₁₀H₁₂N₄ - 188.23.
2] 2-Amino-6-[4-(dimethylamino)methyl]phenoxymethyl-pyridine. MS m/z: 258 (M+H).
3] 2-Amino-6-[1-(N-tert-butoxycarbonyl)amino]-ethoxymethyl-pyridine. MS m/z: 268 (M+H).
4] 2-Amino-6-[4-(methylphenyl)oxymethyl]pyridine. MS m/z: 215 (M+H).
5] 2-Amino-6-[1-(*N*-tert-butoxycarbonyl)amino]-ethoxymethyl-pyridine. MS m/z: 267 (M+H)
6] 2-Amino-5-[1-morpholinylmethyl]pyridine. MS m/z: 194 (M+H).
7] 5-Methoxymethyl-pyridin-2-ylamine.

### Preparation U: Methyl 1-(6-aminopyridin-2-ylmethyl)-pyrrolidine-2-carboxylate

Concentrated sulfuric acid (1.0 mL) was added to a solution of 1-(6-aminopyridin-2-ylmethyl)-pyrrolidine-2-carboxylic acid (620 mg, 2.80 mmol) in MeOH (15 mL) and the resulting mixture was heated at 80°C for 10 h. After cooling to RT, the mixture was quenched with saturated 2 M Na₂CO₃ solution and concentrated in vacuo. CHCl₃ (15 mL) was added and the solution washed with 1.0 N NaOH solution (15 mL). The organics were collected and the aqueous layer was extracted with CHCl₃/IpOH (3/1, 3×10 mL). The combined organics were dried over MgSO₄ and concentrated in vacuo. The crude compound was purified by chromatography on silica gel (CH₂Cl₂/MeOH, 95/5) to give a pale yellow oil. MS m/z: 236.1 (M+H). Calc'd for C₁₂H₁₇N₃O₂ -235.28.

### Preparation V: 3-Methyl-2-(phthalimidyl)pyridine

2-Amino-3-picoline (1.00 mL, 8.62 mmol) was dissolved in DMF (30 mL) at 23°C, and treated with solid carboethoxy-phthalimide (1.89 g, 8.64 mmol), followed by TEA (1.44 mL, 10.3 mmol). The resulting solution was stirred at 23°C for 15 h. After 15 h, the mixture was diluted with EtOAc (50 mL), and washed with saturated NaCl (1X50 mL), H₂O (1X50 mL), dried (MgSO₄), and concentrated in vacuo to a yellow solid. Purification over silica gel (0 to 50% EtOAc/Hexanes) provided the title compound as a white solid.

### Preparation W: 3-(Dibromomethyl)-2-(phthalimidyl)pyridine

3-Methyl-2-(phthalimidyl)pyridine (360 mg, 1.51 mmol) was dissolved in CCl₄ (5 mL), and treated with NBS (267 mg, 1.50 mmol), followed by 2,2'-azobis-isobutyrlnitrile (AIBN) (46.9 mg, 0.29 mmol). The resulting suspension was warmed to reflux for 2 h, treated again with AIBN (55.4 mg, 0.34 mmol), and heated at reflux an additional 12 h. After 12 h, AIBN was again added (96.7 mg, 0.59 mmol) and reflux was continued. After 2 h, more AIBN was added (59.6 mg, 0.36 mmol), and reflux continued. After 2h, additional NBS was added (253 mg, 1.42 mmol) and the mixture was treated with additional AIBN (49.6 mg, 0.30 mmol), and heated at reflux an additional 12 h. The mixture was cooled to RT, diluted with EtOAc (50 mL), washed with saturated NaCl (1X50 mL), then dried (MgSO₄) and concentrated in vacuo. The resulting white solid was purified over silica gel (0 to 40% EtOAc/Hexanes) to provide the title compound. MS *m*/*z:* 397 (M+H).

### Preparation X: 2-(phthalimidyl)-3-(1-piperidinylmethyl)-pyridine

3-(Dibromomethyl)-2-(phthalimidyl)pyridine (185 mg, 0.47 mmol) was dissolved in CH₂Cl₂ (2 mL) and treated with piperidine (.460 mL, 4.66 mmol), and glacial AcOH (.160 mL, 2.80 mmol) in a dropwise fashion. The resulting yellow solution was stirred at 23°C for 2 h, then treated with solid NaBH(OAc)₃ (393 mg, 1.86 mmol) in one portion, and stirring was continued for 14 h. After stirring 14 h at 23°C, the mixture was treated with 2M K₂CO₃ (6 mL), and stirred for 1 h. After 1 h, the mixture was diluted with EtOAc (50 mL) and washed with H₂O (1X50 mL), and saturated NaCl (1X50 mL). The organic phase was then dried (MgSO₄) and concentrated in vacuo to provide the title compound as a yellow residue. The crude material was used in subsequent transformations without further purification. MS m/*z*: 323 (M+H).

### Preparation Y: 2-Amino-3-(1-piperidinylmethyl)-pyridin

2-(Phthalimidyl)-3-(1-piperidinylmethyl)-pyridine (196 mg, 0.609 mmol) was dissolved in EtOH (95%, 2 mL) at 23°C, and treated with hydrazine monohydrate (0.032 mL, 0.670 mmol) in a dropwise fashion. The resulting mixture was warmed to reflux and stirred for 3 h at reflux. The solution was treated with additional hydrazine monohydrate (0.150 mL, 3.050 mmol), and reflux continued. After 14 h at reflux, the mixture was cooled to RT, and concentrated using a rotary evaporator to a white paste. The resulting white paste was dissolved in CHCl₃:IpOH (3:1, 75 mL), and washed with saturated NaHCO₃ (3×50 mL), and H₂O (1×50 mL). The organic layer was dried over MgSO₄ and concentrated in vacuo to provide the title compound as a white solid. MS m/z: 192 (M+H).

### Preparation Z: N-Pivaloyl 2-amino-5-(bromomethyl) pyridine

N-Pivaloyl-2-amino-5-methylpyridine (5.12 g, 26.6 mmol) was dissolved in CCl₄ (75 mL) at 23°C, and treated with NBS (9.69 g, 54.4 mmol), followed by 2,2'-azobisisobutyrlnitrile (AIBN) (937 mg, 5.71 mmol) with stirring. The resulting orange, biphasic suspension was then warmed to reflux for 4h. After 4 h at reflux, the rust-colored mixture was cooled to RT, filtered through a Celite® pad, and concentrated in vacuo to a red residue. Purification over silica gel (gradient, 0 to 25% EtOAc/hexanes) provided the title compound as a light yellow solid. MS m/z: 272 (M+H).

### Preparation AA: N-Pivaloyl-2-amino-5-[2-(N-tert-butoxycarbonyl)amino]ethoxymethylpyridine

N-Pivaloyl-2-amino-5-Dromomethylpyridine (484 mg, 1.78 mmol) was dissolved in THF (6 mL) at 23°C, and treated with 2-(*N*-*tert*-butoxycarbonyl)aminoethanol (0.551 mL, 3.56 mmol), followed by NaH (60% suspension in mineral oil, 221 mg, 5.52 mmol) with stirring. The resulting mixture was stirred at 23°C for 14 h, then treated with additional NaH (75.6 mg, 1.89 mmol) as well as DMSO (1 mL), and stirred an additional 5 h at 23°C. After 5 h at 23°C, the solution was warmed to 55°C for 3 h, and then cooled to RT. The mixture was treated with saturated NaHCO₃ (10 mL), diluted with EtOAc (50 mL), and washed with saturated NaHCO₃ (2X50 mL). The mixture was dried over MgSO₄ and purified over silica gel to provide the title compound as a pale yellow oil. MS m/z: 352 (M+H).

The following amines were prepared from the corresponding bromomethylpyridine in a manner similar to that described in General Procedure AA:
1] 2,2-Dimethyl-N-[6-(N-(tert-butoxycarbonyl)-amino-1-ethoxymethyl)pyridin-2-yl]propionamide. MS *m*/*z*: 352 (M+H).
2]*N*-Pivaloyl-2-amino-6-[(4-methylphenyl)-oxymethyl]-pyridine. MS *m*/*z*: 299 (M+H).
3] *N*-Pivaloyl-2-amino-5-[(4-methylphenyl)-oxymethyl]pyridine. MS *m*/*z:* 299 (M+H).

### Preparation AB: N-Pivaloyl-2-amino-5-[1-morpholinylmethyl]pyridine

*N*-Pivaloyl-2-amino-5-bromomethylpyridine (478 mg, 1.76 mmol) was dissolved in THF at 23°C with stirring and treated with morpholine (0.770 mL, 8.81 mmol) in a dropwise fashion. The resulting brown mixture was stirred at 23°C for 14 h. After stirring 14 h, the mixture was treated with saturated NaHCO₃ (2 mL) and stirred an additional 5 h at 23°C. After 5 h, the brown mixture was warmed to 55°C for 3 h, then cooled to RT and diluted with EtOAc (50 mL). The mixture was then washed with saturated NaHCO₃ (2X50 mL), dried (MgSO₄), and concentrated to a brown residue which was immediately purified over silica gel (0 to 5% MeOH/CHCl₃) to provide the title compound as a yellow oil. MS *m*/*z*: 278 (M+H).

### Preparation AC: 2-(Butyloxycarbonyl)amino-6-methylpyridine

To a 2-L 3-neck Miller flask charged with 2-amino-6-methylpicoline (15 g, 138.7 mmol) and dry THF (1 L) was added di-tert-butyl dicarbonate (33.3 g, 152.6 mmol) then TEA (21.2 mL, 152.6 mmol) via addition funnel at 0°C. The reaction mixture was warmed to RT and added DMAP (1.7 g, 13.9 mmol). After 3.5 h, extracted with EtOAc, washed with saturated NH₄Cl, H₂O (3x), and brine (3x); dried (MgSO₄) and concentrated in vacuo to afford the crude material as a turbid yellow oil. Trituration with hexane formed a precipitate which was filtered and the filtrate was concentrated in vacuo to give the title compound as a yellow oil.

### Preparation AD: 6-Bromomethyl-2-(butyloxycarbonyl)amino-pyridine

To a solution of N-Boc-2-amino-6-picoline (28.7 g, 138 mmol) and CCl₄ (500 mL) was added NBS (27.1 g, 151.8 mmol) and AIBN (2.3 g, 13.8 mmol) and heated to reflux. After 2 h, added 0.1 equivalent of AIBN. The reaction mixture was heated at reflux for 20 h, filtered and concentrated in vacuo to give a dark oil. Purified by silica flash chromatography (100% hexane to 5% EtOAc/Hexane) to afford the desired as a yellow oil. MS m/z: 288.0 (M+H)

### Preparation AE: 2-(Butyloxycarbonyl)amino-6-cyanomethylpyridine

To a solution of N-Boc-2-amino-6-methylbromidepyridine (12 g, 41.8 mmol) and EtOH (250 mL) was added NaCN (2 g, 41.8 mmol). The reaction mixture was heated to reflux for 2 h then cooled to RT and concentrated in vacuo. Purification by silica flash chromatography (100% Hexane to 20% EtOAc/Hexane) afforded the title compound as a yellow oil. MS m/z: 234.0 (M+H).

### Preparation AF: 2-Amino-6-cyanomethylpyridine

To a solution of N-Boc-2-amino-6-methylnitrilepyridine and CH₂Cl₂ (10 mL) was added TFA (8 mL) and stirred at RT. After 3 h, the mixture was concentrated in vacuo, diluted with EtOAc and saturated NaHCO₃ was carefully added. The mixture was washed with saturated NaHCO₃ (2x) and brine, dried (MgSO₄) and concentrated in vacuo to afford the title compound as a yellow solid.

### Preparation AG: 6-Aminoethyl-2-(butyloxycarbonyl)aminopyridine

A solution of N-Boc-2-amino-6-methylnitrile-pyridine (1 g, 4.3 mmol) and EtOH (25 mL) was hydrogenated over 20% Pd(OH)₂/C at RT and 40 psi. After 18 h, the mixture was filtered through Celite® and eluted with EtOAc. The filtrate was concentrated in vacuo to afford the title compound as a white foamy solid.

### Preparation AH: 2-Amino-6-(phthalimidyl)ethyl-pyridine

To a solution of N-Boc-2-amino-6-ethylaminopyridine (1 g, 4.3 mmol) and CHCl₃ (25 mL) was added phthalic anhydride (0.64 g, 4.3 mmol). Heated to 70°C for 15 h then at RT for 5 days. The mixture was washed with H₂O and brine, dried (MgSO₄) and concentrated in vacuo to give crude N-Boc-2-amino-6-ethylphthalamidylpyridine, which was used without further purification. To a solution of crude N-Boc-2-amino-6-ethylphthalamidylpyridine (1.6 g, 4.3 mmol) and CH₂Cl₂ (10 mL) was added 10 mL of TFA and the mixture was stirred at RT. After 30 min, the mixture was concentrated in vacuo. The residue was diluted with 90% MeOH/CH₂Cl₂ and treated with solid NaHCO₃, stirred for 15 min then filtered. The filtrate was concentrated in vacuo to afford the title compound as a yellow solid. MS m/z: 268.2 (M+H).

### Preparation AI: 2-[(6-Bromopyridin-2-yl)methylamino]-propan-1-ol

To a stirred solution of the (6-bromo-2-pyridyl)-formaldehyde (0.52 g, 2.8 mmol) in toluene (14 mL) was added DL-2-amino-1-propanol (0.67 mL). The resulting mixture was heated to reflux with a Dean-Stark trap for 3 h under N₂ until complete formation of the imine was observed. The mixture was brought to RT followed by the addition of a solution of NaBH(OAC)₃ (2.0 g, 9.8 mmol) in AcOH (6 mL). The resulting mixture was stirred at RT and under N₂ for 56 h. The mixture was neutralized (pH 7.0) with a saturated solution of NaHCO₃ (aq) and extracted with CH₂Cl₂ (3x50mL). The aqueous layer was concentrated by rotary evaporation and the residue obtained was extracted with CH₂Cl₂ (3x50mL). The organic layers were combined, dried over MgSO₄, filtered and concentrated by rotary evaporation to afford the title compound as a pale yellow oil. EI-MS m/z 245 (M+H).

### Preparation AJ: (tert-Butoxy)-N-[(6-bromo(2-pyridyl))methyl]-N-(2-hydroxy-isopropyl)carboxamide

To a stirred solution of 2-[(6-bromo-2-pyridyl)-methyl]aminopropan-1-ol (0.55 g, 2.2 mmol) in dry CH₂Cl₂ (11 mL) was added di-tert-butyldicarbonate (0.51 g, 2.42 mmol). The resulting mixture was stirred at RT and under N₂ for 15 h. The mixture was concentrated by rotary evaporation and purified on silica gel (2:1 hexanes/EtOAc, 5:95 MeOH/CH₂Cl₂ and, 10:90 MeOH/CH₂Cl₂) as eluent to afford the title compound as an off-white oil. EI-MS m/z 345 (M+H).

### Preparation AK: (tert-Butoxy-N-[(6-bromo(2-pyridyl))-methyl]-N-(1-methyl-2-oxoethyl)carboxamide

To a dry flask was added oxalyl chloride (72 µL) followed by the addition of dry CH₂Cl₂ (2 mL). The resulting colorless solution was brought to -63°C (dry ice/CHCl₃) and a solution of DMSO (80 µL) in 0.5 mL dry CH₂Cl₂ was slowly added dropwise. A solution of (tert-butoxy)-N-[(6-bromo(2-pyridyl))methyl]-N-(2-hydroxyisopropyl)carboxamide (0.19 g, 0.55 mmol) in dry CH₂Cl₂ (2 mL), was added slowly drop wise. The resulting mixture was kept at -63°C and stirred for 30 min, followed by the slowly addition of a solution of TEA (0.31 mL) in dry CH₂Cl₂ (1 mL). The mixture was stirred at -63°C until all the starting material was consumed (checked by MS). The mixture was brought to -20°C, quenched with a saturated solution of NH₄Cl (aq) and diluted with EtOAc. The organic phase was separated and the aqueous phase was extracted with EtOAc (3x20ml). The organic layers were combined, dried over MgSO₄, filtered and concentrated by rotary evaporation to afford the title compound as a pale yellow semi-solid. EI-MS m/z 343 (M+H).

### Preparation AL: N-[2-(diethylamino)-isopropyl](tert-butoxy)-N-[(6-bromo(2-pyridyl))methyl]-carboxamide

To a stirred solution of (tert-butoxy-N-[(6-bromo(2-pyridyl))methyl]-N-(1-methyl-2-oxoethyl)-carboxamide (0.15 g, 0.44 mmol) in toluene (3 mL) was added DEA (0.2 mL). The resulting mixture was heated to reflux in a Dean-Stark trap under N₂ for 3 h. The mixture was brought to RT followed by the addition of a solution of NaBH(OAC)₃ (0.33 g, 1.54 mmol) in AcOH (6 mL). The yellow-solution was stirred at RT and under N₂ for 15 h. The mixture was diluted with EtOAc (20 ml) and washed with a saturated solution of NaHCO₃ (aq) (50 ml) . The organic phase was separated, dried over MgSO₄, filtered and concentrated by rotary evaporation to afford the title compound as a brown/ yellow oil. EI-MS m/z 400 (M+H).

### Preparation AM: N-[2-(diethylamino)isopropyl](tert-butoxy)-N-[(6-amino(2-pyridyl))methyl]-carboxamide

To a stirred solution of N-[2-(diethylamino)-isopropyl](tert-butoxy)-N-[(6-bromo(2-pyridyl))methyl]-carboxamide (80 mg 0.2 mmol) in IpOH (4 mL) in a sealed tube, was added NH₄OH (28-30%, 6 mL) followed by an excess of Cu. The resulting solution was heated under pressure at 90°C for 24 h. The mixture was brought to RT, diluted with H₂O (20 ml) and extracted with CHCl₃ (3×20mL). The organic layers were combined, dried over MgSO₄, filtered and concentrated by rotary evaporation to afford the title compound as a pale-yellow oil. EI-MS m/z 337 (M+H).

### Preparation AN: Methyl 2-[(6-bromo-2-pyridylmethyl)amino]-3-methyl-butyrate

To a stirred solution of L-valine methyl ester hydrochloride (0.54 g, 3.24 mmol) in dry toluene (15 mL) at 80°C was added DIEA (2.0 mL 11 mmol) followed by (6-bromo-2-pyridyl)formaldehyde (0.50 g, 2.70 mmol) . The resulting mixture was heated at 80°C for 3 h. The reaction was brought to RT and a solution of NaBH (OAc)₃ (1.4 g, 6.75 mmol) in glacial AcOH (4 mL) was added. The resulting mixture was stirred for 15 h and concentrated by rotary evaporation. The resulting yellow oil was dissolved in CH₂Cl₂ (100 ml), washed with a saturated solution of NaHCO₃ (aq) (50 mL), brine (50 ml), dried over Na₂SO₄, filtered, concentrated by rotary evaporation and purified by flash chromatography (2:1 hexanes/EtOAc) to afford the title compound as a pale-yellow oil. EI-MS m/z 301 (M+H).

### Preparation AO: 2-[(6-Bromo-2-pyridylmethyl)amino]-3-methyl-butanol

To a stirred solution of (tert-butoxy)-N-[(6-bromo(2-pyridyl))methyl]-N-[2-oxomethoxide-1-(methylethyl)-ethyl]carboxamide (0.47 g, 1.57 mmol) in dry toluene (25 mL) at -78°C was added dropwise diisobutylaluminum hydride 1.0 M solution in hexane (4.7 mL). The resulting brown-solution was stirred at - 78°C for 3 h, brought to RT and stirred until (tert-butoxy)-N-[(6-bromo(2-pyridyl))methyl]-N-[2-oxomethoxide-1-(methylethyl)ethyl]carboxamide was consumed. The organic layer was separated, dried over Na₂SO₄, filtered, concentrated by rotary evaporation and purified on silica gel (10:90 MeOH/CH₂Cl₂) to afford the title compound as a yellow oil. EI-MS m/z 273 (M+H).

### Preparation AP: tert Butyl (6-bromopyridin-2-ylmethyl)-(1-hydroxymethyl-2-methyl-propyl)-carbamate

To a stirred solution of 2-[(6-bromo-2-pyridylmethyl)amino]-3-methyl-butanol (0.30 g, 1.10 mmol) in CH₂Cl₂ (5 mL) was added di-tert-butyl dicarbonate (0.26 g, 1.21 mmol). The resulting solution was stirred for 15 h, concentrated by rotary evaporation and purified on silica gel (5:95 MeOH/CH₂Cl₂ and 10:90 MeOH/CH₂Cl₂) to afford the title compound as a pale yellow solid. EI-MS m/z 373 (M+H).

### Preparation AQ: tert Butyl (6-bromopyridin-2-ylmethyl)-(1-formyl-2-methyl-propyl) carbamate

To a flame-dried flask was added oxalyl chloride (70 µL) followed by the addition of dry CH₂Cl₂ (2 mL). The resulting colorless solution was brought to -63 °C (dry ice/CHCl₃) and a solution of DMSO (70 µL) in 0.5 mL dry CH₂Cl₂ was slowly added drop wise. The (tert-butoxy)-N-[(6-bromo(2-pyridyl))methyl]-N-[2-hydroxy -1-(methylethyl)ethyl]carboxamide (0.19 g, 0.51 mmol), previously dissolved in dry CH₂Cl₂ (2 mL), was added slowly dropwise. The resulting mixture was kept at - 63°C and stirred for 30 min followed by the slowly addition of a solution of TEA (0.3 mL) in dry CH₂Cl₂ (1 mL). The mixture was stirred at -63°C until all the starting material was consumed (checked by MS) (1.5 h). The mixture was brought to -20°C, quenched with a saturated solution of NH₄Cl (15 mL) and diluted with EtOAc (35 mL) . The organic phase was separated and the aqueous phase was extracted with EtOAc (3x30mL). The organic layers were combined, dried over MgSO₄, filtered and concentrated by rotary evaporation without further purification to afford the title compound as a yellow-semi solid. EI-MS m/z 371 (M+H).

### Preparation AR: tert-Butyl (6-bromopyridin-2-ylmethyl)-(1-diethylaminomethyl-2-methyl-propyl)carbamate

To a stirred solution of (tert-butoxy)-N-[(6-bromo(2-pyridyl))methyl]-N-[1-(methylethyl)-2-oxoethyl]carboxamide (0.17 g, 0.46 mmol) in toluene (5 mL) was added DEA (0.14 mL). The resulting mixture was heated to reflux in a Dean-Stark trap under N₂ for 3 h. The mixture was brought to RT followed by the addition of a solution of NaBH(OAc)₃ (0.34 g, 1.61 mmol) in glacial AcOH (6 mL). The yellow-solution was stirred at RT and under N₂ for 15 h. The mixture was diluted with EtOAc (20 mL) and washed with a saturated solution of NaHCO₃ (aq) (15 mL). The aqueous layer was separated and concentrated under reduced pressure. The solid obtained was extracted with CH₂Cl₂. The extracts were combined, dried over MgSO₄, filtered and, concentrated by rotary evaporation to afford the title compound as a pale yellow oil. EI-MS m/z 438 (M+H).

### Preparation AS: tert-Butyl (6-aminopyridin-2-ylmethyl)-(1-diethylaminomethyl-2-methyl-propyl)carbamate

To a stirred solution of N-{1-[(diethylamino)-methyl]-2-methylpropyl} (tert-butoxy) -N- [(6-bromo (2-pyridyl))methyl]carboxamide (5 mg, 0.012 mmol) in IpOH (5 mL) in a sealed tube, was added NH₄OH (28-30% 6 mL) followed by excess Cu. The resulting solution was heated under pressure at 90°C for 24 h. The mixture was brought to RT, diluted with H₂O (10 mL) and extracted with CHCl₃ (3×20mL). The organic layers were combined, dried over MgSO₄, filtered and concentrated by rotary evaporation to afford the title compound as a green oil. No purification was required. EI-MS m/z 365 (M+H).

### Preparation AT: 2-Bromo-6-(piperidin-1-ylmethyl)pyridine

To a stirred solution of 6-bromo-2-pyridine carboxaldehyde (5.05 g, 27 mmol) in anhydrous CH₂Cl₂ (200 ml) at RT, under N₂, piperidine (2.95 ml, 29 mmol) was added, followed by NaBH(OAc)₃ (11.51 g, 54 mmol) and AcOH (6.2 ml, 108 mmol) 30 min later. After 20 h, a 2M solution of Na₂CO₃(aq) (20 ml) was added. The mixture was vigorously stirred for an additional 30 min, washed successively with a saturated solution of NaHCO₃(aq) until the pH of the aqueous layer reached 7 (2x100ml), H₂O (100 ml) and brine (100 ml). The organic layer was separated, dried over MgSO₄, filtered and concentrated under reduced pressure to yield the title compound as a yellow oil. This was used crude in the next step. MS m/z: 255 (M+H), 257 (M+3).

### Preparation AU: 2-Amino-6-(piperidin-1-ylmethyl)pyridine

To a solution of 2-bromo-6-(piperidylmethyl)pyridine (5.21 g, 20 mmol) in propan-2-ol (30 ml) in a sealed tube at RT a catalytic amount of Cu (100 mg) and 28-30% NH₄OH (35 ml) were added. The stirred suspension was heated to 95°C for 40 h. After cooling to RT, the reaction mixture was diluted with H₂O (100 ml) and extracted with EtOAc (4x80ml). The organic layers were combined, then washed with H₂O (50 ml) followed by brine (50 ml). The organic layer was separated, dried over Na₂SO₄, filtered and concentrated under reduced pressure to yield the title compound (as a dark yellow oil. This was used as crude. MS m/z: 193 (M+H)+.

### Preparation AV: Ethyl 2-(4-aminosulfonylphenyl) thiazole-4-carboxylate

In an oven-dried, 100-mL, round-bottomed flask were placed 4-cyanobenzenesulphonamide (4.1 g, 22.50 mmol), TEA (5 mL) in pyridine (40 mL). H₂S was bubbled through this mixture for 1 h at RT. The reaction was diluted with EtOAc (125 mL) and H₂O (50 mL). The phases were separated, and the organic layer was washed with H₂O (4x25 mL) and brine (15 mL), dried over MgSO₄, and concentrated *in vacuo* to afford the crude thiobenzamide as an oily solid; MS m/z: 217 (M+H). In an oven-dried, 100-mL, round-bottomed flask were placed the crude thiobenzamide, ethyl bromopyruvate (3.0 mL, 23.66 mmol) in EtOH (40 mL). The reaction was heated to 75°C for 12 h, then cooled to RT. The mixture was concentrated *in vacuo* to give the crude sulfonamide as a yellow solid which was filtered, washed with H₂O (1x10 mL) and Et₂O (4×10 mL) to afford the title compound as a yellow solid. MS m/z: 313 (M+H).

### Preparation AW: 2-(4-Aminosulfonlphenyl)thiazole-4-carboxylic acid

In an oven-dried, 100-mL, round-bottomed flask was placed ethyl 2-(4-aminosulfonylphenyl)thiazole-4-carboxylate (1300 mg, 4.2 mmol), LiOH monohydrate (350 mg, 8.3 mmol) in MeOH (40 mL) and H₂O (4 mL). The solution was heated to 75°C for 3 h, cooled to RT, and concentrated. The resulted yellow solid was dissolved in H₂O (10 mL), extracted with EtOAc (1×15 mL). The aqueous layer was acidified with 2N aqueous HCl (4.15 mL). The precipitate was filtered, and washed with H₂O (10 mL) to afford the title compound as a light-yellow solid. M5 m/z: 285 (M+H).

### Preparation AX: 2-(4-(4-morpholinyl)sulfonylphenyl)-thiazole-4-carboxylic acid

In a manner similar to that described for the preparation of 2-(4-aminosulfonylphenyl)thiazole-4-carboxylic acid, 460 mg of 4-(morpholinosulfonyl)-benzonitrile was treated with H₂S, ethyl bromopyruvate, and LiOH successively to give the title compound. MS m/z: 355 (M+H).

### Preparation AY: 2-(4-Boc-aminophenyl)-thiazole-4-carboxylic acid

In a manner similar to that described for the preparation of 2-(4-aminosulfonylphenyl)thiazole-4-carboxylic acid, 4-[(1,1-di-methylethoxy) carbonyl] - aminobenzonitrile was treated with H₂S, ethyl bromopyruvate, and LiOH successively to give the title compound. MS m/z: 321 (M+H).

### Preparation AZ: Ethyl 2-(phenoxy)thiazole-4-carboxylate

A mixture of the bromothiazole (1.03 g, 4.36 mmol) and phenol (10.0 g, 106 mmol) was stirred at 180°C for 1 h, cooled to RT, diluted with 100 ml of EtOAc, washed with 1N NaOH (40x3), H₂O, and brine, then dried over MgSO₄, and concentrated in vacuo to yield a light yellow residue. Purification over silica gel (gradient, 5% to 10% EtOAc/hexanes) provided the title compound. MS m/z: 250 (M+H)+⁺.

### Preparation BA: 2-(Phenoxy)thiazol-4-ylcarbonylazide

TEA (0.17 ml, 1.20 mmol) was added to a solution of the thiazole carboxylic acid (0.13 g, 0.59 mmol) in 10 ml of THF at 0°c. The mixture was stirred at 0°C for 20 min whereupon ethyl chloroformate (0.065 ml, 0.65 mmol) was added. After the mixture was stirred for 30 min, a solution of NaN₃ (0.043 g, 0.65 mmol) in 3 ml of H₂O was added, the reaction was stirred for 30 min, then warmed to RT, diluted with 25 ml of H₂O, and extracted with EtOAc. The combined organic portions were washed with brine, dried over MgSO₄, filtered, and removal of the solvents in vacuo yielded the title compound as a light brownish solid. MS m/z: 247 (M+H).

### Preparation BB: 6-Chloro-thionicotinamide

To a solution of the 4-chloronicotinamide (5 g, 31.9 mmol) and dry THF (200 mL) was added P₂S₅ (15.6 g, 35.1 mmol) and Na₂CO3 (3.7 g, 35. 1 mmol). The mixture was heated at reflux for 1.5 h, cooled reaction mixture to RT and filtered off a yellow solid. The filtrate was extracted with EtOAc, washed with H₂O and brine; dried (MgSO₄) then concentrated in vacuo to give the title compound as a yellow solid. MS m/z: 173.0 (M+H).

### Preparation BC: Ethyl 2-(6-chloro-3-pyridyl)thiazole-4-carboxylate

To a mixture of the 4-chloro-thionicotinamide (5.5 g, 31.9 mmol) and EtOH (300 mL) was added bromo-ethyl-pyruvate (4.4 mL, 35.1 mmol). The mixture was heated at reflux for 15 h, cooled and concentrated in vacuo to afford a yellow solid/orange oil. The oil was diluted with EtOAc and filtered off yellow solid. The filtrate was filtered through Celite® and concentrated in vacuo to give a dark yellow oil. The oil was diluted with 2% MeOH/CH₂Cl₂ and filtered through a pad of silica gel (150 mL). Elution with 2% MeOH/CH₂Cl₂ (500 mL), followed by concentration in vacuo afforded the title compound as a yellow crystalline solid. MS m/z: 269.1 (M+H).

### Preparation BD: 2-(6-Methoxy-3-pyridyl)thiazole-4-carboxylic acid

To a solution of the ethyl 2-(6-chloro-3-pyridyl)thiazole-4-carboxylate (0.61 g, 2.3 mmol) and MeOH (50 mL) was added solid NaOMe (135 mg, 2.5 mmol) and stirred at RT. After 3 h the ethyl ester transesterified to the methyl ester. NaOMe (1 eq, 135 mg) was added and the mixture was heated to reflux. After 15 h, the ester hydrolyzed to the 2-(6-chloro-3-pyridyl)thiazole carboxylic acid. NaOMe (2 eq) was added and the reaction was heated at reflux for 18 h. The mixture was acidified to pH 5 with concentrated HCl, extracted with EtOAc, washed with H₂O and brine; dried (MgSO₄) and concentrated in vacuo to give the desired carboxylic acid as a yellow solid. MS m/z: 237.1 (M+H).

### Preparation BE: 2-(2-Chloropyridin-4-yl)thiazole-4-carbonyl azide

A mixture of 3-(3-chloro-4-pyridyl)-4-thiazole carboxylic acid (0.6 g, 2.5 mmol) and dry THF (20 mL) was cooled to 0°C with stirring. TEA (0.7 mL, 5.0 mmol) was added and the reaction mixture was stirred for 20 min. Ethyl chloroformate (0.24 mL, 2.5 mmol) was added and the solution was stirred for 30 min. A solution of NaN₃ (174 mg, 2.7 mmol) in 3 mL of H₂O was added and the reaction mixture was warmed to RT. After 30 min, 10 mL of H₂O was added and the mixture was extracted with EtOAc (3x), dried (MgSO₄) and concentrated in vacuo to give the title compound as a pink solid. MS m/z: 266.0 (M+H)+

### Preparation BF: Ethyl 2-(3-methoxyphenyl)-thiazole-4-carboxylate

A suspension of 3-methoxyphenyl boronic acid (0.25 g, 1.65 mmol), ethyl 2-bromothiazole-4-carboxylate (0.33 g, 1.4 mmol), PdCl₂(dppf)₂ (0.11 g) and 2M Na₂CO₃ (aq) (2 mL) in DME (10 mL) was heated to reflux for 20 h. The mixture was cooled to RT, filtered, concentrated by rotary evaporation and purified on silica gel (6:1 hexanes/EtOAc and 4:1 hexanes/EtOAc) to afford the title compound as a light-brown oil. EI-MS m/z 264 (M+H).

### Preparation BG: 2-(3-Methoxyphenyl)thiazole-4-carboxylic acid

To a stirred solution of the ethyl 2-(3-methoxyphenyl)thiazole-4-carboxylate (0.23 g, 0.87 mmol) in EtOH (10 mL) was added 1N NaOH (aq) (5 mL). The resulting mixture was heated to reflux until the starting material was consumed (2 h). The mixture was cooled to RT, acidified with 1N HCl (aq) and concentrated by rotary evaporation. The residue was extracted with CH₂Cl₂ (3x15mL). The extracts were combined, dried over MgSO₄, filtered and concentrated by rotary evaporation to afford the title compound as an off-white solid. EI-MS m/z 236 (M+H).

### Preparation BH: Ethyl 2-(2-methoxyphenyl)-thiazole-4-carboxylate

A suspension of 2-methoxyphenyl boronic acid (0.25 g, 1.65 mmol), ethyl 2-bromothiazole-4-carboxylate (0.33 g, 1.4 mmol), PdCl₂ (dppf)₂ (0.11 g, 0.14 mmol) and 2M Na₂CO₃(aq) (2 mL) in DME (10 mL) was heated at reflux for 20 h, cooled to RT, filtered, concentrated by rotary evaporation and purified on silica gel (6:1 hexanes/EtOAc and 4:1 hexanes/EtOAc) to afford the title compound as a light-brown oil. EI-MS m/z 264 (M+H).

### Preparation BI: 2-(2-Methoxyphenyl)thiazole-4-carboxylic acid

To a stirred solution of ethyl 2-(2-methoxyphenyl)thiazole-4-carboxylate (0.27 g, 1.03 mmol) in EtOH (10 mL) was added 1N NaOH (aq) (5 mL). The resulting mixture was heated to reflux for 2 h. The mixture was cooled to RT, acidified with 1N HCl (aq) and concentrated by rotary evaporation. The residue was extracted with CH₂Cl₂ (3x15mL). The extracts were combined, dried over MgSO₄, filtered and concentrated by rotary evaporation to afford the title compound as an off-white solid. EI-MS m/z 236 (M+H).

### Preparation BJ: 2-[(4-Methoxyphenoxy)methyl]thiazole-4-carboxylic acid

To a stirred solution of ethyl 2-(4-methoxyphenoxy)methyl]thiazole4-carboxylate (0.10 g, 0.34 mmol) in EtOH (5 mL) was added 1N NaOH (2.0 mL) and was heated to reflux until the starting material was consumed (2 h). The mixture was brought to RT, acidified with 1N HCl (pH 4.0) and concentrated by rotary evaporation. The residue obtained was partitioned between EtOAc (50 mL) and H₂O (30 mL). The organic phase was separated, dried over MgSO₄, filtered and concentrated by rotary evaporation to afford the title compound as a white solid. EI-MS m/z 266 (M+H).

### Preparation BK: Ethyl 2-aminothiazole-4-carboxylate

To a stirred suspension of thiourea (23.03 g, 0.30 mol) in EtOH (320 ml) at RT, under N₂, ethyl bromopyruvate (59.0 g, 0.30 mol) was added dropwise. The solution was then heated at 45°C for 12 h. After cooling to RT the reaction flask was placed in the fridge overnight. The resulting solid was filtered, washed with cold EtOH (3x50ml) then air dried to yield the title compound as a pale yellow amorphous solid. MS m/z: 173 (M+H).

### Preparation BL: 2-Bromothiazole-4-carboxylic acid

To a well stirred suspension of ethyl 2-aminothiazole-4-carboxylate hydrobromide (29.99 g, 0.17 mol) in 16% HBr(aq) (400 ml) at 0°C, a solution of NaNO₂ (12.49 g, 0.18 mol) in H₂O (22 ml) was added dropwise. The mixture was maintained at 0°C for an additional 35 min then CuBr (28.23 g, 0.20 mol) and an additional volume of 16% HBr(aq) (150 ml) were added. The ice bath was removed and the suspension heated to 70°C for 1 hr. The mixture was filtered hot. The filtrate was saturated with NaCl then extracted with EtOAc (2×400ml). The combined organic layers were dried over MgSO₄, filtered and concentrated under reduced pressure. The crude brown oil/solid residue was used directly in the next step. A solution of the brown residue in EtOH (100 ml) and 1M NaOH (aq) (367 ml, 0.36 mol) was stirred and heated at reflux for 1 h. The reaction mixture was filtered then extracted with EtOAc (100 ml). The aqueous layer was separated and concentrated under reduced pressure to remove the remaining EtOH. The aqueous solution was acidified to pH 1 with 2N HCl(aq). The solid was filtered off and air dried to yield the title compound as a beige amorphous solid. MS m/z: 208 (M+H) 210 (M+3).

### Preparation BM: Ethyl 2-(2,6-dichloro-4-pyridyl)thiazole-4-carboxylate

2,6-Dichloropyridine-4-thiocarboxamide (1.0 g, 4.83 mmol) was dissolved in dry 1,4-dioxane followed by adding ethyl bromopyruvate (0.9 mL, 7.24 mmol) and pyridine (0.4 mL, 4.83 mmol). The resulting mixture was heated to reflux under N₂ for 5 h. After cooling to RT, solvent was removed. The residue was extracted with CHCl₃. The organic layer was washed with H₂O and brine, dried over MgSO₄, and concentrated to give a brownish solid. This crude was purified by chromatography on silica gel. Elution with hexane:acetone (90:10) gave a title compound as yellow solid. MS *m*/*z:* 303 (M+H). Calc'd. for C₁₁H₈Cl₂N₂O₂S - 303.16.

### Preparation BN: 2-(2,6-Dichloro-4-pyridyl)thiazole-4-carboxylic acid

2-(2,6-Dichloropyridin-4-yl)-ethylthiazolo-4-carboxylate (500 mg, 1.65 mmol) was dissolved in MeOH (10 mL) followed by adding 1N NaOH (2.5 mL, 2.47 mmol). The resulting mixture was stirred at RT for 4 h. The pH was adjusted to 5 using 1N HCl. The solvent was removed *in vacuo* and the residue was partitioned between EtOAc and H₂O. The aqueous layer was extracted more with EtOAc. The combined organic layers was dried over MgSO₄ and concentrated to give a white solid. MS *m*/*z*: 275.1 (M+H). Calc'd. for C₃H₄Cl₂N₂O₂S - 275.11.

### Preparation BO: Ethyl 6-[2-(2,2,2-trifluoroethoxy)-3-pyridyl]thiazole-4-carboxylate

6-(2,2,2-Trifluoroethoxy)pyridine-3-thiocarboxamide (800 mg, 3.4 mmol), ethyl bromopyruvate (0.9 mL, 6.8 mmol), and pyridine (0.3 mL, 3.4 mmol) were heated at reflux in dry 1,4-dioxane (20 mL) to yield title compound as pale yellow solid. MS m/z: 333.1 (M+H). Calc'd. for C₁₃H₁₁F₃N₂O₃S - 332.3.

### Preparation BP: 6-[2-(2,2,2-trifluoroethoxy)-3-pyridyl]thiazole-4-carboxylic acid

Ethyl 6-[2-(2,2,2-trifluoroethoxy)-3-pyridyl]thiazole-4-carboxylate (750 mg, 2.25 mmol) and 1N NaOH (3.4 mL, 3.4 mmol) were dissolved in MeOH (10 mL) to afford the title compound as a white solid. MS m/z: 305.1 (M+H). Calc'd. for C₁₁H₇F₃N₂O₃S - 304.25.

### Preparation BQ: 2-(Phenoxy)thiazole-4-carboxylic acid

A mixture of ethyl 2-phenoxythiazole-4-carboxylate (0.17 g, 0.68 mmol) and LiOH monohydrate (0.14 g, 3.40 mmol) in 2 ml of MeOH, 2 ml of H₂O, and 2 ml of THF was stirred at RT overnight, the solvents were removed in vacuo and the residue was diluted with water. The aqueous mixture was acidified with 1N HCl (aq) to pH=1-2, then extracted with EtOAc, the combined organic portions were washed with brine, dried over MgSO₄, filtered, removal of the solvents in vacuo yielded the title compound as a white solid. EI-MS = 222.4 (M+H)+. Calc'd for C₁₀H₇NO₃S: 221.01.

### Preparation BR: 3-(3-Nitrophenyl)pyridine

To a 1-iodo-3-nitrobenzene (1.0 g, 4.01 mmol) in dry DME (20 mL) was added pyridine-3-boronic acid (641 mg, 5.22 mmol), PdCl₂dppf (327 mg, 0.40 mmol), and 2M Na₂CO₃ (3.0 mL). The resulting mixture was heated to reflux under N₂ for 15 h. Solvent was separated from inorganic solid by filtration. The solvent was removed and the residue was extracted with CHCl₃. The organic layer was washed with water, brine, and dried over MgSO₄. The solvent was removed to give dark brown solid which was purified by chromatography on silica gel. Elution with Hexane:acetone mixture (80:20) gave the final compound as a tan solid. MS m/z: 201.3 (M+H). Calc' d. for C₁₁H₈N₂O₂ - 200.23.

### Preparation BS: 3-(3-Aminophenyl)pyridine

To a prehydrogenated solution of Pd(OH)₂ (298 mg, 2.12 mmol) in EtOH (10 mL) was added 3-(3-pyrid-1-yl)-1-nitrobenzene (440 mg, 2.12 mmol) in EtOH (10 mL). The resulting mixture was stirred at RT under H₂ for 2 h. Solvent was separated from Pd(OH)₂ by filtration through Celite®. Solvent was then removed to give final compound as pale yellow solid. MS m/z: 171.3 (M+H). Calc'd. for C₁₁H₁₀N₂ - 170.22.

### Example 1

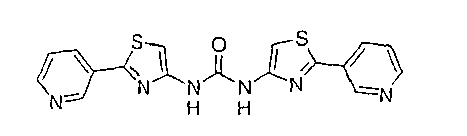

### N,N'-bis [2-(3-Pyridinyl)-4-thiazolyl] urea

To a 50 mL round bottomed flask were added 0.106 g (0.458 mmol) of 2-(3-pyridinyl)-4-thiazolylcarbonylazide, toluene (10 mL) and 5 drops of H₂O. The mixture was heated at 95°C for 4 h then cooled to RT. The precipitate that formed was filtered, washed with a minimum amount of toluene and dried under high vacuum to give the product as a pale yellow solid. MS *m*/*z:* 381.5 (M+H) . Calc'd. for C₁₇H₁₂N₆OS₂ - 380.453.

### Example 2

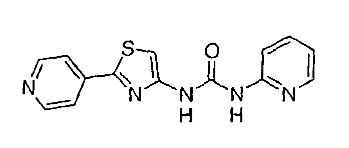

### N-[2-(4-Pyridinyl)-4-thiazolyl]-N'-2-pyridinylurea

To a solution of 2-(4-pyridinyl)-4-thiazolylcarbonylazide (60 mg, 0.260 mmol) in 10 mL toluene was added 2-aminopyridine (35 mg, 0.372 mmol). The mixture was heated at 95°C for 18 h then cooled to RT and filtered. The precipitate was washed with toluene (3mL) and dried under high vacuum to give the product as a pale yellow solid. MS m/z: 298.5 (M+H). Calc'd. for C₁₄H₁₂N₅OS - 297.341.

### Example 3

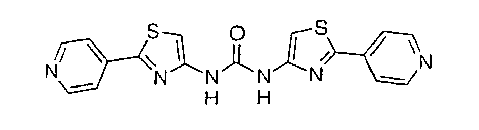

### N,N'-bis [2-(4-Pyridinyl)-4-thiazolyl] urea

In a manner similar to that described in Example 2, 2-(4-pyridinyl)-4-thiazolylcarbonylazide (130 mg, 0.562 mmol) was heated in toluene (10 mL) containing 4 drops of H₂O to give the product as a pale yellow solid. MS m/z: 381.5 (M+H). Calc' d. for C₁₇H₁₂N₆OS₂₋380.453.

### Example 4

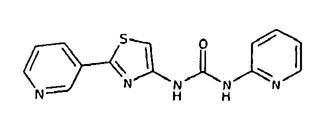

### N-[2-(3-Pyridinyl)-4-thiazolyl]-N'-2-pyridinylurea

In a manner similar to that described in Example 2, 2-(3-pyridinyl)-4-thiazolylcarbonylazide (48 mg, 0.208 mmol) and 2-aminopyridine (24 mg, 0.255 mmol) were heated in toluene (10 mL) to give the product as a pale yellow solid. MS *m*/*z:* 298.4 (M+H). Calc'd. for C₁₄H₁₁N₅OS - 297.341.

### Example 5

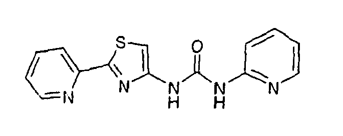

### N-[2-(2-Pyridinyl)-4-thiazolyl]-N'-2-pyridinylurea

2-(2-Pyridinyl)-4-thiazolylcarbonylazide (200 mg, 0.87mmol) and 2-aminopyridine (318 mg, 2.6 mmol) were heated in toluene (10 mL) at 100°C for 14 h. After cooling to RT, the solids were collected by filtration and washed first with toluene (2x20 mL) followed by Et₂O (2×10mL) and cold EtOAc (3x5 mL). The solid was recrystallized from EtOAc to afford the product as an off-white solid: m.p. 233-235°C. MS m/z: 298 (M+H). Calc' d for C₁₄H₁₁N₅OS 297.341.

### Example 6

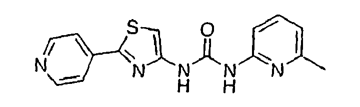

### N-[2-(4-Pyridinyl)-4-thiazolyl]-N'-2-(6-methylpyridinyl)urea

In a manner similar to that described in Example 2, 2-(4-pyridinyl)-4-thiazolylcarbonylazide (69 mg, 0.298 mmol) and 2-amino-6-methylpyridine (101 mg, 0.934 mmol) were heated in toluene (10 mL) to give the product as a pale yellow solid. MS *m*/*z:* 312.5 (M+H). Calc'd. for C₁₅H₁₃N₅OS - 311.368.

### Example 7

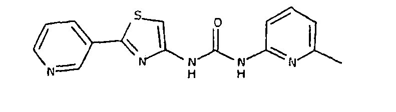

### N-[2-(3-Pyridinyl)-4-thiazolyl]-N'-2-(6-methylpyridinyl)urea

In a manner similar to that described in Example 2, 2-(3-pyridinyl)-4-thiazolylcarbonylazide (78 mg, 0.337 mmol) and 2-amino-6-methylpyridine (101 mg, 0.934 mmol) were heated in toluene (10 mL) to give the product as a pale yellow solid. MS m/z: 312.2 (M+H). Calc'd. for C₁₅H₁₃N₅OS - 311.368.

### Example 8

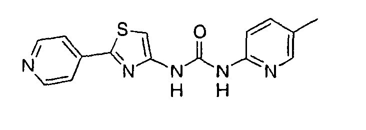

### N-[2-(4-Pyridinyl)-4-thiazolyl]-N'-2-(5-methylpyridinyl)urea

In a manner similar to that described in Example 2, 2-(4-pyridinyl)-4-thiazolylcarbonylazide (72 mg, 0.311 mmol) and 2-amino-5-methylpyridine (106 mg, 0.981 mmol) were heated in toluene (10 mL) to give the product as a pale yellow solid. MS m/z: 312.5 (M+H). Calc'd. for C₁₅H₁₃N₆OS - 311.368.

### Example 9

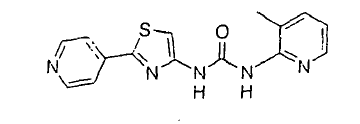

### N-[2-(4-Pyridinyl)-4-thiazolyl]-N'-2-(3-methylpyridinyl)urea

In a manner similar to that described in Example 2, 2-(4-pyridinyl)-4-thiazolylcarbonylazide (135 mg, 0.584 mmol) and 2-amino-3-methylpyridine (200 mg, 1.98 mmol) were heated in toluene (10 mL) to give the product as a pale yellow solid. MS m/z: 312.4 (M+H). Calc' d. for C₁₅H₁₃N₅OS - 311.368.

### Example 10

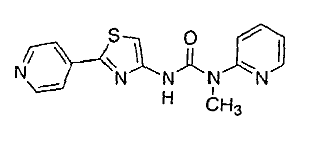

### N-[2-(4-Pyridinyl)-4-thiazolyl]-N'-2-pyridinyl-N'-methylurea

In a manner similar to that described in Example 2, 2-(4-pyridinyl)-4-thiazolylcarbonylazide (71 mg, 0.310 mmol) and 2-methylaminopyridine (210 mg, 1.94 mmol) were heated in toluene (7 mL) to give the product as pale yellow crystals. MS *m*/*z:* 312.5 (M+H). Calc'd. for C₁₅H₁₃N₅OS - 311.368.

### Example 11

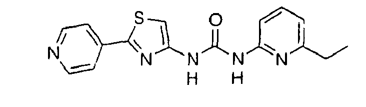

### N-[2-(4-Pyridinyl)-4-thiazolyl]-N'-2-(6-ethylpyridinyl)urea

In a manner similar to that described in Example 2, 2-(4-pyridinyl)-4-thiazolylcarbonylazide (75 mg, 0.324 mmol) and 2-amino-6-ethylpyridine (200 mg, 1.63 mmol) were heated in toluene (8 mL) to give the product as a pale yellow solid. MS m/z: 326.5 (M+H). Calc'd. for C₁₆H₁₅N₅OS - 325.395.

### Example 12

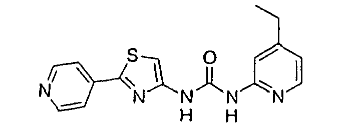

### N-[2-(4-Pyridinyl)-4-thiazolyl]-N'-2-(4-ethylpyridinyl)urea

In a manner similar to that described in Example 2, 2-(9-pyridinyl)-4-thiazolylcarbonylazide (82 mg, 0.355 mmol) and 2-amino-4-ethylpyridine (106 mg, 0.867 mmol) were heated in toluene (10 mL) to give the product as a pale yellow solid. MS m/z: 326.5 (M+H). Calc' d. for C₁₆H₁₅N₅OS - 325.395.

### Example 13

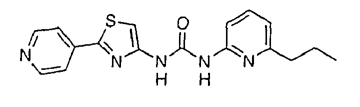

### N-(2-(4-Pyridinyl)-4-thiazolyl]-N'-2-(6-propylpyridinyl)urea

In a manner similar to that described in Example 3, 2-(4-pyridinyl)-4-thiazolylcarbonylazide (89 mg, 0.385 mmol) and 2-amino-6-(n-propyl)pyridine (171 mg, 1.25 mmol) were heated in toluene (10 mL) to give the product as a pale yellow solid. MS *m*/*z*: 339.4 (M+H). Calc'd. for C₁₇H₁₇N₅OS - 339.422.

### Example 14

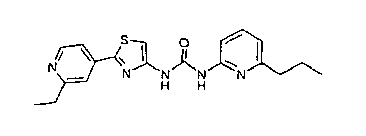

### N-(2-(2-Ethyl-4-pyridinyl)-4-thiazolyl]-N'-2-(6-propylpyridinyl)urea

In a manner similar to that described in Example 6, 2-(4-(2-ethyl)-pyridinyl)-4-thiazolylcarbonylazide (460mg, 1.77mmol) and 2-amino-6-(n-propyl)pyridine (483mg, 3.55 mmol) were heated in toluene (20 mL) at 100°C for 14 h. After cooling to RT, the solids were collected by filtration and washed first with toluene (2 × 20 mL) followed by EtOAc:Et₂O (4:1) (4 × 20 mL) to give the product as an off-white solid: m.p. 204-206°C. MS m/z: 363 (M+H). Calc'd. for C₁₉H₂₂N₅OS - 367.476.

### Example 15

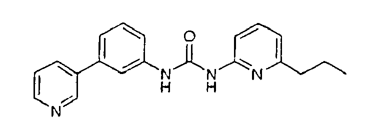

### N-[3-(3-Pyridinyl)phenyl]-N'-2-(6-propylpyridinyl)urea

To a suspended anhydrous solution of 3-pyridylaniline (90 mg, 0.53 mmol) in dry toluene (4 mL) was added phosgene (0.36 mL, 0.69 mmol, 20% in toluene) followed by DIEA (0.20 mL, 1.05 mmol) under an atmosphere of argon. After stirring for 0.5 h at RT, 2-amino-6-n-propylpyridine (72 mg, 0.53 mmol) in dry toluene (4 mL) was added dropwise into the mixture. The resulting mixture was stirred at RT for 18 h. The organic solvent was removed under vacuum. The residue was purified by chromatography on flash silica gel using 2% MeOH/CH₂Cl₂ as eluant to obtain the final urea as an off-white solid. MS m/z :333.4 (M+H). Calc'd. for C₂₀H₂₀N₄O - 332.405.

### Example 16

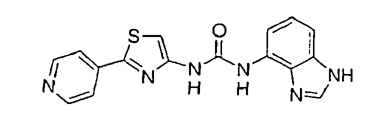

### N-[2-(4-Pyridinyl)-4-thiazolyl]-N'-4-benzimidazolylurea

In a manner similar to that described in Example 2, 2-(4-pyridinyl)-4-thiazolylcarbonylazide (32 mg, 0.138 mmol) and 4-aminobenzimidazole (32 mg, 0.240 mmol) were heated in toluene (8 mL). The crude product was recrystallized with CH₃CN:MeOH (- 10:1) to give the product as a pale brown solid. MS *m*/*z*: 337.5 (M+H). Calc'd. for C₁₆H₁₂N₆OS - 336.378.

### Example 17

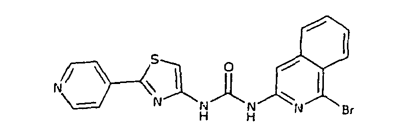

### N-[2-(4-Pyridinyl)-4-thiazolyl]-N'-3-(1-bromoisoquinolinyl)urea

In a manner similar to that described in Example 2, 2-(4-pyridinyl)-4-thiazolylcarbonylazide (61 mg, 0.264 mmol) and 3-amino-1-bromo-isoquinoline (120 mg, 0.538 mmol) were heated in toluene (10 mL) to give the product as a pale yellow solid. MS *m*/*z:* 427.2 (M+H). Calc'd. for C₁₈H₁₂BrN₅OS - 426.297.

### Example 18

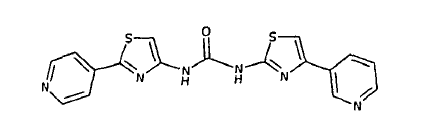

### N-[2-(4-Pyridinyl)-4-thiazolyl]-N'-[4-(3-pyridinyl)-2-thiazolyl] urea

In a manner similar to that described in Example 2, 2-(4-pyridinyl)-4-thiazolylcarbonylazide (36 mg, 0.298 mmol) and 2-amino-4-(3-pyridyl)-thiazole (29 mg, 163 mmol) were heated in toluene (10 mL) to give the product as a pale yellow solid. MS m/z: 381.5 (M+H). Calc. for C₁₇H₂₂N₆OS₂ - 380.453.

### Example 19

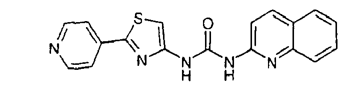

### N-[2-(4-Pyridinyl)-4-thiazolyl]-N'-2-quinolinylurea

In a manner similar to that described in Example 2, 2-(4-pyridinyl)-4-thiazolylcarbonylaside (38 mg, 0.164 mmol) and 2-aminoquinoline (53 mg, 0.370 mmol) were heated in toluene (10 mL) to give the product as a pale yellow solid. MS m/z: 348.4 (M+H). Calc. for C₁₈H₁₃N₅OS - 347.401.

### Example 20

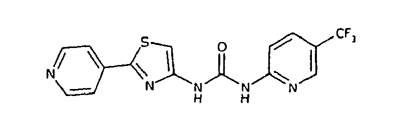

### N-[2-(4-Pyridinyl)-4-thiazolyl]-N'-2-(5-trifluoromethylpyridinyl)urea

In a manner similar to that described in Example 2, 2-(4-pyridinyl)-4-thiazolylcarbonylazide (40 mg, 0.173 mmol) and 2-amino-5-trifluoromethylpyridine (165 mg, 1.02 mmol) were heated in 10 mL toluene to give the product as a pale yellow solid. MS *m*/*z*: 366.3 (M+H). Calc'd. for C₁₅H₁₀F₃N₅OS - 365.339.

### Example 21

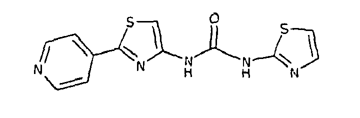

### N-[2-(4-Pyridinyl)-9-thiazolyl]-N'-2-thiazolylurea

In a manner similar to that described in Example 2, 2-(4-pyridinyl)-4-thiazolylcarbonylazide (70 mg, 0.303 mmol) and 2-aminothiazole (38 mg, 0.38 mmol) were heated in toluene (12 mL) to give the product as a pale yellow solid. MS *m*/*z:* 304.4 (M+H). Calc'd. for C₁₂H₉N₅OS₂ - 303.366.

### Example 22

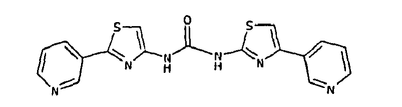

### N-[2-(3-Pyridinyl)-4-thiazolyl]-N'-[4-(3-pyridinyl)-2-thiazolyl] urea

In a manner similar to that described in Example 2, 2-(3-pyridinyl)-4-thiazolylcarbonylazide (36 mg, 0.156 mmol) and 2-amino-4-(3-pyridinyl)thiazole(30 mg, 0.169 mmol) were heated in toluene (8 mL) to give the product as a pale yellow solid. MS m/z: 381.5 (M+H). Calc'd. for C₁₇H₁₂N₆OS₂ - 380.453.

### Example 23

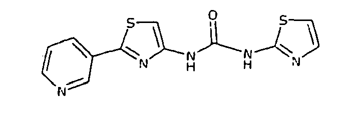

### N-[2-(3-Pyridinyl)-4-thiazolyl]-N'-2-thiazolylurea

In a manner similar to that described in Example 2, 2-(3-pyridinyl)-4-thiazolylcarbonylazide (59 mg, 0.255 mmol) and 2-aminothiazole (27 mg, 268 mmol) were heated in toluene (10 mL) to give the product as a pale yellow solid. MS m/z: 304.3 (M+H). Calc'd. for C₁₂H₉N₆OS₂ - 303.366.

### Example 24

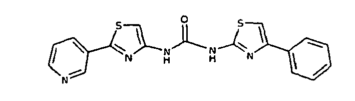

### N-[2-(3-Pyridinyl)-4-thiazolyl]-N'-[4-phenyl-2-thiazolyl]urea

In a manner similar to that described in Example 2, 2-(3-pyridinyl)-4-thiazolylcarbonylazide (49 mg, 0.211 mmol) and 2-amino-4-phenylthiazole (39 mg, 0.218 mmol) were heated in toluene (10 mL) to give the product as a pale yellow solid. MS m/z: 380.5 (M+H). Calc'd. for C₁₈H₁₃N₅OS₂ - 379.465.

### Example 25

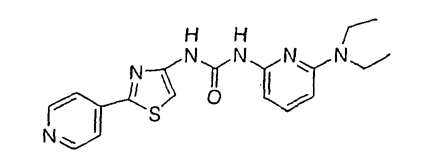

### N-[2-(4-Pyridinyl)-4-thiazolyl]-N'-2-[6-(N",N"-diethylamino)pyridinyl].urea

A mixture of 2-(4-pyridinyl)-4-thiazolylcarbonylazide (100 mg, 0.43 mmol) and 2-amino-6-(N,N-diethylamino)pyridine (150 mg, 0.91 mmol) in toluene (3 mL) was heated at 70°C for 1 h, and then at 80°C for 5 h. After the mixture was cooled to RT the solvent was removed *in vacuo* and the crude product was purified by chromatography on silica gel (1:10 MeOH(NH₃)/CH₂Cl₂) to give N-[2-(4-pyridinyl)-4-thiazolyl]-N'-2-[6-(N",N"-diethylamino)pyridinyl]urea. MS m/z: 369 (M+1). Calc'd. for C₁₈H₂₀N₆OS - 368.463.

### Example 26

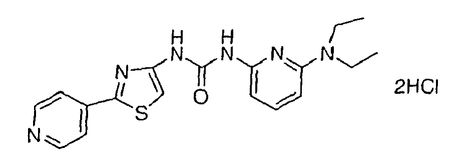

### N-[2-(4-Pyridinyl)-4-thiazolyl]-N'-2-[6-(N",N"-diethylamino)pyridinyl]urea hydrochloride

N-[2-(4-Pyridinyl)-4-thiazolyl}-N'-2-(6-(N",N"-diethylamino)pyridinyl]urea (Example 25) was dissolved in 5 ml of MeOH/CH₂Cl₂ (1:1) and (1 M) HCl (8 mL) in Et₂O solution was added. The solvents were removed in vacuo to afford the title salt as a yellow solid.

### Example 27

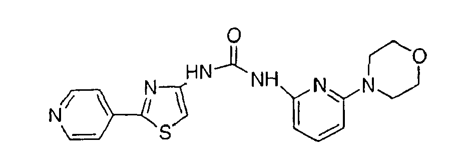

### N-[2-(4-Pyridinyl)-4-thiazolyl]-N'-2-[6-(4-morpholinyl)pyridinyl]urea

A mixture of 2-(4-pyridinyl)-4- thiazolylcarbonylazide (100 mg, 0.43 mmol) and 2-amino-6-(4-morpholinyl)pyridine (150 mg, 0.84 mmol) in toluene (5 mL) was heated at 80°C for 5 h. After the mixture was cooled to RT the solvent was removed in vacuo and the crude product was purified by chromatography on silica gel (1:10 MeOH(NH₃)/CH₂Cl₂) to afford the title compound as a light yellow solid. MS m/z: 383 (M+1). Calc'd. for C₁₈H₂₈N₆O₂S - 382.446.

### Example 28

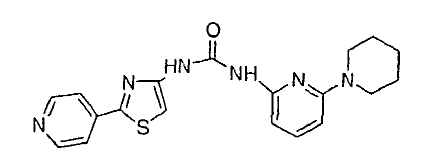

### N-[2-(4-Pyridinyl)-4-thiazolyl]-N'-2-[6-(1-piperdinyl)pyridinyl]urea

A mixture of 2-(4-pyridinyl)-4-thiazolylcarbonylazide (100 mg, 0.43 mmol) and 2-amino-6-(1-piperidinyl)pyridine (100 mg, 0.56 mmol) in toluene (3 mL) was heated at 80°C for 4 h. After cooling to RT, H₂O was added and the mixture was extracted with EtOAc (3x80 mL). The combined organic layers were washed with brine, dried over Na₂SO₄, filtered and concentrated *in vacuo.* The crude product was purified by chromatography on silica gel (1:20 MeOH/CH₂Cl₂) to afford the title compound as a light yellow solid. MS m/z: 381 (M+1). Calc' d for C₁₉H₂₀N₆OS - 380.475.

### Example 29

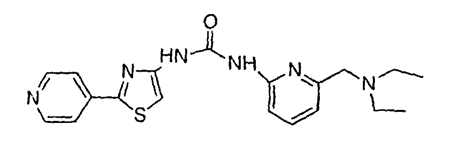

### N-[2-(4-Pyridinyl)-4-thiazolyl]-N'-2-[6-(N",N"-diethylaminomethylamino)pyridinyl]urea

A mixture of 2-(4-pyridinyl)-4-thiazolylcarbonylazide (100 mg, 0.43 mmol) and 2-amino-6-(N,N-diethylaminomethyl)pyridine (150 mg, 0.84 mmol) in toluene (5 mL) was heated at 80°C for 5 h. After the mixture was cooled to RT the solvent was removed *in vacuo* and the crude product was purified by chromatography on silica gel (1:10 MeOH(NH₃)/CH₂Cl₂) to give the base. MS m/z: 383 (M+1). Calc'd. for C₁₉H₂₂N₆OS - 382.49.

### Example 30

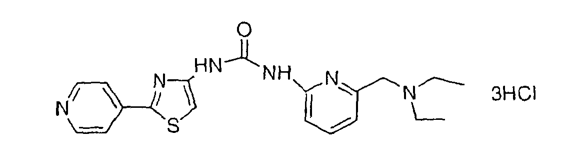

### N-[2-(4-Pyridinyl)-4-thiazolyl]-N'-2-[6-(N",N"-diethylaminomethylamino)pyridinyl]urea hydrochloride

N-[2-(4-Pyridinyl)-4-thiazolyl]-N'-2-(6-(N",N" - diethylaminomethylamino)pyridinyl]urea (Example 29) was dissolved in 5 ml of MeOH/CH₂Cl₂ (1:1) and 1M HCl (8 mL) in Et₂O solution was added. The solvents were removed *in vacuo* to afford the title salt as a yellow solid.

### Example 31

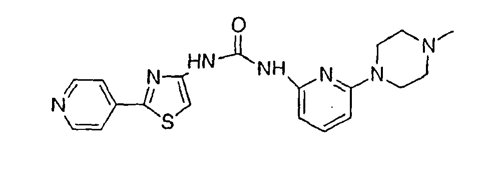

### N-[2-(4-Pyridinyl)-4-thiazolyl]-N'-2-[6-(1-methyl-4-piperazinyl)pyridinyl]urea

A mixture of 2-(4-pyridinyl)-4-thiazolylcarbonylazide (100 mg, 0.43 mmol) and 2-amino-6-(1-(4-methyl)piperazinyl)pyridine (100 mg, 5.21 mmol) in toluene (5 mL) was heated at 80°C for 5 h. After the mixture was cooled to RT the solvent was removed in vacuo. The crude product was purified by chromatography on silica gel (1:10 MeOH(NH₃)/CH₂Cl₂) to give N-[2-(4-pyridinyl)-4-thiazolyl]-N'-2-[6-(1-methyl-4-piperazinyl)pyridinyl]urea. m.p. 251-253°C. MS *m*/*z*: 396 (M+1). Calc'd. for C₁₉H₂₂N₆OS - 395.489.

### Example 32

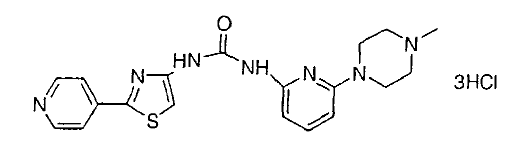

### N-[2-(4-Pyridinyl)-4-thiazolyl]-N'-2-[6-(1-methyl-4-piperazinyl)pyridinyl]urea hydrochloride

N-[2-(4-Pyridinyl)-4-thiazolyl]-N'-2-[6-(1-methyl-4-piperazinyl)pyridinyl]urea (Example 31) was dissolved in 5 ml of MeOH/CH₂Cl₂ (1:1) and 1M HCl (8 mL) in Et₂O solution was added. The solvents were removed *in vacuo* to afford the title salt as a yellow solid.

### Example 33

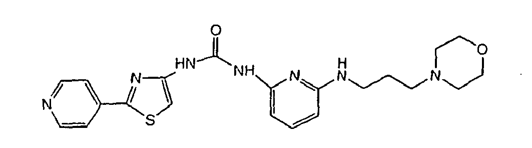

### N-[2-(4-Pyridinyl)-4-thiazolyl]-N'-2-[6-[3-(1-morpholinyl)propyl]amino]pyridinyl] urea

A mixture of 2-(4-pyridinyl)-4-thiazolylcarbonylazide (200 mg, 0.86 mmol) and 2-amino-6-(3-(N-morpholinyl)propylamino)pyridine (300 mg, 1.27 mmol) in toluene (8 mL) was heated at 70°C for 1 h, and then at 80°C for 5 h. After the mixture was cooled to RT the solvent was removed *in vacuo* and the product was purified by chromatography on silica gel (1:10 MeOH(NH₃)/CH₂Cl₂) to afford the title compound as a light yellow solid: m.p. 215-217°C. MS m/z: 440 (M+1). Calc'd. for C₂₁H₂₅N₇O₂S - 439.541.

### Example 34

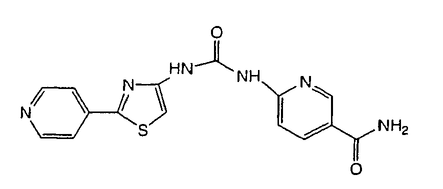

### [[(2-(4-Pyridinyl)-4-thiazolylamino)carbonyl]amino]-2-pyridinyl-5-carboxamide

A mixture of 2-(4-pyridinyl)-4-thiazolylcarbonylazide (100 mg, 0.43 mmol) and 6-aminonicotinamide (200 mg, 1.45 mmol) in toluene (5 mL) was heated at 80°C for 6 h. After the mixture was cooled to RT the solvent was removed in *vacuo* and the crude product was purified by chromatography on silica gel (1:10 MeOH(NH₃)/CH₂Cl₂) to afford the title compound as a light yellow solid: m.p. 255-257°C. MS *m*/*z*: 341 (M+1) . Calc'd for C₁₅H₁₂N₆O₂S - 340.37.

### Example 35

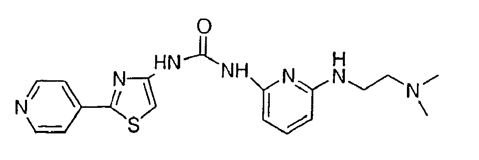

### N-[2-(4-Pyridinyl)-4-thiazolyl]-N'-2-[6-(N",N"-aminoethylamino)pyridinyl]urea

A mixture of 2-(4-pyridinyl)-4-thiazolylcarbonylazide (200 mg, 0.86 mmol) and 2-amino-6-(N,N-dimethylethylenediamino)pyridine (234 mg, 1.30 mmol) in toluene (10 mL) was heated at 70°C for 1 h, and then at 80°C for 5 h. After the mixture was cooled to RT the solvent was removed *in vacuo* and the crude product was purified by chromatography on silica gel (1:10 MeOH(NH₃)/CH₂Cl₂) to afford the title compound as a light yellow solid: m.p. 210-212°C. MS *m*/*z:* 384 (M+1). Calc'd. for C₁₈H₂₂N₇OS - 383.48.

### Example 36

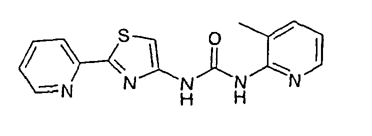

### N-[2-(2-Pyridinyl)-4-thiazolyl]-N'-2-(3-methylpyridinyl)urea

2-(2-Pyridinyl)-4-thiazolylcarbonylazide (500mg, 2.2 mmol) and 2-amino-3-methylpyridine (183mg, 6.6mmol) were heated in toluene (20 mL) at 100°C for 12 h. After cooling to RT, the solids were collected by filtration and washed first with toluene (2x20 mL) followed by Et₂O (3x10 mL). Recrystallization of the product from MeOH afforded the desired material: m.p. 235-237°C. MS *m*/*z:* 312 (M+H) . Calc'd. for C₁₅H₁₃N₅OS - 311.368.

### Example 37

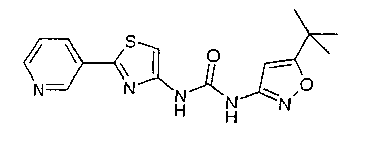

### N-[2-(3-Pyridinyl)-4-thiazolyl]-N'-[5-(1,1-dimethylethyl)-3-isoxazolyl]urea

2-(3-Pyridinyl)-4-thiazolylcarbonylazide (300mg, 1.30 mmol) and 3-amino-5-(tert-butyl)isoxazole (491mg, 3.50 mmol) were heated in toluene (10 mL) at 95°C for 24 h. After cooling to RT, the solids were collected by filtration and washed first with toluene (2x20 mL) followed by cold EtOAc (3x10 mL) to give the product as an off-white solid: m.p. 230-232° C. MS m/z: 344 (M+H). Calc'd. for C₁₆H₁₇N₅O₂S - 343.410.

### Example 38

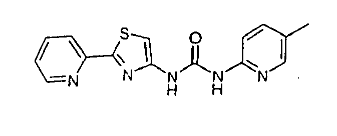

### N-[2-(2-Pyridinyl)-4-thiazolyl]-N'-2-(5-methylpyridinyl)urea

2-(2-Pyridinyl)-4-thiazolylcarbonylazide (200mg, 0.87mmol) and 2-amino-5-methylpyridine (183mg, 1.7mmol) were heated in toluene (15 mL) at 100°C for 12 h. After cooling to RT, the solids were collected by filtration and washed first with toluene (2x20mL) followed by Et₂O:EtOAc (3:1) (3x10 mL) to afford the product as a tan solid: m.p. 228-230°C. MS *m*/*z:* 312 (M+H). Calc'd. for C₁₅H₁₃N₅OS-311.368.

### Example 39

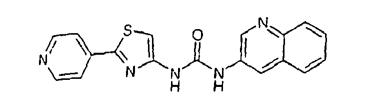

### N-[2-(4-Pyridinyl)-4-thiazolyl]-N'-3-quinolinylurea

In a manner similar to that described in Example 2, 2-(4-pyridinyl)-4-thiazolylcarbonylazide (53 mg, 0.229 mnol) and 3-aminoquinoline (36 mg, 260 mmol) were heated in toluene (10 mL) to give the product as a pale yellow solid. MS m/z: 348.5 (M+H) . Calc. for C₁₈H₁₃N₆OS - 347.401.

### Example 40

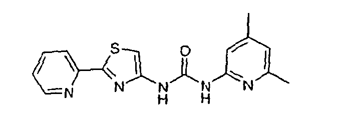

### N-[2-(2-Pyridinyl)-4-thiazolyl]-N'-2-(4,6-dimethylpyridinyl)urea

2-(2-Pyridinyl)-4-thiasolylcarbonylaside (200mg, 0.87mmol) and 2-amino-4,6-dimethylpyridine (210mg, 1.7mmol) were heated in toluene (15 mL) at 100°C for 12 h. After cooling to RT, the solids were collected by filtration and washed first with toluene (2 × 20 mL) followed by Et₂O:EtOAc (3:1) (3 × 10 mL) to afford the product as a tan solid: m.p. 232-234°C. MS *m*/*z*: 326 (M+H). Calc'd. for C₁₆H₁₅N₅OS - 325.394.

### Example 41

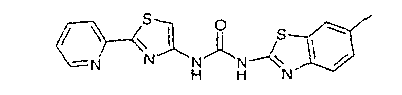

### N-[2-(2-Pyridinyl)-4-thiazolyl]-N'-2-(6-methylbenzthiazolyl)urea

2-(2-Pyridinyl)-4-thiazolylcarbonylazide (200mg, 0.87mmol) and 2-amino-6-methylbenzothiazole (279mg, 1.7mmol) were heated in toluene (15 mL) at 100°C for 12 h. After cooling to RT, the solids were collected by filtration and washed first with toluene (2 × 20 mL) followed by Et₂O:EtOAc (3:1) (3 × 10 mL) to afford the product as a tan solid: m.p. 263-265°C. MS *m*/*z:* 312 (M+H). Calc' d, for C₁₇H₁₃N₅OS₂ - 367.456.

### Example 42

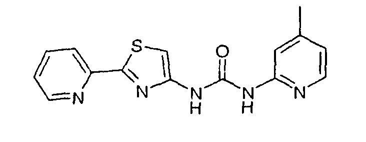

### N-[2-(2-Pyridinyl)-4-thiazolyl]-N'-2-(4-methylpyridinyl)urea

2-(2-Pyridinyl)-4-thiazolylcarbonylazide (200mg, 0.87mmol) and 2-amino-4-methylpyridine (183mg, 1.7mmol) were heated in toluene (15 mL) at 100°C for 12 h. After cooling to RT, the solids were collected by filtration and washed first with toluene (2 × 20 mL) followed by Et₂O:EtOAc (3:1) (3 × 10 mL) to afford the product as an off-white solid: m.p. 217-219°C. MS m/z: 312 (M+H). Calc'd. for C₁₅H₁₃N₅OS - 311.368.

### Example 43

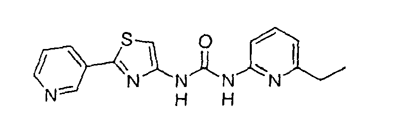

### N-[2-(3-Pyridinyl)-4-thiazolyl]-N'-2-(6-ethylpyridinyl)urea

In a manner similar to that described in Example 2, 2-(3-pyridinyl)-4-thiazolylcarbonylazide (186 mg, 0.804 mmol) and 2-amino-6-ethylpyridine (364 mg, 2.78 mmol) were heated in toluene (12 mL) to give the product as a pale yellow solid. MS *m*/*z*: 326.5 (M+H). Calc'd. for C₁₆H₁₅N₅OS - 325.395.

### Example 44

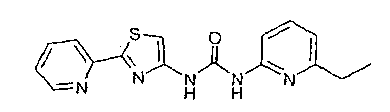

### N-[2-(2-Pyridinyl)-4-thiazolyl]-N'-2-(6-ethylpyridinyl)urea

2-(2-Pyridinyl)-4-thiazolylcarbonylazide (200mg, 0.87mmol) and 2-amino-6-ethylpyridine (318mg, 2.6mmol) were heated in toluene (10 mL) at 100°C for 14 h. After cooling to RT, the solids were collected by filtration and washed first with toluene (2 × 20 mL) followed by Et₂O (2 × 10mL) and cold EtOAc (3 × 5 mL) to give the product as a beige solid: m.p. 213-215°C. MS *m*/*z:* 326 (M+H). Calc'd. for C₁₆H₁₅N₅OS - 325.395.

### Example 45

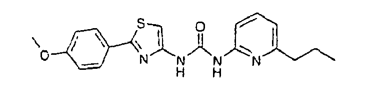

### N-[2-(4-Methoxyphenyl)-4-thiazolyl]-N'-2-(6-propylpyridinyl)urea

2-(4-Methoxyphenyl)-4-thiazolylcarbonylazide (280 mg, 1.1mmol) and 2-amino-6-n-propylpyridine (439 mg, 3.2 mmol) were heated in toluene (20 mL) at 100°C for 14 h. After cooling to RT, the solids were collected by filtration and washed first with toluene (2×20 mL) followed by Et₂O (2 × 10mL) and cold EtOAc (3 ×5mL) to afford the product as an off-white solid. m.p. 223-225°C. MS m/z: 369 (M+H). Calc'd for C₁₉H₂₀N₄O₂S - 368.461.

### Example 46

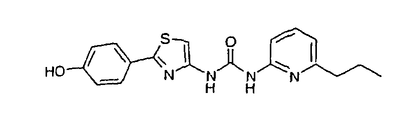

### N-[2-(4-Hydroxyphenyl)-4-thiazolyl]-N'-2-(6-propylpyridinyl)urea

To a stirred solution of Example 45 (100mg, 0.271 mmol) in CH₂Cl₂ (5 mL), boron tribromide was added dropwise at RT. The mixture was stirred for 8 h before adding H₂O (10 ml) and the resulting solids were collected by filtration. This material was washed several times with H₂O and then EtOAc followed by drying *in vacuo* to afford the desired product as a light yellow solid: m.p. 227-229°C. MS m/z: 355 (M+H). Calc'd for C₁₈H₁₈N₄O₂S - 354.434.

### Example 47

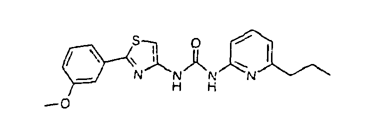

### N-[2-(3-Methoxyphenyl)-4-thiazolyl]-N'-2-(6-propylpyridinyl)urea

2-(3-Methoxyphenyl)-4-thiazolylcarbonylazide (1.0g, 3.8mmol) and 2-amino-6-n-propylpyridine (1.05g, 7.7mmol) were heated in toluene (40 mL) at 100°C for 12 h. After cooling to RT, the solids were collected by filtration and washed first with toluene (2x40 mL) followed by cold EtOAc (3x20 mL) to afford the product as a white solid: m.p. 192-194°C. MS *m*/*z:* 369 (M+H). Calc'd for C₁₉H₂₀N₄O₂S - 368.461.

### Example 48

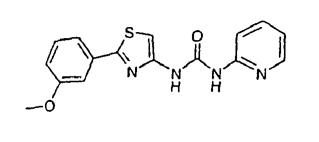

### N-[2-(3-Methoxyphenyl)-4-thiazolyl]-N'-2-pyridinylurea

2-(3-Methoxyphenyl)-4-thiazolylcarbonylazide (1.0g, 3.8mmol) and 2-aminopyridine (0.72g, 7.7mmol) were heated in toluene (40 mL) at 100°C for 12 h. After cooling to RT, the solids were collected by filtration and washed first with toluene (2x40 mL) followed by cold EtOAc (3x20 mL) to afford the product as a white solid: m.p. 201-203°C. MS *m*/*z:* 327 (M+H). Calc'd for C₁₆H₁₄N₄O₂S - 326.380.

### Example 49

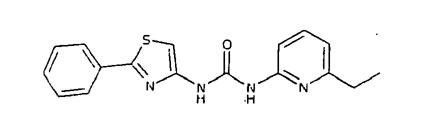

### N-[2-phenyl-4-thiazolyl]-N'-2-(6-ethylpyridinyl)urea

In a manner similar to that described in Example 2, 2-phenyl-4-thiazolylcarbonylazide (150 mg, 0.652 mmol) and 2-amino-6-ethylpyridine (250 mg, 2.05 mmol) were heated in toluene (10 mL) to give the product as a pale yellow solid. MS *m*/*z:* 325.4 (M+H). Calc'd for C₁₇H₁₆N₄OS - 324.407.

### Example 50

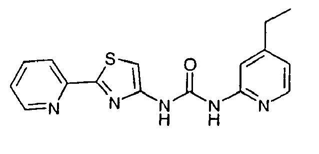

### N-[2-(2-Pyridinyl)-4-thiazolyl]-N'-2-(4-ethylpyridinyl)urea

2-(2-Pyridinyl)-4-thiazolylcarbonylazide (200mg, 0.87mmol) and 2-amino-4-ethylpyridine (208mg, 1.7mmol) were heated in toluene (15 mL) at 100°C for 12 h. After cooling to RT, the solids were collected by filtration and washed first with toluene (2 × 20 mL) followed by Et₂O : EtOAc (3:1) (3 × 10 mL) to afford the product as a tan solid: m.p. 196-198°C. MS m/z: 326 (M+H). Calc'd. for C₁₆H₁₅N₅OS - 325.395.

### Example 51

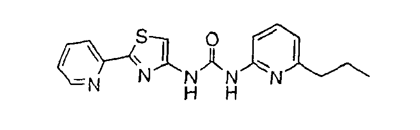

### N-[2-(2-Pyridinyl)-4-thiazolyl]-N'-2-(6-propylpyridinyl)urea

2-(2-Pyridinyl)-4-thiazolylcarbonylazide (200mg, 0.87mmol) and 2-amino-6-(n-propyl)pyridine (350mg, 2.6mmol) were heated in toluene (10mL) at 100°C for 14 h. After cooling to RT, the solids were collected by filtration and washed first with toluene (2 × 20 mL) followed by Et₂O (2 × 10mL) and cold EtOAc (3 × 5 mL) to give the product as a grayish solid: m.p. 210-212°C. MS m/z: 340 (M+H). Calc'd. for C₁₇H₁₇N₅OS - 339.422.

### Example 52

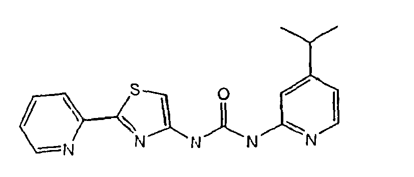

### N-[2-(2-Pyridinyl)-4-thiazolyl]-N'-2-[4-(1-methylethyl)pyridinyl]urea

2-(2-Pyridinyl)-4-thiazolylcarbonylazide (300mg, 1.3mmol) and 2-amino-4-isopropylpyridine (500mg, 3.6mmol) were heated in 10 mL toluene at 100°C for 12 h. After cooling to RT, the solvent was removed by rotary evaporation and the crude oil purified by column chromatography with hexane:EtOAc (7:3) as eluant to give the urea as a light yellow solid. MS *m*/*z:* 340 (M+H). Calc'd for C₁₇H₁₇N₅OS - 339.42.

### Example 53

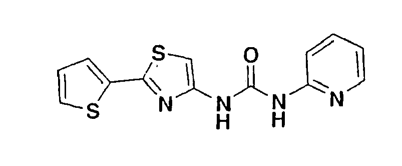

### N-[2-(2-Thienyl)-4-thiazolyl]-N'-2-(pyridinyl)urea

2-(2-Thienyl)-4-thiazolylcarbonylazide (200mg, 0.85mmol) and 2-aminopyridine (154mg, 1.62mmol) were heated in 20 mL toluene at 100°C for 16 h. After cooling to RT, the solids were collected by filtration and washed first with toluene (2 × 20 mL) followed by Et₂O: EtOAc (3:1) (3 × 10 mL) to afford the urea as an off-white solid. MS *m*/*z:* 303 (M+H). Calc'd for C₁₃H₁₀N₄OS₂ - 302.38 .

### Example 54

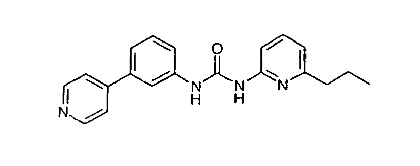

### N-[3-(4-Pyridinyl)phenyl]-N'-2-(6-propylpyridinyl)urea

To a suspended anhydrous solution of 4-pyridylaniline (180 mg, 1.06 mmol) in dry toluene (8 mL) was added phosgene (0.73 mL, 1.38 mmol, 20% in toluene) followed by N,N-diisopropylethylamine (0.37 mL, 2.11 mmol) under an atmosphere of argon. After stirring for 0.5 h at RT, 2-amino-6-(n-propyl)pyridine (144 mg, 1.06 mmol) in dry toluene (3 mL) was added dropwise into the reaction mixture. The resulting mixture was stirred at RT for 18 h. The organic solvent was removed under vacuum. The residue was purified by flash chromatography on silica gel using 5% methanol/dichloromethane as eluant to obtain the final urea as white solid: m.p. 195-198°C. MS *m*/*z*: 333.4 (M+H). Calc'd. for C₂₀H₂₀N₄O - 332.405.

### Example 55

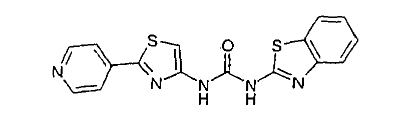

### N-[2-(4-Pyridinyl)-4-thiazolyl]-N'-2-benzthiazolylurea

In a manner similar to that described in Example 2, 2-(4-pyridinyl)-4-thiazolylcarbonylazide (52 mg, 0.225 mmol) and 2-aminobenzothiazole (41 mg, 0.273 mmol) were heated in toluene (10 mL) to give the product as a pale yellow solid. MS *m*/*z:* 354.4 (M+H). Calc'd. for C₁₆H₁₂N₅OS₂ - 353.427.

### Example 56

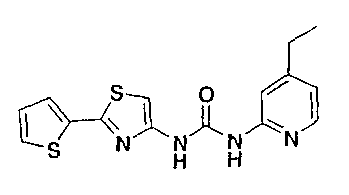

### N-[2-(2-Thienyl)-4-thiazoly1]-N'-2-(4-ethylpyridinyl)urea

2-(2-Thienyl)-4-thiazolylcarbonylazide (500mg, 2.1mmol) and 2-amino-4-etrylpyridine (512mg, 4.2mmol) were heated in 15 mL toluene at 100°C for 16 h. After cooling to RT, the solids were collected by filtration and washed first with toluene (2 × 20 mL) followed by Et₂O:EtOAc (3:1) (3 × 10 mL) to afford the urea as an off-white solid. MS m/z: 331 (M+H). Calc'd for C₁₅H₁₄N₄OS₂ - 330.435.

### Example 57

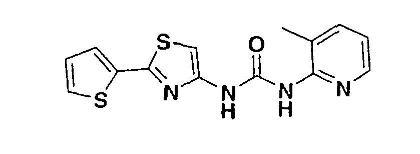

### N-[2-(2-Thienyl)-4-thiazolyl]-N'-2-(3-methylpyridinyl)urea

2-(2-Thienyl)-4-thiazolylcarbonylazide (500mg, 2.1mmol) and 2-amino-3-methylpyridine (449mg, 4.2mmol) were heated in 15 mL toluene at 100°C for 16 h. After cooling to RT, the solids were collected by filtration and washed first with toluene (2×20 mL) followed by Et₂O:EtOAC (3:1) (3x10 mL) to afford the urea as an off-white solid. MS m/z: 317 (M+H). Calc'd for C₁₄H₁₂N₄OS₂ - 316.408.

### Example 58

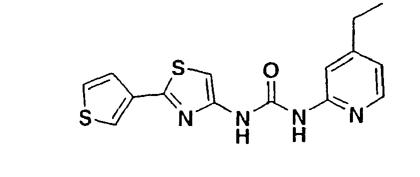

### N-[2-(3-Thienyl)-4-thiazolyl]-N'-2-(4-ethylpyridinyl)urea

2-(3-Thienyl)-4-thiazolylcarbonylazide (200mg, 0.85mmol) and 2-amino-4-ethylpyridine (310mg, 2.54 mmol) were heated in 10 mL toluene at 100°C for 16 h. After cooling to RT, the solids were collected by filtration and washed first with toluene (2x20 mL) followed by Et₂O:EtOAc (3:1; (3×10 mL) to afford the product as an off-white solid. MS *m*/*z*: 331 (M+H). Calc'd for C₁₅H₁₄N₄OS₂ - 330.435.

### Example 59

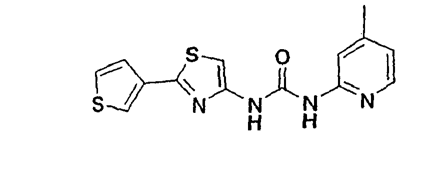

### N-[2-(3-Thienyl)-4-thiazolyl]-N'-2-(4-methylpyridinyl)urea

2-(3-Thienyl)-4-thiazolylcarbonylazide (200mg, 0.85mmol) and 2-amino-4-methylpyridine (272mg, 2.54mmol) were heated in 10 mL toluene at 100°C for 16 h. After cooling to RT, the solids were collected by filtration and washed first with toluene (2x20mL) followed by Et₂O: EtOAc (3:1) (3x10mL) to afford the product as an off-white solid. MS m/z: 317 (M+H). Calc'd for C₁₄H₁₂N₄OS₂ - 316.408.

### Example 60

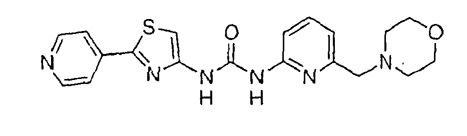

### N-[2-(4-Pyridinyl)-4-thiazolyl]-N'-2-[6-(1-morpholinylmethyl)pyridinyl]urea

2-(4-Pyridinyl)-4-thiazolcarbonylazide (100 mg, 0.43 mmol) in dry toluene (10 mL) was heated to 85°C under N₂ and maintained at for 5 min. A solution of 6-morpholin-4-ylmethyl-pyridin-2-ylamine (101 mg, 0.52 mmol) in dry toluene (2 mL) was added dropwise via syringe and the resulting mixture was heated at 100°C for 12 h. After cooling to RT, a precipitate formed and was collected, rinsing with hexane to give a white solid. MS *m*/*z:* 397.3 (M+H). Calc'd for C₁₉H₂₀N₆O₂S: 396.14.

The following compounds were prepared from the corresponding amines in a manner similar to that described above for Example 60:

### Example 61

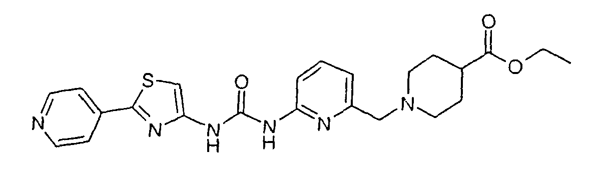

### Ethyl 1-{6-[3-(2-(pyridin-4-yl)thiazol-4-yl)ureido]-pyridin-2-ylmethyl)-piperidine-4-carboxylate

2-(4-Pridinyl)-4-thiazolcarbonylazide (182 mg, 0.87 mmol) heated with ethyl 1-(6-aminopyridin-2-ylmethyl)-piperidine-4-carboxylate (230 mg, 0.87 mmol) in dry toluene (15 mL) gave the final urea. MS m/z: 465.9 (M+H). Calc'd. for C₂₃H₂₆N₆O₃S - 466.50.

### Example 62

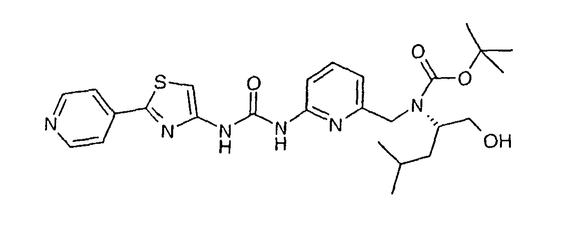

### tert-Butyl (1-hydroxymethyl-3-methyl-butyl)-{6-[3-(2-pyridin-4-yl-thiazol-4-yl)-ureido]-pyridin-2-ylmethyl}-carbamate

2-(4-Pyridinyl)-4-thiazolcarbonylazide (343 mg, 1.48 mmol) was heated with 2-amino-6-[N'-*tert-*butoxycarbonyl-N'-2-(1-hydroxy-4-methyl)pentylamino]methylpyridine (480 mg, 1.48 mmol) in dry toluene (20 mL) to yield the final compound as pale yellow solid. MS m/z: 527.6 (M+H). Calc'd. for C₂₆H₃₄N₆O₄S - 526.66.

### Example 63

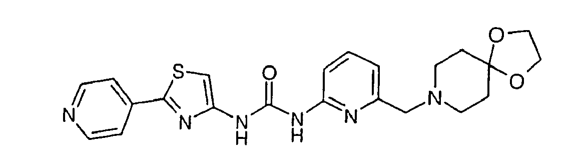

### 1-[6-(1,4-Dioxa-8-aza-spiro[4.5]dec-8-ylmethyl)-pyridin-2-yl]-3-(2-pyridin-4-yl-thiazol-4-yl)-urea

2-(4-Pyridinyl)-4-thiazolcarbonylazide (420 mg, 2.01 mmol) was heated with 2-amino-6-(4-ethoxyacetal)piperidinylmethyl pyridine (500 mg, 2.01 mmol) in dry toluene (30 mL) to yield the final compound as yellow solid. MS m/z: 452.9 (M+H). Calc'd. for C₂₂H₂₄N₆O₃S - 452.23.

### Example 64

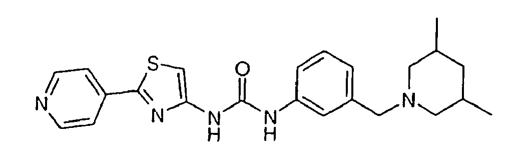

### 1-[6-(3,5-Dimethylpiperidin-1-ylmethyl)pyridin-2-yl]-3-(2-pyridin-4-ylthiazol-4-yl)urea

2-(4-Pyridinyl)-4-thiazolcarbonylazide (200 mg, 0.367 mmol) was heated with 2-amino-6-(3,5-dimethyl)piperidinyl-methylpyridine (190 mg, 0.867 mmol) in dry toluene (20 mL) to yield the final compound as yellow solid. MS m/z: 423.2 (M+H). Calc'd. for C₂₂H₂₆N₆OS - 422.0.

### Example 65

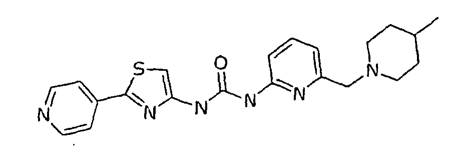

### 1-[6-(4-Methylpiperidin-1-ylmethyl)pyridin-2-yl]-3-(2-pyridin-4-yl-thiazol-4-yl)urea

2-(4-Pyridinyl)-4-thiazolcarbonylazide (348 mg, 1.51 mmol) was heated with 2-amino-6-(4-methyl)piperidinyl-methylpyridine (310 mg, 1.51 mmol) in dry toluene (20 mL) to yield the final compound as pale yellow solid. MS m/z: 409.5(M+H). Calc'd. for C₂₁H₂₄N₆OS - 408.52.

### Example 66

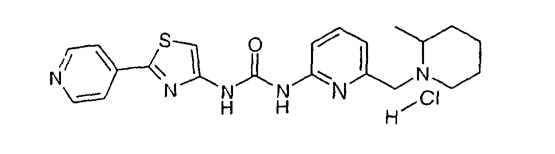

### 1-[6-(2-Methylpiperidin-1-ylmethyl)pyridin-2-yl]-3-(2-pyridin-4-yl-thiazol-4-yl)urea

2-(4-Pyridinyl)-4-thiazolcarbonylaside (101 mg, 0.44 mmol) was heated with 2-amino-6-(2-methyl)piperidinylmethyl pyridine (90 mg, 0.44 mmol) in dry toluene (15 mL) to yield the final compound as pale yellow solid. MS m/z: 409.6 (M+H). Calc'd. for C₂₁H₂₄N₆OS - 408.52.

### Example 67

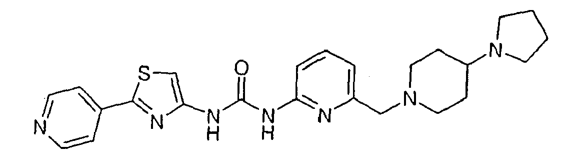

### 1-(2-Pyridin-4-yl-thiazol-4-yl)-3-[6-(4-pyrrolidin-1-yl-piperidin-1-ylmethyl)-pyridin-2-yl]-urea

2-(4-pyridinyl)-4-thiazolcarbonylazide (293 mg, 1.43 mmol) was heated with 2-amino-6-[4-(1-pyrrolidinyl)piperidinylmethyl] pyridine (330 mg, 1.43 mmol) in dry toluene (20 mL) to yield the final compound as pale yellow solid. MS *m*/*z:* 464.2 (M+H). Calc'd. for C₂₄H₂₉N₇OS - 463.

### Example 68

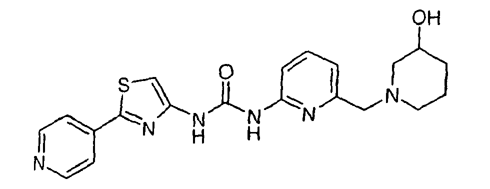

### 1-[6-(3-Hydroxy-piperidin-1-ylmethyl)-pyridin-2-yl]-3-(2-pyridin-4-yl-thiazol-4-yl)-urea

2-(4-Pyridinyl)-4-thiazolcarbonylazide (312 mg, 1.35 mmol) was heated with 2-amino-6-(3-hydroxy)-piperidinylmethyl pyridine (280 mg, 1.35 mmol) in dry toluene (20 mL) to yield the final compound as yellow solid. MS m/z: 410. 9 (M+H). Calc' d. for C₂₀H₂₂N₆O₂S - 410.5.

### Example 69

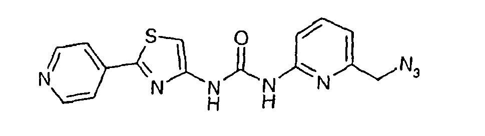

### N-(6-azidomethyl-2-pyridyl)-N'-[2-(4-pyridinyl)-4-thiazolyl]urea

2-(4-Pyridinyl)-4-thiazolcarbonylazide (400 mg, 1.73 mmol) was heated with 2-amino-6-azidomethyl-pyridine (258 mg, 1.73 mmol) in dry toluene (15 mL) to yield the final compound as yellow solid. MS *m*/*z:* 353.4(M-H). Calc'd. for C₁₅H₁₂N₈OS - 352. 8.

### Example 70

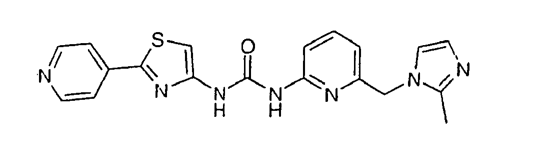

### 1-[6-(2-Methyl-imidazol-1-ylmethyl)-pyridin-2-yl]-3-(2-pyridin-4-yl-thiazol-4-yl)-urea

2-(4-Pyridiny)-4-thiazolcarbonylazide (110 mg, 0.48 mmol) was heated with 2-amino-6-[2-methylimidazol-1-yl]methyl-pyridine (90 mg, 0.48 mmol) in dry toluene (15 mL) to yield the final compound as white solid. MS *m*/*z:* 392.4 (M+H). Calc'd. for C₁₉H₁₇N₇OS - 391.45.

### Example 71

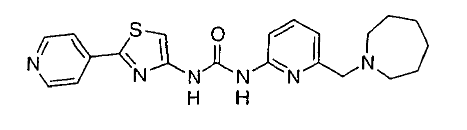

### 1-(6-Azepan-1-ylmethyl-pyridin-2-yl)-3-(2-pyridin-4-yl-thiazol-4-yl)urea

2-(4-Pyridinyl)-4-thiazolcarbonylazide (150 mg, 0.65 mmol) and 2-amino-6-azaperhydroepinylmethylpyridine (147 mg, 0.71 mmol) in dry toluene (15 mL) were heated at 100°C for 12 h to give a pale yellow solid. MS m/z: 409.1 (M+H). Calc'd for C₂₁H₂₄N₆OS - 408.52 .

### Example 72

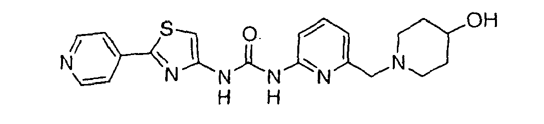

### 1-[6-(4-Hydroxy-piperidin-1-ylmethyl)-pyridin-2-yl]-3-(2-pyridin-4-yl-thiazol-4-yl)-urea

2-(4-Pyridinyl)-4-thiazolcarbonylazide (265 mg, 1.27 mmol) and 2-amino-6-(4-hydroxy)piperidyl-methylpyridine (220 mg, 1.06 mmol) in dry toluene (15 mL) were heated at 100°C for 12 h to give a pale yellow solid which was recrystallized from CHCl₃/meOH/hexane (94:2:1) to give a white solid. MS m/z: 410.9 (M+H). Calc'd for C₂₀H₂₂N₆O₂S - 410.50.

### Example 73

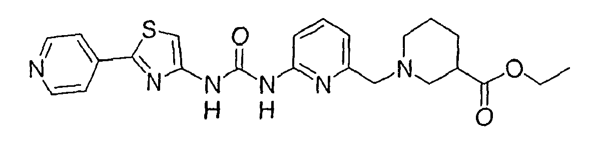

### Ethyl 1-{6-[3-(2-pyridin-4-yl-thiazol-4-yl)ureido]-pyridin-2-ylmethyl}piperidine-3-carboxylate

2-(4-Pyridinyl)-4-thiazolcarbonylazide (150 mg, 0.65 mmol) and 2-amino-ethyl(6-piperidylmethyl-pyridinyl)-3-carboxylate (170 mg, 0.65 mmol) in dry toluene (15 mL) were heated at 100°C for 12 h to give a pale yellow solid which was purified by chromatography on silica gel (CH₂Cl₂/MeOH, 95:5) to give a white solid. MS *m*/*z*: 467.1 (M+H). Calc'd for C₂₃H₂₆N₆O₃S - 466.56.

### Example 74

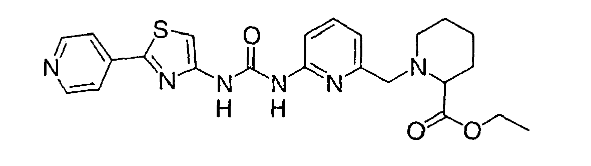

### Ethyl 1-[6-[3-(2-(pyridin-4-yl)thiazol-4-yl)ureido]-pyridin-2-ylmethyl]piperidine-2-carboxylate

2-(4-Pyridinyl)-4-thiazolcarbonylazide (483 mg, 2.09 mmol) and ethyl 2-amino-(6-piperidylmethyl-pyridinyl)-2-carboxylate (550 mg, 2.09 mmol) in dry toluene (20 mL) were heated at 100°C for 8 h to give a pale yellow solid which was purified by chromatography on silica gel (CH₂Cl₂/MeOH, 95:5) to give a white solid. MS *m*/*z:* 466.9 (M+H). Calc'd for C₂₃H₂₆N₆O₃S - 466.56.

### Example 75

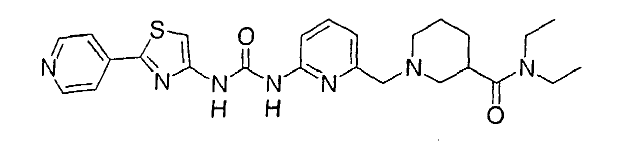

### N,N-Diethyl 1-{6-[3-(2-pyridin-4-yl-thiazol-4-yl)-ureido]pyridin-2-ylmethyl}piperidine-3-carboxamide

2-(4-Pyridinyl)-4-thiazolcarbonylazide (320 mg, 1.38 mmol) and 2-amino-6-[(N'',N''-diethylcarbamoyl)-piperidylmethyl]-3-carboxamide (400 mg, 1.38 mmol) in dry toluene (25 mL) were heated at 100°C for 12 h to give a pale yellow solid which was purified by chromatography on silica gel (CH₂Cl₂/MeOH, 95:5) to give a white solid. MS m/z: 494.1 (M+H). Calc'd for C₂₅H₃₁N₇O₂S - 493.63.

### Example 76

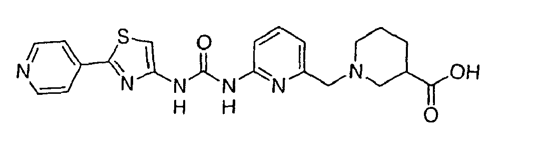

### 1-(6-[3-(2-Pyridin-4-yl-thiazol-4-yl)-ureido]-pyridin-2-ylmethyl)-piperidine-3-carboxylic acid

2-(4-Pyridinyl)-4-thiazolcarbonylazide (196 mg, 0.85 mmol) and 2-amino-6-(piperidylmethylpyridinyl)-3-carboxylate (200 mg, 0.85 mmol) in dry toluene (10 mL) were heated at 100°C for 8 h to give a pale yellow solid which was purified by chromatography on silica gel (CH₂Cl₂/MeOH, 95 :5) to give a white solid. MS *m*/*z:* 437.9 (M+H). Calc'd for C₂₁H₂₂N₆O₃S - 438.51.

### Example 77

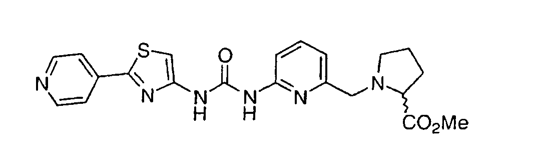

### Methyl 1-{6-[3-(2-pyridin-4-yl-thiazol-4-yl)ureido]-pyridin-2-ylmethyl}-pyrrolidine-2-carboxylate

2-(4-Pyridinyl)-4-thiazolcarbonylazide (104 mg, 0.45 mmol) and 2-amino-6-(2-methoxycarbonyl)-pyrrolidinyl-methylpyridine (105 mg, 0.45 mmol) in dry toluene (10 mL) were heated at 100°C for 12 h to give a pale yellow solid which was purified by chromatography on silica gel (CHCl3/MeOH, 99:5) to give a white solid. MS m/z: 438.7 (M+H). Calc'd for C₂₁H₂₂N₆O₃S - 438.51.

### Example 78

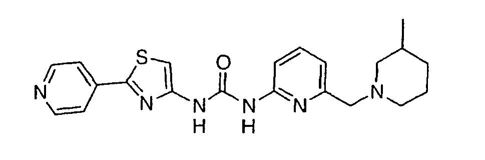

### 1-[6-(3-Methyl-piperidin-1-ylmethyl)-pyridin-2-yl]-3-(2-pyridin-4-yl-thiazol-4-yl)-urea

2-(4-Pyridinyl)-4-thiazolcarbonylazide (259 mg, 1.12 mmol) and 2-amino-6-(3-methyl)piperidinylmethyl-pyridine (230 mg, 1.12 mmol) in dry toluene (15 mL) were heated at 100°C for 12 h to give a pale yellow solid which was purified by chromatography on silica gel (CHCl₃/ MeOH, 99:5) to give a white solid. MS m/z: 408.8 (M+H). Calc'd for C₂₁H₂₄N₆OS - 408.53.

### Example 79

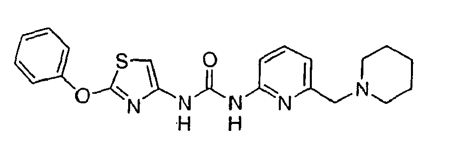

### 1-(2-Phenoxy-thiazol-4-yl)-3-(6-piperidin-1-ylmethyl-pyridin-2-yl)-urea

MS m/z: 410 (M+H). Calc'd for C₂₁H₂₃N₅O₂S: 409.16.

### Example 80

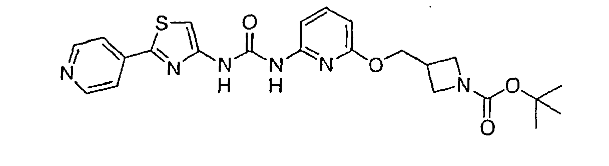

### tert Butyl 3-{6-[3-(2-pyridin-4-yl-thiazol-4-yl)-ureido]-pyridin-2-yloxymethyl}-azetidine-1-carboxylate

MS m/z: 483 (M+H). Calc'd for C₂₃H₂₆N₆O₄S: 482.17.

### Example 81

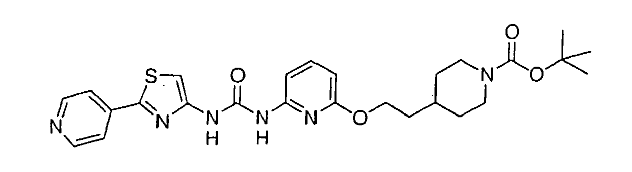

### tert Butyl 4-(2-{6-[3-(2-pyridin-4-yl-thiazol-4-yl)-ureido]pyridin-2-yloxy}ethyl)piperidine-1-carboxylate

MS m/z: 525 (M+H). Calc'.d for C₂₆H₃₂N₆O₄S: 524.22

### Example 82

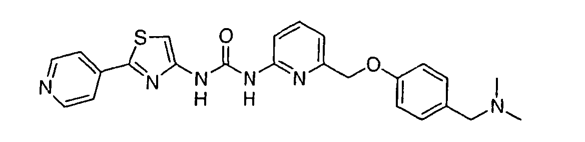

### 1-[6-(4-Dimethylaminomethyl-phenoxymethyl)-pyridin-2-yl]-3-(2-pyridin-4-yl-thiazol-4-yl)-urea

MS m/z: 461 (M+H). Calc'd for C₂₄H₂₄N₆O₂S: 460.17.

### Example 83

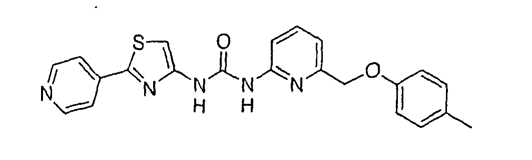

### 1-(2-Pyridin-4-yl-thiazol-4-yl)-3-(6-(4-methylphenyl)oxymethylpyridin-2-yl)urea

MS m/z : 416 (M-H) . Calc'd for C₂₂H₁₉N₅O₂S: 417.13.

### Example 84

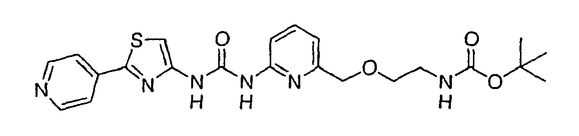

### tert Butyl (2-{6-[3-(2-pyridin-4-yl-thiazol-4-yl)-ureidolpyridin-2-ylmethoxylethyl)carbamate

MS m/z: 471 (M+H). Calc'd for C₂₂H₂₆N₆O₄S: 470.17.

### Example 85

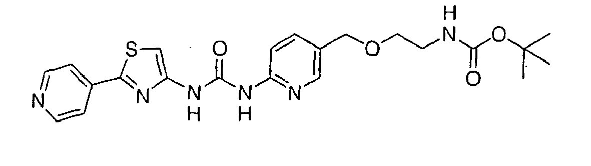

### tert Butyl (2-{6-[3-(2-pyridin-4-yl-thiazol-4-yl)-ureido]pyridin-3-ylmethoxy}ethyl)carbamate

MS m/z: 471 (M+H) . Calc'd for C₂₂H₂₆N₆O₄S: 470.17.

### Example 86

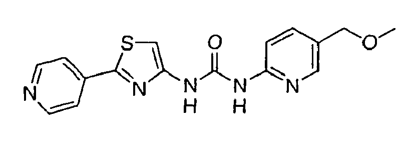

### 1-(5-Methoxymethyl-pyridin-2-yl)-3-(2-pyridin-4-yl-thiazol-4-yl)-urea

MS m/z : 342 (M+H) . Calc' d for C₁₆H₁₅N₅O₂S: 341.09.

### Example 87

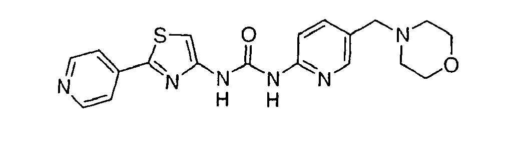

### 1-(5-Morpholin-4-ylmethyl-pyridin-2-yl)-3-(2-pyridin-4-yl-thiazol-4-yl)urea

MS m/z: 397 (M+H). Calc'd for C₁₉H₂₀N₆O₂S: 396.14.

### Example 88

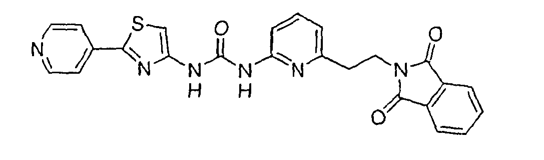

### 1-{6-[2-phthalimidylethyl]pyridin-2-yl}-3-(2-pyridin-4-yl-thiazol-4-yl)urea

Prepared in a manner similar to that described in Example 60 from 3-(4-pyridyl)-thiazole acyl-azide (103 mg, 0.56 mmol) and 2-amino-6-ethylphthalamidylpyridine (150 mg, 0.56 mmol) in toluene (10 mL). Concentrated in vacuo to afford a yellow solid which was treated with EtOH (10 mL) and filtered to give the title compound as a yellow solid. MS m/z: 470.9 (M+H). Calc'd for C₂₄H₁₈N₆O₃S: 470.12.

### Example 89

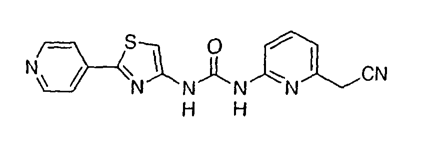

### 1-(6-Cyanomethylpyridin-2-yl)-3-(2-pyridin-4-yl-thiazol-4-yl)urea

Prepared in a manner similar to that described in Example 60 from 2-amino-6-methylnitrile-pyridine (0.32 g, 2.4 mmol) and 3-(4-pyridyl)-4-thiazole acylazide (0.51 g, 2.2 mmol). After 1.5 h, yellow solid precipitated out of toluene solution. The mixture was cooled to RT and the solid filtered. Purified by silica flash chromatography (3% MeOH/CH₂Cl₂) to afford the title compound as a white solid. MS m/z: 337.1 (M+H) . Calc'd for C₁₆H₁₂N₆OS: 336.08.

### Example 90

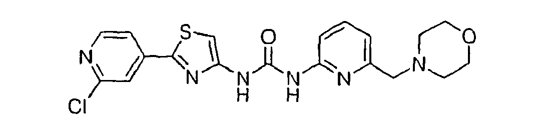

### 1-[2-(2-Chloropyridin-4-yl)thiazol-4-yl]-3-(6-morpholin-4-ylmethyl-pyridin-2-yl)urea

Prepared in a manner similar to that described in Example 60 from 3-(4-pyridyl)-4-thiazole acyl azide (0.51 g, 1.9 mmol) and 2-amino-6-methylmorpholino-pyridine (0.42g, 2.2 mmol) in toluene (50 mL). After 3 h, the reaction mixture was cooled to RT and filtered to afford the title compound as a light purple solid. MS m/z: 431.0 (M+H). Calc'd for C₁₉H₁₉ClN₆O₂S: 430.10.

### Example 91

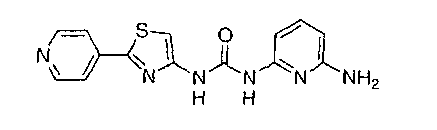

### 1-(6-Aminopyridin-2-yl)-3-(2-pyridin-4-yl-thiazol-4-yl)urea

Prepared in a manner similar to that described in Example 60 from 3-(4-pyridyl)-4-thiazole-acyl azide (148 mg, 0.64 mmol) and 2,6-diaminopyridine (77 mg, 0.70 mmol, Aldrich) in toluene (10 mL). After 2 h, a yellow precipitate formed. The reaction mixture was cooled and filtered to afford the title compound as a yellow solid. MS m/z: 180 (M+H). Calc'd for C₁₉H₁₂N₆OS : 312.08.

### Example 92

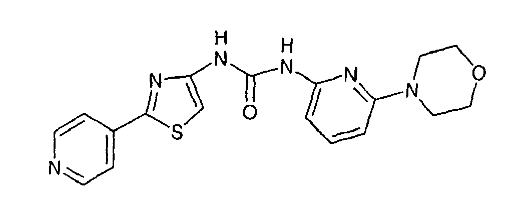

### 1-(6-Morpholin-4-yl-pyridin-2-yl)-3-(2-pyridin-4-yl-thiazol-4-yl)urea

EI-MS m/z 383.4 (M+H). Calc'd for C₁₈H₁₈N₆O₂S: 382.12.

### Example 93

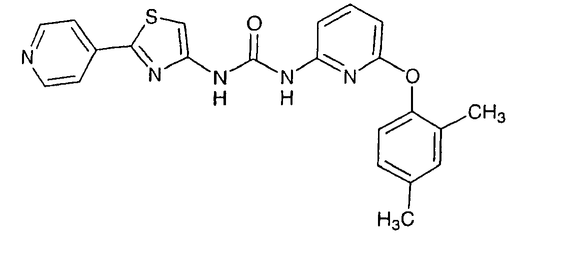

### 1-[6-(2,4-Dimethylphenoxy)pyridin-2-yl]-3-(2-pyridin-4-yl-thiazol-4-yl)urea

EI-MS m/z 418.5 (M+H). Calc'd for C₂₂H₁₉N₅O₂S: 417.13.

### Example 94

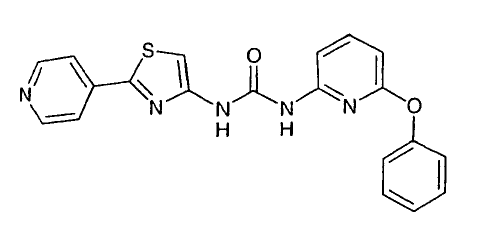

### 1-(6-Phenoxypyridin-2-yl)-3-(2-pyridin-4-yl-thiazol-4-yl)urea

EI-MS m/z 390.4 (M+H). Calc'd for C₂₀H₁₅N₅O₂S: 389.09.3

### Example 95

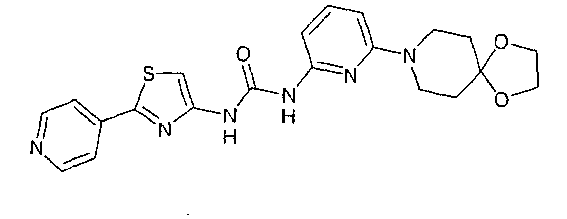

### 1-[6-(1,4-Dioxa-8-aza-spiro[4.5]dec-8-yl)-pyridin-2-yl]-3-(2-pyridin-4-yl-thiazol-4-yl)urea

Prepared in a manner similar to that described in Example 60 using 2-(4-pyridinyl)-4-thiazolcarbonylazide and the requisite 2-aminopyridine. EI-MS m/z 439.5 (M+H) Calc'd for C₂₁H₂₂N₆O₃S: 438.15.

### Example 96

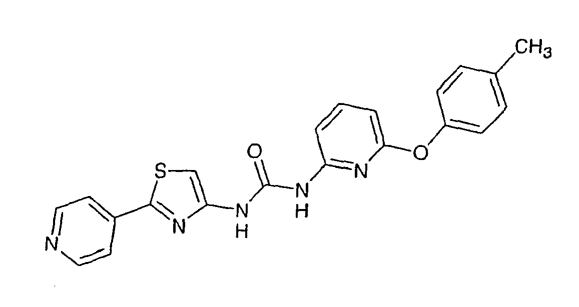

### 1-(2-Pyridin-4-yl-thiazol-4-yl)-3-(6-p-tolyloxy-pyridin-2-yl)-urea

EI-MS m/z 404.4 (M+H) . Calc'd for C₂₁H₁₇N₅O₂S: 403.11.

### Example 97

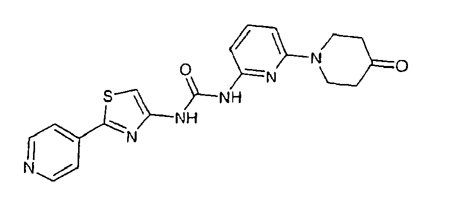

### 1-(4-Oxo-3,4,5,6-tetrahydro-2H-[1,2']bipyridinyl-6'-yl)-3-(2-pyridin-4-yl-thiazol-4-yl)-urea

EI-MS m/z 395.4 (M+H). Calc'd for C₁₉H₁₆N₆O₂S: 394.12.

### Example 98

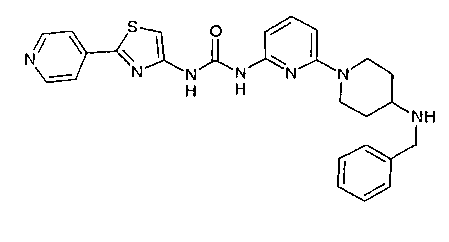

### 1-(4-Benzylamino-3,4,5,6-tetrahydro-2H-[1,2']bipyridinyl-6'-yl)-3-(2-pyridin-4-yl-thiazol-4-yl)-urea

EI-MS m/z 486.7 (M+H). Calc'd for C₂₆H₂₇N₇OS: 435.20.

### Example 99

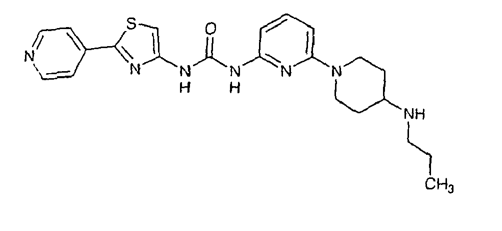

### 1-(4-Propylamino-3,4,5,6-tetrahydro-2H-[1,2']bipyridinyl-6'-yl)-3-(2-pyridin-4-yl-thiazol-4-yl)-urea

EI-MS m/z 438.6 (M+H). Calc'd for C₂₂H₂₇N₇OS: 437.20.

### Example 100

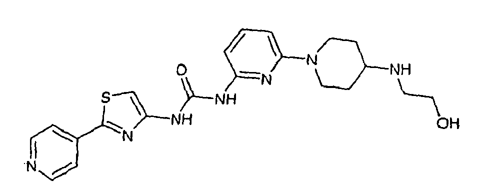

### 1-[4-(2-Hydroxy-ethylamino)-3,4,5,6-tetrahydro-2H-[1,2']bipyridinyl-6'-yl]-3-(2-pyridin-4-yl-thiazol-4-yl)-urea

EI-MS m/z 440.5 (M+H). Calc'd for C₂₁H₂₅N₇O₂S: 439.18.

### Example 101

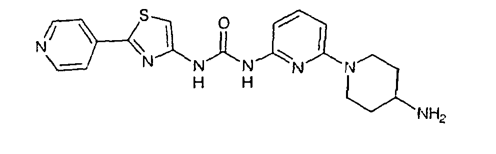

### 1-(4-Amino-3,4,5,6-tetrahydro-2H-[1,2']bipyridinyl-6'-yl)-3-(2-pyridin-4-yl-thiazol-4-yl)-urea

EI-MS m/z 396.6 (M+H). Calc'd for C₁₉H₂₁N₇OS: 395.15.

### Example 102

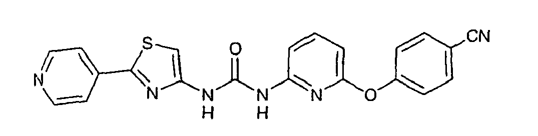

### 1-[6-(4-Cyanophenoxy)-pyridin-2-yl]-3-(2-pyridin-4-yl-thiazol-4-yl)urea

EI-MS m/z 415.5 (M+H). Calc'd for C₂₁H₁₄N₆O₂S: 414.09.

### Example 103

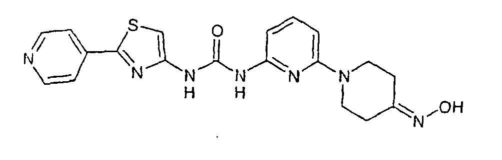

### 1-(4-Hydroxyimino-3,4,5,6-tetrahydro-2H-[1,2']bipyridinyl-6'-yl)-3-(2-pyridin-4-yl-thiazol-4-yl)-urea

EI-MS m/z 410.4(M+H). Calc' d for C₁₉H₁₉N₇O₂S: 409.13.

### Example 104

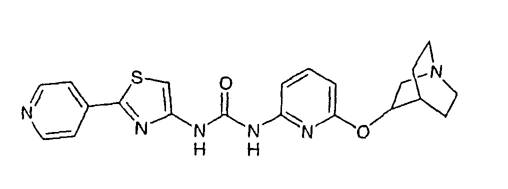

### 1-[6-(1-Aza-bicyclo[2.2.2]oct-3-yloxy)-pyridin-2-yl]-3-(2-pyridin-4-yl-thiazol-4-yl)-urea

EI-MS m/z 423.6 (M+H). Calc'd for C₂₁H₂₂N₆O₂S: 422.15.

### Example 105

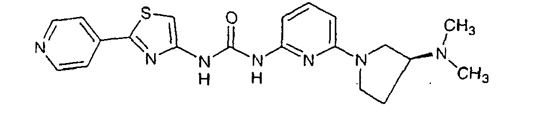

### 1-[6-(3-Dimethylamino-pyrrolidin-1-yl)-pyridin-2-yl]-3-(2-pyridin-4-yl-thiazol-4-yl)-urea

EI-MS m/z 410.5 (M+H). Calc'd for C₂₀H₂₃N₇OS: 409.17.

### Example 106

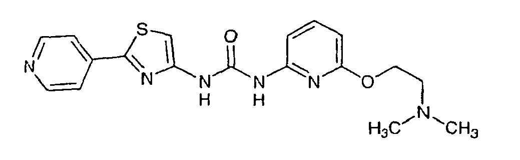

### 1-[6-(2-Dimethylamino-ethoxy)-pyridin-2-yl]-3-(2-pyridin-4-yl-thiazol-4-yl)-urea

EI-MS m/z 385.5 (M+H). Calc'd for C₁₈H₂₀N₆O₂S: 384.14.

### Example 107

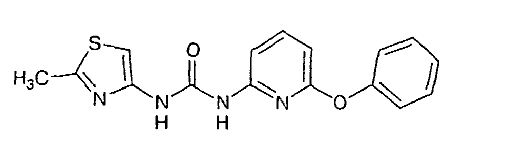

### 1-(2-Methylthiazol-4-yl)-3-(6-phenoxy-pyridin-2-yl)urea

EI-MS m/z 327.4 (M+H). Calc'd for C₁₆H₁₄N₄O₂S: 326.08.

### Example 108

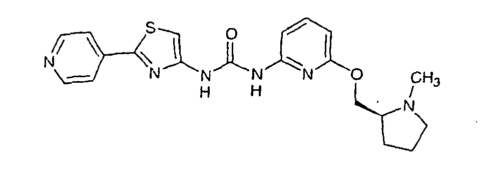

### 1-[6-(1-Methylpyrrolidin-2-ylmethoxy)pyridin-2-yl]-3-(2-pyridin-4-yl-thiazol-4-yl)urea

EI-MS m/z 411.4 (M+H). Calc'd for C₂₀H₂₂N₆O₂S: 410.15.

### Example 109

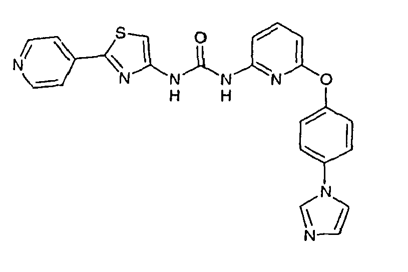

### 1-[6-(4-Imidazol-1-yl-phenoxy)-pyridin-2-yl]-3-(2-pyridin-4-yl-thiazol-4-yl)-urea

EI-MS m/z 456.6 (M+H). Calc' d for C₂₃H₁₇N₇O₂S: 455.12.

### Example 110

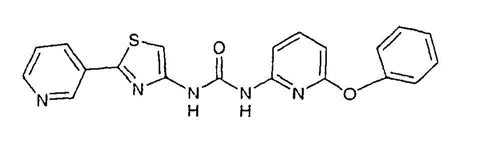

### 1-(6-Phenoxypyridin-2-yl)-3-(2-pyridin-3-yl-thiazol-4-yl)urea

EI-MS m/z 390.5 (M+H). Calc'd for C₂₀H₁₅N₅O₂S: 389.09.

### Example 111

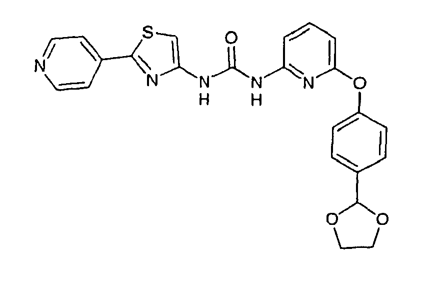

### 1-[6-(4-[1,3]Dioxolan-2-yl-phenoxy)pyridin-2-yl]-3-(2-pyridin-4-yl-thiazol-4-yl)urea

EI-MS m/z 462.5 (M+H). Calc'd for C₂₃H₁₉N₅O₄S: 461.12.

### Example 112

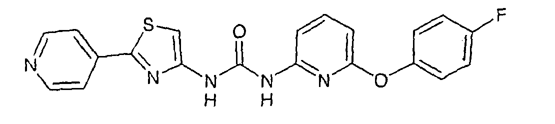

### 1-[6-(4-Fluorophenoxy)pyridin-2-y1]-3-(2-pyridin-4-yl-thiazol-4-yl)urea

EI-MS *m*/*z* 408.5 (M+H). Calc'd for C₂₀H₁₄FN₅O₂S: 407.09.

### Example 113

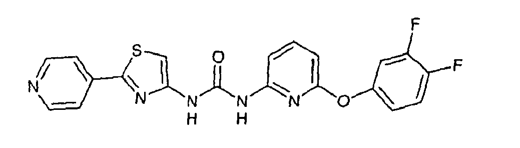

### 1-[6-(3,4-Difluorophenoxy)pyridin-2-yl]-3-(2-pyridin-4-yl-thiazol-4-yl)urea

EI-MS m/z 426.5 (M+H). Calc'd for C₂₀H₁₃F₂N₅O₂S: 425.08.

### Example 114

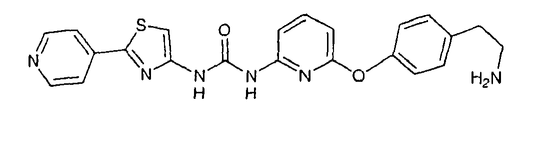

### 1-{6-[4-(2-Aminoethyl)phenoxy]pyridin-2-yl}-3-(2-pyridin-4-yl-thiazol-4-yl)urea

EI-MS m/z 433.5 (M+H). Calc'd for C₂₂H₂₀N₆O₂S: 432.14.

### Example 115

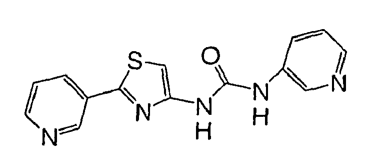

### 1-Pyridin-3-yl-3-(2-pyridin-3-yl-thiazol-4-yl)-urea

EI-MS m/z 396.6 (M+H). Calc'd for C₁₄H₁₁N₅OS: 297.07.

### Example 116

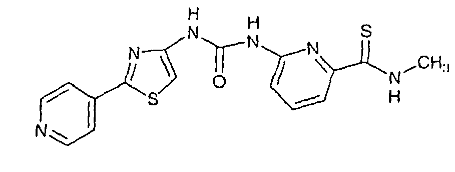

### 6-[3-(2-Pyridin-4-yl-thiazol-4-yl)-ureido]-pyridine-2-carbothioic acid methylamide

EI-MS m/z 371.5 (M+H). Calc'd for C₁₆H₁₄N₆OS₂: 370.07.

### Example 117

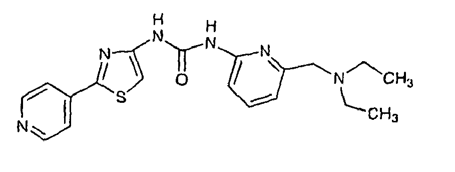

### 1-(6-Diethylaminomethyl-pyridin-2-yl)-3-(2-pyridin-4-yl-thiazol-4-yl)urea

EI-MS m/z 383.5 (M+H). Calc'd for C₁₉H₂₂N₆OS: 382.16.

### Example 118

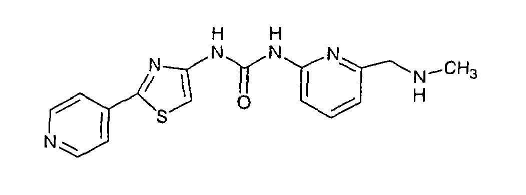

### 1-(6-Methylaminomethyl-pyridin-2-yl)-3-(2-pyridin-4-yl-thiazol-4-yl)urea

EI-MS m/z 341.4 (M+H). Calc'd for C₁₆H₁₆N₆OS: 340.11.

### Example 119

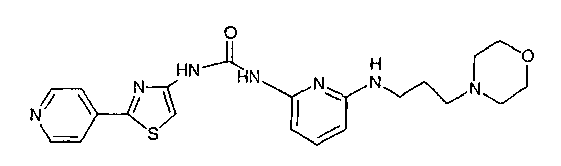

### 1-[6-(3-Morpholin-4-yl-propylamino)-pyridin-2-yl]-3-(2-pyridin-4-yl-thiazol-4-yl)-urea

EI-MS m/z 440.4 (M+H). Calc'd for C₂₁H₂₅N₇O₂S: 439.18.

### Example 120

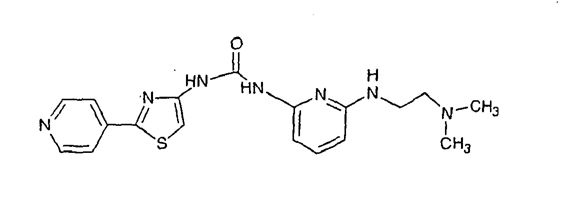

### 1-[6-(2-Dimethylamino-ethylamino)-pyridin-2-yl]-3-(2-pyridin-4-yl-thiazol-4-yl)-urea

EI-MS m/z 384.5 (M+H). Calc'd for C₁₈H₂₁N₇OS: 383.15.

### Example 121

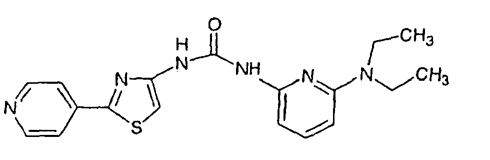

### 1-(6-Diethylamino-pyridin-2-yl)-3-(2-pyridin-4-yl-thiazol-4-yl)-urea

EI-MS m/z 369.3 (M+H). Calc'd for C₁₈H₂₀N₆OS: 368.14.

### Example 122

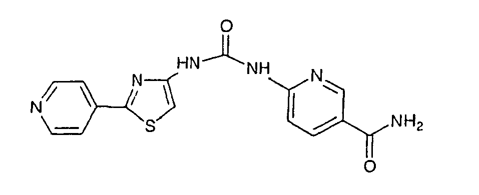

### 6-[3-(2-Pyridin-4-yl-thiazol-4-yl)-ureido]nicotinamide

EI-MS m/z 341.3 (M+H). Calc'd for C₁₅H₁₂N₆O₂S : 340.07.

### Example 123

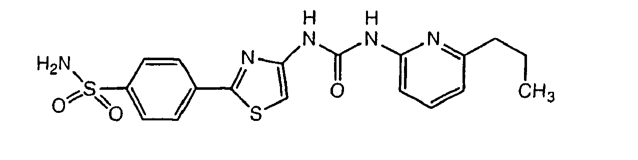

### 4-{4-[3-(6-Propylpyridin-2-yl)ureido]thiazol-2-yl}-benzenesulfonamide

EI-MS m/z = 418.5 (M+H). Calc'd for C₁₈H₁₉N₅O₃S₂: 417.09.

### Example 124

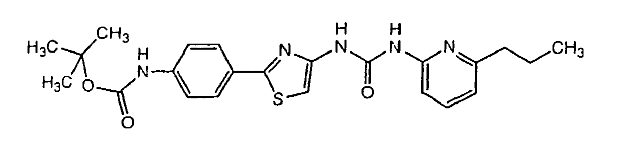

### tert Butyl (4-{4-[3-(6-propylpyridin-2-yl)ureido]-thiazol-2-yl}phenyl)carbamate

EI-MS m/z 454.6 (M+H). Calc' d for C₂₃H₂₇N₅O₃S: 453.18.

### Example 125

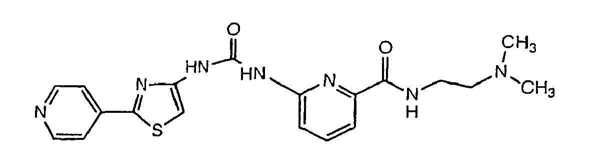

### 2-Dimethylaminoethyl 6-[3-(2-pyridin-4-yl-thiazol-4-yl)ureido]pyridine-2-carboxamide

EI-MS m/z 412.5 (M+H). Calc' d for C₁₉H₂₁N₇O₂S : 411.15.

### Example 126

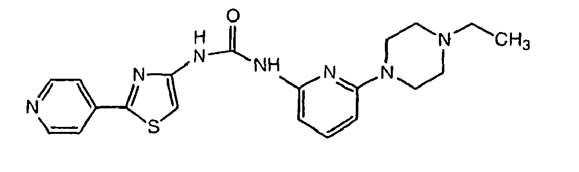

### 1-[6-(4-Ethylpiperazin-1-yl)-pyridin-2-yl]-3-(2-pyridin-4-yl-thiazol-4-yl)urea

EI-MS m/z 410.6 (M+H). Calc'd for C₂₀H₂₃N₇OS: 409.17.

### Example 127

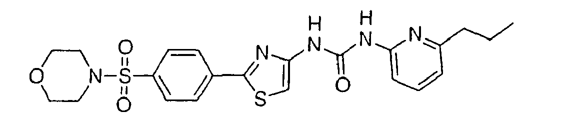

### 1-{2-[4-(4-Morpholinylsulfonyl)phenyl]thiazol-4-yl}-3-(6-propyl-pyridin-2-yl)urea

EI-MS m/z 488.7 (M+H). Calc'd for C₂₂H₂₅N₅O₄S₂: 487.13.

### Example 128

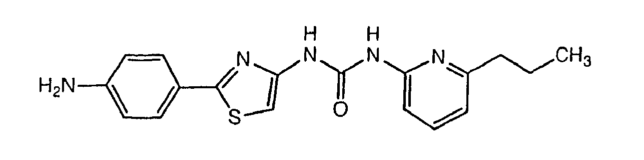

### 1-[2-(4-Aminophenyl)thiazol-4-yl]-3-(6-propylpyridin-2-yl)urea

EI-MS m/z 354.4 (M+H). Calc'd for C₁₈H₁₉N₅OS: 353.13.

### Example 129

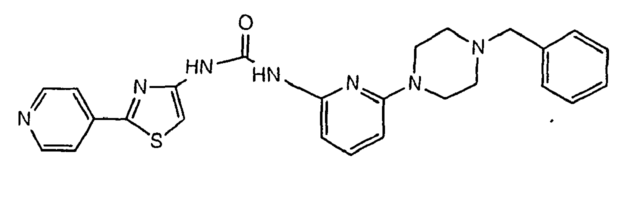

### 1-[6-(4-Benzylpiperazin-1-yl)pyridin-2-yl]-3-(2-pyridin-4-yl-thiazol-4-yl)urea

EI-MS m/z 472.5 (M+H). Calc'd for C₂₅H₂₅N₇OS: 471.18.

### Example 130

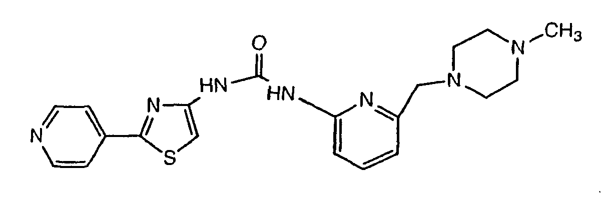

### 1-[6-(4-Methyl-piperazin-1-ylmethyl)-pyridin-2-yl]-3-(2-pyridin-4-yl-thiazol-4-yl)-urea

EI-MS m/z 410.5 (M+H). Calc'd for C₂₀H₂₃N₇OS: 409.17.

### Example 131

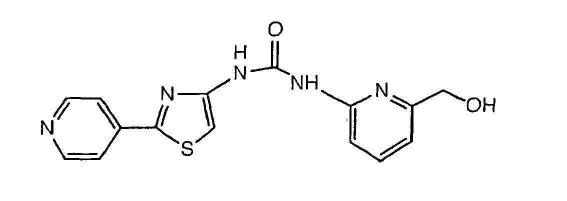

### 1-(6-Hydroxymethyl-pyridin-2-yl)-3-(2-pyridin-4-yl-thiazol-4-yl)-urea

EI-MS m/z 328.4 (M+H). Calc'd for C₁₅H₁₃N₅O₂S: 327.08.

### Example 132

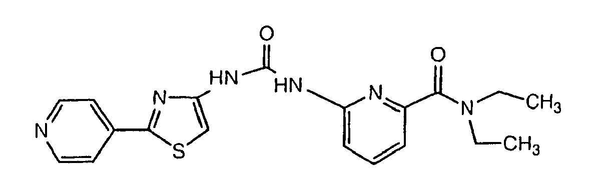

### Diethyl 6-[3-(2-pyridin-4-yl-thiazol-4-yl)ureido]-pyridine-2-carboxamide

EI-MS m/z 397.6 (M+H). Calc'd for C₁₉H₂₀N₆O₂S: 396.14.

### Example 133

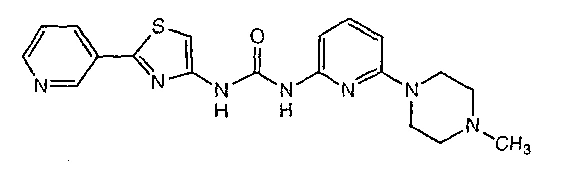

### 1-[6-(4-Methylpiperazin-1-yl)pyridin-2-yl]-3-(2-pyridin-3-yl-thiazol-4-yl)urea

EI-MS m/z 396.5 (M+H). Calc'd for C₁₉H₂₁N₇OS: 395.15.

### Example 134

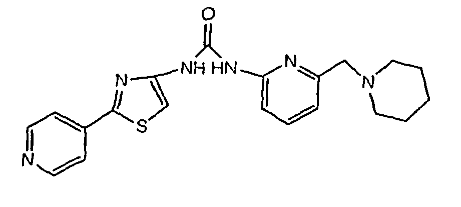

### 1-(6-Piperidin-1-ylmethyl-pyridin-2-yl)-3-(2-pyridin-4-yl-thiazol-4-yl)-urea

EI-MS m/z 395.6 (M+H). Calc'd for C₂₀H₂₂N₆OS: 394.16.

### Example 135

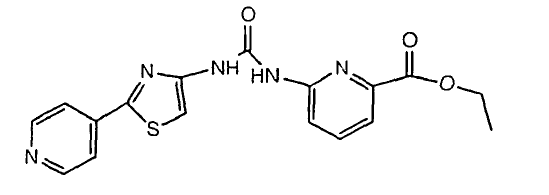

### 6-[3-(2-Pyridin-4-yl-thiazol-4-yl)-ureido]-pyridine-2-carboxylic acid ethyl ester

EI-MS m/z 370.4 (M+H). Calc'd for C₁₇H₁₅N₅O₃S: 309.09.

### Example 136

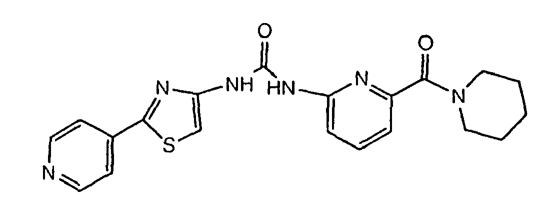

### 1-[6-(Piperidine-1-carbonyl)pyridin-2-yl]-3-(2-pyridin-4-yl-thiazol-4-yl)urea

EI-MS m/z 409.5 (M+H). Calc' d for C₂₀H₂₀N₆O₂S: 408.14.

### Example 137

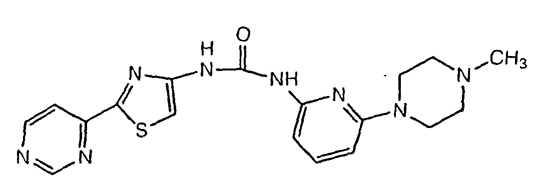

### 1-[6- (4-Methylpiperazin-1-yl)pyridin-2-yl] -3-(2-pyrimidin-4-yl-thiazol-4-yl)urea

EI-MS m/z 397.5 (M+H). Calc'd for C₁₈H₂₀N₈OS: 396.15.

### Example 138

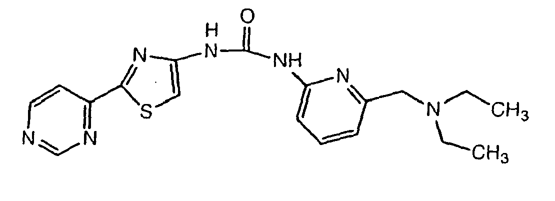

### 1-(6-Diethylaminomethyl-pyridin-2-yl)-3-(2-pyrimidin-4-yl-thiazol-4-yl)urea

EI-MS m/z 384.6 (M+H). Calc'd for C₁₈H₂₁N₇OS: 383.15.

### Example 139

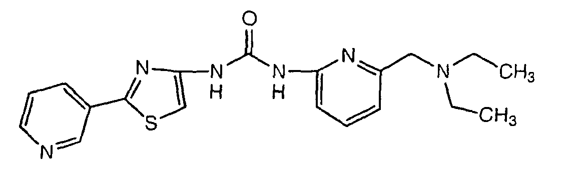

### 1-(6-Diethylaminomethyl-pyridin-2-yl)-3-(2-pyridin-3-yl-thiazol-4-yl)urea

EI-MS m/z 383.5 (M+H). Calc'd for C₁₉H₂₂N₆OS: 382.16.

### Example 140

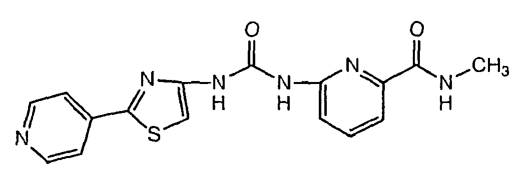

### Methyl 6-[3-(2-pyridin-4-yl-thiazol-4-yl)ureido]pyridine-2-carboxamide

EI-MS m/z 355.3 (M+H). Calc'd for C₁₆H₁₄N₆O₂S: 354.09.

### Example 141

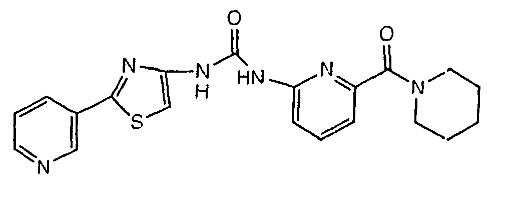

### 1-[6-(Piperidine-1-carbonyl)pyridin-2-yl]-3-(2-pyridin-3-yl-thiazol-4-yl)urea

EI-MS m/z 409.5 (M+H). Calc'd for C₂₀H₂₀N₆O₂S: 402.14.

### Example 142

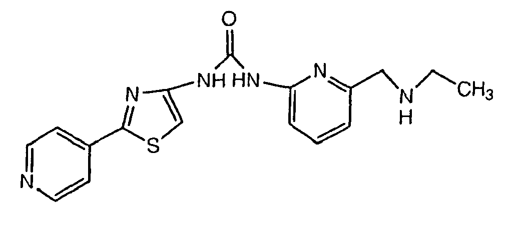

### 1-(6-Ethylaminomethylpyridin-2-yl)-3-(2-pyridin-4-yl-thiazol-4-yl)urea

EI-MS m/z 355.5 (M+H) . Calc'd for C₁₇H₁₆N₆OS: 354.13.

### Example 143

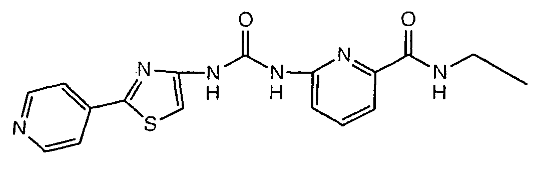

### Ethyl 6-[3-(2-Pyridin-4-yl-thiazol-4-yl)ureido]pyridine-2-carboxamide

EI-MS m/z 369.4 (M+H). Calc'd for C₁₇H₁₆N₆O₂S: 368.11.

### Example 144

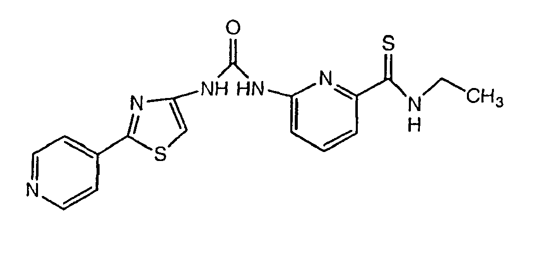

### Ethyl 6-[3-(2-pyridin-4-yl-thiazol-4-yl)ureido]-pyridine-2-thiocarboxamide

EI-MS m/z 385.5 (M+H). Calc'd for C₁₇H₁₆N₆OS₂: 384.08.

### Example 145

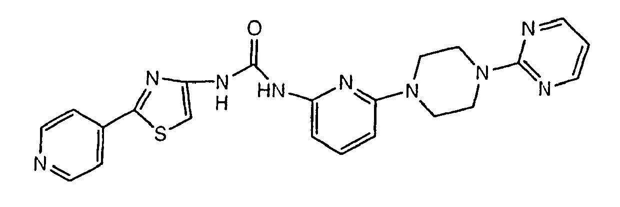

### 1-(2-Pyridin-4-yl-thiazol-4-yl)-3-[6-(4-pyrimidin-2-yl-piperazin-1-yl)pyridin-2-yl]urea

EI-MS m/z 460.5 (M+H). Calc'd for C₂₂H₂₁N₉OS: 459.16.

### Example 146

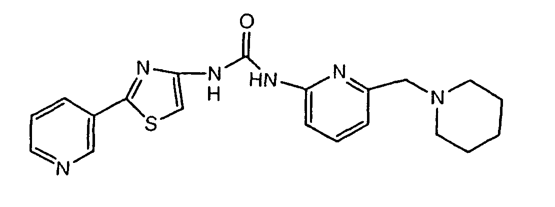

### 1-(6-Piperidin-1-ylmethyl-pyridin-2-yl)-3-(2-pyridin-3-yl-thiazol-4-yl)-urea

EI-MS m/z 395.5 (M+H). Calc'd for C₂₀H₂₂N₆OS: 394.16.

### Example 147

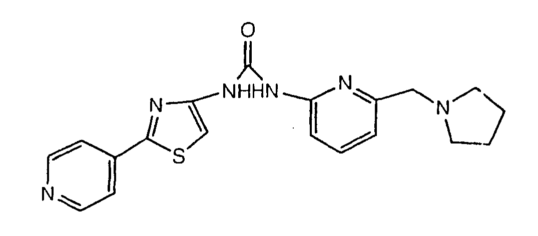

### 1-(2-Pyridin-4-yl-thiazol-4-yl)-3-(6-pyrrolidin-1-ylmethyl-pyridin-2-yl)-urea

EI-MS m/z 381.5 (M+H). Calc'd for C₁₉H₂₀N₆OS: 380.14.

### Example 148

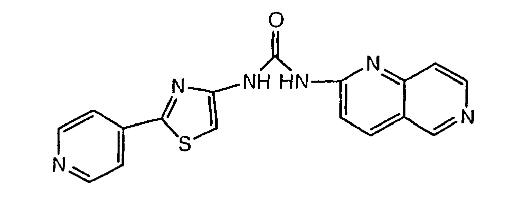

### 1-[1,6]Naphthyridin-2-yl-3-(2-pyridin-4-yl-thiazol-4-yl)-urea

EI-MS m/z 349.5 (M+H). Calc'd for C₁₇H₁₂N₆OS: 348.08.

### Example 149

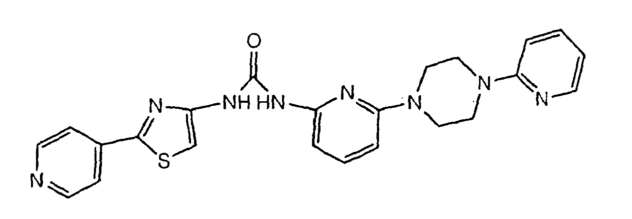

### 1-[6-(4-Pyridin-2-yl-piperazin-1-yl)pyridin-2-yl]-3-(2-pyridin-4-yl-thiazol-4-yl)urea

EI-MS m/z 459.5 (M+H). Calc'd for C₂₃H₂₂N₈OS: 458.16.

### Example 150

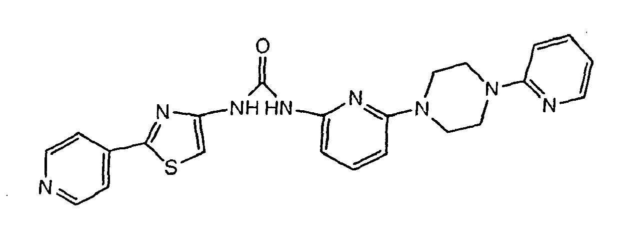

### 1-[6-(4-Pyridin-2-yl-piperazin-1-yl)-pyridin-2-yl]-3-(2-pyridin-4-yl-thiazol-4-yl)-urea

EI-MS m/z 459 . 5 (M+H). Calc'd for C₂₃H₂₂N₈OS: 458.16.

### Example 151

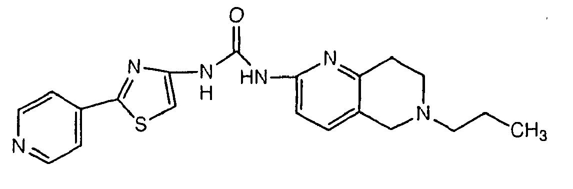

### 1-(6-Propyl-5,6,7,8-tetrahydro-[1,6]naphthyridin-2-yl)-3-(2-pyridin-4-yl-thiazol-4-yl)-urea

EI-MS m/z 395.6 (M+H). Calc'd for C₂₀H₂₂N₆OS: 394.16.

### Example 152

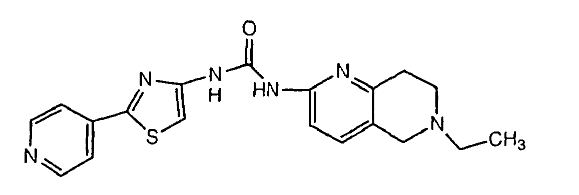

### 1-(6-Ethyl-5,6,7,8-tetrahydro-[1,6]naphthyridin-2-yl)-3-(2-pyridin-4-yl-thiazol-4-yl)-urea

EI-MS m/z 381.5 (M+H). Calc'd for C₁₉H₂₀N₆OS: 380.14.

### Example 153

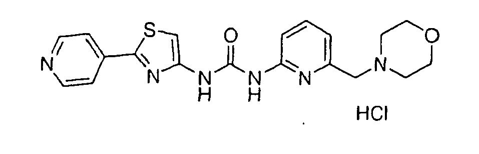

### N-[2-(4-Pyridinyl)-4-thiazolyl]-N'-2-[6-(1-morpholinylmethyl)pyridinyl]urea hydrochloride

To a solution of N-[2-(pyridin-4-yl)-4-thiazolyl]-N'-2-(6-morpholinylmethylpyridinyl)urea (90 mg, 0.23 mmol, Example 60) in MeOH (3 mL) was added HCl (0.25 mL, 0.25 mmol, 1.0 M in Et₂O). The resulting mixture was stirred at RT for 2 h then concentrated in vacuo to give a pale yellow solid.
The following Examples 154-165 were prepared from the corresponding amines in a manner similar to that described above for Example 153:

### Example 154

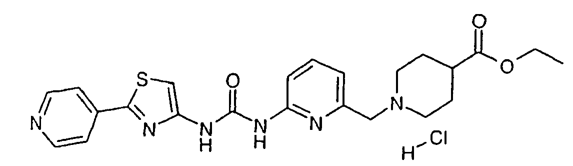

### Ethyl 1-{6-[3-(2-pyridin-4-yl-thiazol-4-yl)ureido]-pyridin-2-ylmethyl}-piperidine-4-carboxylate hydrochloride

Ethyl 1-{6-[3-(2-pyridin-4-yl-thiazol-4-yl)-ureido]-pyridin-2-ylmethyl]-piperidine-4-carboxylate (50 mg, 0.05 mmol, Example 61) in MeOH (5 mL) was treated with HCl (0.12 mL, 0.06 mmol, 1M in Et₂O) to afford the title salt as a yellow solid.

### Example 155

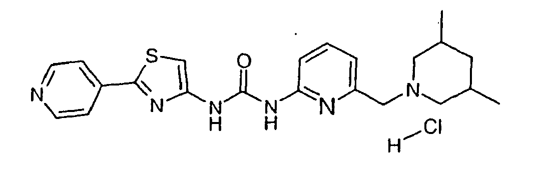

### 1-[6-(3,5-Dimethylpiperidin-1-ylmethyl)pyridin-2-yl]-3-(2-pyridin-4-ylthiazol-4-yl)urea hydrochloride

1-[6-(3,5-Dimethylpiperidin-1-ylmethyl)pyridin-2-yl]-3-(2-pyridin-4-ylthiazol-4-yl)urea (52 mg, 0.123 mmol, Example 64) was treated with HCl (0.08 mL, 0.135 mmol, 1 M in Et₂O) to afford the title salt as a yellow solid.

### Example 156

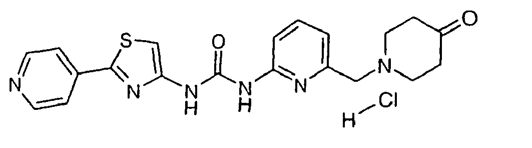

### 1-[6-(4-Oxo-piperidin-1-ylmethyl)pyridin-2-yl]-3-(2-pyridin-4-yl-thiazol-4-yl)urea hydrochloride

1-[6-(4-Oxo-piperidin-1-ylmethyl)pyridin-2-yl]-3-(2-pyridin-4-yl-thiazol-4-yl)urea (30 mg, 0.073 mmol, Example 175) was treated with HCl (0.08 mL, 0.081 mmol, 1M in Et₂O) to afford the title salt as a yellow solid.

### Example 157

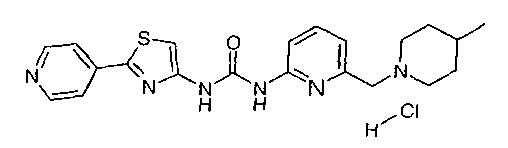

### 1-[6-(4-Methylpiperidin-1-ylmethyl)pyridin-2-yl]-3-(2-pyridin-4-yl-thiazol-4-yl)urea hydrochloride

1-[6-(4-Methylpiperidin-1-ylmethyl)pyridin-2-yl]-3-(2-pyridin-4-yl-thiazol-4-yl)urea (70 mg, 0.171 mmol, Example 65) was treated with HCl (0.19 mL., 0.188 mmol, 1M in Et₂O) to afford the title salt as a yellow solid.

### Example 158

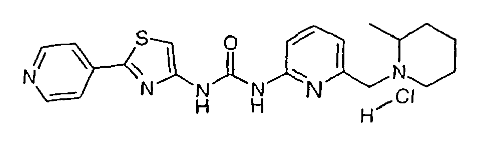

### 1-[6-(2-Methylpiperidin-1-ylmethyl)pyridin-2-yl]-3-(2-pyridin-4-yl-thiazol-4-yl)urea hydrochloride

1-[6-(2-Methylpiperidin-1-ylmethyl)pyridin-2-yl]-3-(2-pyridin-4-yl-thiazol-4-yl)urea (70 mg, 0.171 mmol, Example 66) was treated with HCl (0.19 mL., 0.188 mmol, 1M in Et₂O) to afford the title salt as a yellow solid.

### Example 159

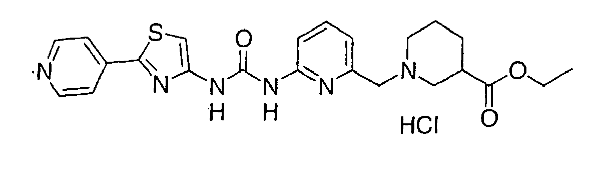

### Ethyl 1-{6-[3-(2-pyridin-4-yl-thiazol-4-yl)ureido]-pyridin-2-ylmethyl}piperidine-3-carboxylate hydrochloride

HCl (0.21 mL, 0.212 mmol, 1.0 M soln in Et₂O) was added to ethyl 1-{6-[3-(2-pyridin-4-yl-thiazol-4-yl)ureido]-pyridin-2-ylmethyl}piperidine-3-carboxylate (90 mg, 0.193 mmol, Example 73) in a solution of MeOH (2 mL) to give a pale yellow solid.

### Example 160

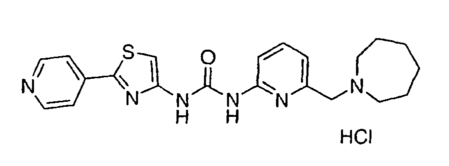

### 1-(6-Azepan-1-ylmethyl-pyridin-2-yl)-3-(2-pyridin-4-yl-thiazol-4-yl)urea hydrochloride

HCl (0.29 mL, 0.28 mmol, 1.0 M soln in Et₂O) was added to 1-(6-azepan-1-ylmethyl-pyridin-2-yl)-3-(2-pyridin-4-yl-thiazol-4-yl)urea (106 mg, 0.26 mmol, Example 71) in a solution of MeOH (4 mL) and the resulting mixture stirred 6 h. Concentration in vacuo gave a yellow solid.

### Example 161

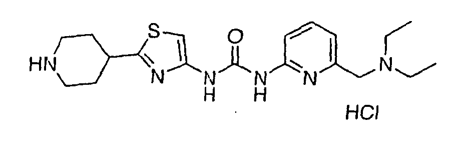

### 1-(6-Diethylaminomethyl-pyridin-2-yl)-3-(2-piperidin-4-yl-thiazol-4-yl)urea hydrochloride

HCl (27 µL, 0.026 mmol, 1.0 M soln in Et₂O) was added to 1-(6-diethylaminomethyl-pyridin-2-yl)-3-(2-piperidin-4-yl-thiazol-4-yl)urea (11 mg, 0.026 mmol, Example 179) in a solution of MeOH (1 mL) and the resulting mixture stirred 3 h. Concentration in vacuo gave a yellow solid.

### Example 162

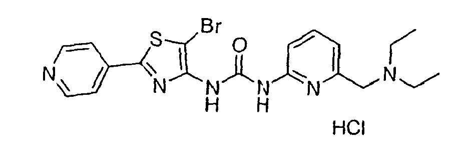

### 1-[5-Bromo-2-(pyridin-4-yl)thiazol-4-yl)-3-(6-diethylaminomethyl-pyridin-2-yl)urea hydrochloride

HCl (54 µL, 0.054 mmol, 1.0 M soln in Et₂O) was added to 1-[5-bromo-2-(pyridin-4-yl)thiazol-4-yl)-3-(6-diethylaminomethyl-pyridin-2-yl)urea (25 mg, 0.054 mmol, Example 180) in a solution of MeOH (0.5 mL) to give a yellow solid.

### Example 163

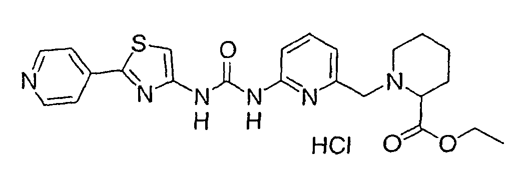

### Ethyl 1-{6-[3-(2-pyridin-4-yl-thiazol-4-yl)-ureido]-pyridin-2-ylmethyl)-piperidine-2-carboxylate hydrochloride

HCl (0.12 mL, 0.12 mmol, 1.0 M soln in Et₂O) was added to ethyl 1-[6-[3-(2-(pyridin-4-yl)thiazol-4-yl)ureido]-pyridin-2-ylmethyl]piperidine-2-carboxylate (50 mg, 0.11 mmol, Example 74) in a solution of MeOH (2 mL) to give a pale yellow solid.

### Example 164

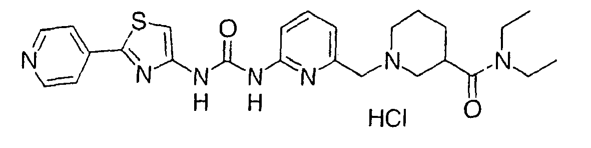

### N,N-Diethyl 1-{6-[3-(2-pyridin-4-yl-thiazol-4-yl)-ureido]pyridin-2-ylmethyl}piperidine-3-carboxamide hydrochloride

HCl (0.15 mL, 0.156 mmol, 1.0 M soln in Et₂O) was added to N,N-diethyl 1-{6-[3-(2-pyridin-4-yl-thiazol-4-yl)-ureido]pyridin-2-ylmethyl}piperidine-3-carboxamide (70 mg, 0.142 mmol, Example 75) in a solution of MeOH (3 mL) to give a pale yellow solid.

### Example 165

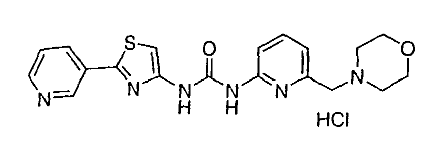

### 1-[6-(Morpholin-4-ylmethyl)-pyridin-2-yl]-3-[(2-pyridin-3-yl)thiazol-4-yl]urea hydrochloride

HCl (55 µL, 0.05 mmol, 1.0 M in Et₂O) was added to 1-[5-(morpholin-4-ylmethyl)-pyridin-2-yl]-3-[(2-pyridin-3-yl)thiazol-4-yl]urea (20 mg, 0.05 mmol, Example 180) in a solution of MeOH (1 mL) and the resulting mixture stirred 3 h. Concentration in vacuo gave a yellow solid.
The following Examples 166-167 were prepared from the corresponding protected amines in a manner similar to that described above for Example 157:

### Example 166

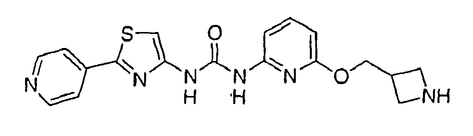

### 1-[6-(Azetidin-3-ylmethoxy)pyridin-2-yl]-3-[2-(pyridin-4-yl)thiazol-4-yl]urea

From tert butyl 3-{6-[3-(2-pyridin-4-yl-thiazol-4-yl)-ureidol-pyridin]-2-yloxymethyl}-azetidine-1-carboxylate (Example 80) EI-MS m/z 382.2 (M+H). Calc'd for C₁₈H₁₈N₆O₂S: 382.12.

### Example 167

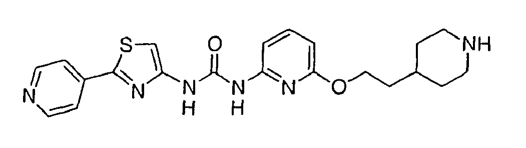

### 1-[6-(2-Piperidin-4-yl-ethoxy)pyridin-2-yl]-3-[2-(pyridin-4-yl)thiazol-4-yl]urea

From tert butyl 4-(2-{6-[3-(2-pyridin-4-yl-thiazol-4-yl)ureido]pyridin-2-yloxy}ethyl)piperidine-1-carboxylate (Example 81) MS m/z: 425 (M+1)⁺. Calc'd for C₂₁H₂₄N₆O₂S: 424.17.

### Example 168

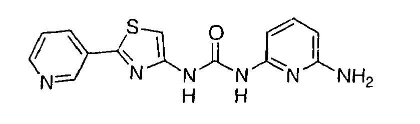

### N-[2-(3-Pyridinyl)-4-thiazolyl]-N'-2-[6-aminopyridin-2-yl]urea

TEA (0.27 mL, 1.94 mmol) was added to a solution of 2-(pyridin-3-yl)thiazole-4-carboxylic acid (200 mg, 0.97 mmol) and 4A molecular sieves in THF (25 mL) under N₂ at RT. (PhO)₂PON₃ (0.33 mL, 1.55 mmol) followed by 2,6-diaminopyridine (265 mg, 2.43 mmol) was added and the resulting mixture was heated at reflux for 12 h. After cooling to RT, the heterogeneous mixture was decanted to remove the molecular sieves. The precipitate was collected, rinsing with EtOAc to give a light tan solid. MS m/z: 313.0 (M+H). Calc'd for C₁₄H₁₂N₆OS: 312.08.
The following compounds were prepared from the corresponding amines in a manner similar to that described above for Example 168:

### Example 169

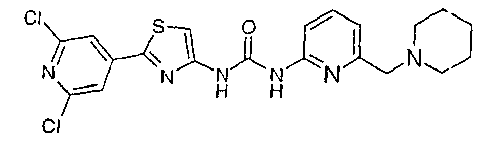

### 1-[2-(2,6-Dichloropyridin-4-yl)thiazol-4-yl]-3-[6-(piperidin-1-ylmethyl)pyridin-2-yl]urea

2-(2,6-Dichloropyridin-4-yl)thiazol-4-carbocylic acid (100 mg, 0.36 mmol), 2-amino-6-piperidinylmethyl-pyridine (76 mg, 0.39 mmol), (PhO)₂PON₃ (0.1 mL, 0.55 mmol), and TEA (0.08 mL, 0.55 mmol) were heated in toluene (15 mL) to yield the title compound as white solid. MS m/z: 464.3 (M+H). Calc'd. for C₂₀H₂₀Cl₂N₆OS - 463.39.

### Example 170

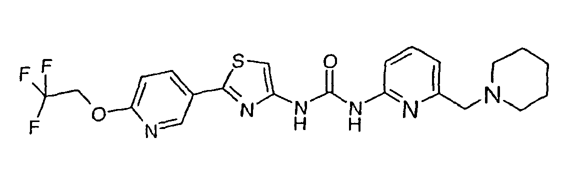

### 1-[6-(Piperidin-1-ylmethyl)pyridin-2-yl]-3-[2-[6-(2,2,2-trifluoroethoxy)pyridin-3-yl]thiazol-4-yl]urea

2-(4-Trifluoroethoxypyridin-4-yl)thiazolyl-4-carboxylic acid (150 mg, 0.49 mmol), 2-amino-6-piperidinylmethyl-pyridine (104 mg, 0.54 mmol), (PhO)₂PON₃ (0.16 mL, 0.74 mmol), and TEA (0.1 mL, 0.74 mmol) were heated in toluene (15 mL) to yield the title compound as white solid. MS *m*/*z:* 493.6 (M+H). Calc'd. for C₂₂H₂₃F₃N₆O₂S - 492.52.

### Example 171

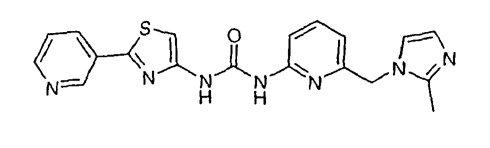

### 1-[6-(2-Methylimidazol-1-ylmethyl)pyridin-2-yl]-3-[2-(pyridin-3-yl)thiazol-4-yl]urea

2-(Pyridin-3-yl)-4-thiazole-4-carboxylic acid (75 mg, 0.36 mmol), 2-amino-6-[2-methylimidazol-1-yl]methyl-pyridine (75 mg, 0.40 mmol), (PhO)₂PON₃ (0.12 mL, 0.54 mmol), and TEA (0.1 mL, 0.54 mmol) were heated in toluene (15 mL) to yield the title compound as light brown solid. MS m/z: 392.3 (M+H). Calc'd. for C₁₉H₁₇N₇OS - 391.45.

### Example 172

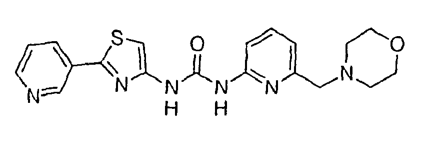

### 1-[6-(Morpholin-4-ylmethyl)-pyridin-2-yl]-3-[(2-pyridin-3-yl)thiazol-4-yl]urea

TEA (0.27 mL, 1.94 mmol) was added to a solution of 2-(pyridin-3-yl)thiazole-4-carboxylic acid (200 mg, 0.97 mmol) and 4A molecular sieves in THF (25 mL) under N₂ at RT. (PhO)₂PON₃ (0.33 mL, 1.55 mmol) followed by 2-amino, 6-morpholinylmethylpyridine (280 mg, 1.45 mmol) was added and the resulting mixture was heated at reflux for 12 h. After cooling to RT, the heterogeneous mixture was decanted to remove the molecular sieves. The precipitate was collected, rinsed with EtOAc and purified by chromatography on silica gel (CH₂Cl₂/MeOH, 95:5) to give a white solid. MS m/z: 397.1 (M+H). Calc'd for C₁₉H₂₀N₆O₂S - 396.47.

### Example 173

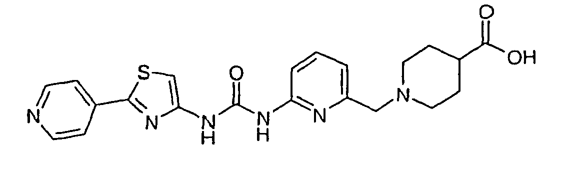

### 1-{6-[3-(2-(4-Pyridinyl)-4-thiazolyl)ureido]-pyridin-2-ylmethyl}-piperidine-4-carboxylic acid

Ethyl 1-(6-[3-(2-(pyridin-4-yl)thiazol-4-yl)ureido]-pyridin-2-ylmethyl}-piperidine-4-carboxylate (55 mg, 0.12 mmol, Example 61) was suspended in MeOH (10 ml) followed by adding LiOH (50 mg, 1.18 mmol) in H₂O (1 ml). The resulting mixture was heated at 45 ^{°}C for 15 h. After cooling to RT, the solvent was removed. The residue was suspended in H₂O (20 mL). The pH was adjusted to 7 using HCl (1N). The resulting mixture was extracted with CHCl₃: IpOH (3:1). The organic layer was washed with H₂O and brine. After being dried over anhydrous MgSO₄, the solvent was removed *in vacuo* to yield the final compound as light yellow solid. MS m/z: 438.7 (M+H). Calc'd. for C₂₁H₂₂N₆O₃S - 438.51.

### Example 174

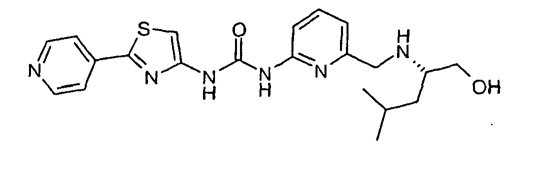

### 1-{6-[(1-Hydroxymethyl-3-methylbutylamino)methyl]-pyridin-2-yl}-3-(2-pyridin-4-yl-thiazol-4-yl)urea

tert-Butyl (1-hydroxymethyl-3-methyl-butyl)-f6-[3-(3-pyridin-4-yl-thiazol-4-yl)-ureido]-pyridin-2-ylmethyl)-carbamate (165 mg, 0.313 mmol, Example 62) in MeOH (5 mL) was treated with HCl (0.16 mL, 0.627 mmol, 4M in dioxane). The resulting stirred solution was heated at 40°C in closed system for 15 h. After cooling to RT, the pH was adjusted to 7 using 1 N NaOH. Solvent was removed and the residue was extracted with CHCl₃. The organic layer was washed with H₂O, brine, dried over MgSO₄, and concentrated to yield a brown liquid crude product. This crude product was purified by chromatography on silica gel. Elution with CH₂Cl₂:MeOH mixture (95:5) gave final compound as a tan solid. MS *m*/*z:* 427.2 (M+H) Calc'd. for C₂₁H₂₆N₆O₂S - 426.54.

### Example 175

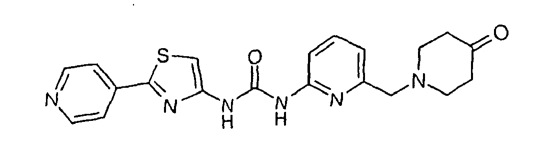

### 1-[6-(4-Oxo-piperidin-1-ylmethyl)pyridin-2-yl]-3-(2-pyridin-4-yl-thiazol-4-yl)urea

N-[2-(4-Pyridinyl)-4-thiazolyl]-N'-2-[6-(4-ethoxyacetal)piperidylmethyl]urea (300 mg, 0.66 mmol) in THF (15 mL) was treated with 5N HCl (5 mL). The resulting mixture was heated to reflux under N₂ for 5 h. After cooling to RT, the mixture was basified using 5 N NaOH. Solvent was removed and the residue was extracted with CHCl₃. The organic layer was washed with H₂O, brine, dried over MgSO₄, and concentrated to yield a pale yellow solid. MS m/z: 409.3(M+H). Calc'd. for C₂₀H₂₀N₆O₂S - 408.32.

### Example 176

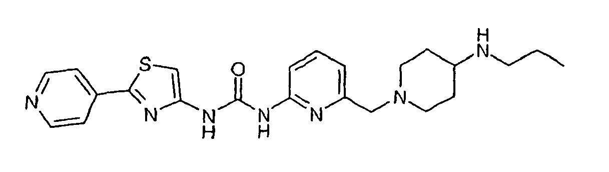

### 1-[6-[4-(Propylamino)piperidin-1-ylmethyl]pyridin-2-yl]-3-(2-pyridin-4-yl-thiazol-4-yl)urea

To a suspension of N-[2-(4-pyridinyl)-4-thiazolyl]-N'-2-[6-(piperidon-4-yl)methyl]urea (50 mg, 0.12 mmol, Example 175) in MeOH (10 mL) was added propylamine (0.1 mL, 1.22 mmol). The resulting mixture was heated at 50ºC for 4 h under N₂. After the mixture was cooled to RT, NaBH₄ (83 mg, 2.20 mmol) was added. The mixture was stirred at RT under N₂ for 3 h.
Solvent was removed in vacuo and the crude product was purified by chromatography on silica gel. Elution with CH₂Cl₂:MeOH (90:10) gave the title compound as a white solid. MS *mlz:* 451.7 (M+H). Calc'd. for C₂₃H₂₉N₇OS - 451.6.

### Example 177

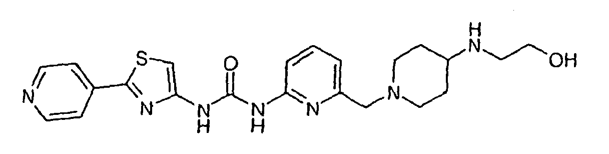

### 1-{6-[4-(2-Hydroxyethylamino)piperidin-1-ylmethyl]-pyridin-2-yl}-3-(2-pyridin-4-yl-thiazol-4-yl)urea

N-[2-(4-Pyridinyl)-4-thiazolyl]-N'-2-(6-(piperidon-4-yl)methyl]urea (60 mg, 0.147 mmol, Example 175) and ethanolamine (0.09 mL, 1.47 mmol) were heated in MeOH (10 mL) yielded the title compound as pale yellow solid. MS *m*/*z*: 454.6 (M+H). Calc'd. for C₂₂H₂₇N₇O₂S - 453.57.

### Example 178

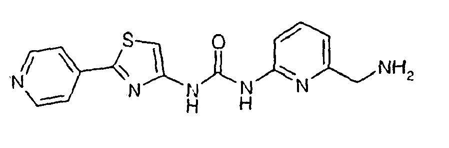

### N-(6-Aminomethyl-2-pyridyl)-N'-[2-(4-pyridinyl)-4-thiazolyl] urea

Pd(OH)₂ (70 mg, 0.5 mmol) was suspended in EtOH (5 mL) followed by adding N-(6-azidomethyl-2-pyridyl)-N'-[2-(4-pyridinyl)-4-thiazolyl]urea (70 mg, 0.198 mmol, Example 69) in EtOH (8 mL). The resulting mixture was heated at 45°C under H₂ balloon for 3 h. After cooling to RT, the mixture was filtered by passing through 2 layers of pleated filtered papers. Solvent was removed *in vacuo* to yield the final compound as a yellow solid. MS *m*/*z*: 327.3 (M+H). Calc'd. for C₁₅H₁₄N₆OS - 326.38.

### Example 179

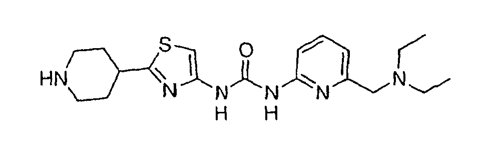

### 1-(6-Diethylaminomethyl-pyridin-2-yl)-3-(2-piperidin-4-yl-thiazol-4-yl)urea

Lithium triethylborohydride (0.84 mL, 0.84 mmol, 1.0 M in THF) was added to a solution of 1-(6-diethylamino-methyl-pyridin-2-yl)-3-(2-pyridin-4-yl-thiazol-4-yl)urea (100 mg, 0.24 mmol, Example 117) and DIEA (63 µL, 0.36 mmol) in THF (5 mL) and the resulting mixture was *stirred* 6 h at RT. The reaction was quenched via dropwise addition of MeOH and concentrated in vacuo. Purification by preparative HPLC (5-60% CH₃CN/H₂O) gave a white solid. MS m/z: 389.2 (M+H). Calc'd for C₁₉H₂₈N₆OS - 388.53.

### Example 180

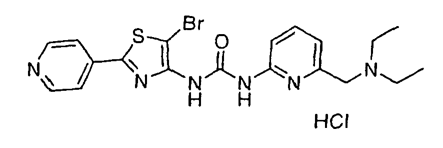

### 1-[5-Bromo-2-(pyridin-4-yl)thiazol-4-yl)-3-(6-diethylaminomethyl-pyridin-2-yl)urea

Bromine (46 µL, 0.90 mmol) was added to a solution of 1-(6-diethylaminomethyl-pyridin-2-yl)-3-(2-pyridin-4-yl-thiazol-4-yl)urea (190 mg, 0.45 mmol, Example 117) in MeOH (8 mL) and the resulting solution was stirred at RT for 1 h. The reaction was quenched with saturated sodium bisulfite solution and concentrated in vacuo. The residue was dissolved in CHCl₃/IpOH (3/1, 10 mL) and washed with H₂O (3x10 mL) followed by 1N NaOH solution (10 mL). The organics were combined, dried over Na₂SO₄, and concentrated in vacuo to give a yellow solid. MS *m*/*z:* 461.1 (M+H). Calc'd for C₁₉H₂₁BrN₆OS - 461.39.

### Example 181

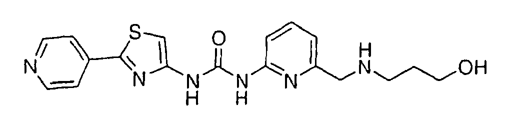

### 1-(6-[(3-Hydroxypropylamino)methyl]-pyridin-2-yl)-3-(2-pyridin-4-yl-thiazol-4-yl)urea

### Step A

2-(4-Pyridinyl)-4-thiazolcarbonylazide (220 mg, 0.78 mmol) and 2-amino-6-[(N"-tert-butoxycarbonyl-N"-3-hydroxypropyl)amino]methylpyridine (196 mg, 0.94 mmol) in dry toluene (10 mL) were heated at 100°C for 12 h to give a pale yellow solid which was purified by chromatography on silica gel (CH₂Cl₂/MeOH, 95:5) to give N-[2-(pyridin-4-yl)-4-thiazolyl)-N'-2-[6-(N"-*tert*-butoxycarbonyl-N''-(3-hydroxypropyl)-amino]methyl-pyridinyl urea as a white solid. MS *m*/*z*: 485.2 (M+H). Calc'd for C₂₃H₂₈N₆O₄S - 484.58.

### Step B

HCl (112 µL, 0.112 mmol, 1.0 M in Et₂O) was added to a solution of N-[2-(pyridin-4-yl)-4-thiazolyl]-N'-2-[6-(*N*''-*tert*-butoxycarbonyl-*N*''-(3-hydroxypropyl)-amino]methylpyridinyl urea (25 mg, 0.051 mmol, Step A) in MeOH (1 mL) and the resulting mixture was heated at 45°C for 12 h. A yellow precipitate formed and was filtered off, rinsing with hexane. The precipitate was added to CH₂Cl₂ (15 mL) and washed with 1N NaOH solution (5 mL). The organics were collected, dried over Na₂SO₄ and concentrated in vacuo to give a pale yellow solid. MS m/z: 385.0 (M+H). Calc' d for C₁₈H₂₀N₆O₂S - 384. 62.

### Example 182

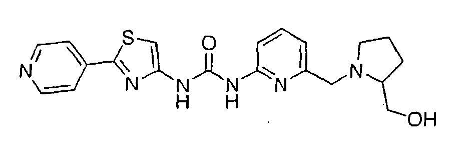

### 1-[6-(2-Hydroxymethylpyrrolidin-1-ylmethyl)-pyridin-2-yl]-3-(2-pyridin-4-yl-thiazol-4-yl)urea

LiAlH₄ (3 mg, 0.079 mmol) was added to a solution of methyl 1-{6-[3-(2-pyridin-4-yl-thiazol-4-yl)ureido]-pyridin-2-ylmethyl}-pyrrolidine-2-carboxylate (15 mg, 0.034 mmol, Example 77) in THF (5 mL) at RT and the resulting mixture was stirred for 8 h. A precipitate formed and was collected. The solid was dissolved in CHCl₃ (5 mL) and washed with saturated NaHCO₃ solution (5 mL). The aqueous layer was adjusted to pH 7 with 1N HCl and extracted with CHCl₃. The organics were combined, dried over MgSO₄ and concentrated in vacuo to give a pale yellow solid. MS *m*/*z*: 411.1 (M+H). Calc'd for C₂₀H₂₂N₆O₂S - 410.50.

### Example 183

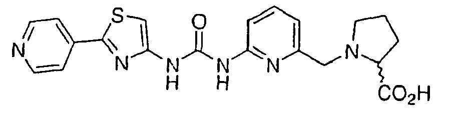

### 1-{6-[3-(2-Pyridin-4-yl-thiazol-4-yl)ureido]-pyridin-2-ylmethyl)-pyrrolidine-2-carboxylic acid

A 1.0 N NaOH solution (0.40 mL) was added to a solution of methyl 1-{6-[3-(2-pyridin-4-yl-thiazol-4-yl)ureido]-pyridin-2-ylmethyl}pyrrolidine-2-carboxylate (3 mg, 6.84 µM, Example 77) in NeOH (1 mL) and the resulting mixture stirred at RT for 12 h. The mixture was adjusted to pH 7 with 1N HCl solution and concentrated in vacuo. The residue was dissolved in CH₂Cl₂ and a few drops of MeOH. A precipitate formed and was collected to give a white solid. MS m/z: 423.5 (M-H) Calc'd for C₂₀H₂₀N₆O₃S - 424.48.

### Example 184

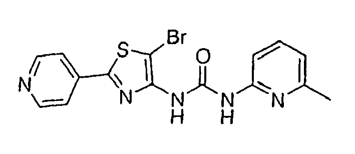

### 1-(5-Bromo-(2-pyridin-4-yl)thiazol-4-yl)-3-(6-methylpyridin-2-yl)urea

NBS (686 mg, 3.85 mmol) and AIBN (158 mg, 0.96 mmol) were added to a heterogeneous solution of 1-((2-pyridin-4-yl)thiazol-4-yl)-3-(6-methylpyridin-2-yl)urea (600 mg, 1.93 mmol, Example 6) in CCl₄(25 mL) and the resulting mixture was heated at reflux for 2 h. After cooling to RT, a precipitate formed and was collected, rinsing with hexane to give a white solid. MS *m*/*z:* 392.0 (M+2H). Calc' d for C₁₅H₁₂BrN₅OS - 390.26.

### Example 185

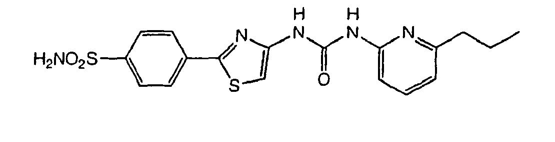

### 4-{4-[3-(6-Propyl-pyridin-2-yl)-ureido]-thiazol-2-yl}-benzenesulfonamide

In an oven-dried, 50-mL, round-bottomed flask were placed 2-(p-sulfamoylphenyl)thiazole-4-carboxylic acid (250 mg, 0.82 mmol), molecular sieves (800 mg) in THF (20 mL). To this mixture was added Et₃N (0.23 mL, 1.64 mmol), followed by DPPA (0.28 mL, 1.28 mmol). The reaction was stirred for 5 min, then 6-propylpyridine-2-amine (280 mg, 2.06 mmol) was added. The suspension was heated to 75ºC for 14 h, cooled to RT, diluted with H₂O (10 mL) and EtOAc (150 mL), and filtered to remove molecular sieves. The filtrate was concentrated *in vacuo* to give the crude product as a yellow solid which was filtered, washed with H₂O (3 × 10 mL), EtOAc (1 × 10 mL) and Et20 (3 × 10 mL) to afford the title compound as a yellow solid. MS *m*/*z*: 418 (M+H). Calc'd for C₁₈H₁₉N₅O₃S₂: 417.09.

### Example 186

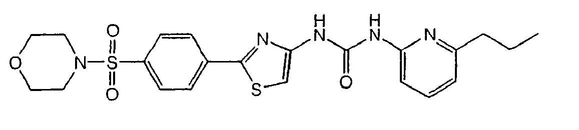

### 1-{2-[4-(4-Morpholinylsulfonyl)phenyl]thiazol-4-yl}-3-(6-propylpyridin-2-yl)urea

In a manner similar to that described for the preparation of Example 185, 2-[(4-morpholinylsulfonyl)-phenyl]thiazole-4-carboxylic acid (354 mg) was treated with DPPA and 6-propylpyridine-2-amine to give the title compound. MS m/z: 488 (M+H). Calc'd for C₂₂H₂₅N₅O₄S₂: 487.13.

### Example 187

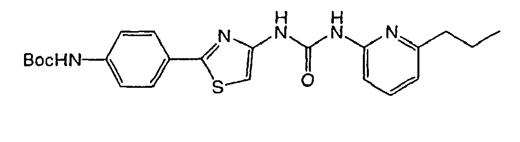

### tert-Butyl (4-{4-[3-(6-propylpyridin-2-yl)ureido]-thiazol-2-yl}phenyl)carbamate

In a manner similar to that described for the preparation of Example 185, 2-[4-[N-Boc-amino]-phenyl]-thiazole-4-carboxylic acid (130 mg) was treated with DPPA and 6-propylpyridine-2-amine to give the title compound. MS m/z: 454.5 (M+H). Calc'd for C₂₃H₂₇N₅O₃S: 453.18.

### Example 188

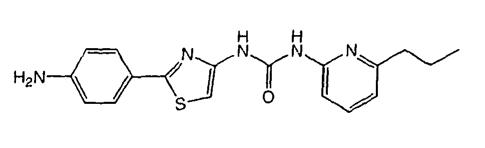

### 1-[2-(4-Aminophenyl) thiazol-4-yl]-3-(6-propylpyridin-2-yl)urea

In an oven-dried, 25-mL, round-bottomed flask were placed N-[6-propylpyridine]-N'-[4-[N-Bocamino]pheny]-4-thiazolyl]urea (55 mg, 0.12 mmol, Example 187), thioanisole (0.35 mL) in CH₂Cl₂ (10 mL). TFA (0.35 mL) was added, the mixture was stirred at RT for 6 h then concentrated in vacuo. Purification by flash chromatography on silica gel [EtOAc/hexane (extracted with aq. NH₄OH), 40:60] afforded the title compound. MS m/z: 354.0 (M+H). Calc'd for C₁₈H₁₉N₅OS: 353.13.

### Example 189

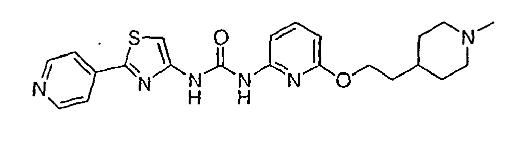

### 1-{6-[2-(1-Methylpiperidin-4-yl)ethoxy]pyridin-2-yl}-3-(2-pyridin-4-yl-thiazol-4-yl)urea

A mixture of N-[2-(4-pyridinyl)-4-thiazolyl]-N'-2-[6-(4-piperidinylethoxy)pyridinyl]urea (0.17 g, 0.40 mmol, Example 167), paraformaldehyde (0.17 g), and NaBH(OAc)₃ (0.21 g, 1.0 mmol) in 40 mL of CH₂Cl₂ was stirred at RT under N₂ for 12 h. After 12 h, the solvent was removed in vacuo, and the residue was diluted with 20 mL of H₂O, then extracted with CHCl₃/IpOH (3:1, 3X20 mL). The combined organic portions were washed with brine, and dried over MgSO₄, and the solvents were removed in vacuo to yield a residue. Purification over silica gel (gradient, 5 % to 7.5% MeOH/CH₂Cl₂ with 0.5% of TEA) provided the title compound as an off-white solid. MS m/z: 439 (M+H) . Calc'd for C₂₂H₂₆N₆O₂S: 438.18.

### Example 190

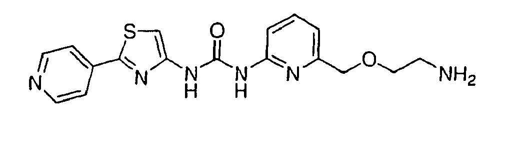

### 1-[6-(2-Aminoethoxymethyl)pyridin-2-yl]-3-(2-pyridin-4-yl-thiazol-4-yl)urea

Prepared in a manner similar to that described in Example 189. MS *m*/*z*: 371 (M+H). Calc'd for C₁₇H₁₈N₆O₂S: 370.12.

### Example 191

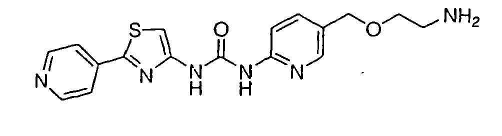

### 1-[5-(2-Aminoethoxymethyl)pyridin-2-yl]-3-(2-pyridin-4-yl-thiazol-4-yl)urea

Prepared in a manner similar to that described in Example 189. MS *mlz:* 371 (M+H) . Calc'd for C₁₇H₁₈N₆O₂S: 370.12.

### Example 192

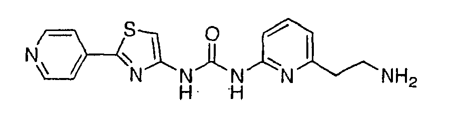

### 1-{6-[2-Aminoethyl]pyridin-2-yl}-3-(2-pyridin-4-yl-thiazol-4-yl)urea

To a mixture of 1-{6-[2-(phthalimidyl)ethyl]-pyridin-2-yl}-3-(2-pyridin-4-yl-thiazol-4-yl)urea (75 mg, 0.16 mmol, Example 88) and EtOH (10 mL) was added hydrazine hydrate (0.1 mL, 0.18 mmol). The mixture was heated at reflux for 2 h then was cooled to RT. The residue was dissolved in 3:1 CHCl₃/IPOH, washed with saturated NaHCO₃; dried (MgSO₄) and concentrated in vacuo to afford the title compound as a yellow solid. MS m/z: 341.0 (N+H). Calc'd for C₁₆H₁₆N₆OS: 340.11.

### Example 193

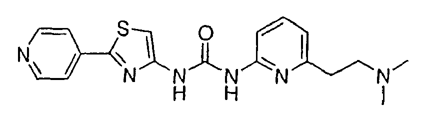

### 1-{6-[2-(N,N-Dimethylamino)ethyl]pyridin-2-yl}-3-(2-pyridin-4-yl-thiazol-4-yl)urea

To a solution of 1-{6-[2-aminoethyl]pyridin-2-yl}-3-(2-pyridin-4-yl-thiazol-4-yl)urea (20 mg, 0.06 mmol, Example 192) and CH₂Cl₂ (5 mL) was added paraformaldehyde (20 mg) and NaBH(OAc)₃ (30 mg, 0.14 mmol). The mixture was stirred at RT for 2.5 h. Extracted with 3:1 CHCl₃/IPOH and washed with brine; dried (MgSO₄) and concentrated in vacuo to afford the desired compound as a yellow solid. MS m/z: 369.1 (M+H). Calc'd for C₁₈H₂₀N₆OS: 368.14.

### Example 194

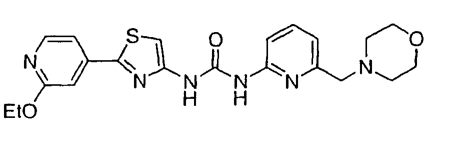

### 1-[2-(2-Ethoxypyridin-4-yl)thiazol-4-yl]-3-(6-morpholin-4-ylmethyl-pyridin-2-yl)urea

To a mixture of 1-[2-(2-chloropyridin-4-yl)thiasol-4-yl]-3-(6-morpholin-4-ylmethyl-pyridin-2-yl)urea (100 mg, 0.23 mmol, Example 90) and EtOH (50 mL) was added a 21 wt% NaOEt/EtOH solution (0.4 mL, 1.2 mmol) and DMF (2 mL). The mixture was heated to reflux for 15 h then additional 21 wt% NaOEt/EtOH solution (10 mL) were added. After 2.5 h, the reaction was complete as judged by LC/MS. The reaction mixture was concentrated in vacuo then diluted with EtOAc and the solid was filtered off. The filtrate was concentrated in vacuo to afford an orange slushy oil which was purified by silica flash chromatography (5-10% MeoH/CH₂Cl₂) to afford the title compound as a yellow solid. MS m/z: 441.1 (M+H). Calc'd for C₂₁H₂₄N₆O₃S: 440.16.

### Example 195

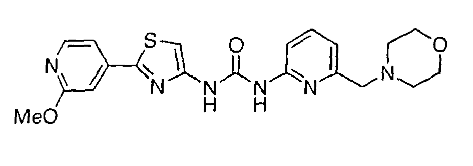

### 1-[2-(2-Methoxypyridin-4-yl)thiazol-4-yl]-3-(6-morpholin-4-ylmethyl-pyridin-2-yl)urea

To a mixture of 1-[2-(2-chloropyridin-4-yl)thiazol-4-yl]-3-(6-morpholin-4-ylmethyl-pyridin-2-yl)urea (100 mg, 0.23 mmol, Example 90) and MeOH (50 mL) was added solid NaOMe (1.6 g, 29.6 mmol) and DMF (20 mL). The reaction mixture was heated to 130ºC. After 2 h, the reaction mixture was cooled to RT and filtered. The filtrate was concentrated in vacuo and diluted with EtOAc and filtered to remove the solid. The filtrate was concentrated in vacuo to afford an orange oil which was purified by silica flash chromatography (5% MeOH/ CH₂Cl₂) to afford the title compound as a white solid. MS m/z: 427.2 (M+H). Calc'd for C₂₀-H₂₂-N₆O₃S: 426.15.

### Example 196

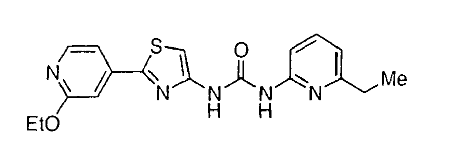

### 1-[2- (2-Ethoxypyridin-4-yl) thiazol-4-yl]-3-(6-ethyl-pyridin-2-yl)urea

To a 10 mL round bottom flask containing 1-[2-(2-chloropyridin-4-yl)thiazol-4-yl]-3-(6-ethylpyridin-2-yl)urea (40 mg, 0.11 mmol) (prepared similar to that described for Example 95) was charged a 21 wt% NaOEt/EtOH solution (5 mL). The reaction mixture was heated to reflux. After 2 h, the reaction mixture was cooled to RT and diluted with H₂O then concentrated in vacuo. The solid residue was washed with CH₂Cl₂ and EtOAc then the solid was diluted with MeOH and concentrated in vacuo. The residue was diluted with EtOAc; washed with saturated NH₄Cl and H₂O; dried (MgSO₄) and concentrated in vacuo to afford the title compound as a light-orange solid. MS m/z: 370.2 (M+H). Calc'd for C₁₈H₁₉N₅O₂S: 369.13.

### Example 197

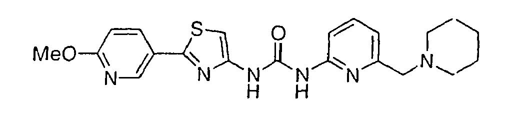

### 1-[2-(6-Methoxypyridin-3-yl)thiazol-4-yl]-3-(6-piperidin-1-ylmethyl-pyridin-2-yl)urea

To a solution of the 3-(4-methoxy-3-pyridyl)thiazole carboxylic acid (200 mg, 0.85 mmol) and dry toluene (20 mL) was added (PhO)₂PON₃ (0.2 mL, 0.94 mmol) and TEA (0.13 mL, 0.94 mmol). The mixture was heated to 85°C for five min then 2-amino-6-methylpiperdinylpyridine (0.16 g, 0.85 mmol) in CH₃CN (3 mL) was added. The reaction was heated at reflux for 15 h then concentrated in vacuo and purified by silica flash chromatography (1% to 5% MeOH/CH₂Cl₂) to give the title compound as an orange oil. Diluted with MeOH (5 mL) and added one equivalent of 1M HCl in Et₂O. Concentrated in vacuo to afford the HCl salt as an orange solid. MS m/z: 424.9 (M+H). Calc'd for C₂₁H₂₄N₆O₂S: 424.17.

### Example 198

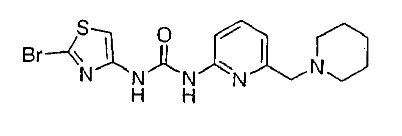

### 1-(2-Bromothiazol-4-yl)-3-(6-piperidin-1-ylmethyl-pyridin-2-yl)urea

To a stirred suspension of 2-bromothiazole-4-carboxylic acid (5.13 g, 2 mmol) in anhydrous CH₃CN (40 ml) at RT, under N₂, TEA (3.80 ml, 27 mmol) and (PhO)₂PON₃ (5.90 ml, 27 mmol) were added. The resulting solution was heated to 85°C. Upon reaching 85°C, a solution of 6-(piperidylmethyl)-2-pyridylamine (4.74 g, 25 mmol) in anhydrous CH₃CN (60 ml) was added. The reaction was maintained at this temperature for 2.25 h. After cooling to RT the mixture was diluted with CH₂Cl₂ (50 ml) then washed with a saturated solution of NH₄Cl (aq) (40 ml). The organic layer was separated, dried over MgSO₄, filtered and concentrated under reduced pressure. The residue was purified by flash chromatography on silica gel (3:1/2:1/1:1, EtOAc:acetone) to yield the title compound as a pale yellow solid. MS m/z: 396 (M+H), 398 (M+3). Calc'd for C₁₅H₁₈BrN₅OS: 395.04.

### Example .199

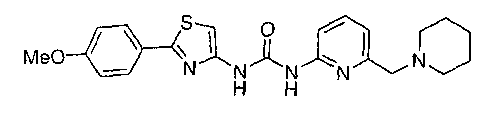

### 1-[2-(4-Methoxyphenyl)thiazol-4-yl]-3-(6-piperidin-1-ylmethyl-pyridin-2-yl)urea

A stirred suspension of N-(2-bromo(1,3-thiazol-4-yl)) {[6-(piperidylmethyl) (2-pyridyl)]amino}carboxamide (2.23 g, 5.64 mmol), 4-methoxyphenylboronic acid (0.94 g, 6.21 mmol), PdCl₂ (dppf)₂ (0.46 g, 0.56 mmol) and Na₂CO₃ (2.10 g, 17.0 mmol) in ethylene glycol dimethyl Et₂O (25 ml) and H₂O (8 ml) was heated at reflux for 12h. After cooling to RT the mixture was filtered through Celite®. The filtrate was concentrated under reduced pressure. The crude product was purified by flash chromatography on silica gel (3:1, EtOAc:acetone) to yield the title compound as a pale yellow amorphous solid. MS m/z: 424 (M+H). Calc' d for C₂₂H₂₅N₅O₂S: 423.17.
The following compounds were prepared from the corresponding boronic acids in a manner similar to Example 199:

### Example 200

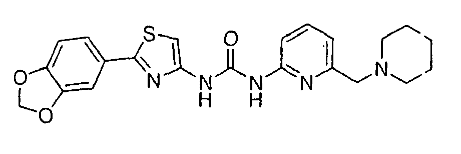

### 1-(2-Benzo[1,3]dioxol-5-yl-thiazol-4-yl)-3-(6-piperidin-1-ylmethyl-pyridin-2-yl)-urea

MS m/z: 438 (M+H). Calc'd for C₂₂H₂₃N₅O₃S: 437.15.

### Example 201

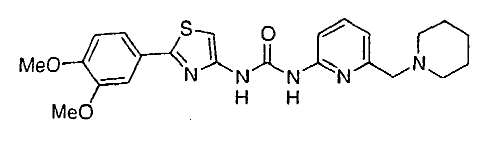

### 1-[2-(3,4-Dimethoxyphenyl)thiazol-4-yl]-3-(6-piperidin-1-ylmethyl-pyridin-2-yl)urea

MS m/z: 454 (M+H) . Calc'd for C₂₃H₂₇N₅O₃S: 453.18.

### Example 202

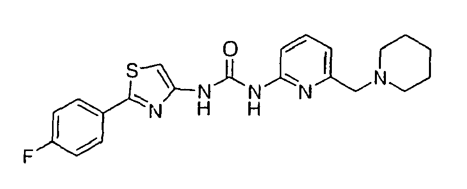

### 1-[2-(4-Fluorophenyl)thiazol-4-yl]-3-(6-piperidin-1-ylmethyl-pyridin-2-yl)urea

EI-MS m/z 412 (M+H). Calc'd for C₂₁H₂₂FN₅OS: 411.15.

### Example 203

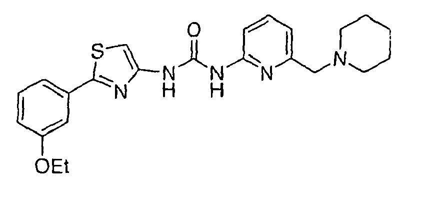

### 1-[2-(3-Ethoxyphenyl)thiazol-4-yl]-3-(6-piperidin-1-ylmethyl-pyridin-2-yl)urea

EI-MS m/z 438 (M+H). Calc'd for C23H27N5O2S: 437.19.

### Example 204

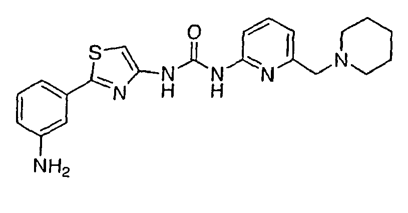

### 1-[2-(3-Aminophenyl)thiazol-4-yl]-3-(6-piperidin-1-ylmethyl-pyridin-2-yl)urea

EI-MS m/z 409 (M+H). Calc'd for C₂₁H₂₄N₆OS: 408.17.

### Example 205

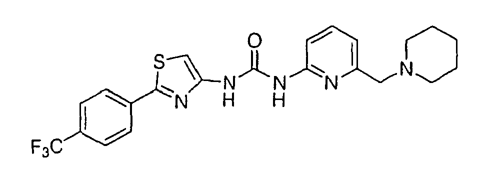

### 1-[2-(4-Trifluoromethylophenyl)thiazol-4-yl]-3-(6-piperidin-1-ylmethyl-pyridin-2-yl)urea

EI-MS m/z 462 (M+H). Calc'd for C₂₂H₂₂F₃N₅OS: 461.15.

### Example 206

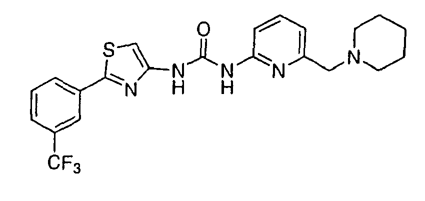

### 1-[2-(3-Trifluoromethylophenyl)thiazol-4-yl]-3-(6-piperidin-1-ylmethyl-pyridin-2-yl)urea

EI-MS m/z 462 (M+H)⁺. Calc'd for Calc'd for C₂₂H₂₂F₃N₅OS: 461.15.

### Example 207

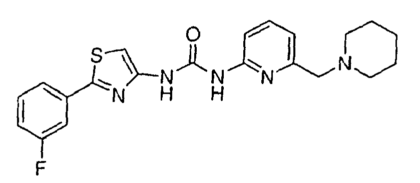

### 1-[2-(3-Fluorophenyl)thiazol-4-yl]-3-(6-piperidin-1-ylmethyl-pyridin-2-yl)urea

EI-MS m/z 412 (M+H). Calc'd for Calc'd for C₂₁H₂₂FN₅OS: 411.15.

### Example 208

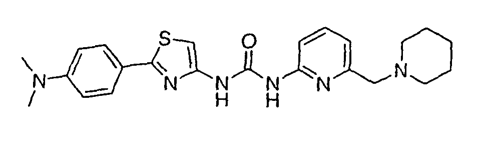

### 1-[2-(4-Dimethylaminophenyl)thiazol-4-yl]-3-(6-piperidin-1-ylmethyl-pyridin-2-yl)urea

EI-MS m/z 437 (M+H). Calc'd for C₂₃H₂₈N₆OS: 436.20.

### Example 209

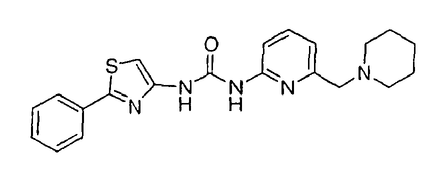

### 1-[2-phenylthiazol-4-yl]-3-(6-piperidin-1-ylmethyl-pyridin-2-yl)urea

EI-MS m/z 394 (M+H). Calc'd for C₂₁H₂₃N₅OS: 393.16.

### Example 210

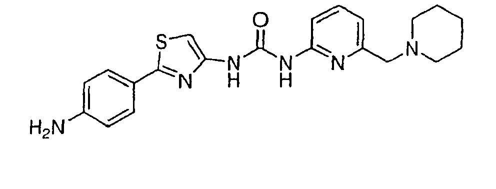

### 1-[2-(4-Aminophenyl)thiazol-4-yl]-3-(6-piperidin-1-ylmethyl-pyridin-2-yl)urea

EI-MS m/z 409 (M+H). Calc'd for C₂₁H₂₄N₆OS: 408.17.

### Example 211

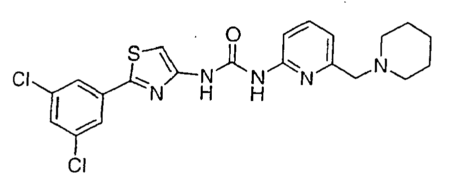

### 1-[2-(3,5-Dichlorophenyl)thiazol-4-yl)-3-(6-piperidin-1-ylmethyl-pyridin-2-yl)urea

EI-MS m/z 462 (M+H). Calc'd for C₂₁-H₂₁Cl₂N₅OS: 461.08.

### Example 212

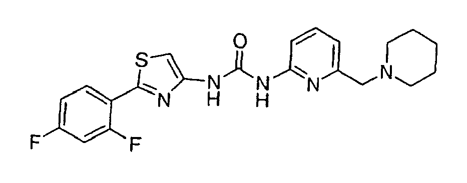

### 1-[2-(2,4-Difluorophenyl)thiazol-4-yl]-3-(6-piperidin-1-ylmethyl-pyridin-2-yl)urea

EI-MS m/z 430 (M+H). Calc'd for C₂₁H₂₁F₂N₅OS: 429.14.

### Example 213

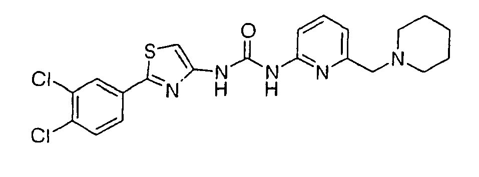

### 1-[2-(3,4-Dichlorophenyl)thiazol-4-yl]-3-(6-piperidin-1-ylmethyl-pyridin-2-yl)urea

EI-MS m/z 462 (M+H). Calc'd for C₂₁H₂₁Cl₂N₅OS: 461.08.

### Example 213a

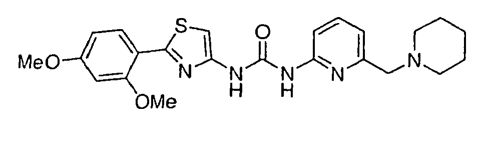

### 1-[2-(2,4-Dimethoxyphenyl)thiazol-4-yl]-3-(6-piperidin-1-ylmethyl-pyridin-2-yl)urea

EI-MS m/z 454 (M+H). Calc'd for C₂₃H₂₇N₅O₃S: 453.18.

### Example 214

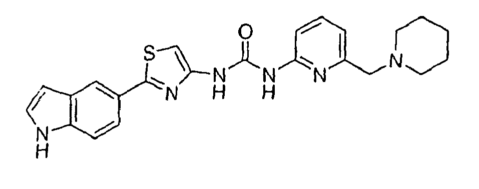

### 1-[2-(1H-Indol-5-yl)-thiazol-4-yl]-3-(6-piperidin-1-ylmethyl-pyridin-2-yl)-urea

EI-MS m/z 433 (M+H). Calc'd for C₂₃H₂₄N₆OS : 432.17.

### Example 215

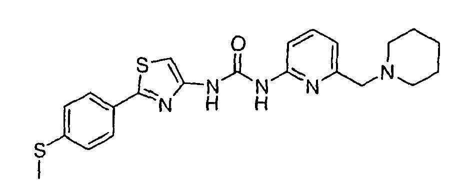

### 1-[2-(4-Methylthiophenyl)thiazol-4-yl]-3-(6-piperidin-1-ylmethyl-pyridin-2-yl)urea

EI-MS m/z 440 (M+H). Calc'd for C₂₂H₂₅N₅OS₂: 439.15.

### Example 216

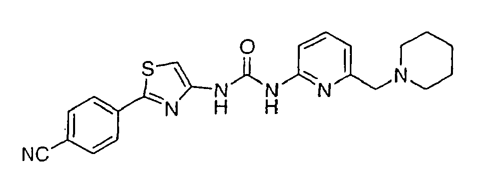

### 1-[2-(4-Cyanophenyl)thiazol-4-yl]-3-(6-piperidin-1-ylmethyl-pyridin-2-yl)urea

EI-MS m/z 419 (M+H). Calc' d for C₂₂H₂₂N₆OS : 418.16 Mol. Wt.: 418.5.

### Example 217

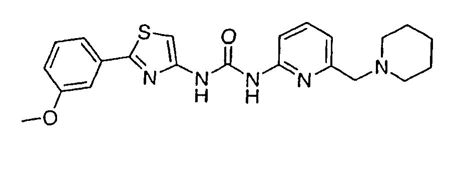

### 1-[2-(3-Methoxyphenyl)thiazol-4-yl]-3-(6-piperidin-1-ylmethyl-pyridin-2-yl)urea

To a stirred solution of 2-(3-methoxyphenyl)-1,3-thiazole-4-carboxylic acid (0.17 g, 0.72 mmol) in toluene (10 mL) at RT and under N2 was added TEA (0.2 mL). After 5 min, (PhO)₂PON₃ (0.2 5mL) was added and the reaction mixture was heated at 85°C for 20 min followed by the addition of 6-(piperidylmethyl)-2-pyridylamine (0.21 g, 1.1 mmol). The resulting mixture was heated at reflux for 4 h using a Dean-Stark trap. The mixture was cooled to RT, concentrated by rotary evaporation and purified on silica gel (5:95 MeOH/CH₂Cl₂). The yellow solid obtained was dissolved in EtOAc (15mL) and washed with a saturated solution of NH₄Cl (aq). The organic phase was separated, dried over MgSO₄, filtered and concentrated by rotary evaporation. The product was recrystallized from hexanes to afford the title compound as a white solid. EI-MS m/z 424 (M+H). Calc' d for C₂₂H₂₅N₅O₂S: 423.17.

### Example 218

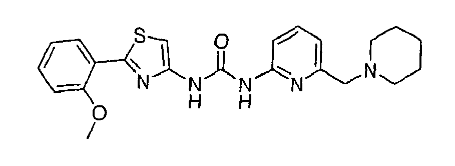

### 1-[2-(2-Methoxyphenyl)thiazol-4-yl]-3-(6-piperidin-1-ylmethyl-pyridin-2-yl)urea

To a stirred solution of 2-(2-methoxyphenyl)-1,3-thiazole-4-carboxylic acid (0.22 g, 0.94 mmol) in toluene (10 mL) at RT and under N₂ was added TEA (0.3 mL). After 5 min, (PhO)₂PON₃ (0.32 mL) was added and the reaction mixture was heated at 85°C for 20 min followed by the addition of 6-(piperidylmethyl)-2-pyridylamine (0.27 g, 1.41 mmol). The resulting mixture was heated at reflux for 4 h using a Dean-Stark trap. The mixture was cooled to RT, concentrated by rotary evaporation and purified on silica gel (5:95 MeOH/CH₂Cl₂). The yellow solid obtained was dissolved in EtOAc (15 mL) and washed with saturated NH₄Cl (10 mL). The organic phase was separated, dried over MgSO₄, filtered and concentrated by rotary evaporation to afford the title compound as a pale-yellow solid. EI-MS m/z 424 (M+H). Calc'd for C₂₂H₂₅N₅O₂S: 423.17.

### Example 219

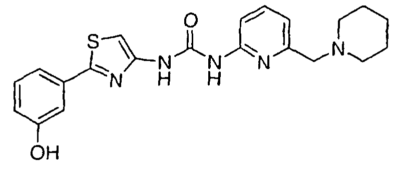

### 1-[2-(3-Hydroxyphenyl)thiazol-4-yl]-3-(6-piperidin-1-ylmethyl-pyridin-2-yl)urea

A mixture of 1-[2-(3-methoxyphenyl)thiazol-4-yl]-3-(6-piperidin-1-ylmethyl-pyridin-2-yl)urea (Example 218) and beryllium chloride (5.0 eq) in dry toluene (0.2 M) and 4A° molecular sieves was heated at reflux for 10 h. The starting material was not totally soluble in toluene. The mixture was brought to RT, diluted with EtOAc and washed with saturated NH₄Cl. The organic phase was separated, dried over MgSO₄, filtered, concentrated by rotary evaporation and purified by prep HPLC (Column Phenomenex type Prodigy 50 ODS3 100A size 250×21.20mm 5u, Gradient 10% to 90% CH₃CN:H₂O containing 1% TFA over 20 min, Detector 254 nm, 4 nm Band) to afford the title compound as an off white solid. EI-MS m/z 410 (M+H). Calc'd for C₂₁H₂₃N₆O₂S: 409.16.

### Example 220

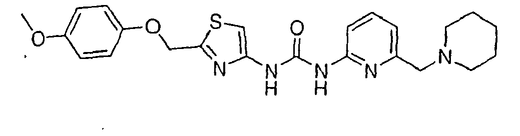

### 1-[2-(4-Methoxyphenoxymethyl)thiazol-4-yl]-3-(6-piperidin-1-ylmethyl-pyridin-2-yl)urea

To a stirred solution of 2-[(4-methoxyphenoxy)-methyl]-1,3-thiazole-4-carboxylic acid (0.10 g, 0.38 mmol) and TEA (0.06 mL, 0.46 mmol) in dry toluene (15mL) and 4A° molecular sieves was added (PhO)₂PON₃ (0.10 mL, 0.46 mmol). The resulting mixture was heated at 85°C for 25 min followed by the addition of 6-(piperidylmethyl)-2-pyridylamine (0.09 g, 0.46 mmol). The resulting mixture was heated to reflux for 15 h, cooled to RT, filtered, concentrated by rotary evaporation and purified on silica gel (5:95MeOH/CH₂Cl₂) to afford the title compound as a yellow oil. EI-MS m/z 454 (M+H). Calc'd for C₂₃H₂₇N₅O₃S: 453.18.

### Example 221

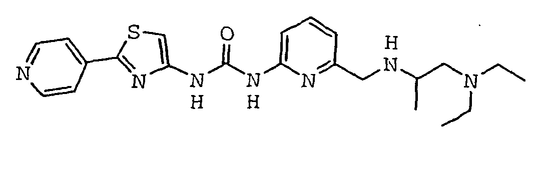

### 1-{6-[(2-Diethylamino-1-methylethylamino)methyl]-pyridin-2-yl}-3-(2-pyridin-4-yl-thiazol-4-yl)urea

### Step a

To a stirred solution of N-[(6-amino(2-pyridyl)) methyl]-N-]-[2-(diethylamino)-isopropyl] (tert-butoxy)-carboxamide (30 mg, 0.09 mmol) in toluene (5 mL) was added 2-aza-2-diazo-1-(2-(4-pyridyl) (1, 3-thiazol-4-yl))ethen-1-one (0.02 g, 0.09 mmol). The resulting green solution was heated to reflux in a Dean-Stark trap for 1.5 h until the starting materials were consumed. The mixture was brought to RT, concentrated by rotary evaporation and the residue obtained was partitioned between H₂O (10 mL) and CHCl₃ (10 ml). The organic phase was separated and the aqueous phase was extracted (3x10 ml) with CHCl₃. The organic layers were combined, dried over MgSO₄, filtered, concentrated by rotary evaporation and purified by prep TLC (10:90 MeOH/CH₂Cl₂) to afford tert butyl (2-dimethylamino-1-methyl-ethyl)-(6-[3-(2-pyridin-4-yl-thiazol-4-yl)ureido]pyridin-2-yl}carbamate as a white solid. EI-MS m/z 540 (M+H). Calc'd for C₂₇H₃₇N₇O₃S: 539.27.

### Step B

To a stirred solution of N-[2-diethylamino)-ethyl](tert-butoxy)-N-[(6-{[N-(2-(4-pyridyl)(1,3-thiazol-4-yl))carbamoyl]amino}(2-pyridyl))methyl]-carboxamide (4 mg, 0.007 mmol) in dry CH₂Cl₂ (1 mL) was added TFA (1 mL). The resulting solution was stirred at RT and under N₂ atmosphere for 2 h, concentrated by rotary evaporation and the residue was diluted with EtOAc (5 mL) and washed with a saturated solution of NaHCO₃ (aq) (15 mL). The organic phase was separated, dried over MgSO₄, filtered, concentrated by rotary evaporation and purified by prep TLC (1:1 MeOH/CH₂Cl₂) to yield 1-{6-[(2-diethylamino-1-methylethylamino)methyl]-pyridin-2-yl}-3-(2-pyridin-4-yl-thiazol-4-yl)urea. EI-MS m/z 540 (M+H). Calc'd for C₂₂H₂₉N₇OS: 439.22.

### Example 222

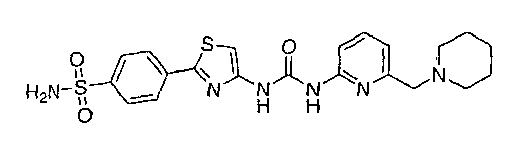

### 4-{4-[3-(6-Piperidin-1-ylmethyl-pyridin-2-yl)-ureido]-thiazol-2-yl}-benzenesulfonamide

To a stirred solution of ethyl 2-(4-sulfamoyl-phenyl)-1,3-thiazole-4-carboxylic acid (90 mg, 0.32 mmol) in dry TFA (3 mL) and 4A° molecular sieves at RT and under N₂ was added TEA (0.1 mL). After 5 min, (PhO)₂PON₃ (0.11 mL) and 6-(piperidylmethyl)-2-pyridylamine (0.10 g, 0.51 mmol) were added and the reaction mixture was heated to reflux for 4h and then cooled to RT. The mixture was washed with 10% HCl (aq) and extracted with EtOAc (3x10 mL). The aqueous layer was brought to a pH 8.0 and extracted with CH₂Cl₂ (3x20mL). The extracts were combined, dried over MgSO₄ concentrated by rotary evaporation and purified on silica gel (*2*:1 hexanes/EtOAc and 1:1 MeOH/CH₂Cl₂) to afford the title compound as a pale yellow solid. EI-MS m/z 473 (M+H). Calc'd for C₂₁H₂₄N₆O₃S₂ : 472.14.

### Example 223

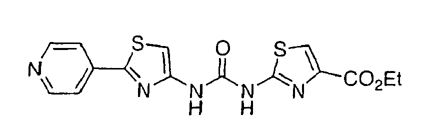

### Ethyl 2-[3-[2-(pyridin-4-yl)-thiazol-4-yl]ureido]-thiazole-4-carboxylate

2-(4-Pyridinyl)-4-thiazolcarbonylazide (420 mg, 1.8 mmol) in dry toluene (20 mL) was heated to 85°C under N₂ and maintained at this temperature for 5 min. A solution of 2-amino-4-thiazolcarboxylic acid ethyl ester (350 mg, 2.0 mmol) was added and the resulting mixture was heated at 85°C for 15 h. After cooling to RT, a precipitate formed and was filtered to give the desired compound as a yellow solid. MS m/z: 376.0 (M+H). Calc'd for C₁₅H₁₃N₅O₃S₂: 375.05.

### Example 224

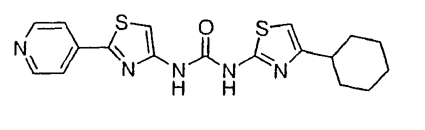

### 1-(4-Cyclohexylthiazol-2-yl)-3-[2-(pyridin-4-yl)-thiazol-4-yl]urea

2-(4-Pyridinyl)-4-thiazolcarbonylazide (200 mg, 0.87 mmol) in dry toluene (10 mL) was heated to 85°C under N₂ and maintained at this temperature for 5 min. A solution of 2-amino-4-cyclohexylthiazole (158 mg, 0.87 mmol) was added and the resulting mixture was heated at 85°C for 15 h. After cooling to RT, a precipitate formed and was filtered to give the desired compound as a yellow solid. MS m/z: 386.0 (M+H). Calc'd for C₁₈H₁₉N₅OS₂: 385.10.

### Example 225

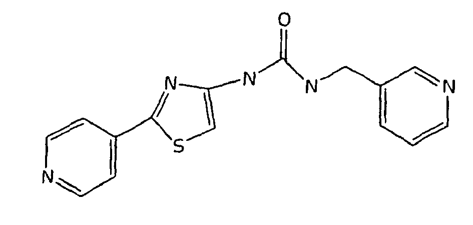

### 1-(Pyridin-3-ylmethyl)-3-(2-pyridin-4-yl-thiazol-4-yl)urea

2-(4-Pyridinyl)-4-thiazolcarbonylazide (100 mg, 0.43 mmol) in dry toluene (3 mL) was heated to 105°C under N₂ and maintained at this temperature for 5 min. A solution of 3-(aminomethyl)pyridine (47 mg, 0.43 mmol) in dry toluene (1 mL) was added dropwise via syringe and the resulting mixture heated at 105°C for 2 h. After cooling to RT, solvent was removed under vacuum and the product was purified by silica gel chromatograpy eluting with MeOH/CH₂Cl₂ (10%) to give the desired compound as a light yellow solid. MS m/z: 312.1 (M+H). Calc'd for C₁₅H₁₃N₅OS: 311.08.

### Example 226

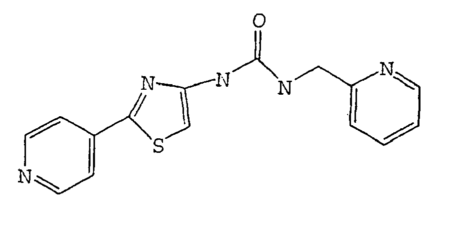

### 1-(Pyridin-2-ylmethyl)-3-(2-pyridin-4-yl-thiazol-4-yl)urea

2-(4-Pyridinyl)-4-thiazolcarbonylazide (100 mg, 0.43 mmol) in dry toluene (3 mL) was heated to 105 °C under nitrogen and maintained at this temperature for 5 min. A solution of 2-(aminomethyl)pyridine (47 mg, 0.43 mmol) in dry toluene (1 mL) was then added dropwise via syringe and the resulting mixture heated at 105°C for 3 h. After cooling to room temperature, solvent was removed under vacuum and the product was purified by silica gel chromatograpy eluting with MeOH/CH₂Cl₂ (10%) to give a light yellow solid. MS m/z: 312.1 (M+H). Calc'd for C₁₅H₁₃N₅OS: 311.08.

### Example 227

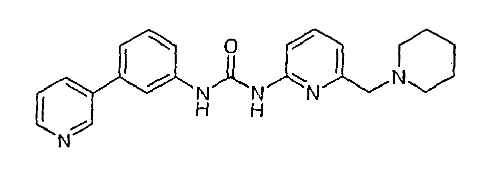

### 1-[6-(Piperidin-1-ylmethyl)pyridin-2-yl]-3-(3-pyridin-3-yl-phenyl)urea

To a stirred solution of phosgene (0.35 mL, 0.65 mmol, 20% in toluene) in dry THF (5 mL) was added 3-(3-pyrid-1-yl)-1-aminobenzene (85 mg, 0.5 mmol) dropwise via the addition funnel. After stirring for 10 min., isopropylethylamine (0.26 mL, 2.0 mmol) was added. The resulting mixture was stirred at RT under N₂ for 30 min. 2-Amino-6-piperidinylmethylpyridine (96 mg, 0.5 mmol) in dry THF (5 mL) was added dropwise into the reaction mixture via the addition funnel. The resulting mixture was stirred at RT for 15 h. Solvent was removed to give a dark brown liquid which was purified by chromatography on silica gel. Elution with CH₂Cl₂:MeoH mixture (95:5) gave the final compound as a pale yellow solid. MS *m*/*z:* 387.9 (M+). Calc'd. for C₂₃H₂₅N₅O - 387.49.

### Example 228

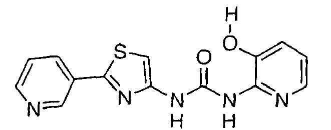

### 1-(3-Hydroxy-pyridin-2-yl)-3-(2-pyridin-3-yl-thiazol-4-yl)-urea

TEA (0.27 mL, 1.94 mmol) was added to a solution of 2-(pyridin-3-yl)thiazole-4-carboxylic acid (200 mg, 0.97 mmol) and 4A molecular sieves in THF (25 mL) under N₂ at RT. (PhO)₂PON₃ (0.33 mL, 1.55 mmol) followed by 2-amino-6-hydroxypyridine (268 mg, 2.43 mmol) was added and the resulting mixture heated at reflux for 12 h. After cooling to RT, the heterogeneous mixture was decanted to remove the molecular sieves. The precipitate was collected, rinsing with EtOAc to give a white solid. MS m/z: 313.0 (M+H). Calc'd for C₁₄H₁₁N₅O₂S - 313.34.

### Example 229

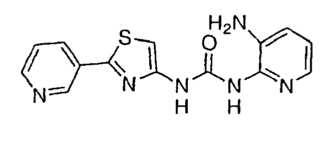

### 1-(3-Amino-pyridin-2-yl)-3-(2-pyridin-3-yl-thiazol-4-yl)-urea

TEA (0.27 mL, 1.94 mmol) was added to a solution of 2-(pyridin-3-yl)thiazole-4-carboxylic acid (200 mg, 0.97 mmol) and 4A molecular sieves in THF (25 mL) under N₂ at RT. (PhO)₂PON₃ (0.33 mL, 1.55 mmol) followed by 2-amino-3-aminomethylpyridine (265 mg, 2.43 mmol) was added and the resulting mixture was heated at reflux for 12 h. After cooling to RT, the heterogeneous mixture was decanted to remove the molecular sieves. The precipitate was collected and discarded. The filtrate was purified by chromatography on silica gel (CH₂Cl₂/MeOH, 95:5) to give a white solid. MS *m*/*z:* 313.8 (M+H) . Calc'd for C₁₄H₁₂N₆OS - 312.36.

### Example 230

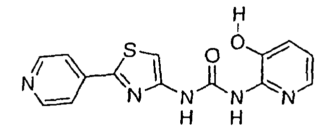

### 1-(3-Hydroxy-pyridin-2-yl)-3-(2-pyridin-4-yl-thiazol-4-yl)-urea

2-(4-Pyridinyl)-4-thiazolcarbonylazide (200 mg, 0.86 mmol) and 2-amino-3-hydroxymethylpyridine (95 mg, 0.86 mmol) in dry toluene (10 mL) were heated at 100°C for 12 h to give a pale yellow solid which was recrystallized from CHCl₃/MeOH (99:5) to give a pale yellow solid. MS *m*/*z:* 314.0 (M+H). Calc'd for C₁₄H₁₁N₅O₂S - 313.34.

### Example 231

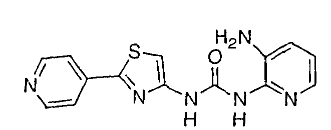

### 1-(3-Amino-pyridin-2-yl)-3-(2-pyridin-4-yl-thiazol-4-yl)-urea

2-(4-Pyridinyl)-4-thiazolcarbonylazide (200 mg, 0.86 mmol) and 2-amino-3-aminomethylpyridine (94 mg, 0.86 mmol) in dry toluene (10 mL) were heated at 100°C for 12 h to give a pale yellow solid which was recrystallized from CHCl3/MeOH (99:5) to give a pale yellow solid. MS *m*/*z:* 313.0 (M+H). Calc'd for C₁₄H₁₂N₆OS - 312.36.

### Example 232

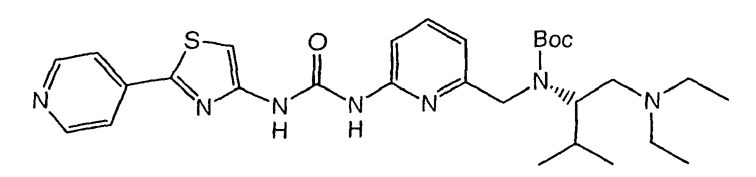

### (1-Diethylaminomethyl-2-methyl-propyl)-{6-[3-(2-pyridin-4-yl-thiazol-4-yl)-ureido]-pyridin-2-ylmethy 1}-carbamic acid tert-butyl ester

To a stirred solution of N-[(6-amino-(2-pyridyl))-methyl]-N-{1-[(diethylamino)methyl]-2-methylpropyl}-(tert-butoxy)carboxamide (6 mg, 0.016 mmol) in toluene (5 mL) was added 6-(piperidylmethyl)-2-pyridylamine (0.004 g, 0.016 mmol). The resulting green solution was heated at reflux in a Dean-Stark trap for 1.5 h until the starting materials were consumed. The mixture was brought to RT, concentrated by rotary evaporation and the residue obtained was partitioned between H₂O (10 mL) and CHCl₃ (35 mL). The organic phase was separated and the aqueous phase was extracted with CHCl₃ (3x10mL). The organic layers were combined, dried over MgSO₄, filtered, concentrated by rotary evaporation and purified by prep TLC (5:95 MeOH/CH₂Cl₂) to afford (1-diethylaminomethyl-2-methyl-propyl)-{6-[3-(2-pyridin-4-yl-thiazol-4-yl)-ureido]-pyridin-2-ylmethyl}-carbamic acid tert-butyl ester as an off-white solid. EI-MS m/z 568 (M+H). Calc'd for C₂₉H₄₁N₇O₃S: 567.30.

### Example 233

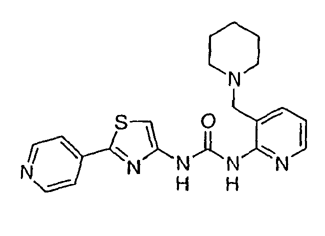

### 1-(3-Piperidin-1-ylmethyl-pyridin-2-yl)-3-(2-pyridin-4-yl-thiazol-4-yl)-urea

MS *m*/*z* : 395 (M+H).

Other compounds included in this invention are set forth in Tables 1-7 below.

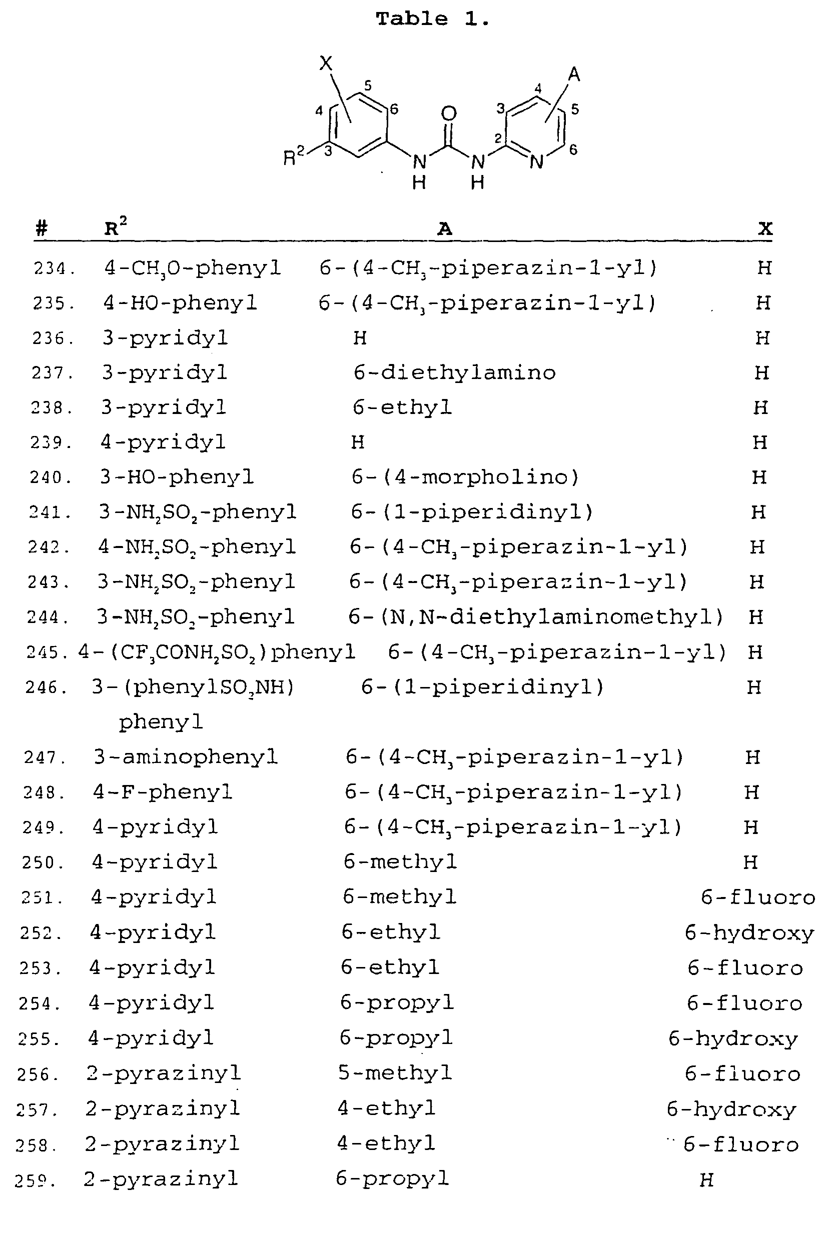

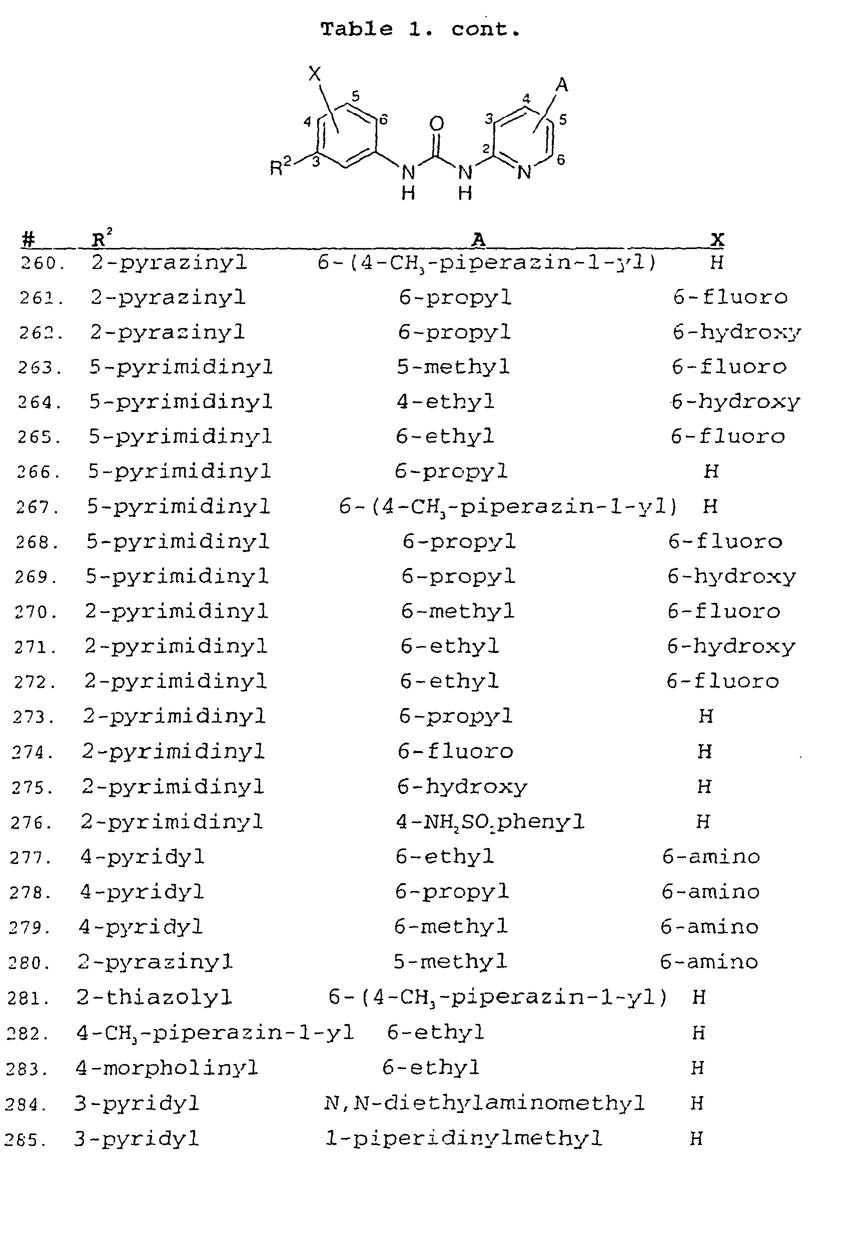

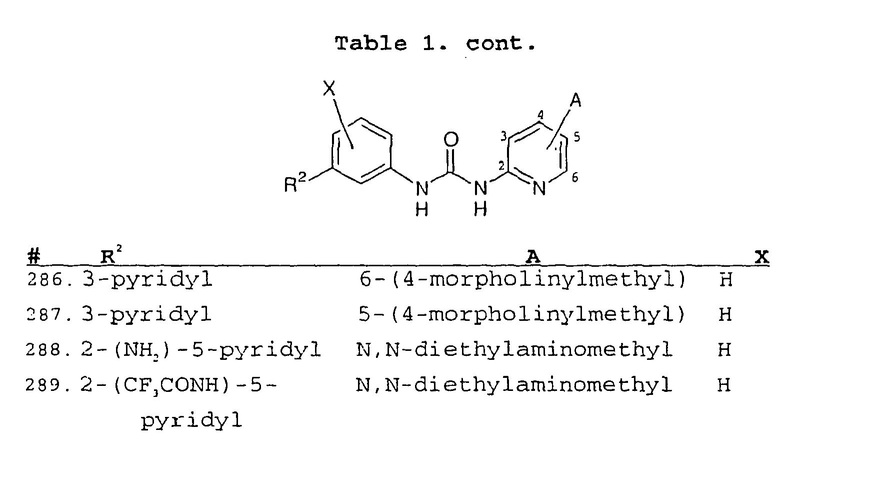

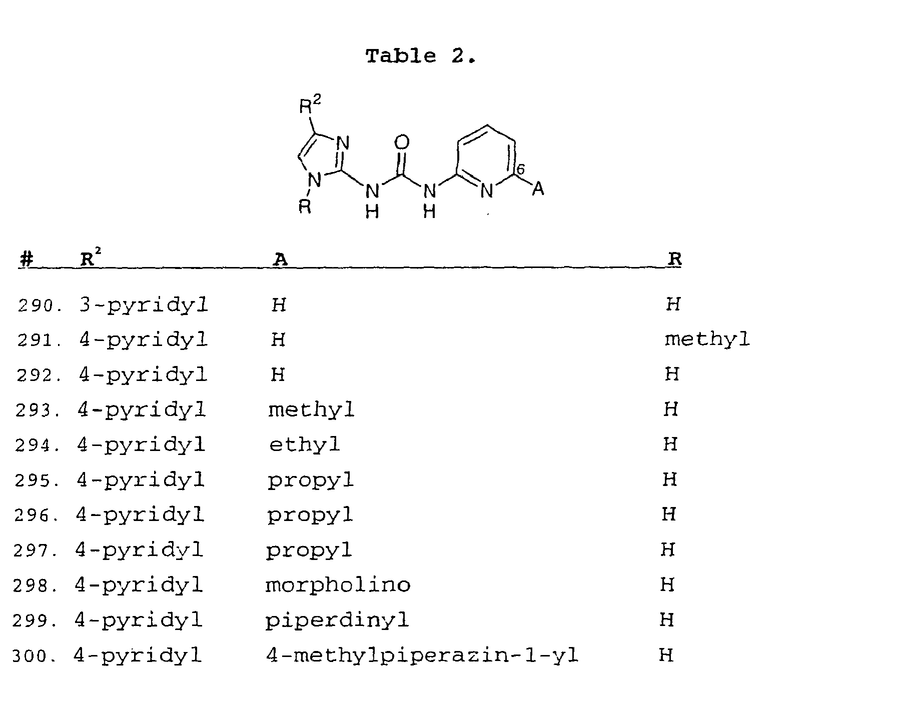

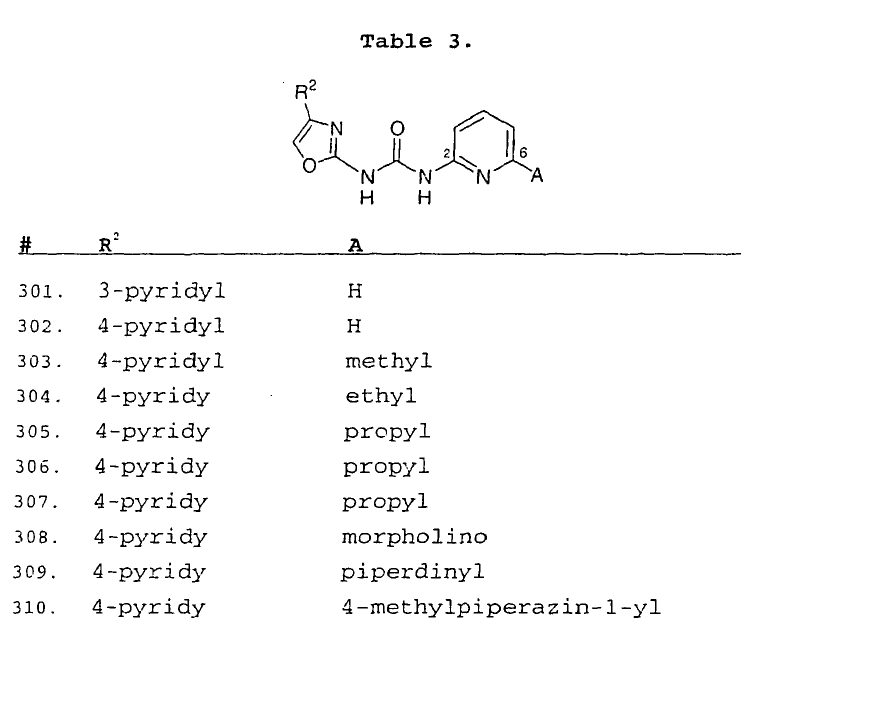

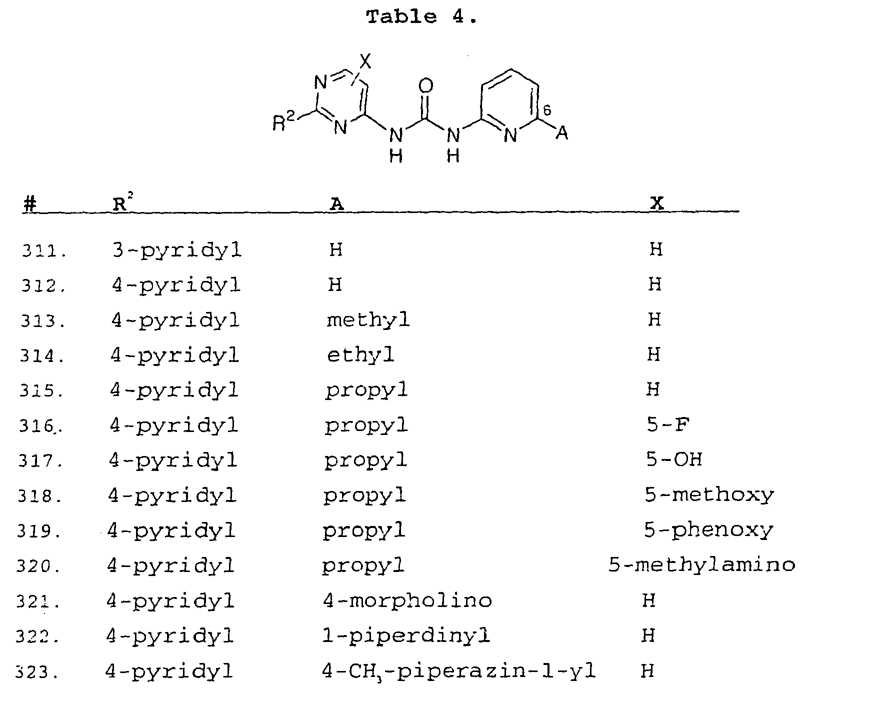

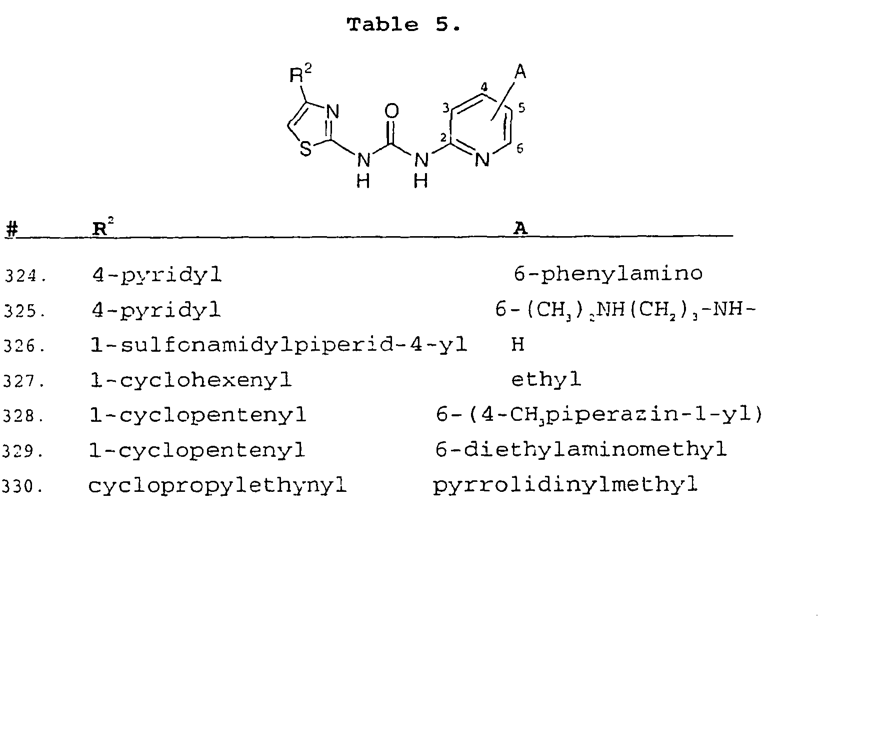

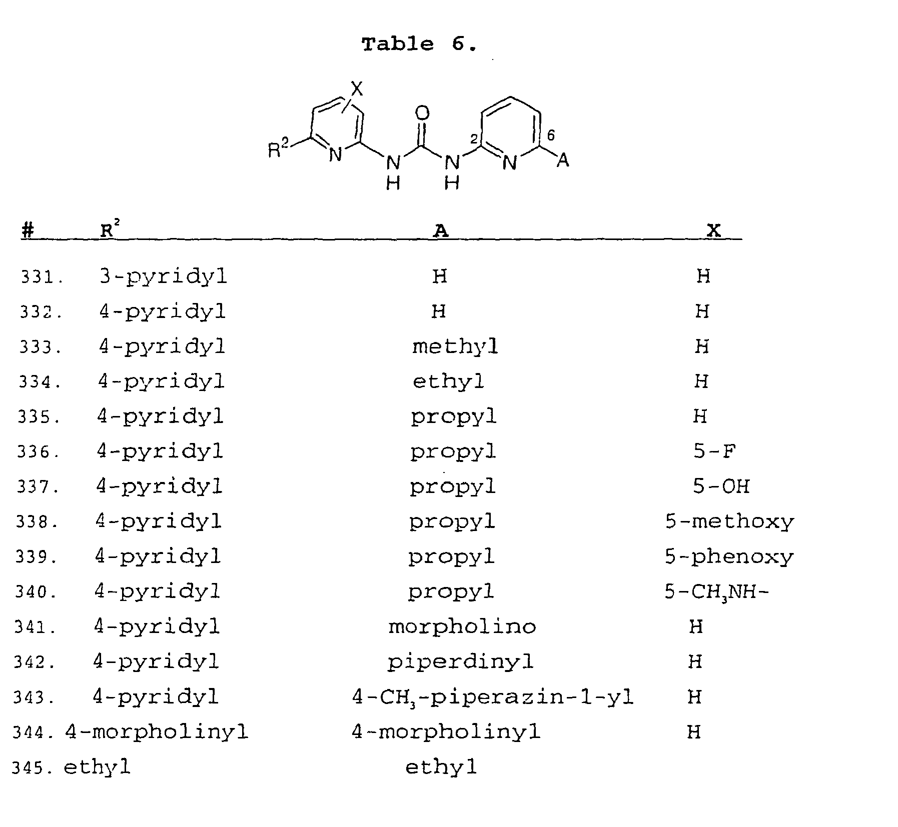

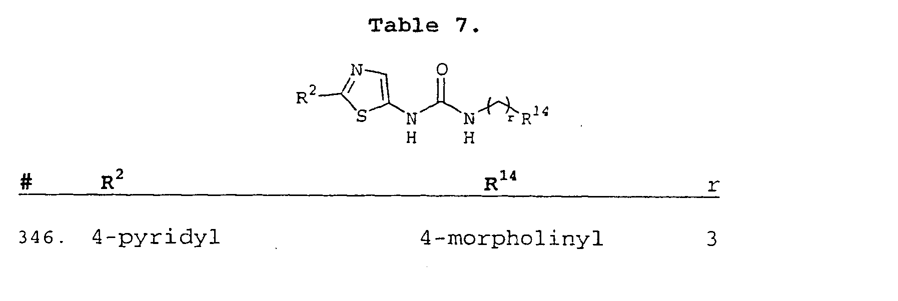

The pharmacological properties of the compounds of this invention may be confirmed by a number of pharmacological assays. The exemplified pharmacological assays which follow have been carried out with the compounds according to the invention and their salts. The compounds of examples 1 to 35 exhibited cdk2/cyclin kinase activity with IC₅₀ values less than 50 µM. The compounds of examples 1 to 35 exhibited cdk5/cyclin kinase activity with IC₅₀ values less than 50 µM. Example 36 exhibited KDR activity with an IC₅₀ value less than 50 µM.

### BIOLOGICAL EVALUATION

### PROTOCOLS FOR CYCLIN E2/CDK2

### Cloning of Cdk2 and cyclin 2/Generation of Cdk2 and cyclin 2 Recombinant Baculovirus

The following oligonucleotide primers flanking the coding sequence of the human Cdk2 cDNA clone were used to amplify the gene and place EcoRI and HindIII restriction sites at the 5' and 3' ends of the gene respectively. [5' oligo-5'-AAGCGCGCGGAATTCATAAATATGGAGAACTTCCAAAAGGTGGAA-3'; 3' oligo-5'-CTCGACAAGCTTATTAGAGTCGAAGATGGGGTAC-3']
The following oligonucleotide primers flanking the coding sequence of the human CycE2 cDNA clone were used to amplify the gene and place XhoI and SphI restriction sites at the 5' and 3' ends of the gene respectively. A His tag was also placed at the N-terminus of the CycE2 protein. [5' oligo-5'-CCCGGGATCTCGAGATAAATATGCATCATCATCATCATTCAAGACGAAGTAGCCG TTTACAA -3'; 3' oligo-5'-CCCGGTACCGCATGCTTAGTGTTTTCCTGGTGGTTTTTC -3']

CycE-2 and Cdk2 PCR fragments were subcloned into the vector pFastBacDual (Gibco/LifeTechnologies) using the restriction sites indicated above. Recombinant virus was made following protocols supplied by the manufacturer.

### Expression of cyclin 2/CDK2 in insect cells

Hi5 cells were grown to a cell density of 1 × 10⁶ cells per ml in 800 ml of Excell 405 media (JRH). Cells were infected with virus at a multiplicity of 1. Infected cultures were incubated with shaking at 28°C. Cells were harvested by centrifugation.

### Cloning of Cdk5 and p25/Generation of CDK5 and p25 Recombinant Baculovirus

Based on the reported sequences of human CDK5 and p35, GenBank accession numbers X66364 and X80343 respectively, oligonucleotide primers flanking the coding sequence of each gene were used to amplify CDK5 (5'-GCGATGCAGAAATACGAGAAACT-3'; 5'-CCCCACTGTCTCACCCTCTCAA-3') and p35 (5'-CGGTGAGCGGTTTTATCCC-TCC-3'; 5'-GCATTGAATCCTTGAGCCATGACG-3') from a human fetal brain cDNA library (Clontech). p25, a C-terminal proteolytic fragment corresponding to amino acids 99-307 of full-length p35 (Lew, et. al), was PCR subcloned from the p35 sequence using oligonucleotide primers (5'-CGGGATCCATGGCCCAGCCCCCACCGGCCCA-3'; 5'-CCAAGCTTTCACCGATCCAGGCCTAG-3'). The p25 PCR product (629bp) was cloned into the pFastBacHTb baculovirus expression vector (Gibco BRL) using *BamHI* and *HindIII.* CDK5 was PCR subcloned using oligonucleotide primers (5'-CGGGATCC -GCCACCATGCAGAAATACGAGAAACTGG-3'; 5'-GGACTAGTCTAGGGCGGAC-AGAAGTCG-3'). The CDK5 PCR product (879 bp) was cloned into the pFastBac1 baculovirus expression vector (Gibco BRL) using *BamHI* and *SpeI.* Recombinant baculovirus expressing human Cdk5 and N-terminally six histidine tagged p25 were generated using the Bac-to-Bac system (Gibco BRL).

### Expression of P25/CDK5 in insect cells

Coinfections of Hi5 cells by recombinant baculovirus containing the P25 gene and another containing the CDK5 gene were done at a multiplicity of infection of 5 (each virus). The Hi5 cultures were set to a cell concentration of 1 × 10⁶ cells per ml in 800 ml of Excell media by JRH. The cultures were grown in 2.6L fernbach flasks with shaking (110 rpm) at 27°C for 60 hours. The cells were harvested by centrifugation.

### Purification of complexes

All steps were performed at 4ºC. Insect cells expressing either cyclin E2/CDK2 or p25/CDK5 were lysed using a microfluidizer (Microfluidics Corporation.) The lysis buffer contained 10mM Hepes, 150mM NaCl, 20mM MgCl₂, 20mm imidazole, 0.5mM EDTA, 10% glycerol, 25*µ*g/ml Aprotinin, 25µg/ml Leupeptin, 1mM Pefabloc, pH 7.5). Total protein was determined on the resulting lysate using the Bradford method with a BSA standard curve. Protamine sulfate was added to the lysate to give a final 30:1 protein:protamine sulfate, incubated for 15-20 minutes and centrifuged at 14000xg for 30 minutes to remove insoluble material. Ni-NTA superflow resin (Qiagen Inc) was equilibrated in lysis buffer and incubated with the centrifugation supernatant for 1 hour while rotating. The slurry was packed in a glass column and washed until a stable UV baseline was reached. Proteins were eluted with a linear gradient of 20-300mM imidazole over 15 column volumes. Fractions were analyzed by SDS-PAGE and Western blot. Appropriate fractions were pooled, total protein determined, and submitted for kinase assay.

### CDK2 Kinase Assay

CDK2 kinase assays were carried out with inhibitor (dissolved in DMSO) in a total volume of 50 *µ*l with 1nM enzyme (His-tagged cyclin 2/CDK2), 1 *µ*M Histone-H1 (Gibco), 25 *µ*M ATP, 20 *µ*Ci/ml ³³P-ATP (Amersham; 2500 Ci/mmole) in kinase buffer (50 mM Tris-HCl, pH 7.5, 5 mM MgCl₂, 1 mM EGTA, 5 mM DTT, 200 *µ*g/ml BSA and 20 mM β-glycerophosphate for 60 minutes at 25 °C. Reactions were stopped by the addition of an equal volume of 30% trichloroacetic acid (Sigma). Precipitates were formed by incubation at 4 °C for 60 minutes and then collected by filtration on Millipore® filter plates (MAFC NOB10). Forty microliters of MicroScint-20 (Packard) was added, and then were counted on a Packard TopCount® . Raw cpms were analyzed with a four-parameter logistic fit using the Levenburg Marquardt algorithm (Xlfit software IDBS LTD). Kinetic parameters were calculated by non-linear regression analysis using Grafit (Erithacus Software LTD). Riscovitine (BIOMOL Research Labs Inc., Plymouth Meeting, PA.) and staurosporin (Sigma, St. Louis MO) were used as standards.

### CDK5 Kinase Assay

CDK5 kinase assays were carried out with inhibitor (dissolved in DMSO) in a total volume of 50 *µ*l with 1nM enzyme (His-tagged p25/CDK5), 1 µM Histone-H1 (Gibco), 25 µM ATP, 20 µCi/ml ³³P-ATP (Amersham; 2500 Ci/mmole) in kinase buffer (50 mM Tris-HCl, pH 7.5, 5 mM MgCl2, 1 mM EGTA, 5 mM DTT, 200 µg/ml bovine serum albumin and 20 mM β-glycerophosphate) for 60 minutes at 25°C. Reactions were stopped by the addition of an equal volume of 30% trichloroacetic acid (Sigma). Precipitates were formed by incubation at 4 °C for 60 minutes and then collected by filtration on Millipore® filter plates (MAFC NOB10). Forty microliters of MicroScint-20 (Packard) was added, and then were counted on a Packard TopCount® . Raw cpms were analyzed with a four-parameter logistic fit using the Levenburg Marquardt algorithm (Xlfit software IDBS LTD). Kinetic parameters were calculated by non-linear regression analysis using Grafit (Erithacus Software LTD). Riscovitine (BIOMOL Research Labs Inc., Plymouth Meeting, PA.) and staurosporin (Sigma, St. Louis MO) were used as standards.

### KDR Assay

KDR kinase assays were carried out in Polypro 96 well clear round bottom plates (Costar). An aqueous kinase reaction buffer was prepared (100 mM Tris-HCl, 125 mM MgCl₂, 25 mM MnCl₂, 2 mM EGTA, 0.25 mM SOV and 2 mM DTT) . To each well buffer (50µl) and biotinylated gastrin peptide substrate (10µl) was added. A pool of KR was diluted (1:10) in buffer. Diluted KDR (10µl) was added to the wells except for the controls. Inhibitor, dissolved in DMSO (5µl) was added and the wells were incubated with shaking for 30 minutes at 25°C. A co-substrate was added (75 mM MnCl₂, 50 µM ATP) (10µL) in one row and the plates were incubated for 60 more minutes with shaking. A portion of the kinase mixture (5µl) was transferred to a Polypro 96 well black bottom plate (costar) containing 40µl TBS (50 mM Tris-HCl, 100 mM NaCl, 0.1% BSA and 0.05% Tween 20), 20 ul 1:200 dilution streptavidin-APC and 20µl 0.45 nM Eu-PT66. The plates were incubated for 30 minutes with shaking then read on a Discovery homogenous time resolved fluorescence (HTRF) analyzer (Wallac). Staurosporin (Sigma) was used as a standard. Kinetic parameters were calculated using Excelfit software.

### CELL PROLIFERATION ASSAY

Cell proliferation was measured using a colorimetric immunoassay (B/M Roche #164 7229), based on the measurement of pyrimidine analog BrdU incorporation during DNA synthesis in proliferating cells. Cells, e.g., human PC-3 prostate carconima cells, huFSF normal human foreskin fibroblast cells, HCT 116 human colon carcinoma cells or HT 29 human colon carcinoma cells, were cultured in a 96-well plate for 24 hours, until a cell count of 3×10³ to 6×10³ cells per well in duplicate wells were achieved, in a well volume of 200 µl. The media was changed and 1 µl of 200X control inhibitors or compounds was added to each well. Cells are incubated for 48 hours at 37°C. The cells were labeled with BrdU for 4 hours at 37°C. The labeling medium was removed and in one step, the cells were fixed and the DNA was denatured (30 minutes at room temperature). Anti-BrdU-POD antibody was added to bind to the BrdU incorporated in newly synthesized cellular DNA (60-90 minutes at room temperature). The cells were washed 3X with washing buffer, substrate (100µl) was added and the cells were incubated for 10 minutes at room temperature. The substrate reaction was stopped by adding H₂SO₄ (25µl of 1M H₂SO₄). The amount of BrdU incorporated was quantified by measuring the absorbance at 450 nm using ELISA reader. IC₅₀'s were calculated using GraFit (Sigma).

### ISCHEMIC STROKE MODEL: MIDDLE CEREBRAL ARTERY OCCLUSION (MCAO) IN VIVO

The compounds' effect on treating stroke was measured in a MCAO rat model. (L. Belayev et al., Stroke, 27, 1616-23 (1996). Male Sprague-Dawley rats (300-330g body weight) were anesthetized with halothane and MCAo was induced by inserting a poly-L-lysine coated monofilament suture to the beginning of the middle cerebral artery (MCA). After various time points (60, 90 or 120 min), the intraluminal suture was carefully removed to start reperfusion. Physiological conditions (blood O₂, CO₂, pH, glucose, blood pressure) were monitored and kept stable during the surgery. The compound was dissolved in 20% Captisol in phosphate buffered saline and administered (orally, IV or IP) 90 minutes after ischemia onset, at the beginning of reperfusion. Further dosing occurred at 4-8 hours and twice a day thereafter.

The use of behavioral tests was directly analogous to the clinical neurological examination for assessing ischemic deficits and rates of behavioral recovery. The battery consisted of four tests: (1) postural reflex test, (2) forelimb placing test (JB Bederson et al., Stroke, 17:472-76 (1986) (L. Belayev et al., Stroke, 26:2313-20 (1995), (3) contralateral foot fault index (A. Tamura et al., J. Cereb Blood Flow Metab., 1:53-60 (1981) (*DM* Freeney, Science, 217:855-57 (1982), and (4) cylinder asymmetry (TA Jones and T. Schallert, J. Neurosci., 14:2140-52 (1994). Tests were performed once a day for three days and then once a week for a period of 30 days. These tests are useful in assessing neurological deficits for short-term studies; the cylinder asymmetry test appeared to be the most useful for long term experiments.

At the end of the experiment, the infarct volume was measured (JB Bederson et al., Stroke, 17:1304-8 (1986) (KA Osborne et al, J. Neurol Neurosurg. Psychiatry, 50:402 (1987) (RA Swanson et al., J. Cereb. Blood Flow Metab., 10:290-3 (1990). The brains were removed and sliced coronally at 1 mm thickness. The brain slices were stained with 2% (w/vol) 2,3,5-triphenyltetrazolium chloride (TTC) which stains the infarcted areas of the brain in white and allows for the measurement of infarct volume by an image-analysis system. Edema volume that contributes to infarct volume was subtracted by comparison with the total volume of the contralateral hemisphere. Example 14 and 43 significantly (-30%) improved the responses in the behavioral tests and reduced the brain infarct volume after 2-3 days at doses of 10-30 mg/kg.

### Formulations

Also embraced within this invention is a class of pharmaceutical compositions comprising the active compounds of Formula I in association with one or more non-toxic, pharmaceutically-acceptable carriers and/or diluents and/or adjuvants (collectively referred to herein as "carrier" materials) and, if desired, other active ingredients. The active compounds of the present invention may be administered by any suitable route, preferably in the form of a pharmaceutical composition adapted to such a route, and in a dose effective for the treatment intended. The compounds and compositions of the present invention may, for example, be administered orally, mucosally, topically, rectally, pulmonarily such as by inhalation spray, or parentally including intravascularly, intravenously, intraperitoneally, subcutaneously, intramuscularly intrasternally and infusion techniques, in dosage unit formulations containing conventional pharmaceutically acceptable carriers, adjuvants, and vehicles.

The pharmaceutically active compounds of this invention can be processed in accordance with conventional methods of pharmacy to produce medicinal agents for administration to patients, including humans and other mammals .

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are tablets or capsules. For example, these may contain an amount of active ingredient from about 1 to 2000 mg, preferably from about 1 to 500 mg, more preferably from about 5 to 150 mg. A suitable daily dose for a human or other mammal may vary widely depending on the condition of the patient and other factors, but, once again, can be determined using routine methods.

The amount of compounds which are administered and the dosage regimen for treating a disease condition with the compounds and/or compositions of this invention depends on a variety of factors, including the age, weight, sex and medical condition of the subject, the type of disease, the severity of the disease, the route and frequency of administration, and the particular compound employed. Thus, the dosage regimen may vary widely, but can be determined routinely using standard methods. A daily dose of about 0.01 to 500 mg/kg body weight, preferably between about 0.5 and about 50 mg/kg body weight and most preferably between about 0.1 to 20 mg/kg body weight, may be appropriate may be appropriate. The daily dose can be administered in one to four doses per day.

For therapeutic purposes, the active compounds of this invention are ordinarily combined with one or more adjuvants appropriate to the indicated route of administration. If administered per os, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose.

In the case of psoriasis and other skin conditions, it may be preferable to apply a topical preparation of compounds of this invention to the affected area two to four times a day.

Formulations suitable for topical administration include liquid or semi-liquid preparations suitable for penetration through the skin (e.g., liniments, lotions, ointments, creams, or pastes) and drops suitable for administration to the eye, ear, or nose. A suitable topical dose of active ingredient of a compound of the invention is 0.1 mg to 150 mg administered one to four, preferably one or two times daily. For topical administration, the active ingredient may comprise from 0.001% to 10% w/w, *e.g.,* from 1% to 2% by weight of the formulation, although it may comprise as much as 10% w/w, but preferably not more than 5% w/w, and more preferably from 0.1% to 1% of the formulation.

When formulated in an ointment, the active ingredients may be employed with either paraffinic or a water-miscible ointment base. Alternatively, the active ingredients may be formulated in a cream with an oil-in-water cream base. If desired, the aqueous phase of the cream base may include, for example at Least 30% w/w of a polyhydric alcohol such as propylene glycol, butane-1,3-diol, mannitol, sorbitol, glycerol, polyethylene glycol and mixtures thereof. The topical formulation may desirably include a compound which enhances absorption or penetration of the active ingredient through the skin or other affected areas, Examples of such dermal penetration enhancers include dimethylsulfoxide and related analogs.

The compounds of this invention can also be administered by a transdermal device. Preferably transdermal administration will be accomplished using a patch either of the reservoir and porous membrane type or of a solid matrix variety. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane.

The oily phase of the emulsions of this invention may be constituted from known ingredients in a known manner. While the phase may comprise merely an emulsifier, it may comprise a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make-up the so-called emulsifying wax, and the wax together with the oil and fat make up the so-called emulsifying ointment base which forms the oily dispersed phase of the cream formulations. Emulsifiers and emulsion stabilizers suitable for use in the formulation of the present invention include Tween 60, Span 80, cetostearyl alcohol, myristyl alcohol, glyceryl monostearate, sodium lauryl sulfate, glyceryl distearate alone or with a wax, or other materials well known in the art.

The choice of suitable oils or fats for the formulation is based on achieving the desired cosmetic properties, since the solubility of the active compound in most oils likely to be used in pharmaceutical emulsion formulations is very low. Thus, the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters may be used. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredients are dissolved or suspended in suitable carrier, especially an aqueous solvent for the active ingredients. The active ingredients are preferably present in such formulations in a concentration of 0.5 to 20%, advantageously 0.5 to 10% and particularly about 1.5% w/w.

Formulations for parenteral administration may be in the form of aqueous or non-aqueous isotonic sterile injection solutions or suspensions. These solutions and suspensions may be prepared from sterile powders or granules using one or more of the carriers or diluents mentioned for use in the formulations for oral administration or by using other suitable dispersing or wetting agents and suspending agents. The compounds may be dissolved in water, polyethylene glycol, propylene glycol, ethanol, corn oil, cottonseed oil, peanut oil, sesame oil, benzyl alcohol, sodium chloride, tragacanth gum, and/or various buffers. Other adjuvants and modes of administration are well and widely known in the pharmaceutical art. The active ingredient may also be administered by injection as a composition with suitable carriers including saline, dextrose, or water, or with cyclodextrin (ie.Captisol), cosolvent solubilization (ie. propylene glycol) or micellar solubilization (ie. tween 80).

The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed, including synthetic mono- or diglycerides. In addition, fatty acids such as oleic acid find use in the preparation of injectables.

For pulmonary administration, the pharmaceutical composition may be administered in the form of an aerosol or with an inhaler including dry powder aerosol.

Suppositories for rectal administration of the drug can be prepared by mixing the drug with a suitable non-irritating excipient such as cocoa butter and polyethylene glycols that are solid at ordinary temperatures but liquid at the rectal temperature and will therefore melt in the rectum and release the drug.

The pharmaceutical compositions may be subjected to conventional pharmaceutical operations such as sterilization and/or may contain conventional adjuvants, such as preservatives, stabilizers, wetting agents, emulsifiers, buffers etc. Tablets and pills can additionally be prepared with enteric coatings. Such compositions may also comprise adjuvants, such as wetting, sweetening, flavoring, and perfuming agents.

The foregoing is merely illustrative of the invention and is not intended to limit the invention to the disclosed compounds. Variations and changes which are obvious to one skilled in the art are intended to be within the scope and nature of the invention which are defined in the appended claims.

From the foregoing description, one skilled in the art can easily ascertain the essential characteristics of this invention, and without departing from the spirit and scope thereof, can make various changes and modifications of the invention to adapt it to various usages and conditions.

All mentioned references, patents, applications and publications, are hereby incorporated by reference in their entirety, as if here written.

## Claims

1. A compound having the formula 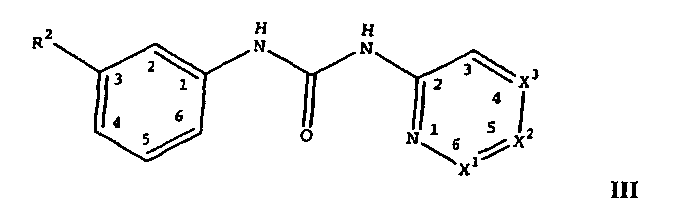
wherein X¹ is CR¹ or N; wherein X² is CR¹ or N; wherein X³ is CH or N; provided only one of X¹, X² and X³ can be N;
wherein R¹ is one or more substituents independently selected from H, optionally substituted pyrrolidinyl, optionally substituted piperazinyl, optionally substituted piperidinyl, morpholinyl, 1,4-dioxa-8-aza-spiro[4.5]decyl, pyridyl, phenyl, C₁-C₆-alkyl, C₁-C₂-haloalkyl, C₁-C₄₋hydroxyalkyl, amino, C₁-C₄-azidoalkyl, C₁-C₄-cyanoalkyl, C₁-C₄-aminoalkyl, halo, hydroxy, (optionally substituted pyrrolidinyl)-C₁-C₂-, (optionally substituted piperidinyl)-C₁-C₂-, (optionally substituted piperazinyl)-C₁-C₂-, morpholinyl-C₁-C₂-, (optionally substituted imidazolyl)-C₁-C₂-, phthalimidyl-C₁-C₂-, optionally substituted azepanyl-C₁-C₂-, 1,4-dioxa-8-aza-spiro[4.5]decyl-C₁-C₂-, optionally substituted phenoxy-C₁-C₂-, C₁-C₄₋alkylaminothiocarbonyl, C₁-C₄-alkoxy-C₁-C₄-alkyl, C₁-C₄-alkylamino-C₁-C₄₋alkyl, C₁-C₄-hydroxyalkylamino-C₁-C₄-alkyl, amino-C₁-C₄-alkoxy-C₁-C₄-alkyl, (1-aza-bicyclo[2.2.2]oct-3-yl)-oxy, optionally substituted pyrrolidinyl-C₁-C₄₋alkoxy, optionally substituted azetidinyl-C₁-C₄-alkoxy optionally substituted piperidinyl-C₁-C₄-alkoxy, C₁-C₄-alkylamino-C₁-C₄-alkoxy, tetrahydrofuryl-O-, tetrahydrofuryl-C₁-C₄-alkoxy, optionally substituted pyridyloxy, optionally substituted phenoxy, C₁-C₄-alkoxycarbonyl, 5-6-membered heterocyclyl-C₁₋C₄-alkylaminocarbonyl, 5-6-membered N-containing heterocyclylcarbonyl, C₁-C₄-alkylamino-carbonyl, C₁-C₄-alkylamino-C₁-C₄-alkylaminocarbonyl, aminocarbonyl, 5-6-membered N-containing heterocyclyl-C₁-C₄-alkylamino, C₁-C₄-alkylamino, C₁-C₄-alkylamino-C₁-C₄-alkylamino-C₁-C₄-alkyl, and C₁₋C₄-alkylamino-C₁-C₄-alkylamino; and
wherein R² is selected from halo, C₁-C₄-alkyl, C₁-C₄-alkylamino-C₂-C₄-alkynyl, C₃-C₆-cycloalkyl, optionally substituted benzodioxolyl, optionally substituted indolyl, optionally substituted phenoxy, unsubstituted 5-membered oxygen or sulfur containing heteroaryl, unsubstituted 5- or 6-membered nitrogen-containing heterocyclyl, phenyl optionally substituted with one or two substituents selected from
halo, C₁-C₄-alkylamino, amino, nitro, C₁-C₄-alkoxy, C₁-C₂-haloalkyl, hydroxy, C₁-C₄-alkylthio, C₁-C₄-alkylcarbonylamino, (optionally substituted phenyl)sulfonylamino, cyano, C₁-C₂-haloalkoxy, 5- or 6-membered N-containing heterocyclyl, aminosulfonyl, (6-membered N-containing heterocyclyl)sulfonyl, C₁-C₂-haloalkylcarbonylaminosulfonyl and (optionally substituted phenyl)aminosulfonyl,
and 6-membered nitrogen-containing heterocyclyl substituted with one or more substituents independently selected from
pyridyl, phenyl, C₁-C₄ alkyl, C₁-C₂ haloalkyl, C₁-C₂ alkoxy, amino, halo, piperidinyl, morpholinyl, C₁-C₂ alkylpiperazinyl, C₁-C₃ alkylaminothiocarbonyl, N,N-di-C₁-C₂-alkylamino-C₁-C₄-alkylenyl, N-C₁-C₂-alkyl-amino-C₁-C₄-alkylenyl, morpholinyl-C₁-C₄-alkylenylaminocarbonyl, aminocarbonyl, C₁-C₂-haloalkylcarbonylamino, morpholinyl-C₁-C₄₋alkylenylamino, N,N-di-C₁-C₂-alkylamino and N,N-di-C₁-C₂-alkylamino-C₁-C₄-alkylenylamino;
and pharmaceutically acceptable salts thereof.

2. The compound of claim 1 wherein X¹ is CR¹; wherein X² is CR¹; wherein X³ is CH; provided X² is CH when X¹ is not CH;
wherein R¹ is independently selected from H, methyl, ethyl, propyl, 1-methyl-4-piperazinyl, 1-benzyl-4-piperazinyl, 1-(2-pyrimidinyl)-4-piperazinyl, 1-(2-pyridyl)-4-piperazinyl, 1-ethyl-4-piperazinyl, 1-piperidinyl-CH₂-, 4-methyl-1-piperidinyl-CH₂-, 3-methyl-1-piperidinyl-CH₂-, 2-methyl-1-piperidinyl-CH₂-, 3,5-dimethyl-1-piperidinyl-CH₂-, 4-oxo-1-piperidinyl-CH₂-, 4-hydroxy-1-piperidinyl-CH₂-, 3-hydroxy-1-piperidinyl-CH₂-, 2-ethoxy-carbonyl-1-piperidinyl-CH₂-, 3-ethoxycarbonyl-1-piperidinyl-CH₂-, 3-carboxy-1-piperidinyl-CH₂-, 4-ethoxycarbonyl-1-piperidinyl-CH₂-, 4-carboxy-1-piperidinyl-CH₂-, 4-(1-pyrrolidinyl)-1-piperidinyl-CH₂-, 4-(N-hydroxyethyl-amino)-1-piperidinyl-CH₂-, 4-(N-propylamino)-1-piperidinyl-CH₂-, 3-(N,N-diethylamino)carbonyl-1-piperidinyl-CH₂-, 4-morpholinyl-CH₂-, N,N-dimethylaminoethylenyl, N,N-diethyl-aminomethylenyl, N-methylamino-methylenyl, N-ethylaminomethylenyl and N, N-diethylamino;
and wherein R² is 3-(N,N-dimethylamino)-1-propynyl, 3-fluorophenyl, 4-fluorophenyl, 4-(N,N-dimethylamino)phenyl, 3-(methylcarbonylamino)phenyl, phenyl, 3-trifluoromethylphenyl, 4-trifluoromethylphenyl, 4-aminophenyl, 3-amino-phenyl, 4-aminosulfonylphenyl, 4-(4-morpholinylsulfonyl)phenyl, 4-(trifluoro-acetylaminosulfonyl)phenyl, 4-(trifluoromethylcarbonylamino-sulfonyl)phenyl, 4-[(4-chlorophenyl)aminosulfonyl]phenyl, 3-(phenylsulfonylamino)phenyl, 2,4-difluorophenyl, 2,4-dimethoxyphenyl, 3-hydroxyphenyl, 4-hydroxyphenyl, 3-ethoxyphenyl, 3,4-dimethoxyphenyl, 4-methylthiophenyl, 4-cyanophenyl, 4-trifluoromethoxyphenyl, 4-methoxyphenyl, 3-nitrophenyl, 3-methoxyphenyl, 2-methoxyphenyl, 2-thiazolyl, 2-pyrazinyl, 5-pyrimidinyl, 4-methyl-1-piperazinyl, 4-morpholinyl, 6-methoxy-3-pyridyl, 2-methoxy-3-pyridyl, 2-ethoxy-3-pyridyl, 3,4-dichloro-4-pyridyl, 6-(trifluoromethylcarbonylamino)-3-pyridyl, 6-amino-3-pyridyl, 3,5-dichloro-4-pyridyl, 2-chloro-4-pyridyl, 3-pyridyl and 4-pyridyl;
and pharmaceutically acceptable salts thereof.

3. The compound of claim 2 wherein R¹ is selected from ethyl, propyl, 1-methyl-4-piperazinyl, 1-piperidinyl-CH₂-, 4-morpholinyl-CH₂-, N,N-diethylamino-methylenyl and N,N-diethylamino;
and wherein R² is 5-pyrimidinyl, 2-pyrazinyl, morpholinyl, 4-methylpiperazinyl, 4-fluorophenyl, 4-(N,N-dimethylamino)propynyl, 3-nitrophenyl, 3-aminophenyl, 4-aminosulfonylphenyl, 3-aminosulfonylphenyl, 3-(phenylsulfonylamino) phenyl, 3-(methylcarbonylamino)phenyl, 4-[(trifluoromethylcarbonyl)aminosulfonyl]phenyl, 4-hydroxyphenyl, 4-methoxyphenyl, 2-thiazolyl, 6-(trifluoromethyl-carbonylamino)-3-pyridyl, 6-amino-3-pyridyl, 3-pyridyl and 4-pyridyl;
and pharmaceutically acceptable salts thereof.

4. Use of a compound of any of claims 1 to 3 for the preparation of a medicament for the treatment of cancer.

5. Use of a compound of any of claims 1 to 3 for the preparation of a medicament for the treatment of a neurological disorder.

6. Use of a compound of any of claims 1 to 3 for the preparation of a medicament for the treatment of cell proliferation.
